(19) Europäisches Patentamt
European Patent Office
Office européen des brevets

(11) EP 1 910 838 B1

(12) **EUROPEAN PATENT SPECIFICATION**

(45) Date of publication and mention of the grant of the patent:
**11.04.2012 Bulletin 2012/15**

(21) Application number: **06764481.5**

(22) Date of filing: **20.07.2006**

(51) Int Cl.:
***G01N 33/574*** *(2006.01)* ***A61K 31/715*** *(2006.01)*

(86) International application number:
**PCT/FI2006/000263**

(87) International publication number:
**WO 2007/010089 (25.01.2007 Gazette 2007/04)**

(54) **USES OF CANCER SPECIFIC GLYCANS**

VERWENDUNGEN VON KREBSSPEZIFISCHEN GLYKANEN

UTILISATIONS DE GLYCANES SPÉCIFIQUES DU CANCER

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.07.2005 FI 20055417**

(43) Date of publication of application:
**16.04.2008 Bulletin 2008/16**

(60) Divisional application:
**11189389.7 / 2 431 743**

(73) Proprietor: **Glykos Finland Oy**
**00790 Helsinki (FI)**

(72) Inventors:
- **SATOMAA, Tero**
  **FI-00700 Helsinki (FI)**
- **NATUNEN, Jari**
  **FI-01520 Vantaa (FI)**
- **HEISKANEN, Annamari**
  **FI-00370 Helsinki (FI)**
- **OLONEN, Anne**
  **FI-15700 Lahti (FI)**
- **SAARINEN, Juhani**
  **FI-00370 Helsinki (FI)**
- **SALOVUORI, Noora**
  **FI-00810 Helsinki (FI)**
- **HELIN, Jari**
  **FI-01680 Vantaa (FI)**

(74) Representative: **Karvinen, Leena Maria et al**
**Oy Jalo Ant-Wuorinen Ab**
**Iso Roobertinkatu 4-6 A**
**00120 Helsinki (FI)**

(56) References cited:
**WO-A1-03/016915 WO-A1-2004/019040**
**WO-A2-03/016464 US-A1- 2004 147 033**

- **HUNT L A ET AL: "Both acidic-type and neutral-type asparaginyl-oligosaccharides of host-cell glycoproteins are altered in Rous-sarcoma-virus-transformed chick-embryo fibroblasts." THE BIOCHEMICAL JOURNAL 15 JUL 1985, vol. 229, no. 2, 15 July 1985 (1985-07-15), pages 441-451, XP002488793 ISSN: 0264-6021**
- **CHAPMAN A. ET AL.: 'Structure of the High Mannose Oligosaccharides of a human IgM Myeloma Protein' THE JOURNAL OF BIOLOGICAL CHEMISTRY vol. 254, 1979, pages 816 - 823, XP003010300**
- **WONG N.K. ET AL.: 'Characterization of the Oligosaccharides Associated with the Human Ovarian Tumor Marker CA125' THE JOURNAL OF BIOLOGICAL CHEMISTRY vol. 278, 2003, pages 28619 - 28634, XP003013156**
- **LLOYD K.O. ET AL.: 'Comparison of O-linked Carbohydrate Chains in MUC-1 Mucin from Normal Breast Epithelial Cell Lines and Breast Carcinoma Cell Lines' THE JOURNAL OF BIOLOGICAL CHEMISTRY vol. 271, 1996, pages 33325 - 33334, XP000942228**
- **ZHU ET AL.: CARBOHYDRATE RESEARCH, vol. 337, no. 3, 11 February 2002 (2002-02-11), pages 207-215,**
- **CIPOLLO ET AL.: JOURNAL OF BIOLOGICAL CHEMISTRY, vol. 277, no. 51, 2002, pages 49143-49157,**
- **SEFTON ET AL.: JOURNAL OF VIROLOGY, vol. 17, no. 1, 1976, pages 85-93,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- HUNT L A ET AL: "Both acidic-type and neutral-type asparaginyl-oligosaccharides of host-cell glycoproteins are altered in Rous-sarcoma-virus-transformed chick-embryo fibroblasts", BIOCHEMICAL JOURNAL, THE BIOCHEMICAL SOCIETY, LONDON, GB, vol. 229, no. 2, 15 July 1985 (1985-07-15) , pages 441-451, XP002488793, ISSN: 0264-6021
- GAGNEUX PASCAL ET AL.: "Evolutionary considerations in relating oligosaccharide diversity to biological function.", GLYCOBIOLOGY, vol. 9, no. 8, 1999, pages 747-755,
- ANON: "N-glycans", WIKIPEDIA, 10 May 2008 (2008-05-10), Retrieved from the Internet: URL: http://en.wikipedia.org/wiki/N-glycosy lation#N-linked_glycosylation> [retrieved on 2009-10-30]
- KOBATA AKIRA: "Structures and functions of the sugar chains of glycoproteins", EUROPEAN JOURNAL OF BIOCHEMISTRY, vol. 209, no. 2, 1992, pages 483-501,

## Description

FIELD OF THE INVENTION

The present invention relates to glycans, which are specifically expressed by certain cancer cells, tumours and other malignant tissues. The present invention describes methods to detect cancer specific glycans as well as methods for the production of reagents binding to said glycans. The invention is also directed to the use of said glycans and reagents binding to them for the diagnostics of cancer and malignancies. Furthermore, the invention is directed to the use of said glycans and reagents binding to them for the treatment of cancer and malignancies.

BACKGROUND OF THE INVENTION

It is generally acknowledged that cancerous transformation of human tissues is associated with changes in the complex carbohydrate structures, *glycans,* which are elementary components of the glycoproteins, glycolipids, and proteoglycans that cover all human cell surfaces. Several individual glycan molecular structures have been identified as cancer-associated glycans (Dube & Bertozzi, 2005). These glycan structures have also been pursued as molecular drug targets for treatment of malignant breast cancer (Holmberg & Sandmeier, 2001) and melanoma (Fernandez, 2003). However, the whole spectrum of cancer-associated glycan changes has remained unknown because of lack of suitable analysis technology. The present invention is directed to such novel analytical methods and the application of the methods to analyses of tissue samples from human patients. The methods are applicable to clinical cancer diagnostics. The novel cancer-associated molecules discovered by the inventors are targets for cancer therapy and diagnostics.

Hunt and Wright, 1985, (The Biochemical Journal 229:441-451) disclose an association between low-mannose structure $Man_3GlcNAC_2$ and Rous-sarcoma virus induced cell transformation in chick-embryo. Due to species specificity (chicken vs human) and tissue specificity (cancer vs. embryonic fibroblasts) of glycosylation, the publication seems not to have relevance with regard to human cancer glycosylation as disclosed in the present invention.

SUMMARY OF THE INVENTION

The present invention provides a method for diagnosing human cancer comprising the step of detecting in a patient sample an increase in expression of an oligosaccharide sequence relative to a control, wherein said oligosaccharide sequence is a low mannose N-glycan consisting of $Man_{1-4}GlcNAc_2$, wherein said oligosaccharide sequence comprises one or more terminal Manα residues, and wherein non-reducing end terminal structures of the oligosaccharide sequence comprise at least one terminal Man-glycan epitope selected from the group consisting of: Manα3Man, Manα6Man, and Manα3(Manα6)Man.

The present invention also provides a pharmaceutical composition comprising an antibody that specifically recognises the combination of terminal structures and glycan core structures of the low mannose N-glycan consisting of $Man_{1-4}GlcNAc_2$ as defined above for use in the treatment of human cancer in a patient diagnosed according to the present invention.

The present invention also provides a pharmaceutical composition consisting of the low mannose N-glycan consisting of $Man_{1-4}GlcNAc_2$ as defined above or an antigenic epitope according to Formula

$$[OS-(X)_n-L-Y]_m-Z \qquad (II),$$

wherein OS is the low mannose N-glycan consisting of $Man_{1-4}GlcNAc_2$ as defined above, Y is a non-carbohydrate spacer or a non-glycosidically linked terminal conjugate, n is 0 or 1 and X is lactosyl-, galactosyl-, N-acetyllactosaminyl, mannosyl-, $Man_2$, $Man_3$-, $Man_3GlcNAc$, $Man_4GlcNAc$, N-acetylglucosaminyl-, or N-acetylgalactosaminyl, and OS is β2-, or β4-, or β6 linked to the mannosylresidue, for use in the treatment of human cancer in a patient diagnosed according to the present invention.

BRIEF DESCRIPTION OF THE DRAWINGS

**Figure 1.** Example of α-mannosidase digestion schemes of, **A.** healthy lung and lung cancer tumor sample pairs from 7 patients with non-small cell lung adenocarcinoma, and **B.** breast cancer tumor samples from 7 patients with ductale breast adenocarcinoma. The m/z figures in the x-axis correspond to the approximate m/z values of $[M+Na]^+$

adduct ions. The figures in the y-axis correspond to relative glycan signal intensities. The difference between glycan intensities before and after α-mannosidase digestion is equal to the amount of non-reducing terminal α-mannose containing structures.

**Figure 2.** Structural features of non-fucosylated glycans Man$\alpha_{0-3}$Manβ4GlcNAcβ4GlcNAc(β-*N*-Asn).

**Figure 3.** Example of mass spectrometric analysis of glycans expressed in tumor and healthy control tissues of a non-small cell lung adenocarcinoma patient. **A.** Neutral glycan mass spectrum from the lung cancer tumor, **B.** neutral glycan mass spectrum from healthy lung tissue from the same patient, and **C.** illustration of the differences in spectra A. and B., with regard to the present structure group glycans. The m/z figures in the x-axis correspond to the approximate m/z values of $[M+Na]^+$ adduct ions. The figures in the y-axis correspond to relative glycan signal intensities.

**Figure 4.** Graph presenting the average relative glycan signal intensities in breast cancer tumor - healthy breast tissue sample pairs from 9 patients with ductale breast adenocarcinoma. The m/z figures in the x-axis correspond to the approximate m/z values of $[M+Na]^+$ adduct ions. The figures in the y-axis correspond to relative glycan signal intensities.

**Figure 5.** Graph presenting the average relative glycan signal intensities in breast cancer tumor - healthy breast tissue sample pairs from 7 patients with lobulare breast adenocarcinoma. The m/z figures in the x-axis correspond to the approximate m/z values of $[M+Na]^+$ adduct ions. The figures in the y-axis correspond to relative glycan signal intensities.

**Figure 6.** Neutral protein-linked glycan profiles from non-small cell lung adenocarcinoma patients. *x-axis*: approximate mass-to-charge ratio (m/z) of the glycan signals, corresponding to mass of $[M+Na]^+$ ions; *y-axis:* relative abundance of the glycan within the neutral glycan fraction. **Light columns:** healthy lung; **Dark columns:** non-small cell lung adenocarcinoma.

**Figure 7.** MALDI-TOF mass spectrometric analysis of sialylated high-molecular weight protein-linked glycans from **A.** normal ovary tissue, **B**. benign ovarian cystadenoma tumor, and C. malignant ovarian cystadenocarcinoma tumor. *x-axis:* mass-to-charge ratio (m/z) of the detected signals at 1500-4000 Da, corresponding to mass of $[M-H]^-$ ions; *y-axis:* relative signal intensity, 0-100 %; *N*: $[M-2H+Na]^-$ ion at +22 Da; *K*: $[M-2H+K]^-$ ion at +38 Da; *asterisk:* $H_2O$ elimination product at -18 Da; *numbering* of glycan signals refers to **Table 3.**

**Figure 8.** Neutral protein-linked glycan profiles from **A.** normal ovary tissue (1 patient), **B.** benign ovarian cystadenoma tumor (average of 5 patients), and **C.** malignant ovarian cystadenocarcinoma tumor (average of 4 patients). *x-axis:* approximate mass-to-charge ratio (m/z) of the glycan signals, corresponding to mass of $[M+Na]^+$ ions; *y-axis:* relative abundance of the glycan within the neutral glycan fraction. Column code from left to right, **dark columns:** normal ovary; **blank columns:** benign tumor; **light columns:** malignant tumor.

**Figure 9.** Discrimination analysis of neutral protein-linked glycans analysis results of malignant and benign tumors and healthy tissue samples from the same tissues of **A.** breast, **B.** ovary, and **C.** colon. The discrimination is based on relative abundancies of three neutral protein-linked glycans identified in principal component analysis and an experimental discrimination formula derived from a randomly picked training group of breast cancer patients, as described in the text. The scores resulting from individual samples are plotted on the y-axis.

**Figure 10.** Example of glycan signal analysis of MALDI-TOF mass spectrometric data. **A.** Mass spectrometric raw data showing a window of neutral N-glycan mass spectrum in positive ion mode, **B.** Glycan profile generated from the data in A.

**Figure 11.** Example of glycan signal analysis of MALDI-TOF mass spectrometric data. **A.** Mass spectrometric raw data showing a window of sialylated N-glycan mass spectrum in negative ion mode, **B.** Glycan profile generated from the data in A.

**Figure 12.** Neutral protein-linked glycans of normal lung tissue **(light columns)** and lung cancer tumor **(dark columns).**

**Figure 13.** Neutral and acidic N-glycan profiles of lysosomal protein sample.

**Figure 14.** Reference neutral N-glycan structures for NMR analysis (A-C) in **Table 5.**

**Figure 15.** Reference neutral N-glycan structures for NMR analysis (D-G) in **Table 6.**

**Figure 16.** Neutral protein-linked glycans of normal breast tissue **(light columns)** and ductale type breast carcinoma tumor **(dark columns)** from the same patient.

**Figure 17.** Neutral protein-linked glycans of normal lymph node tissue **(light columns)** and ductale type breast carcinoma lymph node metastasis **(dark columns)** from the same patient.

**Figure 18.** Acidic protein-linked glycans of normal breast tissue **(light columns)** and ductale type breast carcinoma tumor **(dark columns)** from the same patient.

**Figure 19.** Acidic protein-linked glycans of normal lymph node tissue **(light columns)** and ductale type breast carcinoma lymph node metastasis **(dark columns)** from the same patient.

DETAILED DESCRIPTION OF THE INVENTION

**Analysis of cancer glycomes**

The present invention is directed in a preferred embodiment quantitative mass spectromettic profiling of human cancers according to the invention and analysis of alterations in cancer in comparision with normal corresponding normal tissues. The analysis can be performed based on signals corresponding to glycan structures, these signals were translated to likely monosaccharide compositions and further analysed to reveal structures and correlations between the signals. The invention is especially directed to analysis of N-glycan glycomes derived from cancer proteins. The glycans are analysed as neutral and/or acidic signals and glycan mixtures, multiple analysis methods are preferred to obtain maximal amount of data. The invention is also directed to methods for analysis of mixture of N-glycans and O-glycans released together.

Not with standing any indications to the contrary, the invention is limited to the oligosaccharides specified in the claims, and to human cancers.

<u>Preferred N-glycan glycomes and characteristic glycan groups therein</u>

The invention revealed glycan groups, which are characteristically changed in cancer in protein derived N-glycans. Among N-glycans preferred N-glycans are glycans with non-reducing end terminal Man-residues (terminal Man-glycans).

*Glycome analysis by quantitative analysis of glycan groups in a glycome*

The invention is especially directed to analysis of glycomes. The invention is especially directed to methods of calculation %-part of a specific glycan groups and comparing the % values, e.g. in form of Table. It is notable that the glycan groups may occasionally comprise unusual/uncharacteristics glycans, which do not exactly correspond the title of group, but presence of such material would likely increase the characteristics of the analysis in comparisions between tissue materials. The glycan score analysis according to the invention revealed especial usefulness of the analysis by glycan groups.

*Preferred terminal Man-glycans*

Preferred terminal Man-glycans includes low-Man glycans. The invention revealed that there is especially characteristic alterations in low-Man glycans. The invention revealed that the low-Man glycans share much similarity with glycans produced by lysosomal enzyme for lysosomal proteins, Fig 25. The invention thus revealed that the structures altering reflects major changes in balance of cellular organelles in cancers, which is reasonable when considering morphological and other alterations in cancer. Furthermore the invention revealed additional novel soluble N-glycan type glycome, comprising terminal-Man structures and giving additional characteristics for cancer glycomes, when included in the glycome fraction.

Analysis by specific binding molecules

It is further realized that key alterations in glycomes can be also analysed by other methods such as specific binding reagents after altering structure has been determined. The invention is directed to analysis of altering structures when the amount of the structure increases in a specific cancer.

The analysis by binding method molecule may be preferred as a fast test, though the current mass spectrometric screening method is also quite fast and cost effective, only draw being requirement of mass spectrometer, which includes some capital investment for the method. The analysis by the specific binder may be also performed directly from the tissue and better information of tissue and/or cellular localization of the materials can be obtained. It also realized that combinations of at least two binding molecules recognizing different structures would be especially useful for analysis of cancer and multiple selcted specific binding molecules would approach the effectivity of the mass spectrometric screening methods.

Preferred structures in glycomes to be analyzed by the binding molecules and target epitopes therein

The invention reveled characteristic structures among the N-glycan glycomes. The invention is especially directed to these structures as targets for recognition by specific binding molecules.

*Preferred terminal Man-glycans*

Preferred terminal Man-glycans includes low-Man. The preferred terminal Man glycans comprise non-reducing end Man- residue(s), which Manα- or Manβ-residue, preferably being either one or more Manα-residues or a single Manβ-residue. Preferably the glycan comprise the Man residue(s) as the only non-reducing end terminal monosaccharide types.

*Preferred recognition of terminal Manβ-residue comprising low-Man glycans*

When the terminal Man residue is Manβ-residue, the structure is the minimal low mannose glycan Manβ4GlcNAcβ4GlcNAcβAsn. The preferred minimal epitopes to be recognized includes Manβ and terminal disaccharides and tri/tetrasaccharides either including reducing end anomeric structure or not and/or reducing end amino acid residue and/or part of the peptide chain of the potential carrier protein (marked in following by Asn) Manβ, Manβ4GlcNAc, Manβ4GlcNAcβ, Manβ4GlcNAcβ4GlcNAc, Manβ4GlcNAcβ4GlcNAcβ, and Manβ4GlcNAcβ4GlcNAcβAsn.

The specificity of the binding reagent such as binding protein should such that the binding side covers the terminal Manβ-so that the binding molecule does not cross-react with elongated N-glycans, or less preferably cross reacts only with other preferred low-Man structures according to the invention such as the core structures elongated only by single Manα3/6-residue. The invention is directed to beta-mannosidase enzymes and corresponding engineered lectins used for sequencing N-glycans as examples specific binding reagents. The preferred minimum epitopes includes Manβ, Manβ4GlcNAc, Manβ4GlcNAcβ in a preferred embodiment. It is realized that smaller binding epitopes are generally enough for specific recognition as similar structures are rare/non-existing in animal/human materials.

*Preferred recognition of terminal Manα-residue comprising low-Man glycans*

The preferred Manα-residue comprising target low-Man glycans includes both isomers of dimannosyl structures, preferably ones comprising Manα6Man, which was analysed to have higher predominance at least in part of cancers, the trimannosyl core structure low-Man glycans, and tetra-Mannosylisomers

Low-Man glycans according to the invention. The dimannosyl, and trimannosyls are preferred structures, and the branched trimannosyl are especially preferred due to prevalence observed and larger homogeneity (less isomers in target). The trimannosyl-glycans is separately a preferred control step glycan with different branched accessibility for mannosidases and for synthesis of lower size low mannoses.

The terminal epitopes of antibodies against low-Man structures should recognize linear and/or branched Manα3/6Man-epitopes. The minimal epitopes includes non-reducing end terminal disaccharide and trisaccharide epitopes of the low-Man N-glycans.

Preferred minimal disaccharide epitopes for recognition of Low-Man glycan includes the disaccharides without next anomeric linkage Manα3Man, Manα6Man, and more specific epitopes disaccharides with next anomeric linkage depending on the level of the Manα3Manβ, Manα6Manβ, Manα3Manα and Manα6Manα.

Preferred branched trisaccharides includes Manα3(Manα6)Man, Manα3(Manα6)Manβ, and Manα3(Manα6) Manα. It is furthermore realized that the binding specificity may included further structures in the core of the N-glycans, but the core structures does not interfere with the binding.

The specificity of the binding reagent such as binding protein should such that the binding side covers the terminal Manα3/6- structures so that the binding molecule does not cross-react with elongated complex type N-glycans (GlcNAc-modifications on Manα3/6). The invention is directed to such Manα3/6-specifc N-glycan core specific antibodies and and lectins.

Examples of useful antibody types fro the recognition of low-Man glycans in cancer include:

1) antibodies with specificity similar to antibody IgM mAb 100-4G11-A (van Remoorte A. et al. Glycobiology (2003) vol. 13, 217-225) associated with S. mansoni infection in mouse, which antibody has specificity to branched Manα3 (Manα6)Manβ- epitope, but not to pentamannose epitope of RNAse B Manaα[Manα3(Manα6)Manα6]Manβ-. The antibody is produced naturally in mouse without major autoimmune complications, and it reacts with only few normal mammalian proteins indicating that the antibody is not harmful even for in vivo applications in mammals.
2) Antibodies with specificity similar to L3 and L4 antibodies (Schmitz B et al. et al. Glycobiology (1993) vol. 3, 609-17) with highest binding for Manα3[Manα3(Manα6)Manα6]Manβ- and lower activity for high-Man glycans.

*Additional analysis of glycans in cancer involving recognition of carrier proteins*

The invention is further directed to methods of analysing any of the glycans according to the invention from cancer derived proteins, preferably integral (cell bound/transmembrane) cancer tissue or cell released proteins and assigning the glycan structures with specific carrier protein, preferably by specific purification of the protein, e.g. by affinity methods such as immunoprecipitation or by sequencing, preferably by mass spectrometric sequencing, glycopeptides including sequencing and recognizing peptides and thus proteins linked to the proteins.

**The target cancer tissue**

The present invention is directed to analysis of un-normally transformed tissues, when the transformation is benign and/or malignant cancer type transformation referred as cancer (or tumor). It is realized that benign transformation may be a step towards malignant transformation, and thus the benign cancers are also useful to be analysed and differentiated from normal tissue, which may have also non cancerous or non-transformation related alterations such as swelling or trauma related to physical or e.g. infectious trauma, and it is useful and preferred to differentiate with benign and malignant cancers

Preferably the tissue is human tissue or tissue part such as liquid tissue, cell and/or solid polycellular tumors, and in another embodiment preferably a solid human tissue. The solid tissues are preferred for the analysis and/or targeting specific glycan marker structures from the tissues, including intracellulalarily and extracellullarily, preferably cell surface associated, localized markers. In a preferred embodiment the invention is specifically directed to the recognition of cell surface localized and/or mostly cell surface localized marker structures from solid tumor tissues or parts thereof. It is realized that the contacts between cells and this glycomes madiateing these are are affected by presence of cells as solid tumor or as more individual cells. The preferred individual cell type cancers or tumors include preferably blood derived tumors such as leukemias and lymphomas, while solid tumors are preferably includes solid tumors derived from solid tissues suchs gastrointestinal tract tissues, other internal organs such as liver, kidneys, spleen, lungs, gonads and associated organs including preferably ovary, testicle, and prostate. The invention further reveals markers from individually or multicellularily presented cancer cells in contrast to solid tumors. The preferred cancer cells to be analyzed includes metastatic cells released from tumors/cancer and blood cell derived cancers, such as leukemias and/or lymphomas. Metastasis from solid tissue tumors forms a separately preferred class of cancer samples with specific characteristics.

The invention is furthermore directed to the anlysis of secretions from tumors such as ascites and/or cyst fluids, and cancer secreted materials present in general body fluids such as blood (or its derivatives such as serum nor plasma), urine, mucous secretions, amnion fluid, lymphatic fluid or spinal fluid, preferably blood, urine or mucous secretions, most preferably blood.

The cancer tissue materials to be analyzed according to the invention are in the invention also referred as tissue materials or simply as cells, because all tissues comprise cells, however the invention is preferably directed to unicellularily and/or multicellularily expressed cancer cells and/or solid tumors as separate preferred characterisitics. The invention further reveals normal tissue materials to be compared with the cancer materials. The invention is specifically directed to methods according to the invention for revealing status of transformed tissue or suspected cancer sample when expression of specific structure of a signal correlated with it is compared to a expression level estimated to correspond to expression in normal tissue or compared with the expression level in an standard sample from the same tissue, preferably a tissue sample from healthy part of the same tissue from the same patient.

The invention is in a preferred embodiment directed to analysis of the marker structures and/or glycome profiles from both cancer tissue and corresponding normal tissue of the same patient because part of the glycosylations includes

individual changes for example related to rare glycosylation related diseases such as congenital disorders in glycosylation (of glycoproteins/carbohydrates) and/or glycan storage diseases. The invention is furthermore directed to method of verifying analysing importance and/or change of a specific structure/structure group or glycan group in glycome in specific cancer and/or a subtype of a cancer optionally with a specific status (e.g. primary cancer, metastase, benign transformation related to a cancer) by methods according to the present invention.

The present invention is directed to a set of glycan structures which are expressed by human cancers. The presence or increased amount of one or more of these glycans in a patient sample indicates cancerous status of the sample as described below in detail. The glycan structures are

neutral low-mannose type N-glycans having terminal Man, preferentially Manα structure; for example $Man\alpha_{0-3}Man\beta4GlcNAc\beta4GlcNAc(\beta\text{-}N\text{-}Asn)$

More detailed analysis of low mannose glycans

Low-mannose N-glycans are smaller and more rare than the common high-mannose N-glycans ($Man_{5-9}GlcNAc_2$).

Preferred non-fucosylated low-mannose glycans are according to the formula:

$$[Man\alpha3]_{n1}[(Man\alpha6)]_{n2}[Man\alpha6]_{n3}[(Man\alpha3)]_{n4}Man\beta4GlcNAc\beta4GlcNAc[\beta Asn]_{p}$$

wherein p, n1, n2, n3, n4 are either independently 0 or 1,
with the proviso that when n3 is 0, also n1 and n2 are 0, and preferably either n1 or n2 is 0,
[ ] indicates determinant either being present or absent
depending on the value of n1, n2, n3, n4,
( ) indicates a branch in the structure.

Methods for evaluating the malignancy of patient samples

The present invention is directed to a method of evaluating the malignancy of a patient sample comprising the step of detecting the presence of cancer related oligosaccharide sequences in the sample, said oligosaccharide sequences comprising structures with terminal monosaccharides Manα.

The present invention is directed to a method of evaluating the malignancy of a patient sample comprising the step of detecting the presence or amount of a cancer related oligosaccharide sequence in the sample according to the invention.

The invention is directed to method including analysis of low -mannose (abbreviated low-Man) structure.

Preferred structures according to the present invention are described in the Examples, in which association of the structures with cancer was found in major human cancer types.

Cancer indicating structures and combinations of structures

The inventors found that an increased amount of any one of the abovementioned cancer-related oligosaccharide sequences in said patient sample indicates the cancerous nature of the sample. Furthermore, simultaneous increase of more than one of the abovementioned cancer-related oligosaccharide sequences is highly indicative of cancer, especially when more than one of the abovementioned oligosaccharide sequences is simultaneously expressed in elevated amounts compared to healthy human tissues. The abovementioned glycan structures were found to be cancer-associated in all cancer types studied.

It was found that the increased amount of the abovementioned oligosaccharide sequences was indicative of the malignancy of human tumors, though no such increase was found in benign tumors of the colon and the ovaries. The inventors found that malignant and benign growth in human patients could be distinguished by analyzing protein-linked glycan structures for the expression of the abovementioned oligosaccharide sequences according to the present invention. Furthermore, the analysis specificity was increased by combination with analysis of cancer type specific glycan features, as described below. The present invention is especially directed to cancer diagnostics or analysis of clinical state of cancer by analysing several glycan structures simultaneously.

The present findings are considered medically very interesting. The novel methods to detect and diagnose cancer described in the present invention can be used in the clinical setting e.g. to give tools and data for decisions how to treat human patients. It is realized that ability to detect malignant cancer and to differentiate between benign and malignant tumors is of utmost importance in terms of efficient clinical decision-making and selection of the correct therapy.

The inventors further discovered that individual differences occur in normal tissue glycosylation and tumor-associated glycosylation changes, and in some patients the cancer-associated glycan changes are more prominent than

in others. The present invention is further directed to using the methods for selecting patients for most effective therapy options according to their individual glycosylation profiles and the glycosylation profiles expressed in the disease, preferentially in the malignant tumor.

General structures representing oligosaccharide sequences

The cancer related oligosaccharide sequences described herein can be a part of a glycoprotein, free oligosaccharides, or glycans such as glycopeptides. Defects or changes in biosynthetic and/or biodegradative pathways of tumors lead to the synthesis of the cancer related oligosaccharide sequences both on glycolipids and glycoproteins.

The term "oligosaccharide sequence" indicates that the monosaccharide residue/residues in the sequence are part of a larger glycoconjugate, which contains other monosaccharide residues in a chain, which may be branched, or may have natural substituted modifications of oligosaccharide chains. The oligosaccharide chain is normally conjugated to a lipid anchor or to a protein. In a preferred embodiment the oligosaccharide sequences according to the present invention are non-reducing terminal oligosaccharide sequences, which means here that the oligosaccharide sequences are not linked to other monosaccharide or oligosaccharide structures except optionally from the reducing end of the oligosaccharide sequence. The oligosaccharide sequence when present as conjugate is preferably conjugated from the reducing end of the oligosaccharide sequence, though other linkage positions which are tolerated by the antibody/binding substance binding can also be used. In a more specific embodiment the oligosaccharide sequence according to the present invention means the corresponding oligosaccharide residue which is not linked by natural glycosidic linkages to other monosaccharide or oligosaccharide structures. The oligosaccharide residue is preferably a free oligosaccharide or a conjugate or derivative from the reducing end of the oligosaccharide residue.

In one embodiment of the invention the cancer specific oligosaccharides are detected for the diagnostics of cancer or tumor.

Preferably the tumor specific oligosaccharide sequence is detected by a specific binding substance which can be an aptamer, lectin, peptide, or protein, such as an antibody, a fragment thereof or genetically engineered variants thereof. More preferably the specific binding substance is divalent, oligovalent or polyvalent. Most preferably the binding substance is a lectin or an antibody.

Specific binding combinatorial chemistry libraries can be used to search for the binding molecules. Saccharide binding proteins, antibodies or lectins can be engineered, for example, by phage display methods to produce specific binders for the structures of the invention. Labelled bacteria or cells or other polymeric surfaces containing molecules recognizing the structures can be used for the detection. Oligosaccharide sequences can also be released from cancer or tumor cells by endoglycosidase enzymes. Alternatively oligosaccharides can be released by protease enzymes, resulting in glycopeptides. Chemical methods to release oligosaccharides or derivatives thereof include, e.g., ozonolysis of glycolipids and beta-elimination or hydrazinolysis methods to release oligosaccharides from glycoproteins. Alternatively the glycolipid fraction can be isolated. A substance specifically binding to the cancer specific oligosaccharide sequences can also be used for the analysis of the same sequences on cell surfaces. Said sequences can be detected e.g. as glycoconjugates or as released and/or isolated oligosaccharide fractions. The possible methods for the analysis of said sequences in various forms also include NMR spectroscopy, mass spectrometry and glycosidase degradation methods. Preferably at least two analysis methods are used, especially when methods of limited specificity are used.

Analysis of multiple cancer specific structures simultaneously from mass spectrometric profiles

The present invention is especially directed to the analysis and/or comparision of several analytical signals, preferably mass spectrometry signals produced from a sample comprising total fraction of oligosaccharides released from a cancer or a tumor sample. A single mass spectrum of an oligosaccharide fraction comprise a profile of glycosylation and multiple peaks indicating the potential presence of the oligosaccharide sequences and potential presence of cancer specific oligosaccharide sequences and altered levels thereof in comparison to normal tissue sample or a benign tumour sample. The profiles are determined preferably by MALDI-TOF mass spectrometry as described in the Examples. The total oligosaccharide fraction corresponds preferably to the total fraction of protein oligosaccharides, preferably comprising at least one cancer or tumor specific oligosaccharide sequence according to the invention. The present invention is further directed to analysis of the multiple mass spectrometric signals after the total oligosaccharide fraction is released from a cancer or tumor sample is subjected to an enzymatic or a chemical digestion step. The enzymatic digestion is preferably performed by a glycosidase enzyme, preferably N-acetylglucosaminidase, or mannosidase.

The present specification also discloses the use of the tumor specific oligosaccharide sequences or analogs or derivatives thereof to produce polyclonal or monoclonal antibodies recognizing said structures using following process: 1) producing synthetically or biosynthetically a polyvalent conjugate of an oligosaccharide sequence of the invention or analogue or derivative thereof, the polyvalent conjugate being, for instance, according to the following structure: position C1 of the reducing end terminal of an oligosaccharide sequence (OS) comprising the cancer specific sequence described

in the present invention is linked (-L-) to an oligovalent or a polyvalent carrier (Z), via a spacer group (Y) and optionally via a monosaccharide or oligosaccharide residue (X), forming the following structure

$$[OS - (X)_n - L- Y]_m - Z$$

wherein integer m has values m > 1 and n is independently 0 or 1; L can be oxygen, nitrogen, sulfur, or a carbon atom; X is preferably lactosyl-, galactosyl-, poly-N-acetyl-lactosaminyl, or part of an N-glycan oligosaccharide sequence, Y is a spacer group or a terminal conjugate such as a ceramide lipid moiety or a linkage to Z; 2) immunizing an animal or human with polyvalent conjugate together with an immune response activating substance. Preferably the oligosaccharide sequence is polyvalently conjugated to an immune response activating substance and the conjugate is used for immunization alone or together with an additional immune response activating substance. In a preferred embodiment the oligosaccharide conjugate is injected or administered mucosally to an antibody-producing organism with an adjuvant molecule or adjuvant molecules. For antibody production the oligosaccharide or analogs or derivatives thereof can be polyvalently conjugated to a protein such as bovine serum albumin, keyhole limpet hemocyanin, a lipopeptide, a peptide, a bacterial toxin, a part of peptidoglycan or immunoactive polysaccharide or to another antibody production activating molecule. The polyvalent conjugates can be injected to an animal with adjuvant molecules to induce antibodies by routine antibody production methods known in the art.

Antibody production or vaccination can also be achieved by analogs or derivatives of the cancer specific oligosaccharide sequences. Simple analogs of the N-acetyl-group containing oligosaccharide sequences include compounds with modified N-acetyl groups, for example, N-alkyls, such as N-propanyl.

It is also possible to use the tumor specific oligosaccharide sequences for the purification of antibodies from serum, preferably from human serum. The cancer specific oligosaccharides or derivatives or analogs, such as a close isomer, can also be immobilized for the purification of antibodies from serum, preferably from human serum. The present invention is directed to natural human antibodies that bind strongly to the cancer specific oligosaccharide sequences described in the present invention.

The cancer specific oligosaccharide sequences can also be used for detection and/or quantitation of the human antibodies binding to the cancer specific oligosaccharide sequences, for example, in enzyme-linked immunosorbent assay (ELISA) or affinity chromatography type assay formats. The detection of human antibodies binding to the cancer specific oligosaccharide sequences is preferably aimed for diagnostics of cancer, development of cancer therapies, especially cancer vaccines against the oligosaccharide sequences described in the present invention, and search for blood donors which have high amounts of the antibodies or one type of the antibody.

Furthermore, it is possible to use human antibodies or humanized antibodies against the cancer specific oligosaccharide sequences to reduce the growth of or to destroy a tumor or cancer. Human antibodies can also be tolerated analogs of natural human antibodies against the cancer specific oligosaccharide sequences; the analogs can be produced by recombinant gene technologies and/or by biotechnology and they may be fragments or optimized derivatives of human antibodies. Purified natural anti-tumor antibodies can be administered to a human patient without any expected side effect as such antibodies are transferred during regular blood transfusions. This is true under conditions that the cancer specific structures are not present on normal tissues or cells and do not vary between individuals as blood group antigens do. The production of specific humanized antibodies by gene engineering and biotechnology is also possible: the production of humanized antibodies has been described in US patents Nos. 5,874,060 and 6,025,481, for example. The humanized antibodies are designed to mimic the sequences of human antibodies and therefore they are not rejected by immune system as animal antibodies are, if administered to a human patient. It is realized that the method to reduce the growth of or to destroy cancer applies both to solid tumors and to cancer cells in general. It is also realized that the purified natural human antibodies recognizing any human cancer specific antigen, preferably an oligosaccharide antigen, can be used to reduce the growth of or to destroy a tumor or cancer. In another embodiment species specific animal antibodies are used against a tumor or cancer of the specific animal.

Human antibodies or humanized antibodies against the cancer specific oligosaccharides, or other tolerated substances binding the tumor specific oligosaccharides, are useful to target toxic agents to tumor or to cancer cells. The toxic agent could be, for example, a cell killing chemotherapeutics medicine, such as doxorubicin (Arap et al., 1998), a toxin protein, or a radiochemistry reagent useful for tumor destruction. Such therapies have been demonstrated in the art. The toxic agent may also cause apoptosis or regulate differentiation or potentiate defence reactions against the cancer cells or tumor. The cancer or tumor binding antibodies can be also used for targeting prodrugs active against tumor or enzymes or other substances converting prodrugs to active toxic agents which can destroy or inhibit tumor or cancer, for example in so called ADEPT-approaches.

The therapeutic antibodies described above can be used in pharmaceutical compositions for the treatment or prevention of cancer or tumor.

Other methods for therapeutic targeting of tumors

It is realized that numerous other agents besides antibodies, antibody fragments, humanized antibodies and the like can be used for therapeutic targeting of cancer or tumors similarily with the diagnostic substances. It is specifically preferred to use non-immunogenic and tolerable substances to target cancer or tumor. The targeting substances binding to the cancer or tumor comprise also specific toxic or cytolytic or cell regulating agents which leads to destruction or inhibition of cancer or tumor. Preferably the non-antibody molecules used for cancer or tumor targeting therapies comprise molecules specifically binding to the cancer or tumor specific oligosaccharide sequences are aptamers, lectins, genetically engineered lectins, glycosidases and glycosyltransferase and genetically engineered variants thereof. Labelled bacteria, viruses or cells or other polymeric surfaces containing molecules recognizing the structures can be used for the cancer or tumor targeting therapies.

Furthermore it is possible to produce a pharmaceutical composition comprising the cancer specific oligosaccharide sequences or analogs or derivatives thereof for the treatment of cancer or tumor. Preferably the pharmaceutical composition is used for the treatment of a human patient. Preferably the pharmaceutical composition is used for the treatment of cancer or tumor, when patient is under immunosuppressive medication or he/she is suffering from immunodeficiency. The pharmaceutical compositions described above are especially preferred for the treatment of cancer or tumor diagnosed to express the cancer specific oligosaccharide sequences of the invention. The the pharmaceutical compositions can be used together with other pharmaceutical compositions for the treatment of cancer or tumor. Preferably the other pharmaceutical compositions comprise cytostatics, anti-angiogenic pharmaceuticals, anti-cancer proteins, such as interferons or interleukins, or use of radioactivity.

Use of antibodies for the diagnostics of cancer or tumor and for the targetting of drugs to cancer has been described with other antigens and oligosaccharide structures (US 4,851,511; US 4,904,596; US 5,874,060; US 6,025,481; US 5,795,961; US 4,725, 557; US 5,059,520; US 5,171,667; US 5,173,292; US 6,090,789; US 5,708,163; US 5,902,725 and US 6,203,999). Use of cancer specific oligosaccharides as cancer vaccines has also been demonstrated with other oligosaccharide sequences (US 5,102,663; US 5,660,834; US 5,747,048; US 5,229,289 and US 6,083,929).

Combination of the therapeutic and diagnostic methods

The data in the Examples shows the usefulness of the combination of analysis of the cancer specific structures according to the invention, because there are individual variations in glycosylation of tumors and normal tissues. The normal tissue close to tumor may also be partially contaminated by materials secreted by tumor that may be taken to consideration when analyzing the normal tissue data.

The substance according to the invention can be attached to a carrier. Methods for the linking of oligosaccharide sequences to a monovalent or multivalent carrier are known in the art. Preferably the conjugation is performed by linking the cancer specific oligosaccharide sequences or analogs or derivatives thereof from the reducing end to a carrier molecule. When using a carrier molecule, a number of molecules of a substance according to the invention can be attached to one carrier increasing the stimulation of immune response and the efficiency of the antibody binding. To achieve an optimal antibody production, conjugates larger than 10 kDa carrying typically more than 10 oligosaccharide sequences are preferably used.

The oligosaccharide sequences according to the invention can be synthesized, for example, enzymatically by glycosyltransferases, or by transglycosylation catalyzed by a glycosidase enzyme or a transglycosidase enzyme, for review see Ernst et al. (2000). Specificities of the enzymes and their use of co-factors such as nucleotide sugar donors, can be engineered. Specific modified enzymes can be used to obtain more effective synthesis, for example, glycosynthase is modified to achieve transglycosylation but not glycosidase reactions. Organic synthesis of the saccharides and conjugates of the invention or compounds similar to these are known (Ernst et al., 2000). Carbohydrate materials can be isolated from natural sources and be modified chemically or enzymatically into compounds according to the invention. Natural oligosaccharides can be isolated from milks of various ruminants and other animals. Transgenic organisms, such as cows or microbes, expressing glycosylating enzymes can be used for the production of saccharides.

It is possible to incorporate an oligosaccharide sequence according to the invention, optionally with a carrier, in a pharmaceutical composition, which is suitable for the treatment of cancer or tumor in a patient. Examples of conditions treatable according to the invention are cancers in which the tumor expresses one or more of the tumor specific oligosaccharides described in the invention. The treatable cancer cases can be discovered by detecting the presence of the tumor specific oligosaccharide sequences in a biological sample taken from a patient. Said sample can be a biopsy or a blood sample.

The pharmaceutical composition according to the invention may also comprise other substances, such as an inert vehicle, or pharmaceutically acceptable carriers, preservatives etc., which are well known to persons skilled in the art.

The substance or pharmaceutical composition according to the invention may be administered in any suitable way. Methods for the administration of therapeutic antibodies or vaccines are well known in the art.

The term "treatment" used herein relates to both treatment in order to cure or alleviate a disease or a condition, and to treatment in order to prevent the development of a disease or a condition. The treatment may be either performed in an acute or in a chronic way.

The term "patient", as used herein, relates to any mammal in need of treatment according to the invention.

When a cancer specific oligosaccharide or compound specifically recognizing cancer specific oligosaccharides of the invention is used for diagnosis or typing, it may be included e.g. in a probe or a test stick, optionally in a test kit. When this probe or test stick is brought into contact with a sample containing antibodies from a cancer patient or cancer cells or tissue of a patient, components of a cancer positive sample will bind the probe or test stick and can be thus removed from the sample and further analyzed.

In the present invention the term "tumor" means solid multicellular tumor tissues. Furthermore the term "tumor" means herein premalignant tissue, which is developing to a solid tumor and has tumor specific characteristics. The present invention is preferably directed to primary human cancer samples. It is well known that glycosylations in cultivated cancer cells vary and are not in general relevant with regard to cancer. It is also known that transfections, cell culture media and dividing solid tumor to single cells may have daramatic effects for glycosylations. When referring to therapies tumor specific oligosaccharides or oligosaccharide sequences (possibly occasionally referred as cancer specific oligosaccharides/oligosaccharide sequences) are targeted for treatment of all kinds of cancers and tumors. The term cancer includes tumors.

The present invention is specifically directed to the treatment of all types of cancer or tumors expressing the tumor specific oligosaccharide sequences according to the present invention. Examples of preferred cancer types includes cancers of larynx, colon cancer, stomach cancer, breast cancer, lung cancer, kidney cancer, pancreas cancer, and ovarian cancer.

Glycolipid and carbohydrate nomenclature is according to recommendations by the IUPAC-IUB Commission on Biochemical Nomenclature (Carbohydr. Res. 1998, 322:167; Carbohydr. Res. 1997, 297:1; Eur. J. Biochem. 1998, 257:29).

It is assumed that Gal, Glc, Man, GlcNAc, GalNAc are of the D-configuration, and that all monosaccharide units are in the pyranose form. Glucosamine is referred as GlcN and galactosamine as GalN. Glycosidic linkages are shown partly in shorter and partly in longer nomenclature, the linkages α3 and α6 of the NeuNAc-residues mean the same as α2-3 and α2-6, respectively, and β1-3, β1-4, and β1-6 can be shortened as β3, β4, and β6, respectively. Lactosamine or N-acetyllactosamine or Galβ3/4GlcNAc means either type one structure residue Galβ3GlcNAc or type two structure residue Galβ1-4GlcNAc. Hex is any hexose, preferably Man, Gal, or Glc; HexNAc is any N-acetylhexosamine, preferably GlcNAc or GalNAc.

<u>Description of preferred glycan methods</u>

**Tissue derived glycomes**

**Glycomes - novel glycan mixtures from tissue samples**

The present specification reveals novel methods for producing novel carbohydrate compositions, glycomes from animal tissues, preferably from vertebrates, more preferably human and mammalian tissues. The tissue substrate materials can be total tissue samples and fractionated tissue parts, such as serums, secretions and isolated differentiated cells from the tissues, or artificial models of tissues such as cultivated cell lines.

The invention revealed that the glycan structures on cell surfaces vary between the various tissues and same tissues under changing conditions, especially cancer.

The glycan structures on cell surfaces in general have been known to have numerous biological roles. Thus the knowledge about exact glycan mixtures from cell or tissue surfaces is important for knowledge about the status of cells. The invention revealed that multiple conditions affect the cells and cause changes in their glycomes.

**Molecular weight distribution and structure groups of the glycomes**

**General compositions**

The inventors were able to release or isolate various glycan fractions from tissue materials, which are useful for the characterization of the cancer cellular material. The glycans or major part thereof are released preferably from glycoproteins or glycolipids of tissue samples. The invention is specifically directed to such glycan fractions. The glycan fractions of tissue samples comprise typically multiple, at least about 10 "glycan mass components" typically corresponding at least ten glycans and in most cases clearly more than 10 glycan structures.

## Glycan mass components and corresponding monosaccharide compositions

The glycan mass components correspond to certain molecular weights observable by mass spectrometry and further correspond to specific monosaccharide composition or monosaccharide compositions. Each monosaccharide component is normally present in a glycan as glycosidically linked monosaccharide residue in the nonreducing end part of glycan and the reducing end monosaccharide may be in free alditol form or modified for example by reduction or conjugated to an reducing end modifying reagent well known in the art or to one, two or several amino acids in case of glycopeptides. Monosaccharide composition can be obtained from molecular mass in a mass spectrum (glycan mass component) after correcting potential effect of the ion forms observable by the specific mass spectrometry technology such as protonation/deprotonation, $Na^+$, $K^+$, $Li^+$, or other adduct combinations, or isotope pattern derived effects. The monosaccharide compositions are calculated by fitting mixtures of individual monosaccharide (residue) masses and modification groups to corrected molecular mass of glycan mass component. Typically the molecular mass of fitting composition and the experimental mass correspond to each other very closely with similar first and even second decimals with optimal calibration.

The fitting may be further checked by measuring the experimental mass difference from the smaller and/or larger glycan mass component next in the putative biosynthetic series of a glycan type and comparing the difference with the exact molecular mass of corresponding monosaccharide unit (residue), typically the mass differences of fitting components in a good quality mass spectrum and with correct marking of peaks in decimals, preferaby in second or third decimal of the mass number depending on the resolution of the specific mass spectrometric method. For optimal mass accuracy, an internal calibration may be used, where two or more known component's mass peaks are used to re-calculate masses for each component in the spectrum. Such calibration components are preferably selected among the most abundant glycan signals present in the glycan profiles, in the case of human or other animal cell derived glycan profiles most preferably selected among the most abundant glycan signals present e.g. in Figures described in the present invention.

The monosaccharide composition includes monosaccharide component names and number, typically as subscript, indicating how many of the individual mass components are present in the monosaccharide composition; and names of assigned modifying groups and numbers indicating their abundance.

It is further realized that the masses of glycan mass component may be obtained as exact monoisotopic mass of usually smallest isotope of the glycan mass component or as an average mass of the isotope distribution of the glycan mass component. Exact mass is calculated form exact masses of individual mass components and average from masses average masses of individual mass components. Person skilled in art can recognize from the peak shapes (i.e. by the resolution obtained) in the mass spectrum whether to use monoisotopic or average masses to interpret the spectra. It is further realized that average and exact masses can be converted to each other when isotope abundances of molecules are known, typically natural abundance without enrichment of isotopes can be assumed, unless the material is deliberately labelled with radioactive or stable isotopes.

It is further realized that specific rounded mass numbers can be used as names for glycan mass components. The present invention uses preferably mass numbers rounded down from the exact mass of the monosaccharide composition (and usually observable or observed mass) to closest integer as names of glycan mass components.

The masses of glycan mass components are obtained by calculating molecular mass of individual monosaccharide components (Hex, HexNAc) from the known atom compositions (for example hexose corresponds to $C_6H_{12}O_6$) and subtracting for water in case of monosaccharide residue, followed by calculating the sum of the monosaccharide components (and possible modifications such as $SO_3$ or $PO_3H$) .It is further realized that molecular masses of glycans may be calculated from atomic compositions or any other suitable mass units corresponding molecular masses of these. The molecular masses and calculation thereof are known in the art and masses of monosaccharide components/residues are available in tables with multiple decimals from various sources.

It is further realized that many of the individual monosaccharide compositions described in the present invention further correspond to several isomeric individual glycans. In addition, there exist also monosaccharide compositions that have nearly equal masses. It is realized that the ability to differentiate compositions with nearly equal masses depends on instrumentation, and the present method is especially directed to a possibility to select also such compositions in place of proposed compositions.

The preferred glycans in glycomes comprise at least two of following monosaccharide component residues selected from group: Hexoses (Hex) which are Man; N-acetylhexosamines (HexNAc) which are GlcNAc. The monosaccharide residues are further grouped as major backbone monosaccharides including GlcNAc, Man; and specific terminal modifying monosaccharide unit Glc.

## Detection of glycan modifications

The present invention is directed to analyzing glycan components from biological samples, preferably as mass

spectrometric signals. Specific glycan modifications can be detected among the detected signals by determined indicative signals as exemplified below. Modifications can also be detected by more specific methods such as chemical or physical methods, for example mass spectrometric fragmentation or glycosidase detection as disclosed in the present invention. In a preferred form of the present method, glycan signals are assigned to monosaccharide compositions based on the detected m/z ratios of the glycan signals, and the specific glycan modifications can be detected among the detected monosaccharide compositions.

In a further aspect of the present invention, relative molar abundances of glycan components are assigned based on their relative signal intensities detected in mass spectrometry as described in the Examples, which allows for quantification of glycan components with specific modifications in relation to other glycan components. The present method is also directed to detecting changes in relative amounts of specific modifications in cells at different time points to detect changes in cell glycan compositions.

**Glycome glycan fraction further comprising monosaccharides**

The invention is specifically directed to glycan compositions, which further comprise at least one monosaccharide component in free form, preferably a preferred monosaccharide component described above. The monosaccharide comprising compositions are in a preferred embodiment derived from a cell material or released glycomes, which has been in contact with monosaccharide releasing chemicals or enzymes, preferably with exoglycosidase enzymes or chemicals such as oxidating reagents and/or acid or base, more preferably with a glycosidase enzyme. The invention is further directed to compositions comprising a specific preferred monosaccharide according to the invention, an exoglycosidase enzyme capable releasing all or part of the specific monosaccharide and an glycan composition according to the invention from which at least part of the terminal specific monosaccharide has been released.

**Limit of detection for glycome components**

It is further realized that by increasing the sensitivity of detection the number of glycan mass components in a given sample can be increased. The analysis according to the invention can in most cases be performed from major or significant components in the glycome mixture. The present invention is preferably directed to detection of glycan mass components from a high quality glycan preparation with optimised experimental condition, when the glycan mass components have abundance at least higher than 0.01% of total amount of glycan mass components, more preferably of glycan mass components of abundance at least higher than 0.05%, and most preferably at least higher than 0.10% are detected. The invention is further directed to practical quality glycome compositions and analytic process directed to it, when glycan mass components of at least about 0.5 %, of total amount of glycan mass components, more preferably of glycan mass components of abundance at least higher than 1.0 %, even more preferably at least higher than 2.0%, most preferably at least higher than 4.0% (presenting lower range practical quality glycome), are detected. The invention is further directed to glycomes comprising preferred number of glycan mass components of at least the abundance of observable in high quality glycomes, and in another embodiment glycomes comprising preferred number of glycan mass components of at least the abundance of observable in practical quality glycomes.

**Subglycomes obtainable by purification or specific release method**

It is further realized that fractionation or differential specific release methods of glycans from glycoconjugates can be applied to produce subglycomes containing part of glycome.

The subglycomes produced by fractionation of glycomes are called "fractionated subglycomes".

The glycomes produced by specific release methods are "linkage-subglycomes". The invention is further directed to combinations of linkage-subglycomes and fractionated subglycomes to produce "fractionated linkage-subglycomes", for example preferred fractionated linkage-subglycomes include neutral N-glycans, which were found very practical in characterising target materials according to the invention.

The fractionation can be used to enrich components of low abundance. It is realized that enrichment would enhance the detection of rare components. The fractionation methods may be used for larger amounts of cell material. In a preferred embodiment the glycome is fractionated based on the molecular weight, charge or binding to carbohydrate binding agents. These methods have been found useful for specific analysis of specific subglycomes and enrichment of more rare components. The present invention is in a preferred embodiment directed to charge based separation of neutral and acidic glycans. This method gives rise for an analysis method, preferably mass spectroscopy material of reduced complexity and it is useful for analysis as neutral molecules in positive mode mass spectrometry and negative mode mass spectrometry for acidic glycans.

Differential release methods may be applied to get separately linkage specific subglycomes such as O-glycan, N-glycan, glycolipid or proteoglycan comprising fractions or combinations thereof. Chemical and enzymatic methods

are known for release of specific fractions, furthermore there are methods for simultaneous release of O-glycans and N-glycans.

**Novel complete compositions**

It is realized that at least part of the glycomes have novelty as novel compositions of very large amount of components. The glycomes comprising very broad range substances are referred as complete glycomes.
Preferably the composition is a complete composition comprising essentially all degrees of polymerisation in general from at least about about disaccharides, more preferably from trisaccharides to at least about 25-mers in a high resolution case and at least to about 20-mers or at least about 15-mer in case of medium and practical quality preparations. It is realized that especially the lower limit, but also upper limit of a subglycome depend on the type of subglycome and/or method used for its production. Different complete ranges may be produced in scope of general glycomes by fractionation, especially based on size of the molecules.

**Novel compositions with new combinations of subglycomes and preferred glycan groups**

It is realized that several glycan types are present as novel glycome compositions produced from the tissue samples. The invention is specifically directed to novel mixture compositions comprising different subglycomes and preferred glycan groups.

**Novel quantitative glycome compositions**

It is realised that the glycome compositions as described in the Examples represent quantitatively new data about glycomes from the preferred tissue sample types. The proportions of various components cannot be derived from background data and are very useful for the analysis methods according to the invention. The invention is specifically directed to glycome compositions according to the Examples when the glycan mass components are present in essentially similar relative amounts.

Preferred composition formulas

The present invention is specifically directed to glycomes of tissue samples according to the invention comprising glycan material with monosaccharide composition for each of glycan mass components according to the Formula I:

$$NeuAc_mNeuGc_nHex_oHexNAc_pdHex_qHexA_rPen_sAc_tModX_x,$$

wherein m, n, o, p, q, r, s, t, and x are independent integers with values ≥ 0 and less than about 100,
with the proviso that
for each glycan mass components at least two of the backbone monosaccharide variables o, p, or r is greater than 0, and ModX represents a modification (or N different modifications Mod1, Mod2, ..., ModN), present in the composition in an amount of x (or in independent amounts of x1, x2, ..., xN), Preferably examples of such modifications (Mod) including for example $SO_3$ or $PO_3H$ indicating esters of sulfate and phosphate, respectively
and the glycan composition is preferably derived from isolated human tissue samples or preferred subpopulations thereof according to the invention.
It is realized that usually glycomes contain glycan material for which the the variables are less much less than 100, but large figures may be obtained for polymeric material comprising glycomes with repeating polymer structures, for example ones comprising glycosaminoglycan type materials. It is further realized that abundance of the glycan mass components with variables more than 10 or 15 is in general very low and observation of the glycome components may require purification and enrichment of larger glycome components from large amounts of samples.

**Broad mass range glycomes**

In a preferred embodiment the invention is directed to broad mass range glycomes comprising polymeric materials and rare individual components as indicated above. Observation of large molecular weight components may require enrichment of large molecular weight molecules comprising fraction. The broad general compositions according to the **Formula I** are as described above,
with the proviso that
m, n, o, p, q, r, s, t, and x are independent integers with preferable values between 0 and 50, with the proviso that for each glycan mass components at least two of o, p, or r is at least 1, and the sum of the monosaccharide variables; m,

n, o, p, q, r, and s, indicating the degree of polymerization or oligomerization, for each glycan mass component is less than about 100 and the glycome comprises at least about 20 different glycans of at least disaccharides.

**Practical mass range glycomes**

In a preferred embodiment the invention is directed to practical mass range and high quality glycomes comprising lower molecular weight ranges of polymeric material. The lower molecular weight materials at least in part and for preferred uses are observable by mass spectrometry without enrichment.

In a more preferred general composition according to the **Formula I** as described above,
m, n, o, p, q, r, s, t, and x are independent integers with preferable values between 0 and about 20, more preferably between 0 and about 15, even more preferably between 0 and about 10, with the proviso that at least two of o, p, or r is at least 1,
and the sum of the monosaccharide variables; m, n, o, p, q, r, and s, indicating the degree of polymerization or oligomerization, for each glycan mass component is less than about 50 and more preferably less than about 30,
and the glycome comprises at least about 50 different glycans of at least trisaccharides.

In a preferred embodiment the invention is directed to practical mass range high quality glycomes which may comprise some lower molecular weight ranges of polymeric material. The lower molecular weight materials at least in part and for preferred uses are observable by mass spectrometry without enrichment.

In a more preferred general composition according to the **Formula I** as described above,
m, n, o, p, q, r, s, t, and x are independent integers with preferable values between 0 and about 10, more preferably between 0 and about 9, even more preferably, between 0 and about 8,
with the proviso that at least two of o, p, or r is at least 1, and the sum of the monosaccharide variables; m, n, o, p, q, r, and s, indicating the degree of polymerization or oligomerization, for each glycan mass component is less than about 30 and more preferably less than about 25,
and the glycome comprises at least about 50 different glycans of at least trisaccharides.

The practical mass range glycomes may typically comprise tens of components, for example in positive ion mode MALDI-TOF mass spectrometry for neutral subglycomes it is usually possible to observe even more than 50 molecular mass components, even more than 100 mass components corresponding to much larger number of potentially isomeric glycans. The number of components detected depends on sample size and detection method.

**Preferred Subglycomes**

The present invention is specifically directed to subglycomes of tissue sample glycomes according to the invention comprising glycan material with monosaccharide compositions for each of glycan mass components according to the **Formula I** and as defined for broad and practical mass range glycomes. Each subglycome has additional characteristics based on glycan core structures of linkage-glycomes or fractionation method used for the fractionated glycomes. The preferred linkage glycomes include N-glycans and neutral subglycomes.

*N-glycan subglycome*

Protein N-glycosidase releases N-glycans comprising typically two N-acetylglucosamine units in the core, optionally a core linked fucose unit and typically then 2-3 hexoses (core mannoses), after which the structures may further comprise hexoses being mannose or in complex-type N-glycans further N-acetylglycosamines and optionally hexoses and sialic acids.

N-glycan subglycomes relased by protein N-glycosidase comprise N-glycans containing N-glycan core structure and are releasable by protein N-glycosidase from cells. The N-glycan core structure is Manβ4GlcNAcβ4(Fucα6)$_n$GlcNAc, wherein n is 0 or 1 and the N-glycan structures can be elongated from the Manβ4 with additional mannosylresidues. The protein N-glycosidase cleaves the reducing end GlcNAc from Asn in proteins. N-glycan subglycomes released by endo-type N-glycosidases cleaving between GlcNAc units contain Manβ4GlcNAc-core, and the N-glycan structures can be elongated from the Manβ4 with additional mannosylresidues.

In case the Subglycome and analysis representing it as Glycan profile is formed from N-glycans liberated by N-glycosidase enzyme, the preferred additional constraints for **Formula I** are:
$p > 0$, more preferably $1 \leq p \leq 100$, typically p is between 2 and about 20, but polymeric structures containing glycomes may comprise larger amounts of HexNAc and it is realized that in typical core of N-glycans indicating presence of at least partially complex type structure, when $p \geq 3$ it follows that $o \geq 1$.

*Neutral subglycomes*

Most preferred fractionated Subglycomes includes subglycome of neutral glycans

**Preferred structure groups observable in glycome profiles**

The present invention is specifically directed to the glycomes of tissue samples according to the invention comprising as major components at least one of structure groups selected from the groups described below.

**Glycan groups**

According to the present invention, the glycan signals are optionally organized into glycan groups and glycan group profiles based on analysis and classification of the assigned monosaccharide and modification compositions and the relative amounts of monosaccharide and modification units in the compositions, according to the following classification rules:

1° The glycan structures are described by the formulae:

$$Hex_mHexNAc_ndHex_oNeuAc_pNeuGc_qPen_rMod1_{sMod1}Mod2_{sMod2}...ModX_{sModX},$$

wherein m, n, o, p, q, individual sMod, and X, are each independent variables, and Mod is a functional group covalently linked to the glycan structure.

2° Glycan structures are classified as follows:

a. In the case of mammalian glycans, structures (o = 0) are classified as "non-fucosylated",
b. Structures (n = 2 and 4 ≥ m ≥ 1 and p,q = 0) are classified as "low-mannose N-glycans",

**Glycomes comprising novel glycan types**

The present invention revealed novel unexpected components among in the glycomes studied. The present invention is especially directed to glycomes comprising such unusual materials.

**Preferred glycome types**

**Derivatized glycomes**

It is further realized that the glycans may be derivatized chemically during the process of release and isolation. Preferred modifications include modifications of the reducing end and/or modifications directed especially to the hydroxyls- and/or N-atoms of the molecules. The reducing end modifications include modifications of reducing end of glycans involving known derivatization reactions, preferably reduction, glycosylamine, glycosylamide, oxime (aminooxy-) and reductive amination modifications. Most preferred modifications include modification of the reducing end. The derivatization of hydroxyl- and/or amine groups, such as produced by methylation or acetylation methods including permethylation and peracetylation has been found especially detrimental to the quantitative relation between natural glycome and the released glycome.

**Non-derivatized released glycomes**

In a preferred embodiment the invention is directed to non-derivatized released glycomes. The benefit of the non-derivatized glycomes is that less processing is needed for the production. The non-derivatized released glycomes correspond more exactly to the natural glycomes from which these are released. The present invention is further directed to quantitative purification according to the invention for the non-derivatized released glycomes and analysis thereof.

The present invention is especially directed to released glycomes when the released glycome is not a permodified glycome such as permethylated glycome or peracetyated glycome. The released glycome is more preferably reducing end derivatized glycome or a non-derivatized glycome, most preferably non-derivatized glycome.

### Novel cell surface glycomes and released glycomes of the target material

The present invention is further directed to novel total compositions of glycans or oligosaccharides referred as glycomes and in a more specific embodiment as released glycomes observed from or produced from the target material according to the invention. The released glycome indicates the total released glycans or total specific glycan subfractions released from the target material according to the invention. The present invention is specifically directed to released glycomes meaning glycans released from the target material according to the invention and to the methods according to the invention directed to the glycomes.
The present invention is preferably directed to the glycomes released as truncated and/or non-truncated glycans and/or derivatized according to the invention.
The invention is especially directed to N-linked and/or O-linked and/or lipid-linked released glycomes from the target material according to the invention. The invention is more preferably directed to released glycomes comprising glycan structures according to the invention, preferably glycan structures as defined in **Formula I.** The invention is more preferably directed to N-linked released glycomes comprising glycan structures according to the invention, preferably glycan structures as defined in **Formula I.**

### Non-derivatized released cell surface glycomes and production

In a preferred embodiment the invention is directed to non-derivatized released cell surface glycomes. The non-derivatized released cell surface glycomes correspond more exactly to the fractions of glycomes that are localized on the cell surfaces, and thus available for biological interactions. These cell surface localized glycans are of especial importance due to their availability for biological interactions as well as targets for reagents (e.g. antibodies, lectins, etc.) targeting the cells or tissues of interest. The invention is further directed to release of the cell surface glycomes, preferably from intact cells by hydrolytic enzymes such as proteolytic enzymes, including proteinases and proteases, and/or glycan releasing enzymes, including endo-glycosidases or protein N-glycosidases. Preferably the surface glycoproteins are cleaved by proteinase such as trypsin and then glycans are analysed as glycopeptides or preferably released further by glycan releasing enzyme.

### Analysis of the glycomes

The present invention is especially directed to analysis of glycan mixtures present in tissue samples by chemical, biochemical, or physical means, preferably by mass spectrometry, as described below.

### Quantitative and qualitative analysis of glycan profile data

The invention is directed to novel methods for qualitative analysis of glycome data. The inventors noticed that there are specific components in glycomes according to the invention, the presence or absence of which are connected or associated with specific cell type or cell status. It is realized that qualitative comparison about the presence of absence of such signals are useful for glycome analysis. It is further realized that signals either present or absent that are derived from a general glycome analysis may be selected to more directed assay measuring only the qualitatively changing component or components optionally with a more common component or components useful for verification of data about the presence or absence of the qualitative signal.
The present invention is further specifically directed to quantitative analysis of glycan data from tissue samples. The inventors noted that quantitative comparisons of the relative abundances of the glycome components reveal substantial differences about the glycomes useful for the analysis according to the invention.

### Essential steps of the glycome analysis

The process contains essential key steps which should be included in every process according to the present invention.
The essential key steps of the analysis are:

1. Release of total glycans or total glycan groups from a tissue sample
2. Purification of the glycan fraction/fractions from contaminating biological material of the sample, preferably by a small scale column array or an array of solid-phase extraction steps
3. Analysis of the composition of the released glycans, preferably by mass spectrometry

In most cases it is useful to compare the data with control sample data. The control sample may be for example

from a healthy tissue or cell type and the sample from same tissue altered by cancer or another disease. It is preferable to compare samples from same individual organism, preferably from the same human individual.

**Specific types of the glycome analysis**

**Comparative analysis**

The steps of a comparative analysis are:

1. Release of total glycans or total glycan groups from tissue sample
2. Purification of the glycan fraction/fractions from biological material of the sample, preferably by a small scale column array or an array of solid-phase extraction steps
3. Analysis of the composition of the released glycans, preferably by mass spectrometry
4. Comparing data about the released glycans quantitatively or qualitatively with data produced from another tissue sample

It may be useful to analyse the glycan structural motifs present in the sample, as well as their relative abundances. The ability to elucidate structural motifs results from the quantitative nature of the present analysis procedure, comparison of the data to data from previously analyzed samples, and knowledge of glycan biosynthesis.

**Analysis including characterization of structural motives**

The glycome analysis may include characterization of structural motives of released glycans. The structural motif analysis may be performed in combination with structural analysis.
Preferred methods to reveal specific structural motifs include

a) direct analysis of specific structural modifications of the treatment of glycans preferably by exo- or endoglycosidases and/or chemical modification or
b) indirect analysis by analysis of correlating factors for the structural motives for such as mRNA-expression levels of glycosyltransferases or enzymes producing sugar donor molecules for glycosyltransferases.

The direct analyses are preferred as they are in general more effective and usually more quantitative methods, which can be combined to glycome analysis.
In a preferred embodiment the invention is directed to combination of analysis of structural motifs and glycome analysis.

*The steps of a structural motif analysis are:*

1. Release of total glycans or total glycan groups from a tissue sample
2. Purification of the glycan fraction/fractions from biological material of the sample, preferably by a small scale column array or an array of solid-phase extraction steps
3. Analysis of the composition of the released glycans, preferably by mass spectrometry
4. Analysis of structural motifs present in of the glycan mixture, and optionally their relative abundancies
5. Optionally, comparing data about the glycan structural motifs with data produced from another tissue sample

The steps 3 and 4 may be combined or performed in order first 4 and then 3.

*Preferred detailed glycome analysis including quantative data analysis*

More detailed preferred analysis method include following analysis steps:

1. Preparing a tissue sample containing glycans for the analysis
2. Release total glycans or total glycan groups from a tissue sample
3. Optionally modifying glycans or part of the glycans.
4. Purification of the glycan fraction/fractions
from biological material and reagents of the sample by a small scale column array

5. Optionally modifying glycans and optionally purifying modified glycans
6. Analysis of the composition of the released glycans preferably by mass spectrometry using at least one mass spectrometric analysis method
7. a) Optionally presenting the data about released glycans quantitatively and
7. b) Comparing the quantitative data set with another data set from another tissue sample
and/or alternatively to 7a) and 7b)
8. Comparing data about the released glycans quantitatively or qualitatively with data produced from another tissue sample

The present methods further allow the possibility to use part of the non-modified material or material modified in step 3 or 5 for additional modification step or step and optionally purified after modification step or steps, optionally combining modified samples, and analysis of additionally modified samples, and comparing results from differentially modified samples.

As mentioned above, it is realized that many of the individual monosaccharide compositions in a given glycome further corresponds to several isomeric individual glycans. The present methods allow for generation of modified glycomes. This is of particular use when modifications are used to reveal such information about glycomes of interest that is not directly available from a glycan profile alone (or glycome profiles to compare). Modifications can include selective removal of particular monosaccharides bound to the glycome by a defined glycosidic bond, by degradation by specific exoglycosidases or selective chemical degradation steps such as e.g. periodic acid oxidation. Modifications can also be introduced by using selective glycosyltransferase reactions to label the free acceptor structures in glycomes and thereby introduction of a specific mass label to such structures that can act as acceptors for the given enzyme. In preferred embodiment several of such modifications steps are combined and used to glycomes to be compared to gain further insights of glycomes and to facilitate their comparison.

Such modifications may also include non-covalent modification, such as ion-pairing of charged groups. Sulphate esters may be ion-paired with cationic moiety, which enhances the ionization of sulphated glycans in positive-ion mode mass spectrometry. Such cationic moieties include e.g. lysine or arginine tripeptide (**KKK** or **RRR**), as described previously for glycopeptides. The present invention is specifically directed to using ion-pairing of free oligosaccharides to enhance detection of glycans with charged groups such as sulphate or phosphate. According to the present invention, glycans containing charged groups may also be identified by analysis as differential adduct and/or ion-pairing ions. For example, comparison of spectra obtained from the same sample in the presence of sodium or lithium ions gives information about the presence of charged groups without covalent modification.

**Quantitative presentation of glycome analysis**

The present invention is specifically directed to quantitative presentation of glycome data.

**Two-dimensional presentation by quantitation and component indicators**

The quantitative presentation means presenting quantitative signals of components of the glycome, preferably all major components of the glycome, as a two-dimensional presentation including preferably a single quantitative indicator presented together with component identifier. A preferred purpose of this operation is to allow reliable comparison between data obtained from different samples or to identify glycan structures present in the analyzed sample. Any given glycan can exist as multiple ions in mass spectrometry or multiple modified forms in mass spectrometry and chromatography. For example, a glycan can exist as adduct ions with e.g. sodium or potassium. This may divide the signal to e.g. three or four components in the case of sialylated glycans, and these signals have to be summed up to get the correct signal intensity value for the glycan component. Another example is related to comparison of samples with different sample quality, and normalization is required to be able to reliably compare these samples. The prior art describes comparison between glycan samples without proper correction and normalization of the data.

The preferred two-dimensional presentations includes tables and graphs presenting the two dimensional data. The preferred tables list quantitative indicators in connection with, preferably beside or under or above the component identifiers, most preferably beside the identifier because in this format the data comprising usually large number of component identifier - quantitation indicator pairs.

**Quantitation indicator**

The quantitation indicator is a value indicating the relative abundance of the single glycome component with regard to other components of total glycome or subglycome. The quantitation indicator can be directly derived from quatitative experimental data, or experimental data corrected to be quantitative.

**Normalized quantitation indicator**

The quantitation indicator is preferably a normalized quantitation indicator. The normalized quantitation indicator is defined as the experimental value of a single experimental quantitation indicator divided by total sum of quantitation indicators multiplied by a constant quantitation factor.

Preferred quantitation factors includes integer numbers from 1- 1000 0000 000, more preferably integer numbers 1, 10 or 100, and more preferably 1 or 100, most preferably 100. The quantitation number one is preferred as commonly understandable portion from 1 concept and the most preferred quantitation factor 100 corresponds to common concept of per cent values.

The quantitation indicators in tables are preferably rounded to correspond to practical accuracy of the measurements from which the values are derived from. Preferred rounding includes 2-5 meaningful accuracy numbers, more preferably 2-4 numbers and most preferably 2-3 numbers.

**Component indicators**

The preferred component indicators may be experimentally derived component indicators. Preferred components indicators in the context of mass spectrometric analysis includes mass numbers of the glycome components, monosaccharide or other chemical compositions of the components and abbreviation corresponding to thereof, names of the molecules preferably selected from the group: desriptive names and abbreviations; chemical names, abbreviations and codes; and molecular formulas including graphic representations of the formulas.

It is further realized that molecular mass based component indicators may include multiple isomeric structures. The invention is in a preferred embodiment directed to practical analysis using molecular mass based component indicators. In more specific embodiment the invention is further directed to chemical or enzymatic modification methods or indirect methods according to the invention in order to resolve all or part of the isomeric components corresponding to a molecular mass based component indicators.

**Glycan signals**

The present invention is directed to a method of accurately defining the molecular masses of glycans present in a sample, and assigning monosaccharide compositions to the detected glycan signals.

The glycan signals according to the present invention are glycan components characterized by:

1° mass-to-charge ratio (m/z) of the detected glycan ion,
2° molecular mass of the detected glycan component, and/or
3° monosaccharide composition proposed for the glycan component.

**Glycan profiles**

The present invention is further directed to a method of describing mass spectrometric raw data of glycan signals as two-dimensional tables of:

1° monosaccharide composition, and
2° relative abundance,

which form the glycan profiles according to the invention. Monosaccharide compositions are as described above. For obtaining relative abundance values for each glycan signal, the raw data is recorded in such manner that the relative signal intensities of the glycan signals represent their relative molar proportions in the sample. Methods for relative quantitation in MALDI-TOF mass spectrometry of glycans are known in the art (Naven & Harvey, 1996; Papac et al., 1996) and are described in the present invention. However, the relative signal intensities of each glycan signal are preferably corrected by taking into account the potential artefacts caused by e.g. isotopic overlapping, alkali metal adduct overlapping, and other disturbances in the raw data, as described below.

By forming these glycan profiles and using them instead of the raw data, analysis of the biological data carried by the glycan profiles is improved, including for example the following operations:

1° identification of glycan signals present in the glycan profile,
2° comparison of glycan profiles obtained from different samples,
3° comparison of relative intensities of glycan signals within the glycan profile, and
4° organizing the glycan signals present in the glycan profile into subgroups or subprofiles.

**Analysis of associated signals to produce single quantitative signal (quantitation indicator)**

**Analysis of associated signals: isotope correction**

Glycan signals and their associated signals may have overlapping isotope patterns. Overlapping of isotope patterns is corrected by calculating the experimental isotope patterns and subtracting overlapping isotope signals from the processed data.

**Analysis of associated signals: adduct ion correction in positive ion mode**

Glycan signals may be associated with signals arising from multiple adduct ions in positive ion mode, e.g. different alkali metal adduct ions. Different glycan signals may give rise to adduct ions with similar m/z ratios: as an example, the adduct ions $[Hex+Na]^+$ and $[dHex+K]^+$ have m/z ratios of 203.05 and 203.03, respectively. Overlapping of adduct ions is corrected by calculating the experimental alkali metal adduct ion ratios in the sample and using them to correct the relative intensities of those glycan signals that have overlapping adduct ions in the experimental data. Preferably, the major adduct ion type is used for comparison of relative signal intensities of the glycan signals, and the minor adduct ion types are removed from the processed data. The calculated proportions of minor adduct ion types are subtracted from the processed data.

**Analysis of associated signals: adduct ion correction in negative ion mode**

Also in negative ion mode mass spectrometry, glycan signals may be associated with signals arising from multiple adduct ions. Typically, this occurs with glycan signals that correspond to multiple acidic group containing glycan structures. As an example, the adduct ions $[NeuAc_2\text{-}H+Na]^-$ at m/z 621.2 and $[NeuAc_2\text{-}H+K]^-$ at m/z 637.1, are associated with the glycan signal $[NeuAc_2\text{-}H]^-$ at m/z 599.2. These adduct ion signals are added to the glycan signal and thereafter removed from the processed data. In cases where different glycan signals and adduct ion signals overlap, this is corrected by calculating the experimental alkali metal adduct ion ratios in the sample and using them to correct the relative intensities of those glycan signals that have overlapping adduct ions in the experimental data.

**Analysis of associated signals: removal of elimination products**

Glycan signals may be associated with signals, e.g. elimination of water (loss of $H_2O$), or lack of methyl ether or ester groups (effective loss of $CH_2$), resulting in experimental m/z values 18 or 14 mass units smaller than the glycan signal, respectively. These signals are not treated as individual glycan signals, but are instead treated as associated signals and removed from the processed data.

**Classification of glycan signals into glycan groups**

According to the present invention, the glycan signals are optionally organized into glycan groups and glycan group profiles based on analysis and classification of the assigned monosaccharide and modification compositions and the relative amounts of monosaccharide and modification units in the compositions, according to the classification rules described above.

**Generation of glycan group profiles**

To generate glycan group profiles, the proportions of individual glycan signals belonging to each glycan group are summed. The proportion of each glycan group of the total glycan signals equals its prevalence in the glycan profile. The glycan group profiles of two or more samples can be compared. The glycan group profiles can be further analyzed by arranging glycan groups into subprofiles, and analyzing the relative proportions of different glycan groups in the subprofiles. Similarly formed subprofiles of two or more samples can be compared.

**Specific technical aspects of tissue glycome analysis**

**Preferred sample sizes**

The present invention is especially useful when low sample amounts are available. Practical cellular or tissue material may be available for example for diagnostic only in very small amounts.

*Sample sizes for preferred pico-scale preparation methods*

The inventors found surprisingly that glycan fraction could be produced and analysed effectively from samples containing low amount of material, for example 100 000-1 000 000 cells or a cubic millimetre (microliter) of the cells.
The combination of very challenging biological samples and very low amounts of samples forms another challenge for the present analytic method. The yield of the purification process must be very high. The estimated yields of the glycan fractions of the analytical processes according to the present invention varies between about 50 % and 99 %. Combined with effective removal of the contaminating various biological materials even more effectively over the wide preferred mass ranges according to the present invention show the ultimate performance of the method according to the present invention.

**Isolation of glycans and glycan fractions**

The present invention is directed to a method of preparing an essentially unmodified glycan sample for analysis from the glycans present in a given sample.
A preferred glycan preparation process consists of the following steps:

1° isolating a glycan-containing fraction from the sample,
2° optionally purification the fraction to useful purity for glycome analysis

The preferred isolation method is chosen according to the desired glycan fraction to be analyzed. The isolation method may be either one or a combination of the following methods, or other fractionation methods that yield fractions of the original sample:

1° extraction with water or other hydrophilic solvent, yielding water-soluble glycans or glycoconjugates such as free oligosaccharides or glycopeptides,
2° extraction with hydrophobic solvent, yielding hydrophilic glycoconjugates such as glycolipids,
3° N-glycosidase treatment, especially *F. meningosepticum* N-glycosidase F treatment, yielding N-glycans,
4° alkaline treatment, such as mild (e.g. 0.1 M) sodium hydroxide or concentrated ammonia treatment, either with or without a reductive agent such as borohydride, in the former case in the presence of a protecting agent such as carbonate, yielding β-elimination products such as O-glycans and/or other elimination products such as N-glycans,
5° endoglycosidase treatment, such as endo-β-galactosidase treatment, especially Escherichia freundii endo-β-galactosidase treatment, yielding fragments from poly-N-acetyllactosamine glycan chains, or similar products according to the enzyme specificity, and/or 6° protease treatment, such as broad-range or specific protease treatment, especially trypsin treatment, yielding proteolytic fragments such as glycopeptides.

The released glycans are optionally divided into sialylated and non-sialylated subfractions and analyzed separately. According to the present invention, this is preferred for improved detection of neutral glycan components, especially when they are rare in the sample to be analyzed, and/or the amount or quality of the sample is low. Preferably, this glycan fractionation is accomplished by graphite chromatography and/or ion-exchange chromatography.
According to the present invention, sialylated glycans are optionally modified in such manner that they are isolated together with the non-sialylated glycan fraction in the non-sialylated glycan specific isolation procedure described above, resulting in improved detection simultaneously to both non-sialylated and sialylated glycan components. Preferably, the modification is done before the non-sialylated glycan specific isolation procedure. Preferred modification processes include neuraminidase treatment and derivatization of the sialic acid carboxyl group, while preferred derivatization processes include amidation and esterification of the carboxyl group.

**Glycan release methods**

The preferred glycan release methods include, but are not limited to, the following methods: Free glycans - extraction of free glycans with for example water or suitable water-solvent mixtures.
Protein-linked glycans including N-linked glycans - alkaline elimination of protein-linked glycans, optionally with subsequent reduction of the liberated glycans.
N-glycans - enzymatic liberation, optionally with N-glycosidase enzymes including for example N-glycosidase F from *C. meningosepticum,* Endoglycosidase H from *Streptomyces,* or N-glycosidase A from almonds.
Glycan fragments - specific exo- or endoglycosidase enzymes including for example endoglycosidase enzyme; chemical cleavage methods; physical methods

**Effective purification process**

The invention describes special purification methods for glycan mixtures from tissue samples. Previous glycan sample purification methods have required large amounts of material and involved often numerous chromatographic steps and even purification of specific proteins. It is known that protein glycosylation varies protein specifically and single protein specific data can thus not indicate the total tissue level glycosylation. Purification of single protein is a totally different task than purifying the glycan fraction according to the present invention.

When the purification starts from a tissue or cells, the old processes of prior art involve often laborious homogenisation steps affecting the quality of the material produced. The present purification directly from a biological sample such as cell or tissue material, involves only a few steps and allows quick purification directly from the biological material to analysis preferably by mass spectrometry.

**Purification from cellular materials of cells and/or tissues**

The cellular material contains various membranes, small metabolites, various ionic materials, lipids, peptides, proteins etc. All of the materials can prevent glycan analysis by mass spectrometry if these cannot be separated from the glycan fraction. Moreover, for example peptide or lipid materials may give rise to mass spectrometric signals within the preferred mass range within which glycans are analysed. Many mass spectrometric methods, including preferred MALDI-mass spectrometry for free glycan fractions, are more sensitive for peptides than glycans. With the MALDI method peptides in the sample may be analysed with approximately 1000-fold higher sensitivity in comparision to methods for glycans. Therefore the method according to the present invention should be able to remove for example potential peptide contaminations from free glycan fractions most effectively. The method should remove essential peptide contaminations from the whole preferred mass range to be analysed.

**Purification suitable for mass spectrometry, especially MALDI-TOF mass spectrometry**

The inventors discovered that the simple purification methods would separate released glycans from all possible cell materials so that

1) The sample is technically suitable for mass spectrometric analysis.
This includes two major properties,

   a) the samples is soluble for preparation of mass spectrometry sample and
   b) does not have negative interactions with chemicals involved in the mass spectrometric method, preferably the sample dries or crystallizes properly with matrix chemical used in MALDI-TOF mass spectrometry

When using MALDI-technologies, the sample does not dry or crystallize properly if the sample contains harmful impurity material in a significant amount.

2) The purity allows production of mass spectrum of suitable quality.

   a) the sample has so low level impurities that it gives mass spectrometric signals. Especially when using MALDI-TOF mass spectrometry, signals can be suppressed by background so that multiple components/peaks cannot be obtained.
   b) the sample is purified so that there is no major impurity signals in the preferred mass ranges to be measured.

Preferably the present invention is directed to analysis of unusually small sample amounts. This provides a clear benefit over prior art, when there is small amount amount of sample available from a small region of diseased tissue or diagnostic sample such as tissue slice produced for microscopy or biopsy sample. Methods to achieve such purity (purity being a requirement for the sensitivity needed for such small sample amounts) from tissue or cell samples (or any other complex biological matices e.g. serum, saliva) has not been described in the prior art.

In a preferred embodiment the method includes use of non-derived glycans and avoiding general derived glycans. There are methods of producing glycan profiles including modification of all hydroxyl groups in the sample such as permethylation. Such processes require large sample amounts and produces chemical artefacts such as undermethylated molecules lowering the effectivity of the method. These artefact peaks cover all minor signals in the spectra, and they can be misinterpreted as glycan structures. It is of importance to note that in glycome analyses the important profile-to profile differences often reside in the minor signals.. In a specific embodiment the present invention is directed to site specific modification of the glycans with effective chemical or enzyme reaction, preferably a quantitative reaction.

## Preferred analytical technologies for glycome analysis

### Mass spectrometric analysis of glycomes

The present invention is specifically directed to quantitative mass spectrometric methods for the analysis of glycomes. Most preferred mass spectrometric methods are MALDI-TOF mass spectrometry methods.

### MALDI-TOF analysis

The inventors were able to optimise MALDI-TOF mass spectrometry for glycome analysis.

The preferred mass spectrometric analysis process is MALDI-TOF mass spectrometry, where the relative signal intensities of the unmodified glycan signals represent their relative molar proportions in the sample, allowing relative quantification of both neutral (Naven & Harvey, 1996) and sialylated (Papac et al., 1996) glycan signals. Preferred experimental conditions according to the present invention are described under Experimental procedures of Examples listed below.

### Preferred mass ranges for MALDI-TOF analysis and released non-modified glycomes

For MALDI-TOF mass spectrometry of unmodified glycans in positive ion mode, optimal mass spectrometric data recording range according to the present invention is over m/z 200, more preferentially between m/z 200 - 10000, or even more preferably between m/z 200 - 4000 for improved data quality. In the most preferred form according to the present invention, the data is recorded between m/z 700 - 4000 for accurate relative quantification of glycan signals.

For MALDI-TOF mass spectrometry of unmodified glycans in negative ion mode, optimal mass spectrometric data recording range according to the present invention is over m/z 300, more preferentially between m/z 300 - 10000, or even more preferably between m/z 300 - 4000 for improved data quality. In the most preferred forms according to the present invention, the data is recorded between m/z 700 - 4000 or most preferably between m/z 800 - 4000 for accurate relative quantification of glycan signals.

### Practical m/z-ranges

The practical ranges comprising most of the important signals, as observed by the present invention may be more limited than these. Preferred practical ranges includes lower limit of about m/z 400, more preferably about m/z 500, and even more preferably about m/z 600, and most preferably m/z about 700 and upper limits of about m/z 4000, more preferably m/z about 3500 (especially for negative ion mode), even more preferably m/z about 3000 (especially for negative ion mode), and in particular at least about 2500 (negative or positive ion mode) and for positive ion mode to about m/z 2000 (for positive ion mode analysis). The preferred range depends on the sizes of the sample glycans, samples with high branching or polysaccharide content or high sialylation levels are preferably analysed in ranges containing higher upper limits as described for negative ion mode. The limits are preferably combined to form ranges of maximum and minimum sizes or lowest lower limit with lowest higher limit, and the other limits analogously in order of increasing size.

### Preferred analysis modes for MALDI-TOF for effective glycome analysis

The inventors were able to show effective quantitative analysis in both negative and positive mode mass spectrometry.

### Sample handling

The inventors developed optimised sample handling process for preparation of the samples for MALDI-TOF mass spectrometry.

### Glycan purification

The glycan purification method according to the present invention consists of at least one of purification options, preferably in specific combinations described below, including the following purification options:

1) Precipitation-extraction;
2) Ion-exchange;

3) Hydrophobic interaction;
4) Hydrophilic interaction; and
5) Affinity to graphitized carbon.

*1) Precipitation-extraction* may include precipitation of glycans or precipitation of contaminants away from the glycans. Preferred precipitation methods include:

1. Glycan material precipitation, for example acetone precipitation of glycoproteins, oligosaccharides, glycopeptides, and glycans in aqueous acetone, preferentially ice-cold over 80 % (v/v) aqueous acetone; optionally combined with extraction of glycans from the precipitate, and/or extraction of contaminating materials from the precipitate;
2. Protein precipitation, for example by organic solvents or trichloroacetic acid, optionally combined with extraction of glycans from the precipitate, and/or extraction of contaminating materials from the precipitate;
3. Precipitation of contaminating materials, for example precipitation with trichloroacetic acid or organic solvents such as aqueous methanol, preferentially about 2/3 aqueous methanol for selective precipitation of proteins and other non-soluble materials while leaving glycans in solution;

*2) Ion-exchange* may include ion-exchange purification or enrichment of glycans or removal of contaminants away from the glycans. Preferred ion-exchange methods include:

1. Cation exchange, preferably for removal of contaminants such as salts, polypeptides, or other cationizable molecules from the glycans; and
2. Anion exchange, preferably either for enrichment of acidic glycans such as sialylated glycans or removal of charged contaminants from neutral glycans, and also preferably for separation of acidic and neutral glycans into different fractions.

3) *Hydrophilic interaction* may include purification or enrichment of glycans due to their hydrophilicity or specific adsorption to hydrophilic materials, or removal of contaminants such as salts away from the glycans. Preferred hydrophilic interaction methods include:

1. Hydrophilic interaction chromatography, preferably for purification or enrichment of glycans and/or glycopeptides;
2. Adsorption of glycans to cellulose in hydrophobic solvents for their purification or enrichment, preferably to microcrystalline cellulose, and even more preferably using an n-butanol:methanol:water or similar solvent system for adsorption and washing the adsorbed glycans, in most preferred system n-butanol:methanol:water in relative volumes of 10:1:2, and water or water:ethanol or similar solvent system for elution of purified glycans from cellulose.

4) *Affinity to graphitized carbon* may include purification or enrichment of glycans due to their affinity or specific adsorption to graphitized carbon, or removal of contaminants away from the glycans. Preferred graphitized carbon affinity methods include porous graphitized carbon chromatography.

Preferred purification methods according to the invention include combinations of one or more purification options. Examples of the most preferred combinations include the following combinations:

1) For neutral underivatized glycan purification: 1. cation exchange of contaminants, 2. hydrophobic adsorption of contaminants, and 3. graphitized carbon affinity purification of glycans.

1) For sialylated underivatized glycan purification: 1. cation exchange of contaminants, 2. hydrophobic adsorption of contaminants, 3. optionally adsorption of glycans to cellulose, and 4. graphitized carbon affinity purification of glycans.

**NMR-analysis of glycomes**

The present invention is directed to analysis of released glycomes by spectrometric method useful for characterization of the glycomes. The invention is directed to NMR spectroscopic analysis of the mixtures of released glycans. The inventors showed that it is possible to produce a released glycome from tissue samples in large scale enough and useful purity for NMR-analysis of the glycome. In a preferred embodiment the NMR-analysis of the tissue glycome is one dimensional proton NMR-analysis showing structural reporter groups of the major components in the glycome. The present invention is further directed to combination of the mass spectrometric and NMR analysis of small scale tissue samples.

**Targets of analysis - Tissue materials**

The present invention refers as "tissue materials" all preferred target tissue related material including for example tissues, secretions and cultivated differentiated cells

*Preferred tissue type*

The present invention is preferably directed to specific tissue types for the analysis according to the invention. The tissue type are found to be very suitable and feasible for the analysis according to the invention. The analysis is especially directed to analysis of 1) tissues of gastrointestinal track, preferably mouth, larynx, stomach, large and small intestine

2) internal organs such as ovarian tissue, liver, lungs, or kidney
3) tissues of circulatory system, especially blood
4) cultivated cell line models of the differentiated tissues

*Preferred tissue parts*

The inventors realized that it is possible perform glycomics analysis of specific parts of tissues and reveal differences useful for studies of diseases and disease induced changes and other changes or presence of receptor structures on specific subtissues. Preferred subtissues includes

1) tissues surfaces, especially epithelia of gastrointestinal tract and cell surfaces and
2) components of circulatory system, preferably serum/plasma, and blood cells, especially red cells and white blood cells

**Subcomponents of glycomes, especially from secreted proteins**

The invention is further directed to methods for selecting specific components of glycomes and searching enriched fractions such as specific protein fraction comprising the specific glycome components. Examples of such preferred methods include search of "cancer specific" oligosaccharide structures according to the invention from serum, saliva or urine. The "cancer specific" oligosaccharide can exist as free secreted oligosaccharides or as conjugates to other biomolecules such as proteins or lipids.

*Tissue surface glycomes*

In a preferred embodiment the invention is directed to special methods for the analysis of the surfaces of tissues.
The preferred tissue surfaces includes

1) epithelia or endothelia of the preferred cancer tissues and
2) surfaces of cells according to cells on surface of tissues or separable homogeneously from tissue, such as blood cells and
3) surfaces of cultivated cells which may be used as models for differentiated tissues.

**Non-derivatized released target material surface glycomes and production**

In a preferred embodiment the invention is directed to non-derivatized released cell surface glycomes. The non-derivatized released cell surface glycomes correspond more exactly to the fractions of glycomes that are localized on the cell surfaces, and thus available for biological interactions. These cell surface localized glycans are of especial importance due to their availability for biological interactions as well as targets for reagents (e.g. antibodies, lectins, etc.) targeting the cells or tissues of interest. The invention is further directed to release of the cell surface glycomes, preferably from intact cells by hydrolytic enzymes such as proteolytic enzymes, including proteinases and proteases, and/or glycan releasing enzymes, including endo-glycosidases or protein N-glycosidases. Preferably the surface glycoproteins are cleaveed by proteinase such as trypsin and then glycans are analysed as glycopeptides or preferably relased further by glycan relasing enzyme.
*Purification method*
The invention represents effective methods for purification of oligosaccharide fractions from tissues, especially in very low scale. The prior art has shown analysis of separate glycome components from tissues, but not total glycomes.

It is further realized that the methods according to the invention are useful for analysis of glycans from isolated proteins or peptides.

*Analysis of glycomes*

The invention is further directed to novel quantitative analysis methods for glycomes. The glycome analysis produces large amounts of data. The invention reveals methods for the analysis of such data quantitatively and comparision of the data between different samples. The invention is especially directed to quantitative two-dimensional representation of the data.

*Integrated glycome analysis*

The invention is further directed to integrated glycomics or glycome analysis process including

1) Optional release of glycans from tissues
2) isolation/purification of glycans from sample,
3) analysis of the glycome
4) quantitative presentation of the data

The first step is optional as the method is further directed to analysis of known and novel secretion derivable soluble glycomes.

*Preferred uses of glycomes and analysis thereof with regard to status of cells*

In the present invention the word cell refer to cells of tissue material according to the invention, especially cancer cells.

**Specific characteristic marker structures and glycome marker components/compositions**

The N-glycan analysis of total profiles of released N-glycans revealed beside the glycans above, which were verified to comprise

1) complex biantennary N-glycans, such as Galβ4GlcNAcβ2Manα3(Galβ4GlcNAcβ2Manα6)Manβ4GlcNAcβ4(Fucα6)$_{0-1}$GlcNAcβ-, wherein the reminal N-acetyllactosamines can be elongated from Gal with NeuNAcα3 aand/or NeuNAcα6 and
2) terminal mannose containing N-glycans such as High-mannose glycans with formula $Hex_{5-9}HexNAc_2$ and degradation products thereof comprising low number of mannose residues (Low mannose glycans) $Hex_{1-4}HexNAc_2$.

The specific N-glycan core marker structure

The glycan share common core structure according to the Formula:

[Manα3]$_{n1}$(Manα6)$_{n2}$Manβ4GlcNAcβ4(Fucα6)$_{0-1}$GlcNAcβAsn,

wherein n1 and n2 are integers 0 or 1, independently indicating the presence or absence of the terminal Man-residue, and wherein the non-reducing end terminal Manα3/Manα6- residues can be elongated to the complex type, especially biantennary structures or to mannose type (high-Man and/or low Man) or to hybrid type structures as desribed in examples.
The invention thus describes the major core structure of N-glycans in human tissue materials verified by NMR-spectroscopy and by specific glycosidase digestions and further reveals that the core structure is a useful target structure for analysis of tissue materials.
The characteristic monosaccharide composition of the core structure will allow recognition of the major types of N-glycan structure groups present as additional modification of the core structure. Furthermore composition of the core structure is characteristic in mass spectrometric analysis of N-glycan and allow immediate recognition for example from $Hex_xHexNAc_1$ -type (preferentially $Man_xGlcNAc_1$) glycans also present in total glycome compostion.

Preferred glycomes

The present invention is specifically directed to tissue material glycomes, which are essentially pure glycan

mixtures comprising various glycans as described in the invention preferably in proportions shown by the invention. The essentially pure glycan mixtures comprise the key glycan components in proportions which are characteristics to tissue material glycomes. The preferred glycomes are obtained from human tissue materials according to the invention.

The invention is further directed to glycomes as products of purification process and variations thereof according to the invention. The products purified from tissue materials by the simple, quantitative and effective methods according to the invention are essentially pure. The essentially pure means that the mixtures are essentially devoid of contaminations disturbing analysis by MALDI mass spectrometry, preferably by MALDI-TOF mass spectrometry. The mass spectra produced by the present methods from the essentially pure glycomes reveal that there is essentially no non-carbohydrate impurities with weight larger than trisaccharide and very low amount of lower molecular weight impurities so that crystallization of MALDI matric is possible and the glycan signals can be observed for broad glycomes with large variations of monosaccharide compositions and ranges of molecular weight as described by the invention. It is realized that the purification of the materials from low amounts of tissue materials comprising very broad range of cellular materials is very challenging task and the present invention has accomplished this.

<u>Combination compositions of the preferred glycome mixtures with matrix for analysis</u>

*Mass spectrometric matrix*

The invention further revealed that it is possible to combine the glycomes with matrix useful for a mass spectrometric analysis and to obtain combination mixture useful for spectrometric analysis. The preferred mass spectrometric matrix is matrix for MALDI (matrix assisted laser desorption ionization mass spectrometry) with mass spectrometric analysis (abbreviated as MALDI matrix), MALDI is preferably performed with TOF (time of flight) detection.

Preferred MALDI matrices include aromatic preferably benzene ring structure comprising molecules with following characteristic. The benzene ring structure molecules preferably comprises 1-4 substituents such as hydroxyl, carboxylic acid or ketone groups. Known MALDI matrixes have been reviewed in Harvey, Mass. Spec. Rev. 18, 349 (1999). The present invention is especially and separately directed to specific matrixes for analysis in negative ion mode of MALDI mass spectrometry, preferred for analysis of negatively charged (acidic, such as sialylated and/or sulfated and/or phosphorylated) subglycome, and in positive ion mode of MALDI mass spectrometry (preferred for analysis of neutral glycomes). It is realized that the matrices can be optimized for negative ion mode and positive ion mode.

The present invention is especially directed to glycome matrix composition optimized for the use in positive ion mode, and to the use of the MALDI-TOF matrix and matrix glycome composition, that is optimized for the use in the analysis in positive ion mode, for the analysis of glycome, preferably neutral glycome. The preferred matrices for positive ion mode are aromatic matrices, e.g. 2,5-dihydroxybenzoic acid, 2,5-dihydroxybenzoic acid/2-hydroxy-5-methoxybenzoic acid, 2,4,6-trihydroxyacetophenone or 6-aza-2-thiothymine, more preferably 2,5-dihydroxybenzoic acid. The present invention is especially directed to glycome matrix composition optimized for the use in negative ion mode, and to the use of the MALDI-TOF matrix and the matrix glycome compositions, that is optimized for the negative ion mode, for the analysis of glycome, preferably acidic glycome. The preferred matrices for negative ion mode are aromatic matrices, e.g. 2,4,6-trihydroxyacetophenone, 3-hydroxypicolinic acid, 2,5-dihydroxybenzoic acid, 2,5-dihydroxybenzoic acid/2-hydroxy-5-methoxybenzoic acid, or 6-aza-2-thiothymine, more preferably 2,4,6-trihydroxyacetophenone. The invention is further directed to analysis method and glycome-matrix compostion for the analysis of glycome compositions, wherein the glycome composition comprises both negative and neutral glycome components. Preferred matrices for analysis of negative and neutral glycome components comprising glycome are aromatic matrices, e.g. 2,4,6-trihydroxyacetophenone, 3-hydroxypicolinic acid, 2,5-dihydroxybenzoic acid, 2,5-dihydroxybenzoic acid/2-hydroxy-5-methoxybenzoic acid, or 6-aza-2-thiothymine, more preferably 2,4,6-trihydroxyacetophenone.

The MALDI-matrix is a molecule capable of

1) Specifically and effectively co-crystallizing with glycome composition with the matrix, crystallizing meaning here forming a solid mixture composition allowing analysis of glycome involving two steps below
2) absorbing UV-light typically provided by a laser in MALDI-TOF instrument, preferred wavelength of the light is 337 nm as defined by the manuals of MALDI-TOF methods
3) transferring energy to the glycome compostion so that these will ionize and be analyzable by the MALDI-TOF mass spectrometry. The present invention is especially directed to compositions of glycomes in complex with MALDI mass spectrometry matrix.

The present invention is specifically directed to methods of analysis of glycomes by MALDI-TOF including the

steps:

1) Specifically and effectively co-crystallizing the glycome composition with the MALDI-TOF-matrix, crystallizing meaning here forming a solid mixture composition allowing analysis of glycome involving two steps below
2) Providing UV linght to crystalline sample by a laser in MALDI-TOF instrument allowing the ionization of sample
3) Analysis of the ions produced by the MALDI mass spectrometer, preferably by TOF analysis. The invention is further directed to the combination of glycome purification methods and/or quantitative and qualitative data analysis methods according to the invention.

*Crystalline compositions of glycomes*

The invention revealed that it is possible to analyze glycomes using very low amount of sample. The preferred crystalline glycome composition comprises between 0.1 - 100 pmol, more preferably 0.5- 10 pmol, more preferably 0.5-5 pmol and more preferably about 0.5-3 pmol, more preferably about 0.5 - 2 pmol of sample co-crystallized with optimized amount of matrix preferably about 10-200 nmol, more preferably 30-150 nmol, and more preferably about 50-120 nmol and most preferably between 60-90 nmols of the matrix, preferably when the matrix is 2,5-dihydroxybenzoic acid. The matrix and analyte amounts are optimized for a round analysis spot with radius of about 1 mm and area of about 0.8 $mm^2$. It is realized that the amount of materials can be changed in proportion of the area of the spot, smaller amount for smaller spot. Examples of preferred amounts per area of spot are 0.1-100 pmol/0.8 $mm^2$ and 10-200 pmol/3 $mm^2$. Preferred molar excess of matrix is about 5000-1000000 fold, more preferably about 10000-500000 fold and more preferably about 15000 to 200 000 fold and most preferably about 20000 to 100000 fold excess when the matrix is 2,5-dihydroxybenzoic acid.

It is realized that the amount and relative amount of new matrix is optimized based on forming suitable crystals and depend on chemical structure of the matrix. The formation of crystals is observed by microscope and further tested by performing test analysis by MALDI mass spectrometry.

The invention is further directed to specific methods for crystallizing MALDI-matrix with glycome. Preferred method for crystallization in positive ion mode includes steps: (1) optionally, elimination of impurities, like salts and detergents, which interfere with the crystallization, (2) providing solution of glycome in $H_2O$ or other suitable solvent in the preferred concentration, (3) mixing the glycome with the matrix in solution or depositing the glycome in solution on a precrystallized matrix layer and (4) drying the solution preferably by a gentle stream of air.

Preferred method for crystallization in negative ion mode includes steps: (1) optionally, elimination of impurities, like salts and detergents, which interfere with the crystallization, (2) providing solution of glycome in $H_2O$ or other suitable solvent in the preferred concentration, (3) mixing the glycome with the matrix in solution or depositing the glycome in solution on a precrystallized matrix layer and (4) drying the solution preferably by vacuum.

<u>Other preferred glycome analysis compostions</u>

*Binder glycome compositions*

The invention is further directed to compositions of essentially pure glycome composition with specific glycan binding molecules such as lectins, glycosidases or glycosyltransferases and other glycosyl modifying enzymes such as sulfateses and/or phosphatases and antibodies. It is realized that these composition are especially useful for analysis of glycomes.

The present invention revealed that the complex glycome compositions can be effectively and even quantitatively modified by glycosidases even in very low amounts. It was revealed that the numerous glycan structures similar to target structures of the enzymes do not prevent the degradation by competive inhibition, especially by the enzymes used. The invention is specifically directed to preferred amounts directed to MALDI analysis for use in composition with a glycosyl modifying enzyme, preferably present in low amounts. Preferred enzymes suitable for analysis include enzymes according to the Examples.

The invention is further directed to binding of specific component of glycome in solution with a specific binder. The preferred method further includes affinity chromatography step for purification of the bound component or analysis of the non-bound fraction and comparing it to the glycome solution without the binding substance. Preferred binders include lectins engineered to be lectins by removal of catalytic amino acids (methods published by Roger Laine, Anomeric, Inc., USA, and Prof. Jukka Finne, Turku, Finland), lectins and antibodies or antibody fragments or minimal binding domains of the proteins.

**Additional data analysis and related methods**

The present invention is especially directed to the use of glycome data for production of mathematical formulas, or algorithms, for specific recognition or identification of specific tissue materials. Data analysis methods are presented in **Examples.**

The invention is especially directed to selecting specific "structural features" such as mass spectrometric signals (such as individual mass spectrometric signal corresponding to one or several monosaccharide compositions and/or glycan structures), or signal groups or subglycomes or signals corresponding to specific glycan classes, which are preferably according to the invention, preferably the signal groups (preferably defined as specific structure group by the invention), from quantitative glycome data, preferably from quantitative glycome data according to the invention, for the analysis of status of tissue materials. The invention is furthermore directed to the methods of analysis of the tissue materials by the methods involving the use of the specific signals or signal groups and a mathematical algorithm for analysis of the status of a tissue material.

Preferred algorithm includes use of proportion (such as %-proportion) of the specific signals from total signals as specific values (structural features) and creating a "glycan score", which is algorithm showing characteristics/status of a tissue material based on the specific proportional signal intensities (or quantitative presence of glycan structures measured by any quantitation method such as specific binding proteins or quantitative chromatographic or electrophoresis analysis such as HPLC analysis). Preferably signals which are, preferably most specifically, upregulated in specific tissue materials and signals which are, preferably most specifically, downregulated in the tissue material in comparison to control tissue materials are selected to for the glycan score. In a preferred embodiment value(s) of downregulated signals are subtracted from upregulated signals when glycan score is calculated. The method yields largest score values for a specific tissue material type or types selected to be differentiated from other tissue materials.

The invention is specifically directed to methods for searching characteristic structural features (values) from glycome profiling data, preferably quantitative or qualitative glycome profiling data. The preferred methods include methods for comparing the glycome data sets obtained from different samples, or from average data sets obtained from a group of similar samples such as paraller samples from same or similar tissue material preparations. Methods for searching characteristic features are briefly described in the section: identification and classification of differences in glycan datasets. The comparison of datasets of the glycome data according to the invention preferably includes calculation of relative and/or absolute differences of signals, preferably each signal between two data sets, and in another preferred embodiment between three or more datasets. The method preferably further includes step of selecting the differing signals, or part thereof, for calculating glycan score.

It is further realized that the analyzed glycome data has other uses such as use of the selected characteristic signals and corresponding glycan material:

1) for targets for structural analysis of glycans (preferably chemically by glycosidases, fragmentation mass spectrometry and/or NMR spectroscopy as shown by the present invention and/or structural analysis based on the presence of other signals and knowledge of biosynthesis of glycans). The preferred use for targets includes estimation of chemical characteristics of potential corresponding glycans for complete or partial purification/separation of the specific glycan(s). The preferred chemical characteristics to be analysed preferably include one or several of following properties: a) acidity (e.g. by presence of acidic residues such as sialic acid and/or sulfate and/or phosphate) for charge based separation, b) molecular weight or hydrodymanamic volume affecting chromatographic separation, e.g. estimation of the elution volume in gel filtration methods (the effect of acidic residue can be estimated from effects of similar structures and the "size" of HexNAc (GalNAc/GlcNAc) is in general twice the size of Hex (such as Gal, Man or Glc), c) estimation (e.g. based on composition and biosynthetic knowledge of glycans) of presence of epitopes for specific binding reagents for labelling identification in a mixture or for affinity purification, d) estimation of presence of target epitopes for specific glycosylmodifying enzymes including glycosidases and/or glycosyltransferases (types of binding reagents) or for specific chemical modification reagents (such as periodate for specific oxidation or acid for specific acid hydrolysis), for modification of glycans and recognition of the modification by potential chemical change such as incorporation of radioactive label or by change of mass spectrometric signal of the glycan for labelling identification in a mixture.

2) use of the signals or partially or fully analysed glycan structures corresponding to the signals for searching specific binding reagents for recognition of tissue materials which are preferably selected as described by the present invention (especially as described above) and in the methods for identification and classification of differences in glycan datasets and/or signals selected and/or tested by glycan score methods, are preferably selected for targets for structural analysis of glycans (preferably by glycosidases, fragmentation mass spectrometry and/or NMR spectroscopy as shown by the present invention) and/or for use of the signals or partially or fully analysed glycan structures corresponding to the signals for searching specific binding reagents for recognition of tissue materials.

The preferred method includes the step of comparing the values, and preferably presenting the score values in graphs, and preferably evaluating the statistic significance of the result by a statistic analysis methods such as a mathematical test for statistic significance tissue material type refers here to tissue materials with specific status and/or identity, e.g. malignancy, with possible individual variability, e.g. between individual patients.

It is realized that to differentiate a tissue materials type from other(s) different characteristic signals may be selected than for another tissue material type. The invention however revealed that for tissue materials and especially for human cancer patients preferred characteristic signals include ones selected in the Examples as described above. It is realized that a glycan score can be also created with less characteristic signals or with only part of signals and still relevant results can be obtained. The invention is further directed to methods for optimisation of glycan score algorithms and methods for selecting signals for glycan scores.

In case the specific proportion (value) of a characteristic signal is low in comparision to other values a specific factor can be selected for increase the relative "weight" of the value in the glycan scores to be calculated for the cell populations.

The preferred statuses of tissue materials, to be analysed by mathematical methods such as algorithms using quantitative glycome profiling data according to the invention include differentiation status, individual characteristics and mutation, cell culture or storage conditions related status, effects of chemicals or biochemicals on cells, and other statuses described by the invention.

**Preferred qualitative and quantitative complete N-glycomes of tissue materials**

**High-mannose type and glucosylated N-glycans**

The present invention is especially directed to glycan compositions (structures) and analysis of high-mannose type and glucosylated N-glycans according to the formula:

$Hex_{n3}HexNAc_{n4}$,

wherein n3 is 5, 6, 7, 8, 9, 10, 11, or 12, and n4 = 2.

According to the present invention, within total N-glycomes of tissue materials the major high-mannose type and glucosylated N-glycan signals preferentially include the compositions with 5≤ n3≤ 10: Hex5HexNAc2 (1257), Hex6HexNAc2 (1419), Hex7HexNAc2 (1581), Hex8HexNAc2 (1743), Hex9HexNAc2 (1905), and Hex10HexNAc2 (2067);
and more preferably with 5≤ n3≤ 9: Hex5HexNAc2 (1257), Hex6HexNAc2 (1419), Hex7HexNAc2 (1581), Hex8HexNAc2 (1743), and Hex9HexNAc2 (1905).

**Low-mannose type N-glycans**

The present invention is especially directed to glycan compositions (structures) and analysis of low-mannose type N-glycans according to the formula:

$Hex_{n3}HexNAc_{n4}dHex_{n5}$,

wherein n3 is 1, 2, 3, or 4, n4 = 2, and n5 is 0 or 1.

According to the present invention, within total N-glycomes of tissue materials the major low-mannose type N-glycan signals preferably include the compositions with 2 ≤ n3 ≤ 4: Hex2HexNAc2 (771), Hex3HexNAc2 (933), Hex4HexNAc2 (1095), Hex2HexNAc2dHex (917), Hex3HexNAc2dHex (1079), and Hex4HexNAc2dHex (1241); and more preferably when n5 is 0: Hex2HexNAc2 (771), Hex3HexNAc2 (933), and Hex4HexNAc2 (1095).

As demonstrated in the present invention by glycan structure analysis of tissue materials, preferably this glycan group in tissue materials includes the molecular structures:

(Manα)$_{1-3}$Manβ4GlcNAcβ4(Fucα6)$_{0-1}$GlcNAc within the glycan signals 771, 917, 933, 1079, 1095, and 1095, and the preferred low-Man structures includes structures common all tissue material types, tri-Man and tetra-Man structures according to the Examples,
(Manα)$_{0-1}$Manα6(Manα3)Manβ4GlcNAcβ4(Fucα6)$_{0-1}$GlcNAc, more preferably the tri-Man structures:

Manα6(Manα3)Manβ4GlcNAcβ4(Fucα6)$_{0-1}$GlcNAc
even more preferably the abundant molecular structure:

Manα6(Manα3)Manβ4GlcNAcβ4GlcNAc within the glycan signal 933.

Quantitative analysis directed to the low-Man components

Beside the qualitative variations the low-Man glycans have specific value in quantitative analysis of tissue materials. The present invention revealed that the low-Man glycans are especially useful for the analysis of status of the cells. For example the analysis in the Examples revealed that the amounts of the glycans vary between total tissue profiles and specific organelles, preferably lysosomes.

The group of low-Man glycans form a characteristic group among glycome compositions. The relative total amount of neutral glycans is notable in average human tissues. The glycan group was revealed also to be characteristic in cancerous tissues and tumorsa with total relative amount of neutral glycomes increased. The difference is more pronounced within lysosomal organelle-specific glycome, wherein low-Man structures accounted nearly 50% of the neutral protein-linked glycome. Glycome analysis of tissue materials is especially useful for methods for development of binder reagents for separation of different tissue materials.

The invention is directed to analysis of relative amounts of low-Man glycans, and to the specific quantitative glycome compositions, especially neutral glycan compositions, comprising about 0 to 50 % of low-Man glycans, more preferably between about 1 to 50 % of solid tissue glycomes, for the analysis of tissue materials according to the invention, and use of the composition for the analysis of tissue materials.

**Soluble glycans**

The present invention is especially directed to glycan compositions (structures) and analysis of neutral soluble N-glycan type glycans according to the formula:

$$Hex_{n3}HexNAc_{n4},$$

wherein n3 is 1, 2, 3, 4, 5, 6, 7, 8, or 9, and n4 = 1.

Within total N-glycomes of tissue materials the major soluble N-glycan signals include the compositions with $4 \leq n3 \leq 8$, more preferably $4 \leq n3 \leq 7$: Hex4HexNAc (892), Hex5HexNAc (1054), Hex6HexNAc (1216), Hex7HexNAc (1378). In the most preferred embodiment of the present invention, the major glycan signal in this group within total neutral glycomes of tissue materials is Hex5HexNAc (1054).

**Preferred combinations of glycan types in complete glycomes**

The preferred complete glycomes of tissue materials include low-mannose type, hybrid-type or monoantennary, hybrid, and complex-type N-glycans,
and most preferentially also including soluble glycans as described in the present invention.

In a preferred embodiment of the present invention the tissue material total N-glycome contains the three glycan types: 1) high-mannose type, 2) hybrid-type or monoantennary, and 3) complex-type N-glycans; and more preferably, in the case of solid tissues or cells also 4) low-mannose type N-glycans; and further more preferably, in the case of solid tissues or cells additionally 5) soluble glycans.

In a preferred embodiment of the preferred glycan type combinations within the tissue material complete glycomes, their relative abundances are as described in Tables of

**Examples.**

***More detailed structure and method descriptions***

***Structures of N-linked glycomes***

***Common core structure of N-linked glycomes***

The inventors revealed that the N-glycans released by specific N-glycan release methods from the cells according to the invention, and preferred cells according to the invention, comprise mostly a specific type of N-glycan core structure.

The preferred N-glycan structure of each cell type is characterised and recognized by treating cells with a N-glycan releasing enzyme releasing practically all N-glycans with core type according to the invention. The N-glycan relasing enzyme is preferably protein N-glycosidase enzyme, preferably by protein N-glycosidase releasing effectively

the N-glycomes according to the invention, more preferably protein N-glycosidase with similar specificity as protein N-glycosidase F, and in a specifically preferred embodiment the enzyme is protein N-glycosidase F from *F. meningosepticum.* Alternative chemical N-glycan release method was used for controlling the effective release of the N-glycomes by the N-glycan relasing enzyme.

The inventors used the NMR glycome analysis according to the invention for further characterization of released N-glycomes from small cell samples available. NMR spectroscopy revealed the N-glycan core signals of the preferred N-glycan core type of the cells according to the invention.

**The minimum formula**

The present invention is directed to glycomes derived from cells and comprising a common N-glycosidic core structures. The invention is specifically directed to minimum formulas covering both $GN_1$-glycomes and $GN_2$-glycomes with difference in reducing end structures.

The minimum core structure includes glycans from which reducing end GlcNAc or Fucα6GlcNAc has been released. These are referred as $GN_1$-glycomes and the components thereof as $GN_1$-glycans. The present invention is specifically directed to natural N-glycomes from cells comprising $GN_1$-glycans. In a preferred embodiment the invention is directed to purified or isolated practically pure natural $GN_1$-glycome from human cells. The release of the reducing end GlcNAc-unit completely or partially may be included in the production of the N-glycome or N-glycans from cells for analysis. The invention is specifically directed to soluble high/low mannose glycome of $GN_1$-type.

The glycomes including the reducing end GlcNAc or Fucα6GlcNAc are referred as $GN_2$-glycomes and the components thereof as $GN_2$-glycans. The present invention is also specifically directed to natural N-glycomes from cells and tissues comprising $GN_2$-glycans. In a preferred embodiment the invention is directed to purified or isolated practically pure natural $GN_2$-glycome from cells.

The preferred N-glycan core structure(s) and/or N-glycomes from cells according to the invention comprise structure(s) according to
the formula NC1:

$$R_1M\beta 4GNX_yR_2,$$

Wherein X is glycosidically linked disaccharide epitope $\beta 4(Fuc\alpha 6)_nGN$, wherein n is 0 or 1, or X is nothing and y is anomeric linkage structure α and/or β or linkage from derivatized anomeric carbon, and $R_1$ indicates 1-4, preferably 1-3, natural type carbohydrate substituents linked to the core structures,
$R_2$ is reducing end hydroxyl, chemical reducing end derivative or natural asparagine N-glycoside derivative such as asparagine N-glycosides including asparagines N-glycoside aminoacids and/or peptides derived from protein.

It is realized that when the invention is directed to a glycome, the formula indicates mixture of several or typically more than ten or even higher number of different structures according to the Formulas describing the glycomes according to the invention.

The possible carbohydrate substituents $R_1$ comprise at least one mannose (Man) residue.

When the glycome is released by N-glycosidase the free N-glycome saccharides comprise in a preferred embodiment reducing end hydroxyl with anomeric linkage A having structure α and/or β, preferably both α and β. In another embodiment the glycome is derivatized by a molecular structure which can be reacted with the free reducing end of a released glycome, such as amine, aminooxy or hydrazine or thiol structures. The derivatizing groups comprise typically 3 to 30 atoms in aliphatic or aromatic structures or can form terminal group spacers and link the glycomes to carriers such as solid phases or microparticels, polymeric carries such as oligosaccharides and/or polysaccharide, peptides, dendrimer, proteins, organic polymers such as plastics, polyethyleneglycol and derivatives, polyamines such as polylysines.

When the glycome comprises asparagine N-glycosides, A is preferably beta and R is linked asparagine or asparagine peptide. The peptide part may comprise multiple different aminoacid residues and typically multiple forms of peptide with different sequences derived from natural proteins carrying the N-glycans in cell materials according to the invention. It is realized that for example proteolytic release of glycans may produce mixture of glycopeptides. Preferably the peptide parts of the glycopeptides comprises mainly a low number of amino acid residues, preferably two to ten residues, more preferably two to seven amino acid residues and even more preferably two to five aminoacid residues and most preferably two to four amino acid residues when "mainly" indicates preferably at least 60 % of the peptide part, more preferably at least 75 % and most preferably at least 90 % of the peptide part comprising the peptide of desired low number of aminoacid residues.

**The preferred $GN_2$- N-glycan core structure(s)**

The preferred $GN_2$- N-glycan core structure(s) and/or N-glycomes from cells according to the invention comprise structure(s) according to the formula NC2:

$$R_1M\beta4GN\beta4(Fuc\alpha6)_nGN_yR_2,$$

wherein n is 0 or 1 and
wherein y is anomeric linkage structure α and/or β or linkage from derivatized anomeric carbon and
$R_1$ indicates 1-4, preferably 1-3, natural type carbohydrate substituents linked to the core structures,
$R_2$ is reducing end hydroxyl, chemical reducing end derivative or natural asparagine N-glycoside derivative such as asparagine N-glycosides including asparagines N-glycoside aminoacid and/or peptides derived from protein.

The preferred compositions thus include one or several of the following structures NC2a: $M\alpha3\{M\alpha6\}M\beta4GN\beta4\{Fuc\alpha6\}_{n1}GN_yR_2$

NC2b: $M\alpha6M\beta4GN\beta4\{Fuc\alpha6\}_{n1}GN_yR_2$

NC2c: $M\alpha3M\beta4GN\beta4\{Fuc\alpha6\}_{n1}GN_yR_2$

More preferably compositions comprise at least 3 of the structures or most preferably both structures according to the formula NC2a and at least both fucosylated and non-fucosylated with core structure(s) NC2b and/or NC2c.

**The preferred $GN_1$- N-glycan core structure(s)**

The preferred $GN_1$- N-glycan core structure(s) and/or N-glycomes from cells according to the invention comprise structure(s) according to the formula NC3:

$$R_1M\beta4GN_yR_2,$$

wherein y is anomeric linkage structure α and/or β or linkage from derivatized anomeric carbon and $R_1$ indicates 1-4, preferably 1-3, natural type carbohydrate substituents linked to the core structures,
$R_2$ is reducing end hydroxyl, chemical reducing end derivative or natural asparagine N-glycoside derivative such as asparagine N-glycosides including asparagine N-glycoside aminoacids and/or peptides derived from protein.

**Multi-mannose $GN_1$- N-glycan core structure(s)**

The invention is specifically directed glycans and/or glycomes derived from preferred cells according to the present invention when the natural glycome or glycan comprises Multi-mannose $GN_1$- N-glycan core structure(s) structure (s) according to the formula NC4:

$$[R_1M\alpha3]_{n3}\ \{R_3M\alpha6\}_{n2}M\beta4GNX_yR_2,$$

$R_1$ and R3 indicate nothing or one or two, natural type carbohydrate substituents linked to the core structures, when the substituents are α-linked mannose monosaccharide and/or oligosaccharides and the other variables are as described above.

Furthermore common elongated $GN_2$- N-glycan core structures are preferred types of glycomes according to the invention

**The preferred N-glycan core structures further include differently elongated $GN_2$- N-glycan core structures according to the** formula NC5:

$$[R_1M\alpha3]_{n3}\{R_3M\alpha6\}_{n2}M\beta4GN\beta4\{Fuc\alpha6\}_{n1}GN_yR_2,$$

wherein n1, n2 and n3 are either 0 or 1 and
wherein y is anomeric linkage structure α and/or β or linkage from derivatized anomeric carbon and
$R_1$ and $R_3$ indicate nothing or 1-4, preferably 1-3, most preferably one or two, natural type carbohydrate substituents

linked to the core structures,
$R_2$ is reducing end hydroxyl, chemical reducing end derivative or natural asparagine N-glycoside derivative such as asparagine N-glycosides including asparagine N-glycoside aminoacids and/or peptides derived from protein,
GN is GlcNAc, M is mannosyl-, [ ] indicate groups either present or absent in a linear sequence.
{ }indicates branching which may be also present or absent.
with the provision that at least n2 or n3 is 1. Preferably the invention is directed to compositions comprising with all possible values of n2 and n3 and all saccharide types when R1 and/or are R3 are oligosaccharide sequences or nothing.

Preferred N-glycan types in glycomes comprising N-glycans

The present invention is preferably directed to N-glycan glycomes comprising one or several of the preferred N-glycan core types according to the invention. The present invention is specifically directed to specific N-glycan core types when the compositions comprise N-glycan or N-glycans from one or several of the groups Low mannose glycans, High mannose glycans, Hybrid glycans, and Complex glycans, in a preferred embodiment the glycome comrise substantial amounts of glycans from at least three groups, more preferably from all four groups.

***Major subtypes of N-glycans in N-linked glycomes***

The invention revealed certain structural groups present in N-linked glycomes. The grouping is based on structural features of glycan groups obtained by classification based on the monosaccharide compositions and structural analysis of the structurel groups. The glycans were analysed by NMR, specific binding reagents including lectins and antibodies and specific glycosidases releasing monosaccharide residues from glycans. The glycomes are preferably analysed as neutral and acidic glycomes

**The major neutral glycan types**

The neutral glycomes mean glycomes comprising no acidic monosaccharide residues such as sialic acids (especially NeuNAc and NeuGc), HexA (especially GlcA, glucuronic acid) and acid modification groups such as phosphate and/or sulphate esters. There are four major types of neutral N-linked glycomes which all share the common N-glycan core structure: High-mannose N-glycans, low-mannose N-glycans, hydrid type and complex type N-glycans. These have characteristic monosaccharide compositions and specific substructures. The complex and hybrid type glycans may include certain glycans comprising monoantennary glycans.

*Preferred Mannose type structures*

The invention is forther directed to glycans comprosing terminal Mannose such as Mα6-residue or both Manα6- and Manα3-residues, respectively, can additionally substitute other Mα2/3/6 units to form a Mannose- type structures including hydrid, low-Man and High-Man structures according to the invention.

Preferred high- and low mannose type structures with GN2-core structure are according to the Formula M2:

$$[M\alpha2]_{n1}[M\alpha3]_{n2}\{[M\alpha2]_{n3}[M\alpha6)]_{n4}\}[M\alpha6]_{n5}\{[M\alpha2]_{n6}[M\alpha2]_{n7}[M\alpha3]_{n8}\}M\beta4GN\beta4[\{Fuc\alpha6\}]_{m}\ GN_{y}R_{2}$$

wherein p, n1, n2, n3, n4, n5, n6, n7, n8, and m are either independently 0 or 1; with the proviso that when n2 is 0, also n1 is 0; when n4 is 0, also n3 is 0; when n5 is 0, also n1, n2, n3, and n4 are 0; when n7 is 0, also n6 is 0; when n8 is 0, also n6 and n7 are 0;
y is anomeric linkage structure α and/or β or linkage from derivatized anomeric carbon, and $R_2$ is reducing end hydroxyl, chemical reducing end derivative or natural asparagine N-glycoside derivative such as asparagine N-glycosides including asparagines N-glycoside aminoacid and/or peptides derived from protein;
[ ] indicates determinant either being present or absent depending on the value of n1, n2, n3, n4, n5, n6, n7, n8, and m; and
{ } indicates a branch in the structure.

Preferred $_yR_2$-structures include $[\beta\text{-N-Asn}]_p$, wherein p is either 0 or 1.

**Preferred Mannose type glycomes comprising GN1-core structures**

As described above a preferred variant of N-glycomes comprising only single GlcNAc-residue in the core. Such structures are especially preferred as glycomes produced by endo-N-acetylglucosaminidase enzymes and Soluble glycomes. Preferred Mannose type glycomesnclude structures according to the
Formula M2

$$[M\alpha2]_{n1}[M\alpha3]_{n2}\{[M\alpha2]_{n3}[M\alpha6)]_{n4}\}[M\alpha6]_{n5}\{[M\alpha2]_{n6}[M\alpha2]_{n7}[M\alpha3]_{n8}\}M\beta4GN_{y}R_{2}$$

Fucosylated high-mannose N-glycans according to the invention have molecular compositions $Man_{5-9}GlcNAc_2Fuc_1$. For the fucosylated high-mannose glycans according to the formula, the sum of n1, n2, n3, n4, n5, n6, n7, and n8 is an integer from 4 to 8 and m is 0.

The low -mannose structures have molecular compositions $Man_{1-4}GlcNAc_2FuC_{0-1}$. They consist of two subgroups based on the number of Fuc residues: 1) nonfucosylated low - mannose structures have molecular compositions $Man_{1-4}GlcNAc_2$ and 2) fucosylated low - mannose structures have molecular compositions $Man_{1-4}GlcNAc_2Fuc_1$. For the low mannose glycans the sum of n1, n2, n3, n4, n5, n6, n7, and n8 is less than or equal to (m + 3); and preferably n1, n3, n6, and n7 are 0 when m is 0.

*Low mannose glycans*

The invention revealed a very unusual group glycans in N-glycomes of invention defined here as low mannose N-glycans. These are not clearly linked to regular biosynthesis of N-glycans, but may represent unusual biosynthetic midproducts or degradation products. The low mannose glycans are especially characteristics changing during the changes of cell status, the differentiation and other changes according to the invention, for examples changes associated with differentiation status of cells and their differentiated products and control cell materials.

The invention is especially directed to recognizing low amounts of low-mannose type glycans in cell types, such as with low degree of differentiation.

The invention revealed large differences between the low mannose glycan expression in the cell and tissue glycomes and material from tissue secretions such as human serum.

The invention is especially directed to the use of specific low mannose glycan comprising glycomes for analysis of tissues and cells, preferably cultivated cells.

The invention further revealed specific mannose directed recognition methods useful for recognizing the preferred glycomes according to the invention. The invention is especially directed to combination of glycome analysis and recognition by specific binding agents, most preferred binding agent include enzymes and theis derivatives. The invention further revealed that specific low mannose glycans of the low mannose part of the glycomes can be recognized by degradation by specific α-mannosidase ($Man_{2-4}GlcNAc_2Fuc_{0-1}$) or β-mannosidase ($Man_1GlcNAc_2Fuc_{0-1}$) enzymes and optionally further recognition of small low mannose structures, even more preferably low mannose structures comprising terminal Manβ4-structures according to the invention.

The low mannose N-glycans, and preferred subgroups and individual structures thereof, are especially preferred as markers of the novel glycome compositions of the cells according to the invention useful for characterization of the cell types.

The low-mannose type glycans includes a specific group of α3- and/or α6-linked mannose type structures according to the invention including a preferred terminal and core structure types according to the invention.

The inventions further revealed that low mannose N-glycans comprise a unique individual structural markers useful for characterization of the cells according to the invention by specific binding agents according to the invention or by combinations of specific binding agents according to the invention.

Neutral low-mannose type N-glycans comprise one to four or five terminal Man-residues, preferentially Manα structures; for example Man$\alpha_{0-3}$Manβ4GlcNAcβ4GlcNAc(β-N-Asn) or Man$\alpha_{0-4}$Manβ4GlcNAcβ4(Fucα6)GlcNAc(β-N-Asn).

Low-mannose N-glycans are smaller and more rare than the common high-mannose N-glycans ($Man_{5-9}GlcNAc_2$). The low-mannose N-glycans detected in cell samples fall into two subgroups: 1) non-fucosylated, with composition $Man_nGlcNAc_2$, where $1 \leq n \leq 4$, and 2) core-fucosylated, with composition $Man_nGlcNAc_2Fuc_1$, where $1 \leq n \leq 5$. The largest of the detected low-mannose structure structures is $Man_5GlcNAc_2Fuc_1$ (m/z 1403 for the sodium adduct ion), which due to biosynthetic reasons most likely includes the structure below (in the figure the glycan is free oligosaccharide and β-anomer; in glycoproteins in tissues the glycan is N-glycan and β-anomer):

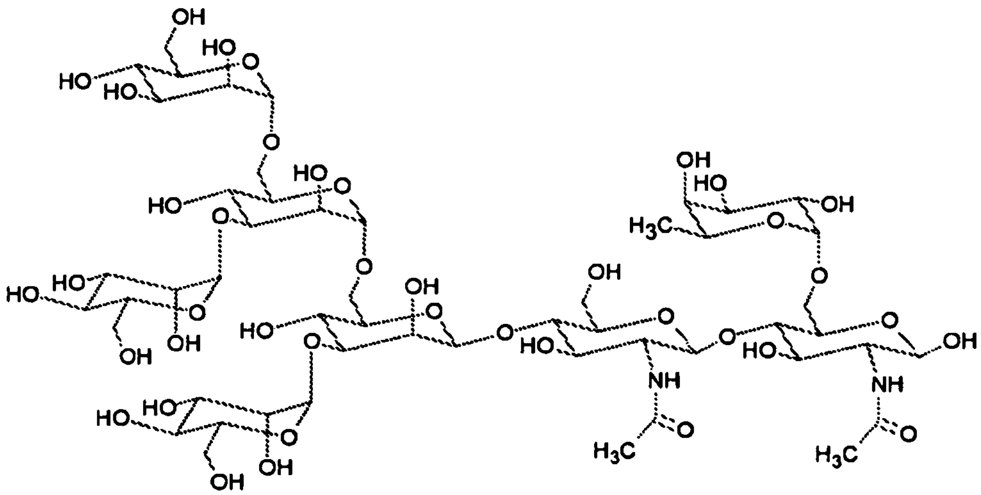

*Preferred general molecular structural features of low Man glycans*

According to the present invention, low-mannose structures are preferentially identified by mass spectrometry, preferentially based on characteristic $Hex_{1-4}HexNAc_2dHex_{0-1}$ monosaccharide composition. The low-mannose structures are further preferentially identified by sensitivity to exoglycosidase digestion, preferentially α-mannosidase ($Hex_{2-4}HexNAc_2dHexc_{0-1}$) or β-mannosidase ($Hex_1HexNAc_2dHex_{0-1}$) enzymes, and/or to endoglycosidase digestion, preferentially N-glycosidase F detachment from glycoproteins, Endoglycosidase H detachment from glycoproteins (only $Hex_{1-4}HexNAc_2$ liberated as $Hex_{1-4}HexNAc_1$), and/or Endoglycosidase F2 digestion (only $Hex_{1-4}HexNAc_2dHex_1$ digested to $Hex_{1-4}HexNAc_1$). The low-mannose structures are further preferentially identified in NMR spectroscopy based on characteristic resonances of the Manβ4GlcNAcβ4GlcNAc N-glycan core structure and Manα residues attached to the Manβ4 residue.

Several preferred low Man glycans described above can be presented in a single Formula:

$$[M\alpha 3]_{n2}\{[M\alpha 6)]_{n4}\}[M\alpha 6]_{n5}\{[M\alpha 3]_{n8}\}M\beta 4GN\beta 4[\{Fuc\alpha 6\}]_m GN_y R_2$$

wherein p, n2, n4, n5, n8, and m are either independently 0 or 1; with the proviso that when n2 is 0, also n1 is 0; when n4 is 0, also n3 is 0; when n5 is 0, also n1, n2, n3, and n4 are 0; when n7 is 0, also n6 is 0; when n8 is 0, also n6 and n7 are 0; the sum of n1, n2, n3, n4, n5, n6, n7, and n8 is less than or equal to (m + 3); [ ] indicates determinant either being present or absent depending on the value of n2, n4, n5, n8, and m; and

{ } indicates a branch in the structure;

y and R2 are as indicated above.

Preferred non-fucosylated low-mannose glycans are according to the formula:

$$[M\alpha 3]_{n2}([M\alpha 6)]_{n4})[M\alpha 6]_{n5}\{[M\alpha 3]_{n8}\}M\beta 4GN\beta 4GN_y R_2$$

wherein p, n2, n4, n5, n8, and m are either independently 0 or 1,

with the provisio that when n5 is 0, also n2 and n4 are 0, and preferably either n2 or n4 is 0,

[ ] indicates determinant either being present or absent depending on the value of, n2, n4, n5, n8,

{} and () indicates a branch in the structure,

y and R2 are as indicated above.

**Preferred individual structures of low-mannose glycans**

**Special small structures**

Small non-fucosylated low-mannose structures are especially unsual among known N-linked glycans and characteristic glycans group useful for separation of cells according to the present invention. These include:

$$M\beta 4GN\beta 4GN_y R_2$$

$M\alpha 6M\beta 4GN\beta 4GN_yR_2$

$M\alpha 3M\beta 4GN\beta 4GN_yR_2$

and

$M\alpha 6\{M\alpha 3\}M\beta 4GN\beta 4GN_yR_2$.

$M\beta 4GN\beta 4GN_yR_2$ trisaccharide epitope is a preferred common structure alone and together with its mono-mannose derivatives $M\alpha 6M\beta 4GN\beta 4GN_yR_2$ and/or $M\alpha 3M\beta 4GN\beta 4GN_yR_2$, because these are characteristic structures commonly present in glycomes according to the invention. The invention is specifically directed to the glycomes comprising one or several of the small non-fucosylated low-mannose structures. The tetrasaccharides are in a specific embodiment preferred for specific recognition directed to α-linked, preferably α3/6-linked Mannoses as preferred terminal recognition element.

## Special large structures

The invention further revealed large low-mannose structures that are unusual among known N-linked glycans and have special characteristic expression features among the preferred cells according to the invention. The preferred large structures include

$[M\alpha 3]_{n2}([M\alpha 6]_{n4})M\alpha 6\{M\alpha\}M\beta 4GN\beta 4GN_yR_2$

more specifically

$M\alpha 6M\alpha 6\{M\alpha 3\}M\beta 4GN\beta 4GN_yR_2$

$M\alpha 3M\alpha 6\{M\alpha 3\}M\beta 4GN\beta 4GN_yR_2$

and

$M\alpha 3(M\alpha 6)M\alpha 6\{M\alpha 3\}M\beta 4GN\beta 4GN_yR_2$.

The hexasaccharide epitopes are preferred in a specific embodiment as rare and characteristic structures in preferred cell types and as structures with preferred terminal epitopes. The heptasaccharide is also preferred as structure comprising a preferred unusual terminal epitope Mα3(Mα6)Mα useful for analysis of cells according to the invention.

## Preferred non-reducing end terminal Mannose-epitopes

The inventors revealed that mannose-structures can be labeled and/or otherwise specifically recognized on cell surfaces or cell derived fractions/matrials of specific cell types. The present invention is directed to the recognition of specific mannose epitopes on cell surfaces by reagents binding to specific mannose structures from cell surfaces.

The preferred reagents for recognition of any structures according to the invention include specific antibodies and other carbohydrate recognizing binding molecules. It is known that antibodies can be produced for the specific structures by various immunization and/or library technologies such as phage display methods representing variable domains of antibodies. Similarily with antibody library technologies, including aptamer technologies and including phage display for peptides, exist for synthesis of library molecules such as polyamide molecules including peptides, especially cyclic peptides, or nucleotide type molecules such as aptamer molecules.

The invention is specifically directed to specific recognition high-mannose and low-mannose structures according to the invention. The invention is specifically directed to recognition of non-reducing end terminal Manα-epitopes, preferably at least disaccharide epitopes, according to the formula:

$$[M\alpha 2]_{m1}[M\alpha x]_{m2}[M\alpha 6]_{m3}\{\{[M\alpha 2]_{m9}[M\alpha 2]_{m8}[M\alpha 3]_{m7}\}_{m10}(M\beta 4[GN]_{m4})_{m5}\}_{m6}yR_2$$

wherein m1, m 2, m3, m4, m5, m6, m7, m8, m9 and m10 are independently either 0 or 1; with the proviso that when m3 is 0, then m1 is 0 and, when m7 is 0 then either m1-5 are 0 and m8 and m9 are 1 forming Mα2Mα2 -disaccharide or both m8 and m9 are 0

y is anomeric linkage structure α and/or β or linkage from derivatized anomeric carbon, and

$R_2$ is reducing end hydroxyl, chemical reducing end derivative and x is linkage position 3 or 6 or both 3 and 6 forming branched structure,
{ } indicates a branch in the structure.

The invention is further directed to terminal Mα2-containing glycans containg at least one Mα2-group and preferably Mα2-group on each, branch so that m 1 and at least one of m8 or m9 is 1. The invention is further directed to terminal Mα3 and/or Mα6-epitopes without terminal Mα2-groups, when all m1, m8 and m9 are 1.

The invention is further directed in a preferred embodiment to the terminal epitopes linked to a Mβ-residue and for application directed to larger epitopes. The invention is especially directed to Mβ4GN-comprising reducing end terminal epitopes.

The preferred terminal epitopes comprise typically 2-5 monosaccharide residues in a linear chain. According to the invention short epitopes comprising at least 2 monosaccharide residues can be recognized under suitable background conditions and the invention is specifically directed to epitopes comprising 2 to 4 monosaccharide units and more preferably 2-3 monosaccharide units, even more preferred epitopes include linear disaccharide units and/or branched trisaccharide non-reducing residue with natural anomeric linkage structures at reducing end. The shorter epitopes may be preferred for specific applications due to practical reasons including effective production of control molecules for potential binding reagents aimed for recognition of the structures.

The shorter epitopes such as Mα2M-may is often more abundant on target cell surface as it is present on multiple arms of several common structures according to the invention.

**Preferred disaccharide epitopes includes**

Manα2Man, Manα3Man, Manα6Man, and more preferred anomeric forms Manα2Manα, Manα3Manβ, Manα6Manβ, Manα3Manα and Manα6Manα.

Preferred branched trisaccharides includes Manα3(Manα6)Man, Manα3(Manα6)Manβ, and Manα3 (Manα6) Manα.

The invention is specifically directed to the specific recognition of non-reducing terminal Manα2-structures especially in context of high-mannose structures.

The invention is specifically directed to following linear terminal mannose epitopes:

a) preferred terminal Manα2-epitopes including following oligosaccharide sequences:

Manα2Man,

Manα2Manα,

Manα2Manα2Man, Manα2Manα3Man, Manα2Manα6Man,

Manα2Manα2Manα, Manα2Manα3Manβ, Manα2Manα6Manα,

Manα2Manα2Manα3Man, Manα2Manα3Manα6Man, Manα2Manα6Manα6Man

Manα2Manα2Manα3Manβ, Manα2Manα3Manα6Manβ, Manα2Manα6Manα6Manβ;

The invention is further directed to recognition of and methods directed to non-reducing end terminal Manα3- and/or Manα6-comprising target structures, which are characteristic features of specifically important low-mannose glycans according to the invention. The preferred structural groups includes linear epitopes according to b) and branched epitopes according to the c3) especially depending on the status of the target matrial.

b) preferred terminal Manα3- and/or Manα6-epitopes including following oligosaccharide sequences:

Manα3Man, Manα6Man, Manα3Manβ, Manα6Manβ, Manα3Manα, Manα6Manα,

Manα3Manα6Man, Manα6Manα6Man, Manα3Manα6Manβ, Manα6Manα6Manβ

and to following

c) branched terminal mannose epitopes, are preferred as characteristic structures of especially high.mannose structures (c1 and c2) and low-mannose structures (c3), The preferred branched epitopes include:

c1) branched terminal Manα2-epitopes

Manα2Manα3(Manα2Manα6)Man, Manα2Manα3(Manα2Manα6)Manα,

Manα2Manα3(Manα2Manα6)Manα6Man, Manα2Manα3(Manα2Manα6)Manα6Manβ,

Manα2Manα3(Manα2Manα6)Manα6(Manα2Manα3)Man,

Manα2Manα3(Manα2Manα6)Manα6(Manα2Manα2Manα3)Man,

Manα2Manα3(Manα2Manα6)Manα6(Manα2Manα3)Manβ

Manα2Manα3(Manα2Manα6)Manα6(ManαManα2Manα3)Manβ

c2) branched terminal Manα2- and Manα3 or Manα6-epitopes according to formula when m 1 and/or m8 and/m9 is 1 and the molecule comprise at least one nonreducing end terminal Manα3 or Manα6-epitope

c3) branched terminal Manα3 or Manα6-epitopes

Manα3(Manα6)Man, Manα3(Manα6)Manβ, Manα3(Manα6)Manα,

Manα3(Manα6)Manα6Man, Manα3(Manα6)Manα6Manβ,

Manα3(Manα6)Manα6(Manα3)Man, Manα3(Manα6)Manα6(Manα3)Manβ

The present invention is further directed to increase of selectivity and sensitivity in recognition of Target glycans by combining recognition methods for terminal Manα2 and Manα3 and/or Manα6-comprising structures. Such methods would be especially useful in context of cell material according to the invention comprising both high-mannose and low-mannose glycans.

**Uses of glycan structure grouping and analysis**

In the present invention, glycan signals with preferential monosaccharide compositions can be grouped into structure groups based on classification rules described in the present invention. The present invention includes parallel and overlapping classification systems that are used for the classification of the glycan structure groups.

Glycan signals isolated from the N-glycan fractions from the tissue material types studied in the present invention are grouped into glycan structure groups based on their preferential monosaccharide compositions according to the invention, in glycan group Tables of Examples for neutral glycan fractions and for acidic glycan fractions. Taken together, the analyses revealed that all the structure groups according to the invention are present in the studied tissue material types. In another aspect of the present invention, the glycan structure grouping is used to compare different tissue materials and characterize their specific glycosylation features. According to the present invention the discovered and analyzed differencies between the glycan signals within the glycan signal groups between different tissue material samples are used for comparison and characterization.

The quantitative glycan profiling combined with glycan structural classification is used according to the present invention to characterize and identify glycosylation features occurring in tissue materials, glycosylation features specific for certain tissue materials as well as differencies between different tissue materials. According to the present invention, the classification is used to characterize and compare glycosylation features of different tissues, of normal and diseased tissues, preferentially cancerous tissues, and solid tissues such as lung tissue and fluid tissues such as blood and/or serum. In another aspect of the present invention, the glycan structure grouping is used to compare different tissue materials and characterize their specific glycosylation features. According to the present invention differencies between relative proportions of glycan signal structure groups are used to compare different tissue material samples.

In a further aspect of the present invention, analysis of the glycan structure groups, preferentially including terminal HexNAc and/or low-mannose and optionally other groups separately or in combination, is used to differentiate between different tissue materials or different stages of tissue materials, preferentially to identify human disease and more preferentially human cancer. In a futher preferred form the present method is used to differentiate between benign and malignant tumors. According to the present invention analysis of human serum glycan groups or combinations

thereof according to the present invention can be used to identify the presence of other tissue materials in blood or serum samples, more preferably to identify disease and preferably malignant cancer.

**Integrated glycome analysis technology**

The invention is directed to analysis of present cell materials based on single or several glycans (glycome profile) of cell materials according to the invention. The analysis of multiple glycans is preferably performed by physical analysis methods such as mass spectrometry and/or NMR.

The invention is specifically directed to integrated analysis process for glycomes, such as total glycomes and cell surface glycomes. The integrated process represent various novel aspects in each part of the process. The methods are especially directed to analysis of low amounts of cells. The integrated analysis process includes

A) preferred preparation of substrate cell materials for analysis, including one or several of the methods: use of a chemical buffer solution, use of detergents, chemical reagents and/or enzymes.
B) release of glycome(s), including various subglycome type based on glycan core, charge and other structural features, use of controlled reagents in the process
C) purification of glycomes and various subglycomes from complex mixtures
D) preferred glycome analysis, including profiling methods such as mass spectrometry and/or NMR spectroscopy
E) data processing and analysis, especially comparative methods between different sample types and quantitative analysis of the glycome data.

***A. Preparation of cell materials***

Cell substrate material and its preparation for total and cell surface glycome analysis. The integrated glycome analysis includes preferably a cell preparation step to increase the availability of cell surface glycans. The cell preparation step preferably degrades either total cell materials or cell surface to yield a glycome for more effective glycan release. The degradation step preferably includes methods of physical degradation and/or chemical degradation. In a preferred embodiment at least one physical and one chemical degradation methods are combined, more preferably at least one physical method is combined with at least two chemical methods, even more preferably with at least three chemical methods.

The physical degration include degration by energy including thermal and/or mechanical energy directed to the cells to degrade cell structures such as heating, freezing, sonication, and pressure. The chemical degradation include use of chemicals and specific concentrations of chemicals for distruption distruption of cells preferably detergents including ionic and neutral detergents, chaotropic salts, denaturing chemicals such as urea, and non-physiological salt concentrations for distruption of the cells.

The glycome analysis according to the invention is divided to two methods including Total cell glycomes, and Cell surface glycomes. The production of Total cell glycomes involves degradation of cells by physical and/or chemical degradation methods, preferably at least by chemical methods, more preferably by physical and chemical methods. The Cell surface glycomes is preferably released from cell surface preserving cell membranes intact or as intact as possible, such methods involve preferably at least one chemical method, preferably enzymatic method. The cell surface glycomes may be alternatively released from isolated cell membranes, this method involves typically chemical and/or physical methods similarity as production of total cell glycomes, preferably at least use of detergents.

**a. Total Cell glycomes**

The present invention revealed special methods for effective purification of released glycans from total cell derived materials so that free oligosaccharides can be obtained. In a preferred embodiment a total glycome is produced from a cell sample, which is degraded to form more available for release of glycans. A preferred degraded form of cells is detergent lysed cells optionally involving physical distruption of cell materials.

Preferred detergents and reaction conditions include,

a1) ionic detergents, preferably SDS type anionic detergent comprising an anionic group such as sulfate and an alkyl chain of 8-16 carbon atoms, more preferably the anionic detergent comprise 10-14 carbon atoms and it is most preferably sodium dodecyl sulfate (SDS), and/or
a2) non-ionic detergents such as alkylglycosides comprising a hexose and 4-12 carbon alkyl chain more preferably the alkyl chain comprises a hexoses being galactose, glucose, and/or mannose, more preferably glucose and/or mannose and the alkyl comprises 6-10 carbon atoms, preferably the non-ionic detergent is octylglucoside .

It is realized that various detergent combinations may be produced and optimized. The combined use of an ionic, preferably anionic, and non-ionic detergents according to the invention is especially preferred.

**Preferred cell preparation methods for production of Total cell glycome**

The preferred methods of cell degration for Total cell glycomes include physical degration including at least heat treatment heat and chemical degration by a detergent method or by a non-detergent method preferably enzymatic degradation, preferably heat treatment. Preferably two physical degradation methods are included.

**A preferred non-detergent method includes**

A non-detergent method is preferred for avoiding detergent in later purification. The preferred non-detergent method involves physical degradation of cells preferably pressure and or by heat and a chemical degradation by protease. A preferred non-detergent method includes: i)cell degradation by physical methods, for example by pressure methods such as by French press.
The treatment is preferably performed quickly in cold temperatures, preferably at 0-2 degrees of Celsius, and more preferably at about 0 or 1 degree of celsius and/or in the presence of glycosidase inhibitors.

ii) The degraded cells are further treated with chemical degradation, preferably by effective general protease, more preferably trypsin is used for the treatment. Preferred trypsin preparation according to the invention does not cause glycan contamination to the sample/does not contain glycans releasable under the reaction conditions.
iii) optionally the physical degradation and chemical degradation are repeated.
iv) At the end of protease treatment the sample is boiled for further denaturing the sample and the protease. The boling is performed at temperature denaturing/degrading further the sample and the protease activity (conditions thus depend on the protease used) preferably about 100 degrees Celsius for time enough for denaturing protease activity preferably about 10-20 minutes for trypsin, more preferably about 15 minutes.

*Preferred detergent method for production of total glycomes*

The invention is in another preferred embodiment directed to detergent based method for lysing cells. The invention includes effective methods for removal of detergents in later purification steps. The detergent methods are especially preferred for denaturing proteins, which may bind or degrade glycans, and for degrading cell membranes to increase the accessibility of intracellular glycans.
For the detergent method the cell sample is preferably a cell pellet produced at cold temperature by centrifuging cells but avoiding distruption of the cells, optionally stored frozed and melted on ice. Optionally glycosidase inhibitors are used during the process.
The method includes following steps:

i) production of cell pellet preferably by centrifugation,
ii) lysis by detergent on ice, the detergent is preferably an anionic detergent according to the invention, more preferably SDS. The concentration of the detergent is preferably between about 0.1 % and 5 %, more preferably between 0.5 %- 3 %, even more preferably between 0.5- 1.5% and most preferably about 1 % and the detergent is SDS (or between 0.9- 1.1%). the solution is preferably produced in ultrapure water,
iii) mixing by effective degradation of cells, preferably mixing by a Vortex-mixer as physical degradation step,
iv) boiling on water bath, preferebly for 3- 10 min, most preferably about 5 min (4-6 min) as second physical degradation step, it is realized that even longer boiling may be performed for example up to 30 min or 15 min, but it is not optimal because of evaporation sample v)adding one volume of non-ionic detergent, preferably alkyl-glycoside detergent according to the invention, most preferably n-octyl-β-D-glucoside, the preferred amount of the detergent is about 5-15 % as water solution, preferably about 10% of octyl-glucoside. The non-ionic detergent is especially preferred in case an enzyme sensitive to SDS, such as a N-glycosidase, is to be used in the next reaction step.
and
vi)incubation at room temperature for about 5 min to about 1-4 hours, more preferably less than half an hour, and most preferably about 15 min.

Preferred amount of detergents in the detergent method

Preferably the anionic detergent and cationic detergent solutions are used in equal volumes. Preferably the solutions are about 1 % SDS and about 10 % octyl-glucoside. The preferred amounts of the solutions are preferably

from 0.1 μl to about 2 μl, more preferably 0.15 μl to about 1.5 μl per and most preferably from 0.16 μl to 1 μl per 100 000 cells of each solution. Lower amounts of the detergents are preferred if possible for reduction of the amount of detergent in later purification, highest amounts in relation to the cell amounts are used for practical reasons with lowest volumes. It is further realized that corresponding weight amounts of the detergents may be used in volumes of about 10% to about 1000%, or from about 20 % to about 500 % and even more effectively in volumes from 30 % to about 300 % and most preferably in volumes of range from 50 % to about 150 % of that described. It is realized that critical micellar concentration based effects may reduce the effect of detergents at lowest concentrations.

In a preferred embodiment a practical methods using tip columns as described in the invention uses about 1-3 μl of each detergent solution, more preferably 1.5-2.5 μl, and most preferably about 2 ul of the preferred detergent solutions or corresponding detergent amounts are used for about 200 000 or less cells (preferably between 2000 and about 250 000 cells, more preferably from 50 000 to about 250 000 cells and most preferably from 100 000 to about 200 000 cells). Another practical method uses uses about 2-10 ul of each detergent solution, more preferably 4-8 μl, and most preferably about 5 μl (preferably between 4 and 6 μl and more preferably between 4.5 and 5.5 μl) of detergent solutions or corresponding amount of the detergents for lysis of cell of a cell amount from about 200 000 - 3 million cells (preferred more exact ranges include 200 000- 3.5 million, 200 000 to 3 million and 200 000 to 2,5 million cells), preferably a fixed amount (specific amount of microliters preferably with the accuracy of at least 0.1 microliter) in a preferred range such as of 5.0 μl is used for the wider range of cells 200 000 - 3 million. It was invented that is possible to handle similarily wider range of materials. It is further realized that the method can be optimized so that exact amount of detergent, preferably within the ranges described, is used for exact amount of cells, such method is preferably an automized when there is possible variation in amounts of sample cells.

b. Cell surface glycomes

In another preferred embodiment the invention is directed to release of glycans from intact cells and analysis of released cell surface glycomes. The present invention is directed to specific buffer and enzymatic cell pre-modification conditions that would allow the efficient use of enzymes for release and optionally modification and release of glycans.

***B. The glycan release methods***

The invention is directed to various enzymatic and chemical methods to release glycomes. The release step is not needed for soluble glycomes according to the invention. The invention further revealed soluble glycome components which can be isolated from the cells using methods according to the invention.

C. Purification of glycans from cell derived materialsThe purification of glycome materials form cell derived molecules is a difficult task. It is especially difficult to purify glycomes to obtain picomol or low nanomol samples for glycome profiling by mass spectrometry or NMR-spectrometry. The invention is especially directed to production of material allowing quantitative analysis over a wide mass range. The invention is specifically directed to the purification of non-derivatized or reducing end derivatized glycomes according to the invention and glycomes containing specific structural characteristics according to the invention. The structural characteristics were evaluated by the preferred methods according to the invention to produce reproducible and quantitative purified glycomes.

**Glycan purification process steps**

*The glycan purification method according to the present invention consists of at least one of purification options, preferably in specific combinations described below, including one or several of following the following purification process steps in varying order:*

6) Precipitation-extraction;
7) Ion-exchange;
8) Hydrophobic interaction;
9) Hydrophilic interaction; and
10) Affinity to carbon materials especially graphitized carbon.

**Prepurification and purification process steps**

In general the purification steps may be divided to two major categories:

Prepurification steps to remove major contaminations and purification steps usually directed to specific binding and optionally fractionation og glycomes

a)Prepurification to remove non-carbohydrate impurities

The need for prepurification depends on the type and amounts of the samples and the amounts of impurities present. Certain samples it is possible to omit all or part of the prepurification steps. The prepurification steps are aimed for removal of major non-carbohydrate impurities by separating the impurity and the glycome fraction(s) to be purified to different phases by precipitation/extraction or binding to chromatography matrix and the separating the impurities from the glycome fraction(s).

The prepurification steps include one, two or three of following major steps: Precipitation-extraction, Ion-exchange, Hydrophobic interaction. The precipitation and/or extraction is based on the high hydrophilic nature of glycome compositions and components, which is useful for separation from different cellular components and chemicals. The prepurification ion exchange chromatography is directed to removal of classes molecules with different charge than the preferred glycome or glycome fraction to be studied. This includes removal of salt ions and aminoacids, and peptides etc. The glycome may comprise only negative charges or in more rare case also only positive charges and the same charge is selected for the chromatography matrix for removal of the impurities for the same charge without binding the glycome at prepurification. In a preferred embodiment the invention is directed to removal of cationic impurities from glycomes glycomes containing neutral and/or negatively charged glycans. The invention is further directed to use both anion and cation exchange for removal of charged impurities from non-charged glycomes. The preferred ion exchange and cation exhange materials includes polystyrene resins such as Dowex resins.

The hydrophilic chromatography is preferably aimed for removal of hydrophobic materials such as lipids detergents and hydrophobic protein materials. The preferred hydrophobic chromatography materials includes.

It is realized that different combinations of the prepurification are usuful depending on the cell preparation and sample type. Preferred combinations of the prepurification steps include: Precipitation-extraction and Ion-exchange; Precipitation-extraction and Hydrophobic interaction; and Ion-exchange and Hydrophobic interaction. The two prepurification steps are preferably performed in the given order.

**Purification steps including binding and optionally fractionation of glycomes**

The purification steps utilize two major concepts for binding to carbohydrates and combinations thereof: a) Hydrophilic interactions and b) Ion exhange

a) Hydrophilic interactions

The present invention is specifically directed to use of matrices with repeating polar groups with affinity for carbohydrates for purification of glycome materials according to the invention in processes according ot the invention. The hydrophilic interaction material may include additional ion exchange properties.

The preferred hydrophilic interaction materials includes carbohydrate materials such as carbohydrate polymers in presence of non-polar organic solvents. A especially preferred hydrophilic interaction chromatography matrix is cellulose.

A specific hydrophilic interaction material includes graphitized carbon. The graphitized carbon separates noncharged carbohydrate materials based mainly on the size on the glycan. There is also possible ion exchange effects. In a preferred embodiment the invention is directed to graphitized carbon chromatography of prepurified samples after desalting and removal of detergents.

The invention is specifically directed to purification of non-derivatized glycomes and neutral glycomes by cellulose chromatography. The invention is further directed to purification of non-derivatized glycomes and neutral glycomes by graphitized carbon chromatography. In a preferred embodiment the purification according to the invention includes both cellulose and graphitized carbon chromatography.

b) Ion exchange

The glycome may comprise only negative charges or in more rare case also only positive charges. At purification stage the ion exchange material is selected to contain opposite charge than the glycome or glycome fraction for binding the glycome. The invention is especially directed to the use of anion exchange materials for binding of negatively charged Preferred ion exchange materials includes ion exchange and especially anion exhange materials includes polystyrene resins such as Dowex-resins, preferably quaternary amine resins anion exchange or sulfonic acid cation exchange resins

It was further revealed that even graphitized carbon can be used for binding of negatively charged glycomes and the materials can be eluted from the carbon separately from the neutral glycomes or glycome fractions according to the invention.

The invention is specifically directed to purification of anionic glycomes by anion exchange chromatography.

The invention is specifically directed to purification of anionic glycomes by anion exchange chromatography.

The invention is further directed to purification of anionic glycomes by cellulose chromatography. The preferred anionic glycomes comprise sialic acid and/or sulfo/fosfo esters, more preferably both sialic acid and sulfo/fosfo esters. A preferred class of sulfo/fosfoester glycomes are complex type N-glycans comprising sulfate esters.

Prepurification and purification steps in detail

*1) Precipitation-extraction* may include precipitation of glycans or precipitation of contaminants away from the glycans. Preferred precipitation methods include:

4. Glycan material precipitation, for example acetone precipitation of glycoproteins, oligosaccharides, glycopeptides, and glycans in aqueous acetone, preferentially ice-cold over 80 % (v/v) aqueous acetone; optionally combined with extraction of glycans from the precipitate, and/or extraction of contaminating materials from the precipitate;
5. Protein precipitation, for example by organic solvents or trichloroacetic acid, optionally combined with extraction of glycans from the precipitate, and/or extraction of contaminating materials from the precipitate;
6. Precipitation of contaminating materials, for example precipitation with trichloroacetic acid or organic solvents such as aqueous methanol, preferentially about 2/3 aqueous methanol for selective precipitation of proteins and other non-soluble materials while leaving glycans in solution;

2) *Ion-exchange* may include ion-exchange purification or enrichment of glycans or removal of contaminants away from the glycans. Preferred ion-exchange methods include:

3. Cation exchange, preferably for removal of contaminants such as salts, polypeptides, or other cationizable molecules from the glycans; and
4. Anion exchange, preferably either for enrichment of acidic glycans such as sialylated glycans or removal of charged contaminants from neutral glycans, and also preferably for separation of acidic and neutral glycans into different fractions.

3) *Hydrophilic interaction* may include purification or enrichment of glycans due to their hydrophilicity or specific adsorption to hydrophilic materials, or removal of contaminants such as salts away from the glycans. Preferred hydrophilic interaction methods include:

3. Hydrophilic interaction chromatography with specific organic or inorganic polar interaction materials, preferably for purification or enrichment of glycans and/or glycopeptides;
4. Preferably adsorption of glycans to carbohydrate materials, preferably to cellulose in hydrophobic solvents for their purification or enrichment, preferably to microcrystalline cellulose, and elution by polar solvents such as water and or alchol, which is preferably ethanol or methanol. The solvent system for absorption comprise preferably

i)a hydrophobic alcohol comprising about three to five carbon atoms, including propanols, butanols, and pentanols, more preferably being n-butanol;
ii) a hydrophilic alcohol such as methanol or ethanol, more preferably methanol, or a hydrophilic weak organic acid, preferably acetic acid and;
iii) water. The hydrophobic alcohol being the major constituent of the mixture with multifold exess to other components. The absorbtion composition is preferably using an n-butanol:methanol:water or similar solvent system for adsorption and washing the adsorbed glycans, in most preferred system n-butanol:methanol: water in relative volumes of 10:1:2.
The preferred polar solvents for elution of the glycomes are water or water:ethanol or similar solvent system for elution of purified glycans from cellulose. Fractionation is possible by using first less polar elution solvent to elute a fraction of glycome compositions and the eluting rest by more polar solvent such as water 3 *Affinity to carbon* may include purification or enrichment of glycans due to their affinity or specific adsorption to specific carbon materials preferably graphitized carbon, or removal of contaminants away from the glycans. Preferred graphitized carbon affinity methods includes porous graphitized carbon chromatography.

Preferred purification methods according to the invention include combinations of one or more prepurification

and/or purification steps. The preferred method include preferably at least two and more preferably at least three prepurification steps according to the invention. The preferred method include preferably at least one and more preferably at least two purification steps according to the invention. It is further realized that one prepurification step may be performed after a purification step or one purification step may be performed after a prepurification step. The method is preferably adjusted based on the amount of sample and impurities present in samples. Examples of the preferred combinations include the following combinations:

For neutral underivatized glycan purification:

A. 1. precipitation and/or extraction 2. cation exchange of contaminants, 3. hydrophobic adsorption of contaminants, and 4. hydrophilic purification, preferably by carbon, preferably graphitized carbon, and/or carbohydrate affinity purification of glycans.

B. 1. precipitation and/or extraction ,2. hydrophobic adsorption of contaminants 3. cation exchange of contaminants, 4. hydrophilic purification by carbon, preferably graphitized carbon, and/or carbohydrate affinity purification of glycans

The preferred method variants further includes preferred variants when

1. both carbon and carbohydrate chromatography is performed in step 4,
2. only carbon chromatography is performed in step 4
3. only carbohydrate chromatography is performed in step 4
4. order steps three and four is exchanged
5. both precipitation and extraction are performed in prepurification step 2) For sialylated/acidic underivatized glycan purification: The same methods are preferred but preferably both carbon and carbohydrate chromatography is performed in step 4. The carbohydrate affinity chromatography is especially preferred for acidic and/sialylated glycans. In a preferred embodiment for additional purification one or two last chromatograpy methods are repeated.

### *D. Analysis of the glycomes*

The present invention is specifically directed to detection various component in glycomes by specific methods for recognition of such components. The methods includes binding of the glycome components by specific binding agents according to the invention such as antibodies and/or enzymes, these methods peferebly include labeling or immobilization of the glycomes. For effective analysis of the glycome a large panel of the binding agents are needed.

The invention is specifically directed to physicochemical profiling methods for exact analysis of glycomes. The preferred methods includes mass spectrometry and NMR-spectroscopy, which give simultaneously information of numerous components of the glycomes. In a preferred embodiment the mass spectrometryand NMR-spectroscopy methods are used in a combination.

### *E. Quantitative and qualitative analysis of glycome data*

The invention revealed methods to create reproducible and quantitative profiles of glycomes over large mass ranges and degrees of polymerization of glycans. The invention further reveals novel methods for quantitative analysis of the glycomics data produced by mass spectrometry. The invention is specifically directed to the analysis of nonderivatized or reducing end derivatized glycomes according to the invention and the glycomes containing specific structureal characteristics according ot the invention.

The invention revealed effective means of comparision of glycome profiles from different cell types or tissue materials with difference in cell status or cell types. The invention is especially directed to the quantitative comparision of relative amount of individual glycan signal or groups of glycan signals described by the invention.

The invention is especially directed to

i)calculating average value and variance values of signal or signals, which have obtained from several experiments/samples and which correspond to an individual glycan or glycan group according to the invention for a first cell sample and for a second cell sample ii) comparing these with values derived for the corresponding signal(s)

iii) optionally calculating statistic value for testing the probability of similarity of difference of the values obtained for the cell types or estimating the similarity or difference based on the difference of the average and optionally also based on the variance values.

iv) preferably repeating the comparision one or more signals or signal groups, and further preferably performing combined statistical analysis to estimate the similarity and/or differences between the data set or estimating the difference or similarity

v) preferably use of the data for estimating the differences between the first and second cell samples indicationg

difference in cell status and/or cell type

The invention is further directed to combining information of several quantitative comparisions of between corresponding signals by method of

i)calculating differences between quantitative values of corresponding most different glycan signals or glycan group signals, changing negative values to corresponding positive values, optionally multiplying selected signals by selected factors to adjust the weight of the signals in the calculation
ii) adding the positive difference values to a sum value
iii) comparing the sum values as indicators of cell status or type.

It was further revealed that there is characteric signals that are present in certain cell types according to the invention but absent or practically absent in other cell types. The invention is therefore directed to the qualitative comparision of relative amount of individual glycan signal or groups of glycan signals described by the invention and observing signals present or absent/practically absent in a cell type. The invention is further directed to selection of a cut off value used for selecting absent or practically absent signals from a mass spectrometric data, for example the preferred cut off value may be selected in range of 0-3 % of relative amount, preferably the cut off value is less than 2 %, or less than 1% or less than 0.5 %. In a preferred embodiment the cut off value is adjusted or defined based on quality of the mass spectrum obtained, preferably based on the signal intensities and/or based on the number of signals observable.

The invention is furher directed to automized qualitative and/or quantitative comparisions of data from corresponding signals from different samples by computer and computer programs prosessing glycome data produced according to the invention. The invention is further directed to raw data based analysis and neural network based learning system analysis as methods for revealing differences between the glycome data according to the invention.

***Methods for low sample amounts***

The present invention is specifically directed to methods for analysis of low amounts of samples.

The invention further revealed that it is possible to use the methods according to the invention for analysis of low sample amounts. It is realized that the cell materials are scarce and difficult to obtain from natural sources. The effective analysis methods would spare important cell materials. Under certain circumstances such as in context of cell culture the materials may be available from large scale. The required sample scale depends on the relative abundancy of the characteristic components of glycome in comparision to total amount of carbohydrates. It is further realized that the amount of glycans to be measured depend on the size and glycan content of the cell type to be measured and analysis including multiple enzymatic digestions of the samples would likely require more material. The present invention revealed especially effective methods for free released glycans.

The picoscale samples comprise preferably at least about 1000 cells, more preferably at least about 50 000 cells, even more more preferably at least 100 000 cells, and most preferably at least about 500 000 cells. The invention is further directed to analysis of about 1 000 000 cells. The preferred picoscale samples contain from at least about 1000 cells to about 10 000 000 cells according to the invention. The useful range of amounts of cells is between 50 000 and 5 000 000, even more preferred range of of cells is between 100 000 and 3 000 000 cells. A preferred practical range for free oligosaccharide glycoomes is between about 500 000 and about 2 000 000 cells. It is realized that cell counting may have variation of less than 20 %, more preferably 10 % and most preferably 5 %, depending on cell counting methods and cell sample, these variations may be used instead of term about. It is further understood that the methods according to the present invention can be upscaled to much larger amounts of material and the pico/nanoscale analysis is a specific application of the technology.

The invention is specifically directed to use of microcolumn technologies according to the invention for the analysis of the preferred picoscale and low amount samples according to the invention,

The invention is specifically directed to purification to level, which would allow production of high quality mass spectrum covering the broad size range of glycans of glycome compositions according to the invention.

**Glycan preparation and purification for glycome analysis of cell materials according to the invention, especially for mass spectrometric methods**

**Use of microfluidistic methods including microcolumn chromatography**

The present invention is especially directed to use microfluidistic methods involving low sample volumes in handling of the glycomes in low volume cell preparation, low volume glycan release and various chromatographic steps. The invention is further directed to integrated cell preparation, glycan release, and purification and analysis steps to

reduce loss of material and material based contaminations. It is further realized that special cleaning of materials is required for optimal results.

**Low volume reaction in cell preparation and glycan release**

The invention is directed to reactions of volume of 1-100 microliters, preferably about 2-50 microliters and even more preferably 3-20 microliters, most preferably 4-10 microliter. The most preferred reaction volumes includes 5-8 microliters+/- 1 microliters. The minimum volumes are preferred to get optimally concentrated sample for purification. The amount of material depend on number of experiment in analysis and larger amounts may be produced preferably when multiple structural analysis experiments are needed.
It is realized that numerous low volume chromatographic technologies may be applied, such low volume column and for example disc based microfluidistic systems.
The inventors found that the most effective methods are microcolumns. Small colomn can be produced with desired volume. Preferred volumes of microcolumns are from about 2 Microliters to about 500 microliters, more preferably for rutine sample sizes from about 5 microliter to about 100 microliters depending on the matrix and size of the sample. Preferred microcolumn volumes for graphitised carbon, cellulose chromatography and other tip-columns are from 2 to 20 μl, more preferably from 3 to 15 μl, even more preferably from 4 to 10 μl, For the microcolumn technologies in general the samples are from about 10 000 to about million cells. The methods are useful for production of picomol amounts of total glycome mixtures from tissue materials according to the invention.
In a preferred embodiment microcolumns are produced in regular disposable usually plastic pipette tips used for example in regular "Finnpipette"-type air-piston pipettes. The pipette-tip microcolumn contain the preferred chromatographic matrix. In a preferred embodiment the microcolumn contains two chromatographic matrixes such as an anion and cation exchange matrix or a hydrophilic and hydrophobic chromatography matrix.
The pipette tips may be chosen to be a commercial tip contain a filter. In a preferred embodiment the microcolumn is produced by narrowing a thin tip from lower half so that the preferred matrix is retained in the tip. The narrowed tip is useful as the volume of filter can be omitted from washing steps
The invention is especially directed to plastic pipette tips containing the cellulose matrix, and in an other embodiment to the pipette tip microclumns when the matrix is graphitised carbon matrix. The invention is further directed to the preferred tip columns when the columns are narorrowed tips, more preferably with column volumes of 1 microliter to 100 microliters.
The invention is further directed to the use of the tip columns containing any of the preferred chromatographic matrixes according to the invention for the purification of glycomes according to the invention, more preferably matrixes for ion exchange, especially polystyrene anion exchangers and cation exchangers according to the invention; hydrophilic chromatographic matrixes according to the invention, especially carbohydrate matrixes and most cellulose matrixes.

***The binding methods for recognition of structures from cell surfaces***

***Recognition of structures from glycome materials and on cell surfaces by binding methods***

The present invention revealed that beside the physicochemical analysis by NMR and/or mass spectrometry several methods are useful for the analysis of the structures. The invention is especially directed to two methods:

i) Recognition by enzymes involvingbinding and alteration of structures.
This method alters specific glycan structures by enzymes cabable of altering the glycan structures. The preferred enzymes includes

a) glycosidase-type enzymes capable of releasing monosaccharide units from glycans
b) glycosyltransferring enzymes, including transglycosylating enzymes and glycosyltransferases
c) glycan modifying enzymes including sulfate and or fosfate modifying enzymes

ii) Recognition by molecules binding glycans referred as the binders These molecules bind glycans and include property allowing observation of the binding such as a label linked to the binder. The preferred binders include

a) Proteins such as antibodies, lectins and enzymes
b) Peptides such as binding domains and sites of proteins, and synthetic library derived analogs such as phage display peptides
c) Other polymers or organic scaffold molecules mimicking the peptide materials

The peptides and proteins are preferably recombinant proteins or corresponding carbohydrate recognition domains derived therereof, when the proteins are selected from the group monoclonal antibody, glycosidase, glycosyl transferring enzyme, plant lectin, animal lectin or a peptide mimetic thereof, and wherein the binder includes a detectable label structure..

## Preferred binder molecules

The present invention revealed various types of binder molecules useful for characterization of tissue materials according to the invention and more specifically the preferred cell groups and cell types according to the invention. The preferred binder molecules are classified based on the binding specificity with regard to specific structures or structural features on carbohydrates of cell surface. The preferred binders recognize specifically more than single monosaccharide residue.

It is realized that most of the current binder molecules such as all or most of the plant lectins are not optimal in their specificity and usually recognize roughly one or several monosaccharides with various linkages. Furthermore the specificities of the lectins are usually not well characterized with several glycans of human types.

The preferred high specificity binders recognize

A) at least one monosaccharide residue and a specific bond structure between those to another monosaccharides next monosaccharide residue referred as MS 1B 1-binder,
B) more preferably recognizing at least part of the second monosaccharide residue referred as MS2B1-binder,
C) even more preferably recognizing second bond structure and or at least part of third mono saccharide residue, referred as MS3B2-binder, preferably the MS3B2 recognizes a specific complete trisaccharide structure.
D) most preferably the binding structure recognizes at least partially a tetrasaccharide with three bond structures, referred as MS4B3-binder, preferably the binder recognizes complete tetrasaccharide sequences.

The preferred binders includes natural human and or animal, or other proteins developed for specific recognition of glycans. The preferred high specificity binder proteins are specific antibodies preferably monoclonal antibodies; lectins, preferably mammalian or animal lectins; or specific glycosyltransferring enzymes more preferably glycosidase type enzymes, glycosyltransferases or transglycosylating enzymes.

## Target structures for specific binders and examples of the binding molecules

### Combination of terminal structures in combination with specific glycan core structures

It is realized that part of the structural elements are specifically associated with specific glycan core structure. The recognition of terminal structures linked to specific core structures are especially preferred, such high specificity reagents have capacity of recognition almost complete individual glycans to the level of physicochemical characterization according to the invention. For example many specific mannose structures according to the invention are in general quite characteristic for N-glycan glycomes according to the invention. The present invention is especially directed to recognition terminal epitopes.

### Common terminal structures on several glycan core structures

The present invention revealed that there are certain common structural features on several glycan types and that it is possible to recognize certain common epitopes on different glycan structures by specific reagents when specificity of the reagent is limited to the terminal without specificity for the core structure. The invention especially revealed characteristic terminal features for specific cell types according to the invention. The invention realized that the common epitopes increase the effect of the recognition. The common terminal structures are especially useful for recognition in the context with possible other cell types or material, which do not contain the common terminal structure in substancial amount.

### Specific preferred structural groups

The present invention is directed to recognition of oligosaccharide sequences comprising specific terminal monosaccharide types, optionally further including a specific core structure. The preferred oligosaccharide sequences classified based on the terminal monosaccharide structures.

**Structures with terminal Mannose monosaccharide**

Preferred mannose-type target structures have been specifically classified by the invention. These include various types of high and low-mannose structures and hybrid type structures according to the invention.
**Low or uncharacterised specificity binders**
preferred for recognition of terminal mannose structures includes mannose-monosaccharide binding plant lectins.
*Preferred high specific high specificity binders* include

i) Specific mannose residue releasing enzymes such as linkage specific mannosidases, more preferably an α-mannosidase or β-mannosidase.
Preferred α-mannosidases includes linkage specific α-mannosidases such as α-Mannosidases cleaving preferably non-reducing end terminal
α2-linked mannose residues specifically or more effectively than other linkages, more preferably cleaving specifically Manα2-structures; or
α6-linked mannose residues specifically or more effectively than other linkages, more preferably cleaving specifically Manα6-structures;
Preferred β-mannosidases includes β-mannosidases capable of cleaving β4-linked mannose from non-reducing end terminal ofN-glycan core Manβ4GlcNAc-structure without cleaving other β-linked monosaccharides in the glycomes.
ii) Specific binding proteins recognizing preferred mannose structures according to the invention. The preferred reagents include antibodies and binding domains of antibodies (Fab-fragments and like), and other engineered carbohydrate binding proteins. The invention is directed to antibodies recognizing MS2B1 and more preferably MS3B2-structures

**Binder-label conjugates**

The present invention is specifically directed to the binding of the structures according to the present invention, when the binder is conjugated with "a label structure". The label structure means a molecule observable in a assay such as for example a fluorescent molecule, a radioactive molecule, a detectable enzyme such as horse radish peroxidase or biotin/streptavidin/avidin. When the labelled binding molecule is contacted with the cells or tissue materials according to the invention, the cells can be monitored, observed and/or sorted based on the presence of the label on the cell surface. Monitoring and observation may occur by regular methods for observing labels such as fluorescence measuring devices, microscopes, scintillation counters and other devices for measuring radioactivity.

Use of binder and labelled binder-conjugates for cell sorting

The invention is specifically directed to use of the binders and their labelled cojugates for sorting or selecting cells from biological materials or samples including cell materials comprising other cell types. The preferred cell types includes cultivated cells and associated cells such as feeder cells. The labels can be used for sorting cell types according to invention from other similar cells. In another embodiment the cells are sorted from different cell types such as blood cells or in context of cultured cells preferably feeder cells, for example in context of complex cell cultures corresponding feeder cells such as human or mouse feeder cells. A preferred cell sorting method is FACS sorting. Another sorting methods utilized immobilized binder structures and removal of unbound cells for separation of bound and unbound cells.

**Use of immobilized binder structures**

In a preferred embodiment the binder structure is conjugated to a solid phase. The cells are contacted with the solid phase, and part of the material is bound to surface. This method may be used to separation of cells and analysis of cell surface structures, or study cell biological changes of cells due to immobilization. In the analytics involving method the cells are preferably tagged with or labelled with a reagent for the detection of the cells bound to the solid phase through a binder structure on the solid phase. The methods preferably further include one or more steps of washing to remove unbound cells.
Preferred solid phases include cell suitable plastic materials used in contacting cells such as cell cultivation bottles, petri dishes and microtiter wells; fermentor surface materials

*mRNA corresponding to glycosylation enzymes*

The present invention is further directed to correlation of specific messenger mRNA molecules with the preferred

glycan structures according to the present invention. It is realized that glycosylation can be controlled in multiple levels and one of them is transcription. The presence of glycosylated structures may in some case correlate with mRNAs involved in the synthesis of the structures.

The present invention is especially directed to analysis of mRNA-species having correlation with expressed fucosylated glycan structures and "terminal HexNAc" containing structures preferred according to the present invention. The preferred mRNA-species includes mRNA corresponding to fucosyltransferases and N-acetylglucosaminyltransferases.

NMR-analysis of glycomes

The present invention is directed to analysis of released glycomes by spectrometric method useful for characterization of the glycomes from tissue specimens or cells. The invention is directed to NMR spectroscopic analysis of the mixtures of released glycans.

The invention is especially directed to methods of producing NMR from specific subglycomes, preferably N-linked glycome. The NMR-profiling according to the invention is further directed to the analysis of the novel and rare structure groups revealed from cell glycomes according to the invention. The general information about complex cell glycome material directed NMR-methods are limited.

Preferably the NMR-analysis is performed from an isolated subglycome. The preferred isolated subglycomes include acidic glycomes and neutral glycomes.

*NMR-glycome analysis of larger tissue specimens or larger amounts of cells*

It is realized that numerous methods have been desribed for purification of oligosaccharide mixtures useful for NMR from various materials, including usually purified individual proteins. It is realized that present methods are useful for NMR-profiling even for larger tissue specimens or higher amounts of cells according to the invention, especially in combination with NMR-profiling according to the invention and/or when directed to the analysis specific and preferred structure groups according to the invention. The preferred purification methods are effective and form an optimised process for purification of glycomes from even larger amounts of cells and tissues than described for nanoscale methods below. The methods are preferred also for any larger amount of cells.

*Purification method for low amount nanoscale NMR-profiling of samples*

Moreover, when purification methods for larger amounts of carbohydrate materials exists, but very low and complex carbohydrate materials with very complex impurities such as cell-derived materials have been less studied as low amounts, especially when purity useful for NMR-analysis is needed.

*Preferred sample amounts allowing effective NMR analysis of cell glycomes*

The invention is directed to analysis of NMR-samples that can be produced from very low amounts of cells according to the invention. Preferred sample amounts of cells or corresponding amount of tissue material for a one-dimensional proton-NMR profiling are from about 2 million to 100 million cells, more preferably 10-50 million cells. It is further realized that good quality NMR data can be obtained from samples containing at least about 10-20 million cells.

The preferred analysis methods is directed to high resolution NMR observing oligosaccharide/saccharide conjugate mixture from an amount of at least 4 nmol, more preferably at least 1 nmol and the cell amount yielding the preferred amount of saccharide mixture. For nanoscale analysis according to the invention cell material is selected so that it would yield at least about 50 nmol of oligosaccharide mixture, more preferably at least about 5 nmol and most preferably at least about 1 nmol of oligosaccharide mixture. Preferred amounts of major components in glycomes to be observed effectively by the methods according to the invention include yield at least about 10 nmol of oligosaccharide component, more preferably at least about 1 nmol and most preferably at least about 0.2 nmol of oligosaccharide component.

The preferred cell amount for analysis of a subglycome from a cell type is preferably optimised by measuring the amounts of glycans produced from the cell amounts of preferred ranges.

It is realized that depending on the cell and subglycome type the required yield of glycans and the heterogeneity of the materials vary yielding different amounts of major components.

**Preferred purification methods**

For the production of sample for nanoscale NMR, the methods described for preparation of cell samples and

release of oligosaccharides for mass spectrometric profiling according to the invention may be applied.

For the purification of sample for nanoscale NMR the methods described for purification mass spectrometry profiling samples according to the invention may be applied.

The preferred purification method for nanoscale NMR- profiling according to the invention include following general purification process steps:

a. Precipitation/extraction;
b. Hydrophobic interaction;
c. Affinity to carbon material, especially graphitized carbon.
d. Gel filtration chromatography

The more preferred purification process includes precipitation/extraction aimed for removal of major non-carbohydrate impurities by separating the impurity and the glycome fraction(s) to be purified to different phases. Hydrophobic interaction step aims to purify the glycome components from more hydrophobic impurities as these are bound to hydrophobic chromatography matrix and the glycome components are not retained. Chromatography on graphitized carbon may include purification or enrichment of glycans due to their Affinity or specific adsorption to graphitized carbon, or removal of contaminants from the glycans. The glycome components obtained by the aforementioned steps are then subjected to gel filtration chromatography, separating molecules according to their hydrodynamic volume, i.e. size in solution. The gel filtration chromatography step allows detection and quantitation of glycome components by absorption at low wavelenghts (205-214 nm).

The most preferred purification process includes precipitation/extraction and hydrophobic interaction steps aimed for removal of major non-carbohydrate impurities and more hydrophobic impurities. Chromatography on graphitized carbon is used for removal of contaminants from the glycans, and to devide the glycome components to fractions of neutral glycome components and acidic glycome components. The neutral and acidic glycome component fractions are then subjected to gel filtration chromatography, which separates molecules according to their size. Preferably, a high-performance liquid chromatography

(HPLC) type gel filtration column is used. The neutral glycome component fraction is preferebly chromatographed in water and the acidic glycome component fraction is chromatographed in 50-200 mM aqueous ammonium bicarbonate solution. Fractions are collected and evaporated prior to further analyses. The gel filtration chromatography step allows detection and quantitation of glycome components by absorption at low wavelenghts (205-214 nm). Quantitation is performed against external standards. The standards are preferably N-acetylglucosamine, N-acetylneuraminic acid, or oligosaccharides containing the same. Fractions showing absorbance are subjected to MALDI-TOF mass spectrometry. Preferably, the neutral glycome components are analyzed in the positive-ion mode and the acidic glycome components in the negative-ion mode in a delayed-extraction MALDI-TOF mass spectrometer.

## Preferred methods for producing enriched glycome fractions for NMR

The fractionation can be used to enrich components of low abundance. It is realized that enrichment would enhance the detection of rare components. The fractionation methods may be used for larger amounts of cell material. In a preferred embodiment the glycome is fractionated based on the molecular weight, charge or binding to carbohydrate binding agents such as lectins and/or other binding agents according to the invention.

These methods have been found useful for specific analysis of specific subglycomes and enrichment more rare components. The present invention is in a preferred embodiment directed to charge based separation of neutral and acidic glycans. This method gives for analysis method, preferably mass spectroscopy material of reduced complexity and it is useful for analysis as neutral molecules in positive mode mass spectrometry and negative mode mass spectrometry for acidic glycans.

It is realized that preferred amounts of enriched glycome oligosacccharide mixtures and major component comprising fractions can be produced from larger glycome preparations.

In a preferred embodiment the invention is directed to size based fractionation methods for effective analysis of preferred classes of glycans in glycomes. The invention is especially directed to analysis of lower abundance components with lower and higher molecular weight than the glycomes according to the invention. The preferred method for size based fractionation is gel filtration. The invention is especially directed to analysis of enriched group glycans of N-linked glycomes preferably including lower molecular weight fraction including low-mannose glycans, and one or several preferred low mannose glycan groups according to the invention.

## Preferred NMR-methods

In a preferred embodiment the NMR-analysis of the glycome is one-dimensional proton-NMR analysis showing

structural reporter groups of the major components in the glycome. The invention is further directed to specific two- and multidimensional NMR-experiments of the glycomes when enough sample is available. It is realized that two-dimensional NMR-experiments require about a ten-fold increase in sample amount compared to proton-NMR analyses.

**Combination of NMR- and mass spectrometry for glycome analysis**

The present invention is further directed to combination of the mass spectrometric and NMR glycome analyses. The preferred method include production of any mass spectrometric profile from any glycome according to the invention from a cell sample according to the invention, optionally characterizing the glycome by other methods like glycosidase digestion, fragmentation mass spectrometry, specific binding agents, and production of NMR-profile from the same sample glycome or glycomes to compare these profiles.

***Recombinant proteins from lower eukayots***

It is furher realized that multiple recombinant protein host cell systems such as yeast and fungal cells can be engineered for production specific low-Mannnose and/or high-Mannose structres (described e.g. in glycoprotein production patents of Kirin Bier Japan, Glycofi US and prof Roland Contreras Belgium).

The present invention is directed to the methods of clinical evaluation of effect of immunization by glycoproteins/ peptides enriched with the preferred cancer antigens according to the invention for patients with one or several types of cancers containing the structures.

**Quantitative synthesis and analysis method for oxidation and reduction of a protein linked glycan**

The present invention revealed a novel quantitative synthesis and analysis method for quantitative periodate (/vicinal hydroxyl) oxidation and reduction of a protein linked glycan. The preferred glycans to be oxidized and analyzed are terminal-Man glycans according to the invention, more preferably lower high-man and/or low-Man N-glycans according to the invention, more preferably low-Man glycans, including fucosylated and/or non-fucosylated glycans, which were revealed to be processable/analysable according to the invention, in preferred embodiment the glycans are non-fucosylated.. It is realized that the glycan structures have effect on the oxidation potential. The invention further revealed a quantitative analysis of reaction products of the oxidation and reduction method by MALDI-TOF mass spectrometry, preferably in positive ion mode according to the example. The mass spectra revealed that novel highly oxidized and reduced molecules ionize at least semiquantitatively similarily from oligosaccharides with various degrees of oligomerization, and the signals correlated to the quantitative amounts of corresponding raw material oligosaccharides with different lengths. The example further includes useful purification method for the glycans preserving the quantities of the samples. Before the invention there was not no quantitative, effective and quick method for this analysis of such reaction especially from low sample amounts such as lower picomol level of samples. It is realized that the method is useful for effective modification and characterization of all types glycans, especially terminal-Man glycans.

<u>Preferred cancer types According to the present invention</u>

As described in the Examples, the inventors detected the presence and/or altered expression levels of the glycans widely in human cancerous tissues, e.g. in following cancer types: lung cancer, both small cell lung adenocarcinoma and non-small cell lung adenocarcinoma, and lung carcinoma liver metastases; breast cancer; ductale type breast adenocarcinoma and lymph node metastases thereof; lobulare type breast adenocarcinoma and lymph node metastases thereof; ovarian cystadenocarcinoma; colon cancer / carcinoma, carcinoma adenomatosum, and liver metastases thereof; kidney cancer / carcinoma, and kidney hypernephroma; gastric cancer / carcinoma, and lymph node metastases thereof, liver cancer /carcinoma; larynx cancer / carcinoma; pancreas cancer / carcinoma; melanoma and liver metastases thereof; gall bladder cancer / carcinoma, and liver metastases thereof; salivary gland cancer / carcinoma, and skin metastases thereof; and lymph node cancer / carcinoma (lymphoma). The present invention is especially directed to uses of identified glycan structures according to the present invention in these preferred cancer types.

**Uses of metastasis-associated glycans**

The present invention is specifically directed to methods studying metastasis-associated, metastasis-specific, metastasis-enriched, and/or metastasis-inducing glycans according to the invention. As described in Examples 17-20, the inventors identified metastasis-associated and metastasis-enriched glycans in various human cancer types, including mannosylated glycans, preferentially low-mannose type glycans.

The present invention is especially directed to methods for identifying metastasis-associated glycans according to the invention. The present invention is further directed to methods for identifying primary cancer type and/or site of metastasis based on identifying the glycans according to the invention. In a further embodiment, the present invention

is directed to methods for studying metastasis growth and/or initiation based on identifying the glycans according to the invention.

It is further realized that cancer metastasis is directly related to cancer malignancy. The present invention is specifically directed to methods for analyzing cancer malignancy based on use of the metastasis-associated glycans according to the present invention.

The present invention is further illustrated in Examples, which in no way are intended to limit the scope of the invention.

## EXAMPLE 1. Structure analysis of glycans that are expressed in various human cancer types.

## EXPERIMENTAL PROCEDURES

*Isolation of glycans from formalin-fixed and paraffin-embedded tissue samples.* Prior to glycan isolation from formalin-fixed and paraffin-embedded samples, the samples were deparaffinised. Glycans were detached from sample glycoproteins by non-reductive β-elimination essentially as described previously (Huang *et al.*, 2001) and purified and analyzed essentially as described in **Examples 11 and 12**.

*MALDI-TOF MS.* MALDI-TOF mass spectrometry was performed with a Voyager-DE STR BioSpectrometry Workstation, essentially as described previously (Saarinen *et al.*, 1999; Harvey *et al.*, 1993).

### Neutral low-mannose type N-glycans

*Exoglycosidase digestions.* All exoglycosidase reactions were performed essentially as described previously (Nyman *et al.*, 1998; Saarinen *et al.*, 1999) and analysed by MALDI-TOF MS. The enzymes and their specific control reactions with characterised oligosaccharides were (R denotes reducing end oligosaccharide sequences in the following examples): β1,4-galactosidase (*Streptococcus pneumoniae*, recombinant, *E. coli*; Calbiochem, USA) digested Galβ1-4GlcNAc-R but not Galβ1-3GlcNAc-R; β-N-acetylglucosaminidase (*Streptococcus pneumoniae*, recombinant, *E. coli*; Calbiochem, USA) digested GlcNAcβ1-6Gal-R in β1,4-galactosidase treated lacto-N-hexaose but not GalNAcβ1-4GlcNAcβ1-3/6Gal-R in a synthetic oligosaccharide; α-mannosidase (Jack bean; Glyko, UK) transformed a mixture of high-mannose N-glycans to the $Man_1GlcNAc_2$ N-glycan core trisaccharide; β-mannosidase (*Helix pomatia*; Calbiochem, USA) digested the β1,4-linked mannose residue from the N-glycan core trisaccharide Manβ4GlcNAcβ4GlcNAc, without affecting the α-linked mannose residues of high-mannose N-glycans. Control digestions were performed in parallel and analysed similarly to the analytical exoglycosidase reactions. Endoglycosidase digestions were performed essentially as described previously (Plummer & Tarentino, 1991), and the reaction products were analyzed by MALDI-TOF MS after purification. The specific N-glycosidase F1 control oligosaccharides and the control reactions were as follows: the enzyme transformed all $Hex_{5-9}HexNAc_2$ oligosaccharides in a sample of high-mannose N-glycans into $He_{5-9}HexNAc_1$ oligosaccharides; in contrast, the enzyme was not able to digest a core-fucosylated N-glycan, namely the hexasaccharide Manα6(Manα3)Manβ4GlcNAcβ4(Fucα6)GlcNAc.

## RESULTS

*Glycan isolation and analysis.* Detached and purified glycans from paraffin-embedded formalin-fixed tissue samples from cancer patients were analysed by MALDI-TOF mass spectrometry after isolation of the neutral glycan fraction. Relative quantification of the glycans were done by comparing relative MALDI-TOF MS signal intensities, which is accurate for the obtained mixtures of purified glycans (Saarinen *et al.*, 1999; Harvey *et al.*, 1993).

*Specific mannosidase digestion analyses.* The proportions of the non-reducing terminal α-mannose containing glycans were determined by their sensitivity towards hydrolysis with α-mannosidase from Jack beans. After the specific exoglycosidase digestion, the presence of high-mannose and low-mannose type glycans in the original sample can be deduced by their disappearance from the recorded mass spectra, and the simultaneous increase in signal intensities of the expected reaction products at m/z 609 and 755, which correspond to the sodium adduct ions $[Hex_1HexNAc_2+Na]^+$ (calc. m/z 609.21) and $[Hex_1HexNAc_2dHex_1+Na]^+$ (calc. m/z 755.27), respectively. An example of the reaction scheme is presented in Figure 1. The results are summarized in Table 1. According to the digestion results, the majority of the detectable signals in the original samples with proposed compositions $Hex_{4-9}HexNAc_2$ and $Hex_{3-5}HexNAc_2dHex_1$, correspond to glycans that are sensitive to α-mannosidase and contain non-reducing terminal α-mannose residues, whereas signals with proposed compositions of $Hex_{2-3}HexNAc_2$ and $Hex_2HexNAc_2dHex_1$ most likely partially correspond to other glycan types, although they also contain variable amounts of non-reducing terminal α-mannose residues.

The $Hex_1HexNAc_2dHex_{0-1}$ components were studied with specific β-mannosidase digestion both before and after α-mannosidase digestion. The results are summarized in Table 1. The results indicate that 1) the original components contain variable amounts of non-reducing terminal β-mannose containing oligosaccharides with the compositions

$Hex_1HexNAc_2dHex_{0-1}$, and 2) the major α-mannosidase digestion products with the compositions $Hex_1HexNAc_2dHex_{0-1}$ have non-reducing terminal β-mannose residues, as they are susceptible towards digestion with β-mannosidase enzyme.

*N-glycosidase digestion analyses.* The assignment of the majority of the $Hex_{1-9}HexNAc_2$ and $Hex_{1-5}HexNAc_2dHex_1$ glycan components as low-mannose and high-mannose type N-glycans was confirmed by their isolation and digestion analysis by specific endoglycosidase enzymes, namely N-glycosidase F and N-glycosidase F1 from *Chryseobacterium meningosepticum*. The first series of experiments was done with glycan samples isolated from a lung tumor of a patient with non-small cell lung adenocarcinoma. In addition to chemical detachment, the glycans in question could also be isolated by N-glycosidase F digestion, indicating that they are N-glycans. However, all $Hex_{1-9}HexNAc_2$ components, but not any of the $Hex_{1-5}HexNAc_2dHex_1$ components, could be digested with N-glycosidase F1, resulting in transformation of the first glycan group into peaks with masses of one less HexNAc residue with monosaccharide compositions $Hex_{1-9}HexNAc_1$. In combination with the α- and β-mannosidase digestion results, these experiments indicate that all the components are N-glycans that have the chitobiose disaccharide sequence in their reducing end, and that the latter components have a dHex residue linked to the reducing terminal GlcNAc. Furthermore, as the latter components are susceptible to digestion with N-glycosidase F, they must have a reducing terminal sequence dHex-6(GlcNAcβ-4)GlcNAc.

*Chemical analyses.* In individual samples, the $Hex_{1-9}HexNAc_2$ and $Hex_{1-5}HexNAc_2dHex_1$ components were also studied with periodate oxidation, subsequent reduction with alkaline sodium borohydride, and MALDI-TOF mass spectrometry. Also post-source decay (PSD) MALDI-TOF mass spectrometric analyses were performed to specific components of the structure group. The results from these analyses support the structural features deduced from the experiments described above. Periodate reaction cleaves structures with vicinal hydroxyl groups including non-reducing terminal monosaccharides, and isomeric structures differ from each other in this reaction. The data is in accordance with low-mannose and high-mannose type N-glycans produced by regular biosynthesis.

More specifically, cancer cell N-glycans with the compositions $Hex_2HexNAc_2$ and $Hex_2HexNAc_2dHex_1$, were studied with periodate oxidation, subsequent borohydride reduction, and MALDI-TOF MS. From the $Hex_2HexNAc_2$ component at m/z 771.59 (calc. m/z 771.26 for the ion $[Hex_2HexNAc_2+Na]^+$), two product ions were observed after the reaction at m/z 717.34 (calc. m/z 717.29 for the ion $[Hex_2HexNAc_2-C_2O_2+H_2+Na]^+$) and 745.34 (calc. m/z 745.29 for the ion $[Hex_2HexNAc_2-C_1O_1+H_2+Na]^+$), which can result from Manα6Manβ4GlcNAcβ4GlcNAc and Manα3Manβ4GlcNAcβ4GlcNAc N-glycan oligosaccharide isomers, respectively. These products occurred in respective relative amounts of approximately 60 % and 40 %, indicating that the original sample contained nearly equal amounts of the both α-mannose linkage isomers. From the $Hex_2HexNAc_2dHex_1$ component at m/z 917.63 (calc. m/z 917.32 for the ion $[Hex_2HexNAc_2dHex_1+Na]^+$), two product ions were observed after the reaction at m/z 835.41 (calc. m/z 835.35 for the ion $[Hex_2HexNAc_2dHex_1-C_3O_3+H_2+Na]^+$) and 863.42 (calc. m/z 863.35 for the ion $[Hex_2HexNAc_2dHex_1-C_2O_2+H_2+Na]^+$), which can result from Manα6Manβ4GlcNAcβ4(Fucα6)GlcNAc and Manα3Manβ4GlcNAcβ4(Fucα6)GlcNAc N-glycan oligosaccharide isomers, respectively. These components occurred in relative amounts of approximately 80% and 20%, respectively, indicating that the original sample contained significantly more of the isomer containing α6-linked mannose, but that the both isomers were present in the sample.

Taken together, all the experiments suggest that the terminal mannose-containing oligosaccharides, which have monosaccharide compositions $Hex_{1-9}HexNAc_2$ and $Hex_{1-5}HexNAc_2dHex_1$, include the structures presented in Figure 2.

## EXAMPLE 2. Expression of glycans in tissue samples of various cancer patients.

### EXPERIMENTAL PROCEDURES

*Statistical calculations.* Statistical analyses were performed with the SAS Software (SAS System, version 8.2, SAS Institute Inc., Cary, NC, USA), using SAS/STAT and SASBASE modules. All tests were performed as two-sided. The distributions of the experimental data were evaluated as 1) normal and symmetric, 2) only symmetric, or 3) non-symmetric and not normal, and the statistical test used was accordingly chosen as 1) Student's *t* Test, 2) Wilcoxon Signed Rank Test, or 3) Sign Test. A p value of less than 0.05 was considered statistically significant.

### RESULTS

#### Neutral low-mannose type N-glycans

*Neutral low-mannose type N-glycans are more abundant in tumor tissue samples than in healthy control tissue samples from cancer patients.* Formalin-fixed samples, from tumor and surrounding healthy tissue, were obtained from patients with various types of cancer. The studied cancer types included non-small cell lung adenocarcinoma, ductale breast carcinoma, lobulare breast carcinoma, stomach cancer, colon cancer, kidney cancer, ovarian carcinoma, pancreatic cancer, and cancers of the lymph nodes and the larynx. There were significant differences between the neutral low-mannose type N-glycans isolated from tumor samples and healthy tissue samples (Table 1), more specifically the $Hex_{2-4}HexNAc_2$ and $Hex_{2-5}HexNAc_2dHex_1$ neutral glycans, as described above. Neutral low-mannose type N-glycans were shown to be expressed in statistically significant manner in lung cancer and in two types of breast cancer (Table 2). In the examples below, it must be taken into account that at least m/z 609, 755, 771, and 917, glycan peaks may contain multiple oligosaccharide structures.

**EXAMPLE 3. Expression of glycans in lung cancer patients.**

**Neutral low-mannose type N-glycans**

In a group of lung cancer patients, more specifically non-small cell lung adenocarcinoma, the glycan peaks at m/z 609, 755, 771, 917, 1079, 1095, 1241, and/or 1403 were expressed in significantly elevated amounts in the tissue samples from the tumor (Table 1), when compared to healthy control tissues from the same patients. These glycan peaks correspond to $Hex_1HexNAc_2$, $Hex_1HexNAc_2dHex_1$, $Hex_2HexNAc_2$, $Hex_2HexNAc_2dHex_1$, $Hex_3HexNAc_2dHex_1$, $Hex_4HexNAc_2$, $Hex_4HexNAc_2dHex_1$, and $Hex_5HexNAc_2dHex_1$ glycan epitopes, respectively. An example pair of mass spectra from a lung cancer patient is presented in Figure 3.

**EXAMPLE 4. Expression of glycans in ductale breast cancer patients.**

**Neutral low-mannose type N-glycans**

In a group of breast cancer patients, more specifically ductale breast carcinoma, the glycan peaks at m/z 609, 771, 917, 933, 1079, 1095, 1241, and/or 1403 were expressed in significantly elevated amounts in the tissue samples from the tumor (Table 1), when compared to healthy control tissues from the same patients. These glycan peaks correspond to $Hex_1HexNAc_2$, $Hex_2HexNAc_2$, $Hex_2HexNAc_2dHex_1$, $Hex_3HexNAc_2$, $Hex_3HexNAc_2dHex_1$, $Hex_4HexNAc_2$, $Hex_4HexNAc_2dHex_1$, and $Hex_5HexNAc_2dHex_1$ glycan epitopes, respectively. This difference was shown to be statistically significant (Table 2). An example pair of mass spectra from a ductale breast cancer patient is presented in Figure 4.

**EXAMPLE 5. Expression of glycans in lobulare type breast cancer patients.**

**Neutral low-mannose type N-glycans**

In a group of breast cancer patients, more specifically lobulare breast carcinoma, the glycan peaks at m/z 609, 755, 771, 917, 933, 1079, 1095, 1241, and/or and 1403 were expressed in significantly elevated amounts in the tissue samples from the tumor (Table 1), when compared to healthy control tissues from the same patients. These glycan peaks correspond to $Hex_1HexNAc_2$, $Hex_1HexNAc_2dHex_1$, $Hex_2HexNAc_2$, $Hex_2HexNAc_2dHex_1$, $Hex_3HexNAc_2$, $Hex_3HexNAc_2dHex_1$, $Hex_4HexNAc_2$, $Hex_4HexNAc_2dHex_1$, and $Hex_5HexNAc_2dHex_1$ glycan epitopes, respectively. An example pair of mass spectra from a lobulare breast cancer patient is presented in Figure 5.

**EXAMPLE 6. Expression of glycans in individual patient samples of various cancer types.**

**Neutral low-mannose type N-glycans**

In addition to the statistically studied larger patient populations in lung and breast cancers, neutral low-mannose type N-glycans were expressed in many cancer types, when compared to healthy control tissues from the same patients. These results are summarized in Table 1.

**EXAMPLE 7. Detection of lung tissue and lung tumor-specific glycan structures.**

**EXPERIMENTAL PROCEDURES**

*Tissue samples and glycan isolation.* Archival paraffin-embedded and formalin-fixed tissue samples were from patients with non-small cell adenocarcinoma. After deparaffinisation, protein-linked glycans were detached from tissue sections with non-reductive alkaline β-elimination in concentrated ammonia-ammonium carbonate essentially as described previously (Huang et al., 2001). The isolated glycans were purified and divided into sialylated and non-sialylated glycan fractions as described in the other Examples of the present invention.

*MALDI-TOF mass spectrometry.* MALDI-TOF mass spectrometry was performed with a Voyager-DE STR

BioSpectrometry Workstation, essentially as described previously (Saarinen *et al.*, 1999; Harvey *et al.*, 1993). Relative molar abundancies of both neutral (Naven & Harvey, 1996) and sialylated (Papac *et al.*, 1996) glycan components were assigned based on their relative signal intensities.

## RESULTS AND DISCUSSION

*Occurrence of multiple cancer-associated protein-linked glycans in tissue glycan profiles.*

**Figure 6** shows the neutral glycan profiles averaged from multiple lung cancer samples. In the healthy tissue profiles, signals corresponding to neutral O-glycans (m/z 771, 917), sialylated O-glycans (m/z 899), and several signals among the low-mannose N-glycans (m/z 917, 1079, 1095, 1241, and 1403), are indicated as cancer-associated, as discussed in the preceding Examples. Also a profile change in the relative proportions of the signals at m/z 1485, 1647, and 1809 is visible: in the cancer samples the relative glycan abundancies are in the order 1485 > 1647 > 1809, whereas in the healthy samples the signals are approximately of the same abundance. The signals at m/z 1485 and 1647, corresponding to $Hex_3HexNAc_4dHex_1$ and $Hex_4HexNAc_4dHex_1$, contain non-reducing terminal GlcNAcβ residues, as evidenced by *S. pneumoniae* β-glucosaminidase digestion. It is concluded that terminal GlcNAcβ residues are associated with lung cancer and also with the three other cancer-associated glycan groups discussed above. Furthermore, the present method can detect these and other potential cancer-associated glycosylation changes simultaneously, allowing for multiparameter cancer diagnostics.

## EXAMPLE 8. Detection of ovarian tissue and ovarian tumor-specific glycan structures.

## EXPERIMENTAL PROCEDURES

*Tissue samples and glycan isolation.* Archival paraffin-embedded and formalin-fixed tissue samples were from patients with malignant ovarian cystadenocarcinoma or benign ovarian cystadenoma. After deparaffinisation, protein-linked glycans were detached from tissue sections with non-reductive alkaline β-elimination in concentrated ammonia-ammonium carbonate essentially as described previously (Huang et al., 2001). The isolated glycans were purified and divided into sialylated and non-sialylated glycan fractions as described in the other Examples of the present invention.

*MALDI-TOF mass spectrometry.* MALDI-TOF mass spectrometry was performed with a Voyager-DE STR BioSpectrometry Workstation, essentially as described previously (Saarinen *et al.*, 1999; Harvey *et al.*, 1993). Relative molar abundancies of both neutral (Naven & Harvey, 1996) and sialylated (Papac *et al.*, 1996) glycan components were assigned based on their relative signal intensities. The mass spectrometric fragmentation analysis was done with the Voyager-DE STR BioSpectrometry Workstation according to manufacturer's instructions. For the fragmentation analysis, sialylated glycans were further purified by gel filtration HPLC and permethylated essentially as described previously (Nyman *et al.*, 1998).

*Exoglycosidase digestions.* Digestions with *A. ureafaciens* neuraminidase (Glyko, UK), *S. pneumoniae* β-glucosaminidase (Calbiochem, USA), and Jack bean β-hexosaminidase (*C. ensiformis*; Calbiochem, USA) were performed essentially as described previously (Saarinen et al., 1999). The specificity of the two latter enzymes was controlled with synthetic oligosaccharides with terminal 1) Galβ1-4GlcNAcβ, 2) GlcNAcβ, and 3) GalNAc-β1-4GlcNAcβ epitopes: β-hexosaminidase digested the HexNAc residues in 2) and 3), but not 1), and β-glucosaminidase digested 2) but not 1) or 3).

## RESULTS

*Occurrence of HexNAcβHexNAcβ sequences in sialylated and neutral protein-linked glycans*

*from ovarian tissue samples.* **Figure 7** shows MALDI-TOF mass spectra from normal ovarian tissue (**Fig. 7A**) and ovarian tumors. Especially in benign ovarian tumors (**Fig. 7B**) there occurs high amounts of sialylated glycan structures with more HexNAc than Hex units in their proposed monosaccharide compositions (**Table 3**). Such glycans include glycans No **26**, **27**, **36**, and **38** (**Table 3**) which are major glycans in the benign tumors. However, only one of these glycans occurs in the malignant tissue and in a significantly reduced amount. In fact, in the sample from normal ovary tissue these glycan signals are more abundant than in the malignant tumour samples. In the neutral glycan fraction, the situation is similar (**Figure 8**). The normal ovary sample and all the benign ovarian tumor samples resemble each other in that they contain more glycans at m/z 1850 and 1891, corresponding to $Hex_4HexNAc_5dHex_1$ and $Hex_3HexNAc_6dHex_1$, respectively.

*Structures of the glycans.* The sialylated glycans were indicated to contain sialic acid residues upon neuraminidase digestion and subsequent analysis by MALDI-TOF mass spectrometry (data not shown). Exoglycosidase digestions of both neutral and sialylated fractions of benign ovarian tumor glycan samples showed that e.g. sialylated glycans **26** and **27**, as well as neutral glycans at m/z 1850 and 1891 were resistant to the action of β-glucosaminidase, but upon β-hexosaminidase digestion they lost either two or four HexNAc units. This indicates that the terminal residue in these glycans is not GlcNAcβ but that it may be GalNAcβ which is terminal to another HexNAcβ unit. The possible such structures include LacdiNAc (GalNAcβ4GlcNAcβ) that has been indicated previously in an ovarian glycoprotein.

*Cancer-ssociated glycan signals and glycosylation changes.* The present Example shows that multiple glycans and the glycan profiles are different between normal and malignant ovarian tissue, and that benign and malignant tumors differ from each other in multiple glycosylation features (**see Figures 18 and 19**). Significantly, also neutral O-glycan, sialylated O-glycan, and low-mannose N-glycan signals are elevated in all malignant ovarian tumor samples compared to the normal ovary and benign ovarian tumor samples (**Figure 8**).

**EXAMPLE 9. Discrimination analysis of major cancer types based on mass spectrometric glycan profiling.**

**EXPERIMENTATION AND RESULTS**

Relative abundancy profiles were obtained for the neutral protein-linked glycan fractions of cancer patient tissue samples with ductale and lobulare type breast adenocarcinoma, non-small cell lung adenocarcinoma, colon carcinoma or benign colon tumor, and ovarian cystadenocarcinoma (malignant tumor) or cystadenoma (benign tumor) as described in the other Examples.

*Generation of a discrimination formula.* A principal component and discrimination analysis was done to the results of a randomly picked group of four cancer and four healthy tissue samples from patients with ductale type breast carcinoma. It was found that three glycan signals could resolve the samples into cancer and healthy groups (**Fig. 9A**, 'training group'), and the experimental formula is:

$$5.36 \times \mathrm{I}\,(m/z\ 771) + 20.0 \times \mathrm{I}\,(m/z\ 899) + 8.13 \times \mathrm{I}\,(m/z\ 917) - 60.6,$$

where 'I (glycan signal)' refers to the relative abundance of the glycan signal marked in parenthesis, the three glycan signals correspond to sodium adduct ions of $Hex_2HexNAc_2$, $NeuAc_1Hex_1HexNAc_1$(deoxyamino)HexNAc, and $Hex_2HexNAc_2dHex_1$, respectively, and the resulting 'scores' for each sample are plotted on the y-axis in **Figure 9**. As described in the preceding Examples, these glycan signals more specifically correspond to neutral O-glycans, sialylated Core 2 type O-glycans, and low-mannose N-glycans.

*Testing the discrimination formula.* The obtained experimental discrimination formula was first applied to neutral protein-linked glycan analysis results of a group of ductale type breast carcinoma patients. As seen in Figure xA ('test group'), the formula could correctly discriminate 10 out of 10 samples (100 %). Similarly, the formula was applied to samples from lobulare type breast carcinoma and lung cancer patients, and it discriminated correctly 12 out of 14 (86 %) and 15 out of 17 (88 %) samples from these patients, respectively. When applied to samples of ovarian tumors and healthy ovarian tissue (**Fig. 9B**), the formula correctly discriminated 11 out of 11 samples (100 %) and placed the normal sample into the group of benign tumors. Similarly, when applied to samples of colon tumors and healthy colon tissue (**Fig. 9C**), the formula correctly discriminated 6 out of 6 samples (100 %) and placed the normal samples into the group of benign tumors.

**CONCLUSIONS**

The present results show that a simple experimental discrimination formula derived from a small group of breast cancer patients, applied on the results of neutral protein-linked glycan profiling, could effectively discriminate between cancerous and healthy samples of multiple cancer types. In particular, the same formula could correctly differentiate between malignant and benign tumors in both colon and ovary. In addition, it was shown that benign tumors resemble normal tissues and that malignant tumors differ significantly from both normal tissues and benign tumors with respect to the relative abundancies of glycan signals including neutral O-glycans, sialylated Core 2 type O-glycans, and low-mannose N-glycans. In conclusion, similar discrimination procedures can be used in diagnostics of human tumors and cancer. The present results indicate that there are individual differences in the expression of the cancer-associated glycan structures in both normal tissues between persons and in cancerous tissues between individual tumors. However, this did not effect the detection results of the present method.

Due to the presence of tissue-specific cancer-associated glycosylation revealed in the present invention, such as HexNAcβHexNAcβ glycans in tumors of the ovary (described in the other Examples), it is indicated that tissue-specific discrimination formulas based on the results of the present invention can differentiate between cancer and healthy

samples and/or benign tumors even more efficiently than the exemplary formula of the present Example.

**EXAMPLE 10.** Glycan isolation and analysis.

## EXAMPLES OF GLYCAN ISOLATION METHODS

*Glycan isolation.* N-linked glycans are preferentially detached from cellular glycoproteins by *F. meningosepticum* N-glycosidase F digestion (Calbiochem, USA) essentially as described previously (Nyman *et al.*, 1998), after which the released glycans are preferentially purified for analysis by solid-phase extraction methods, including ion exchange separation, and divided into sialylated and non-sialylated fractions.

*Example of a glycan purification method.* Isolated oligosaccharides can be purified from complex biological matrices as follows, for example for MALDI-TOF mass spectrometric analysis. Optionally, contaminations are removed by precipitating glycans with 80-90 % (v/v) aqueous acetone at -20 °C, after which the glycans are extracted from the precipitate with 60 % (v/v) ice-cold methanol. After glycan isolation, the glycan preparate is passed in water through a strong cation-exchange resin, and then through $C_{18}$ silica resin. The glycan preparate can be further purified by subjecting it to chromatography on graphitized carbon material, such as porous graphitized carbon (Davies, 1992). To increase purification efficiency, the column can be washed with aqueous solutions. Neutral glycans can be washed from the column and separated from sialylated glycans by elution with aqueous organic solvent, such as 25 % (v/v) acetonitrile. Sialylated glycans can be eluted from the column by elution with aqueous organic solvent with added acid, such as 0.05 % (v/v) trifluoroacetic acid in 25 % (v/v) acetonitrile, which elutes both neutral and sialylated glycans. A glycan preparation containing sialylated glycans can be further purified by subjecting it to chromatography on microcrystalline cellulose in n-butanol:ethanol:water (10:1:2, v/v) and eluted by aqueous solvent, preferentially 50 % ethanol:water (v/v). Preferentially, glycans isolated from small sample amounts are purified on miniaturized chromatography columns and small elution and handling volumes. An efficient purification method comprises most of the abovementioned purification steps. In an efficient purification sequence, neutral glycan fractions from small samples are purified with methods including carbon chromatography and separate elution of the neutral glycan fraction, and glycan fractions containing sialylated glycans are purified with methods including both carbon chromatography and cellulose chromatography.

*MALDI-TOF mass spectrometry.* MALDI-TOF mass spectrometry is performed with a Voyager-DE STR BioSpectrometry Workstation or a Bruker Ultraflex TOF/TOF instrument, essentially as described previously (Saarinen *et al.*, 1999; Harvey *et al.*, 1993). Relative molar abundancies of both neutral (Naven & Harvey, 1996) and sialylated (Papac *et al.*, 1996) glycan components are assigned based on their relative signal intensities. The mass spectrometric fragmentation analysis is done with the Bruker Ultraflex TOF/TOF instrument according to manufacturer's instructions.

## RESULTS

*Examples of analysis sensitivity.* Protein-linked and free glycans, including N- and O-glycans, are typically isolated from as little as about 5 x $10^4$ cells in their natual biological matrix and analyzed by MALDI-TOF mass spectrometry.

*Examples of analysis reproducibility and accuracy.* The present glycan analysis methods have been validated for example by subjecting a single biological sample, containing human cells in their natural biological matrix, to analysis by five different laboratory personnel. The results were highly comparable, especially by the terms of detection of individual glycan signals and their relative signal intensities, indicating that the reliability of the present methods in accurately describing glycan profiles of biological samples including cells is excellent. Each glycan isolation and purification phase has been controlled by its reproducibility and found to be very reproducible. The mass spectrometric analysis method has been validated by synthetic oligosaccharide mixtures to reproduce their molar proportions in a manner suitable for analysis of complex glycan mixtures and especially for accurate comparison of glycan profiles from two or more samples. The analysis method has also been successfully transferred from one mass spectrometer to another and found to reproduce the analysis results from complex glycan profiles accurately by means of calibration of the analysis.

*Examples of biological samples and matrices for successful glycan analysis.* The method has been successfully implied on analysis of e.g. blood cells, cell membranes, aldehyde-fixated cells, glycans isolated from glycolipids and glycoproteins, free cellular glycans, and free glycans present in biological matrices such as blood. The experience indicates that the method is especially useful for analysis of oligosaccharide and similar molecule mixtures and their optional and optimal purification into suitable form for analysis.

**EXAMPLE 11.** Glycan profiling.

*Generation of glycan profiles from mass spectrometric data.* **Figure 10A** shows a MALDI-TOF mass spectrum recorded in positive ion mode from a sample of neutral N-glycans. The profile includes multiple signals that interfere

with the interpretation of the original sample's glycosylation, including non-glycan signals and multiple signals arising from single glycan signals. According to the present invention, the mass spectrometric data is transformed into a glycan profile (**Fig. 10B**), which represents better the original glycan profile of the sample. An exemplary procedure is briefly as follows, and it includes following steps: 1) The mass spectrometric signals are first assigned to proposed monosaccharide compositions e.g. according to **Table 4**. 2) The mass spectrometric signals of ions in the molecular weight are of glycan signals typically show isotopic patterns, which can be calculated based on natural abundancies of the isotopes of the elements in the Earth's crust. The relative signal intensities of mass spectrometric signals near each other can be overestimated or underestimated, if their isotopic patterns are not taken into account. According to the present method, the isotopic patterns are calculated for glycan signals near each other, and relative intensities of glycan signals corrected based on the calculations. 3) Glycan ions are predominantly present as [M+Na]+ ions in positive ion mode, but also as other adduct ions such as [M+K]+. The proportion of relative signal intensities of [M+Na]+ to [M+K]+ ions is deduced from several signals in the spectrum, and the proportion is used to remove the effect of [M+K]+ adduct ions from the spectrum. 4) Other contaminating mass spectrometric signals not arising from the original glycans in the sample can optionally be removed from the profile, such as known contaminants, products of elimination of water, or in a case of permethylated oligosaccharides, undermethylated glycan signals. 5) The resulting glycan signals in the profile are normalized, for example to 100 %, for allowing comparison between samples.

**Figure 11A** shows a MALDI-TOF mass spectrum recorded in negative ion mode from a sample of neutral N-glycans. The profile includes multiple signals that interfere with the interpretation of the original sample's glycosylation, including non-glycan signals and multiple signals arising from single glycan signals. According to the present invention, the mass spectrometric data is transformed into a glycan profile (**Fig. 11B**), which represents better the original glycan profile of the sample. An exemplary procedure is briefly as follows, and it includes following steps: 1) The mass spectrometric signals are first assigned to proposed monosaccharide compositions. 2) The mass spectrometric signals of ions in the molecular weight are of glycan signals typically show isotopic patterns, which can be calculated based on natural abundancies of the isotopes of the elements in the Earth's crust. The relative signal intensities of mass spectrometric signals near each other can be overestimated or underestimated, if their isotopic patterns are not taken into account. According to the present method, the isotopic patterns are calculated for glycan signals near each other, and relative intensities of glycan signals corrected based on the calculations. 3) Glycan ions are predominantly present as [M-H]- ions in negative ion mode, but also as ions such as [M-2H+Na]- or [M-2H+K]-. The proportion of relative signal intensities of e.g. [M-H]- to [M-2H+Na]- and [M-2H+K]- ions is deduced from several signals in the spectrum, and the proportion is used to remove the effect of e.g. these adduct ions from the spectrum. 4) Other contaminating mass spectrometric signals not arising from the original glycans in the sample can optionally be removed from the profile, such as known contaminants or products of elimination of water. 5) The resulting glycan signals in the profile are normalized, for example to 100 %, for allowing comparison between samples.

**EXAMPLE 12.** Glycan structures of Keyhole limpet hemocyanin (KLH) and their modification.

## EXPERIMENTATION AND RESULTS

*Analysis of KLH glycosylation.* Keyhole limpet hemocyanin was from Sigma (*Megathura crenulata*; USA). N-linked and O-linked glycans were released from KLH by N-glycosidase enzyme and alkaline β-elimination, respectively, and the released glycans were purified and analyzed essentially as described in the previous Examples. The detected N-glycan masses, as resolved by MALDI-TOF mass spectrometry, were essentially similar to those described by Kurokawa et al. (2001). However, novel glycan components could be engineered and/or identified on KLH by exoglycosidase digestions with α-mannosidase (Jack beans, *C. ensiformis*, Sigma) and combined β-galactosidase digestion (β1,4-galactosidase from *S. pneumoniae* and recombinant β1,3/6-galactosidase, Calbiochem, USA). More specifically, based on the susceptibility of the $Hex_3HexNAc_2dHex_1$ glycan to β-galactosidase (one hexose removed) and N-glycosidase F (detachment from the glycoprotein) it was found that KLH contains Manα3(Galβ6)Manβ4GlcNAcβ4(Fucα6) GlcNAc that can be converted to Manα3Manβ4GlcNAcβ4(Fucα6)GlcNAc. The latter structure is a novel component in KLH. High-mannose and low-mannose N-glycans were similarly identified based on susceptibility to α-mannosidase. It was concluded that KLH contains at least the following human-type glycans in significant amounts: $(Man\alpha)_3$Manβ4GlcNAcβ4GlcNAc, $(Man\alpha)_4$Manβ4GlcNAcβ4GlcNAc, $(Man\alpha)_5$Manβ4GlcNAcβ4GlcNAc, (Manα) Manβ4GlcNAcβ4GlcNAc, $(Man\alpha)_1$Manβ4GlcNAcβ4(Fucα6)GlcNAc, $(Man\alpha)_2$Manβ4GlcNAcβ4(Fucα6)GlcNAc, $(Man\alpha)_3$Manβ4GlcNAcβ4(Fucα6)GlcNAc, $(Man\alpha)_4$Manβ4GlcNAcβ4(Fucα6)GlcNAc, and $(Man\alpha)_5$Manβ4GlcNAcβ4(Fucα6) GlcNAc. About 1/5 of the non-fucosylated and 1/3 of the fucosylated glycans with similar masses as the glycans listed above were assigned as containing β1,6-linked galactose residues based on their resistance to the action of α-mannosidase (Kurokawa et al., 2001). The latter are not human-type glycans.

*Production of KLH with human-type low-mannose N-glycans.* β-galactosidase was used to removal of, and the reaction result was characterized by MALDI-TOF mass spectrometry of released glycans, verifying that significant

amounts of β-galactose residues, occurring in KLH glycans and capping the human-type low-mannose N-glycans, were removed. Based on the structural knowledge described previously (Kurokawa et al., 2001), it was concluded that a modified spectrum of KLH glycans was produced, containing more low-mannose N-glycans than original KLH, and especially the novel human-type low-mannose glycan Manα3Manβ4GlcNAcβ4(Fucα6)GlcNAc described above. As described in the preceding Examples, the increased glycans present in KLH are associated with malignant tumors in major human cancer types.

**EXAMPLE 13.** Analyses of human tissue material and cell protein-linked glycan structures.

## EXPERIMENTAL PROCEDURES

Protein-linked glycans were isolated by non-reductive alkaline elimination essentially as described by Huang et al. (2000), or by N-glycosidase digestion to specifically retrieve N-glycans as described in the preceding **Examples**.

## RESULTS AND DISCUSSION

*Tissue-specific glycosylation analyses and comparison of glycan profiles between tissues.*

Human tissue protein-linked glycan profiles were analyzed from lung, breast, kidney, stomach, pancreas, lymph nodes, liver, colon, larynx, ovaries, and blood cells and serum. In addition, cultured human cells were analyzed similarly. **Tables 7** and **8** show neutral and acidic protein-linked glycan signals, respectively, observed in these human tissues and cells together with their classification into glycan structure groups. However, the individual glycan signals in each structure group varied from sample type to sample type, reflecting tissue material and cell type specific glycosylation. Importantly, in analyses of multiple samples, such as 10 samples from an individual human tissue type, glycan group feature proportions remain relatively constant with respect to variation in the occurrence of individual glycan signals.

Furthermore, it was observed that each tissue demonstrated a specific glycan profile that could be distinguished from the other tissues, cells, or blood or serum samples by comparison of glycan profiles according to the methods described in the present invention. It was also found out that glycan profile difference could be quantitated by comparing the difference between two glycan profiles, for example according to the Equation (resulting in difference expressed in %):

$$difference = \frac{1}{2}\sum_{i=1}^{n}\left|p_{i,a} - p_{i,b}\right|,$$

wherein $p$ is the relative abundance (%) of glycan signal $i$ in profile $a$ or $b$, and $n$ is the total number of glycan signals. For example, the Equation reveals that human lung and ovary tissue protein-linked glycan profiles differ from each other significantly more than human lung and kidney tissue protein-linked glycan profiles differ from each other. Each tissue or cell type could be compared in this manner.

*Comparison of glycosylation features between human tissue materials.* **Table 9** shows how glycan signal structural classification according to the present invention was applied to the comparison of quantitative differences in glycan structural features in glycan profiles between human tissue materials. The results show that each sample type was different from each other with respect to the quantitative glycan grouping and classification. Specifically, normal human lung and lung cancer tissues were different from each other both in the neutral glycan and sialylated glycan fractions with respect to the quantitative glycan structure grouping. In particular, lung cancer showed increased amounts of glycan signals classified into terminal HexNAc containing glycans. In analysis of individual glycan signals by β-glucosaminidase digestion, it was found that lung cancer associated glycan signals, such as $Hex_3HexNAc_4dHex_1$, contained terminal β-linked GlcNAc residues, correlating with the classification of these glycan signals into the terminal HexNAc (N>H and/or N=H) glycan groups. Furthermore, the human serum protein-linked glycan profile showed significantly lower amounts of high-mannose and especially low-mannose type N-glycan signals. It is concluded that the glycan grouping profile of human serum is significantly different from the corresponding profiles of solid tissues, and the present methods are suitable for identification of normal and diseased human tissue materials and blood or serum typical glycan profiles from each other.

*Disease- and tissue-specific differences in glycan structure groups.* **Fig. 8** shows a neutral protein-linked glycan profile of human ovary with abnormal growth. As described above, there are clear differences in the overall glycan profiles of **Fig. 8** and other human tissue samples. In analyses of multiple samples of ovarian tissues, it was found that benign abnormal growth of the ovary is especially characterized by increased amounts of glycan signals classified as terminal HexNAc (N>H). In structural analyses by fragmentation mass spectrometry and combined β-hexosaminidase and β-glucosaminidase digestions, the corresponding terminal HexNAc glycan signals were found to include structures

with terminal and sialylated β-GalNAc, more specifically terminal and sialylated GalNAcβ4GlcNAcβ (LacdiNAc) structures. According to the glycan structure classification, the protein-linked glycan profiles of normal ovarian tissue also contain increased amounts of terminal HexNAc glycans compared to other human tissues studied in the present invention, and normal human ovary preferentially also contains higher amounts of terminal and/or sialylated LacdiNAc structures than other human tissues on average. However, in malignant transformation the proportion of LacdiNAc structures among the protein-linked glycans of the ovary are decreased, and this is also reflected in the glycan grouping classification of malignant ovarian glycan profiles.

The analysis of protein-linked glycan profiles of human tissues revealed also that tissues with abundant epithelial structures, such as stomach, colon, and pancreas, contain increased amounts of small glycan structures, preferentially mucin-type glycans, and fucosylated glycan structures compared to the other glycan structure groups in structure classification. Similarly as epithelial tissues, mucinous carcinomas were differentiated from other carcinoma types based on analysis of their protein-linked glycan profiles and structure groups according to the methods of the present invention.

**EXAMPLE 14.** Proton-NMR analysis of glycan fractions

EXPERIMENTAL NMR PROCEDURES

Glycan material is liberated from biological material by enzymatic or chemical means. To obtain a less complex sample, glycans are fractionated into neutral and acidic glycan fractions by chromatography on a graphitized carbon as described above. A useful purification step prior to NMR analysis is gel filtration high-performance liquid chromatography (HPLC). For glycans of glycoprotein or glycolipid origin, a Superdex Peptide HR10/300 column (Amersham Pharmacia) may be used. For larger glycans, chromatography on a Superdex 75 HR10/300 column may yield superior results. Superdex columns are eluted at a flow rate of 1 ml per minute with water or with 50-200 mM ammonium bicarbonate for the neutral and acidic glycan fractions, respectively, and absorbance at 205-214 nm is recorded. Fractions are collected (typically 0.5 - 1 ml) and dried. Repeated dissolving in water and evaporation may be necessary to remove residual ammonium bicarbonate salts in the fractions. The fractions are subjected to MALDI-TOF mass spectrometry and all fractions containing glycans are pooled.

Prior to NMR analysis, the pooled fractions are dissolved in deuterium oxide and evaporated. With glycan preparations containing about 100 nmol or more material, the sample is finally dissolved in 600 microliters of high-quality deuterium oxide (99.9-99.996%) and transferred to a NMR analysis tube. A roughly equimolar amount of an internal standard, e.g. acetone, is commonly added to the solution. With glycan preparations derived from small tissue specimens or from a small number of cells (5-25 million cells), the sample is preferably evaporated from very high quality deuterium oxide (99.996%) twice or more to eliminate $H_2O$ as efficiently as possible, and then finally dissolved in 99.996% deuterium oxide. These low-material samples are preferebly analyzed by more sensitive NMR techniques. For example, NMR analysis tubes of smaller volumes can be used to obtain higher concentration of glycans. This kind of tubes include e.g. nanotubes (Varian) in which sample is typically dissolved in a volume of 37 microliters. Alternatively, higher sensitivity is achieved by analyzing the sample in a cryo-NMR instrument, which increases the analysis sensitivity through low electronic noise. The latter techniques allow gathering of good quality proton-NMR data from glycan samples containing about 1-5 nmol of glycan material.

ANALYSIS OF NMR DATA

It is realized that numerous studies have shown that proton-NMR data has the ability to indicate the presence of several structural features in the glycan sample. In addition, by careful integration of the spectra, the relative abundancies of these structural features in the glycan sample can be obtained.

For example, the proton bound to monosaccharide carbon-1, i.e. H-1, yields a distinctive signal at the lower field, well separated from the other protons of sugar residues. Most monosaccharide residues e.g. in N-glycans are identified by their H-1 signals. In addition, the H-2 signals of mannose residues are indicative of their linkages.

Sialic acids do not possess a H-1, but their H-3 signals (H-3 axial and H-3 equatorial) reside well separated from other protons of sugar residues. Moreover, differently bound sialic acids may be identified by their H-3 signals. For example, the Neu5Ac H-3 signals of Neu5Acα2-3Gal structure are found at 1.797 ppm (axial) and 2.756 ppm (equatorial). On the other hand, the Neu5Ac H-3 signals of Neu5 Acα2-6Gal structure are found at 1.719 ppm (axial) and 2.668 ppm (equatorial). By comparing the integrated areas of these signals, the molar ratio of these structural features is obtained.

Other structural reporter signals are commonly known and those familiar with the art use the extensive literature for reference in glycan NMR assignments.

RESULTS OF MALIGNANT TUMOR GLYCAN ANALYSES BY NMR

**Samples:** N-glycan fractions were liberated from pancreas carcinoma samples, purified as described in the preceding Examples, ultimately by gel filtration HPLC, and fractionated into 1) large neutral, 2) small neutral, and 3) acidic N-glycan fractions. These samples were analyzed by cryo-probe 1H-NMR as described above.

**Large neutral N-glycan fraction:** This fraction contained high-mannose N-glycans corresponding to the structural elements of reference structures in **Figure 14**. Correlation with these structures is described in **Table 5**, demonstrating that such structures were the major glycan signals in the glycan fraction.

**Small neutral N-glycan fraction:** This fraction contained low-mannose N-glycans as well as $Man_nGlcNAc_1$ glycans corresponding to the structural elements of reference structures in **Figure 15**. Correlation with these structures is described in **Table 6**, demonstrating that such structures were major glycan signals in the glycan fraction. In particular, the results demonstrated the presence of Mana3(Manα6)Manβ4GlcNAcβ4GlcNAc low-mannose N-glycan, as well as the presence of $(Man\alpha2)_{0-1}$Manα3 N-glycan antenna, and the presence of $(Man\alpha2)_{0-1}$Manα3(Manα6) Manβ4GlcNAc glycans in the glycan fraction.

**NMR References:**

Fu D., Chen L. and O'Neill R.A. (1994) Carbohydr. Res. 261, 173-186
Hård K., Mekking A., Kamerling J.P., Dacremont G.A.A. and Vliegenthart J.F.G. (1991) Glycoconjugate J. 8, 17-28
Hård K., Van Zadelhoff G., Moonen P., Kamerling J.P. and Vliegenthart J.F.G. (1992) Eur. J. Biochem. 209, 895-915
Helin J., Maaheimo H., Seppo A., Keane A. and Renkonen O. (1995) Carbohydr. Res. 266, 191-209

**EXAMPLE 15. Lysosomal organelle-specific N-glycosylation.**

EXPERIMENTAL PROCEDURES

*Lysosomal protein sample* including human myeloperoxidase was chosen to represent lysosomal organelle glycoproteins. The sample was digested with N-glycosidase F to isolate N-glycans, and they were purified for MALDI-TOF mass spectrometric analysis as described in the preceding Examples.

*Alkaline phosphatase digestion* was performed essentially according to manufacturer's instructions. After the digestion glycans were purified for MALDI-TOF mass spectrometric analysis as above.

RESULTS AND DISCUSSION

*Neutral N-glycan profiles.* The neutral N-glycan profile is presented in **Figure 13** (upper panel). The profile is dominated by low-mannose type and high-mannose-type N-glycan signals, comprising 49% and 46% of the total signal intensity, respectively. Especially the high proportion of low-mannose type N-glycans is characteristic to the sample (**Table 10**, **upper panel**).

**EXAMPLE 16. Identification of specific glycosylation signatures from glycan profiles of malignant and normal human tissue samples based on quantitative glycomics.**

EXPERIMENTAL PROCEDURES

Normal lung (Sample I) and malignant lung tumor samples (Sample II) were archival formalin-fixed and paraffin-embedded tissue sections from cancer patients with small cell lung cancer. Protein-linked glycans were isolated from the representative samples by non-reductive β-elimination, purified, and analyzed by MALDI-TOF mass spectrometry as described in the preceding Examples. In the present analysis, the total desialylated protein-linked glycomes from each sample were used.

To analyze the data and to find the major glycan signals associated with either the normal state or the disease, two variables were calculated for the comparison of glycan signals between the two samples:

1. absolute difference $A = (SII - SI)$, and
2. relative difference $R = A / SI$,

wherein *SI* and *SII* are relative abundances of a given glycan signal in Sample I (normal human lung tissue) and Sample II (small cell lung cancer), respectively.

The glycan signals were further classified into structure classes by a one letter code: *a b c d*, wherein *a* is either N (neutral) or S (sialylated); *b* is either L (low-mannose type), M (high-mannose type), H (hybrid-type or monoantennary), C (complex-type), S (soluble), or O (other); *c* is either - (nothing), F (fucosylated), or E (multifucosylated); and *d* is either - (nothing), T (terminal HexNAc, N>H), or B (terminal HexNAc, N=H); as described in the present invention.

RESULTS

To identify protein-linked glycan signals correlating with malignant tumors in total tissue glycomes from cancer patient, major signals specific to either normal lung tissue or malignant small cell lung cancer tumors were selected based on their relative abundances. When *A* and *R* were calculated for the glycan profile datasets of the two samples, and the glycan signals thereafter sorted according to the values of *A* and *R*, the most significant differing glycan signals between the two samples could be identified (**Table 11**). Among the most abundant protein-linked glycan signals in the data, the following three signals had emerged in II (new in Table 11): 1955, 2685, and 2905, corresponding to fucosylated complex-type N-glycans. The absolute differences of these signals were among the ten most large in the data, indicating that they were significant. The signals that experienced the highest relative increase in *R* were: 771 (*R* = 2.4, corresponding to 3.4-fold increase), 1905 (*R* = 2.2, corresponding to 3.2 fold increase), and 1485 (*R* = 1.3, corresponding to 2.3 fold increase). The latter signal corresponded to complex-type N-glycans with terminal HexNAc. Significantly, its +2Hex counterpart 1809 was the most drastically reduced glycan signal in II with A = -8.9 and R = - 0.4 (corresponding to 40% decrease in II), indicating a large change in terminal HexNAc expression. Moreover, the data easily shows that the glycan signals 1704, 1866, 1136, and 755 were not present in II.

Further, the obtained results, especially the identified major glycan signals indicative of either Sample II (high A and R) or Sample I (low A and R) were used to compile two alternative algorithms to produce glycan score with which lung cancer sample could be identified from normal lung sample based on the glycan signal values of the quantitative glycome data:

1. glycan score = I(1485) - I(1809),
wherein I(1485) is the relative abundance of glycan signal 1485 and I(1809) is the relative abundance of glycan signal 1809;
and alternatively:
2. glycan score = I(1485) / I(1809)

These glycan score algorithms yield high numerical value when applied to lung cancer sample and low numerical value when applied to normal lung sample.

DISCUSSION

The present identification analysis produced selected glycan signal groups, from where indifferent glycan signals have been removed and that have reduced noise or background and less observation points, but have the resolving power of the initially obtained glycan profiles. Such selected signal groups and their patterns in different sample types can serve as a signature for the identification of for example **1)** normal human glycosylation, **2)** tissue-specific glycosylation, **3)** disease states affecting tissue glycosylation, **4)** malignant cancer, **5)** malignancy in comparison to benign tumors, and grade of malignancy, or **6)** glycan signals that have individual variation. Moreover, glycan signals can be identified that do not change between samples, including major glycans that can be considered as invariant or housekeeping glycans.

The present data analysis identified potential glycan marker signals for future identification of either the normal lung of the lung tumor glycome profiles. Further, glycan classes that are associated with e.g. disease state in humans can be identified. Specifically, the analysis revealed that within the total complex-type N-glycan structure class in the tissue glycomes, terminal HexNAc (N>H) were typical to small cell lung cancer.

The method also allows identification of major glycans or major changes within glycan structure classes. For example, the proportion of multifucosylated glycans within the total tissue glycome profile was increased in II (1.1%) compared to I (0.3%). The data analysis identified this change predominantly to the appearance of glycan signals 1955 and 2685 in II.

**EXAMPLE 17.** Analysis of large cancer patient sample panel with respect to expression of cancer-associated glycan antigens.

EXPERIMENTAL PROCEDURES

Protein-linked glycans were isolated from formalin-fixed and paraffin-embededed tissue sections and analyzed as describe in the preceding Examples. Following cancer types were analyzed: lung cancer, both small cell lung adenocarcinoma and non-small cell lung adenocarcinoma, and lung carcinoma liver metastases; breast cancer; ductale type breast adenocarcinoma and lymph node metastases thereof; lobulare type breast adenocarcinoma and lymph node metastases thereof; ovarian cystadenocarcinoma; colon cancer / carcinoma, carcinoma adenomatosum, and liver metastases thereof; kidney cancer / carcinoma, and kidney hypernephroma; gastric cancer / carcinoma, and lymph node metastases thereof, liver cancer / carcinoma; larynx cancer / carcinoma; pancreas cancer / carcinoma; melanoma and liver metastases thereof; gall bladder cancer / carcinoma, and liver metastases thereof; salivary gland cancer / carcinoma, and skin metastases thereof; and lymph node cancer /carcinoma (lymphoma).

RESULTS

**Low-mannose type N-glycans** were identified based on their indicative mass spectrometric glycan signals for $[M+Na]^+$ ions of $Hex_{1-4}HexNAc_2dHex_{0-1}$, as described in to the present invention. In the present analyses the preferred indicative signals of the glycan structure group were m/z 771, 917, 933, 1079, and 1095 due to their abundance in mass spectrometric glycan profiles; 771, 933, and 1095 especially in case of non-fucosylated low-mannose type N-glycans; 917 and 1079 especially in case of detecting fucosylated low-mannose type N-glycans; 933, 1079, and 1095 especially in case of detecting low-mannose type N-glycans when N- and O-glycans were analyzed simultaneously; 1079 and 1095 as preferred sensitive indicators of the group; and/or 1079 as a preferred sensitive single indicator of the group.

Using these criteria, low-mannose type N-glycans were detected to be cancer-associated and expressed in malignant tumors in following cancer types: lung cancer, both small cell lung adenocarcinoma and non-small cell lung adenocarcinoma, and lung carcinoma liver metastases; breast cancer; ductale type breast adenocarcinoma and lymph node metastases thereof; lobulare type breast adenocarcinoma and lymph node metastases thereof; ovarian cystadenocarcinoma; colon cancer / carcinoma, carcinoma adenomatosum, and liver metastases thereof; kidney cancer / carcinoma, and kidney hypernephroma; gastric cancer /carcinoma, and lymph node metastases thereof, liver cancer / carcinoma; larynx cancer /carcinoma; pancreas cancer / carcinoma; melanoma and liver metastases thereof; gall bladder cancer / carcinoma, and liver metastases thereof; and lymph node cancer / carcinoma (lymphoma). Further the expression of the glycans was detected in abundant amounts in salivary gland cancer / carcinoma, and skin metastases thereof.

In all these cancer types, both fucosylated and non-fucosylated low-mannose type N-glycans were detected and expression levels were found to be higher in cancer in comparison to normal human tissue samples. In addition, in benign tumors of the ovary and colon, low-mannose type glycan signals were significantly lower than in the corresponding malignant tumors, indicating that low-mannose type glycans are specifically associated with malignancy in human cancers.

**EXAMPLE 18.** Neutral and acidic protein-linked tissue glycan profiles of ductale and lobulare type breast carcinomas.

Protein-linked glycans were isolated from formalin-fixed and paraffin-embededed tissue sections of ductale-type breast cancer and lymph node metastases derived therefrom, and analyzed as described in the preceding Examples.

The results are described as protein-linked glycan profiles of the primary breast cancer tumors and normal brease tissues, as well as metastases and normal lymph node tissues from the same patients in Figures **29, 30, 31, and 32**, respectively. The profiles indicate that major cancer- and metastasis-associated glycan signals and signal groups include low-mannose type glycans and O-glycas, based on the indicative signals for each glycan signal group, respectively.

Further, the acidic glycan profiles were quantitatively analyzed by composition classification into glycan structural features, as described in **Table 13**. Importantly, the classification analysis revealed that especially sulphation and/or phosphorylation and complex fucosylation of acidic glycans were associated with the present tumors as well as the corresponding lymph node metastases in comparison with either of the primary and the secondary normal tissues. Major glycan signals expressing these features were $NeuAc_1Hex_2HexNAc_2SP_1$ and $NeuAc_1Hex_3HexNAc_3SP_2$ wherein SP is either sulphate or phosphate ester, and $NeuAc_1Hex_5HexNAc_4dHex_2$ and $NeuAc_1Hex_5HexNAc_4dHex_3$, respectively.

**EXAMPLE 19.** Metastasis-associated glycans in malignancy and site-specific metastasis to the liver

EXPERIMENTAL PROCEDURES

A series of malignant primary tumors of the lung, colon, skin, and gall bladder, and corresponding liver metastases were analyzed in order to identify glycans associated with malignancy and metastasis formation, especially liver metastases. Protein-linked glycans were isolated from formalin-fixed and paraffin-embedded tissue sections or protein fractions of tumor tissues and analyzed by MALDI-TOF mass spectrometry as described in the preceding Examples.

By comparing the quantitative expression of indicative glycan signals of 1) liver metastases and normal liver tissue, and 2) malignant primary tumor and corresponding normal tissue, malignancy and metastasis-associated glycan signals and glycan structure groups were identified as described in the preceding Examples.

RESULTS AND DISCUSSION

Glycan groups that were associated with liver metastases were 1) low-mannose type glycans, 2) non-reducing terminal HexNAc glycans, especially non-reducing terminal GlcNAc glycans, and 3) neutral and acidic O-glycans, especially neutral and sialylated O-glycans. Preferential glycan signals associated with malignant liver metastases included 933, 1079, and 1095 (1); 1485 (2); and 771, 917, 899, 1038, and 1329 (3), especially 771 and 899 (3); wherein the numbering (1-3) refers to the identified metastasis-associated glycan groups. The glycans were present in the primary tumor in elevated amounts in comparison to corresponding normal tissue, and significantly, further elevated in comparison to normal liver tissue i.e. enriched in the liver metastases. The results indicate that the present glycans identified in metastases are associated with metastasis formation, more specifically liver metastasis formation. Specifically, the present results indicate that low-mannose type, non-reducing terminal GlcNAc, and O-glycans are associated with malignant metastasis formation, more specifically liver metastasis formation; most specifically, low-mannose type and terminal GlcNAc glycans are associated with liver metastases.

**EXAMPLE 20.** Metastasis-associated glycans in malignancy and site-specific metastasis to lymph nodes.

EXPERIMENTAL PROCEDURES

A series of malignant primary tumors of the stomach and breast, and corresponding lymph node metastases were analyzed in order to identify glycans associated with malignancy and metastasis formation, especially lymph node metastases. Protein-linked glycans were isolated from formalin-fixed and paraffin-embedded tissue sections or protein fractions of tumor tissues and analyzed by MALDI-TOF mass spectrometry as described in the preceding Examples.

By comparing the quantitative expression of indicative glycan signals of 1) lymph node metastases and normal lymph node tissue, and 2) malignant primary tumor and corresponding normal tissue, malignancy and metastasis-associated glycan signals and glycan structure groups were identified as described in the preceding Examples.

RESULTS AND DISCUSSION

Glycan groups that were associated with lymph node metastases were 1) low-mannose type glycans and 2) neutral and acidic O-glycans, especially neutral and sialylated O-glycans. Preferential glycan signals associated with malignant liver metastases included 933, 1079, and 1095 (1); and 771, 917, 899, 1038, and 1329 (2), especially 771 and 899 (2); wherein the numbering (1-2) refers to the identified metastasis-associated glycan groups. The glycans were present in the primary tumor in elevated amounts in comparison to corresponding normal tissue, and significantly, further elevated in comparison to normal liver tissue i.e. enriched in the lymph node metastases. The results indicate that the present glycans identified in metastases are associated with metastasis formation, more specifically lymph node metastasis formation. Specifically, the present results indicate that low-mannose type and O-glycans are associated with malignant metastasis formation, more specifically lymph node metastasis formation; most specifically, low-mannose type and sialylated O-glycans are associated with lymph node metastases.

A sample array from different cancer patients with gastric cancer revealed an interesting phenomenon: lymph node metastases of primary gastric cancers transformed the lymph node glycan profiles with glycan signals originating from primary tissue specific glycosylation. For example, in lymph node metastases small O-glycan type signals with either blood group O, A, or B characteristics were detected. These signals in the primary tissue location included in both O, A, and B patients: 1063 (Hex2HexNAc2dHex2) i.e. increased amounts of dHex in glycans with 3 or less HexNAc residues and more clearly in glycans with 2 or less HexNAc residues; specifically in A patients: 1120 (Hex2HexNAc3dHex1) and 1266 (Hex2HexNAc3dHex2) i.e. increased amounts of HexNAc and dHex in glycans with 3 or less Hex residues and more clearly in glycans with 2 or less Hex residues; and specifically in B patients: 714

(Hex2HexNAc1dHex1) and/or 1225 (Hex3HexNAc2dHex2) i.e. increased amounts of Hex and dHex in glycans with 3 or less HexNAc residues and more clearly in glycans with 2 or less HexNAc residues. The present results demonstrated that the origin of the primary tumor is reflected in the glycan profile of the metastasis, and that the metastatic cancer cells carry with them abnormal glycan antigens to the site of the metastasis.

**Table 1.** Overexpression data of low-mannose type N-glycans in studied cancer types.

| | m/z | | | | | |
|---|---|---|---|---|---|---|
| **Cancer type** | **917** | **933** | **1079** | **1095** | **1241** | **1403** |
| Non-small cell lung adenocarcinoma | + | - | + | - | + | + |
| Ductale breast adenocarcinoma | + | + | + | + | + | + |
| Lobulare breast adenocarcinoma | + | + | + | - | + | + |
| Ovarian cystadenocarcinoma | + | + | + | - | + | + |
| Colon carcinoma | + | - | + | - | + | - |
| Kidney cancer | - | + | - | - | - | - |
| Gastric cancer | - | + | + | + | - | - |
| Liver cancer | - | + | - | - | - | - |
| Larynx cancer | + | + | + | - | - | - |
| Pancreas cancer | - | + | + | + | - | - |

*CODE:*
+ overexpressed in cancer
- no overexpression detected
m/z 917 $Hex_2HexNAc_2dHex_1$
m/z 933 $Hex_3HexNAc_2$
m/z 1079 $Hex_3HexNAc_2dHex_1$
m/z 1095 $Hex_4HexNAc_2$
m/z 1241 $Hex_4HexNAc_2dHex_1$
m/z 1403 $Hex_5HexNAc_2dHex_1$

**Table 2.** Statistical analysis of low-mannose type N-glycans in breast cancer.

| **DUCTALE BREAST ADENOCARCINOMA** | | | |
|---|---|---|---|
| 9 sample pairs | | | |
| **Approx. m/z** | **Composition** | **P** | **Test** |
| 917 | $Hex_2HexNAc_2dHex_1$ | 0.0039 | Signed Rank |
| 933 | $Hex_3HexNAc_2$ | 0.0031 | Student's t |
| 1079 | $Hex_3HexNAc_2dHex_1$ | 0.0039 | Signed Rank |
| 1241 | $Hex_4HexNAc_2dHex_1$ | 0.0071 | Student's t |

**Table 3.** Proposed monosaccharide compositions for MALDI-TOF mass spectrometric profiling of sialylated protein-linked glycans isolated from **A.** healthy ovarian tissue, **B.** benign ovarian cystadenoma, and **C.** malignant ovarian cystadenocarcinoma in **Figure y1**. Experimental masses (exp. m/z) refer to spectrum **B.**; SO3, sulfate/phosphate.

| No. | calc. m/z | exp. m/z | proposed composition |
|---|---|---|---|
| 1 | 1550.40 | 1550.36 | NeuAc1Hex3HexNAc3dHex1 |
| 2 | 1558.40 | 1558.37 | Hex4HexNAc4S03 |
| 3 | 1566.40 | 1566.93 | NeuAc1 Hex4HexNAc3 |

(continued)

| No. | calc. m/z | exp. m/z | proposed composition |
|---|---|---|---|
| 4 | | 1588.17 | unknown |
| 5 | | 1617.27 | unknown |
| 6 | | 1654.70 | unknown |
| 7 | 1712.54 | 1712.13 | NeuAc1Hex4HexNAc3dHex1 |
| 8 | | 1719.82 | unknown |
| 9 | 1728.54 | 1728.33 | NeuAc1Hex5HexNAc3 |
| 10 | 1753.60 | 1753.61 | NeuAc1Hex3HexNAc4dHex1 |
| 11 | 1769.60 | 1769.86 | NeuAc1Hex4HexNAc4 |
| 12 | 1785.59 | 1785.98 | NeuGc1Hex4HexNAc4 |
| 13 | 1816.61 | 1816.45 | NeuAc2Hex5HexNAc2 |
| 14 | 1857.66 | 1857.37 | NeuAc2Hex4HexNAc3 |
| 15 | 1874.68 | 1874.51 | NeuAc1Hex5HexNAc3dHex1 |
| 16 | 1882.69 | 1882.09 | Hex6HexNAc4SO3 |
| 17 | 1915.74 | 1915.68 | NeuAc1Hex4HexNAc4dHex1 |
| 18 | 1931.74 | 1931.73 | NeuAc1Hex5HexNAc4 |
| 19 | 1947.74 | 1947.80 | NeuGc1Hex5HexNAc4 |
| 20 | | 1978.32 | unknown |
| 21 | | 1988.68 | unknown |
| 22 | 2020.83 | 2020.05 | NeuAc1Hex5HexNAc3dHex2 |
| 23 | 2044.85 | 2044.17 | NeuAc2Hex3HexNAc4dHex1 |
| 24 | 2077.88 | 2077.81 | NeuAc1Hex5HexNAc4dHex1 |
| 25 | | 2110.12 | unknown |
| 26 | 2118.93 | 2118.81 | NeuAc1Hex4HexNAc5dHex1 |
| 27 | 2159.98 | 2159.72 | NeuAc1Hex3HexNAc6dHex1 |
| 28 | | 2198.95 | unknown |
| 29 | 2223.00 | 2223.03 | NeuAc2Hex5HexNAc4 |
| 30 | 2281.07 | 2281.08 | NeuAc1Hex5HexNAc5dHex1 |
| 31 | | 2308.09 | unknown |
| 32 | 2328.08 | 2326.60 | NeuAc2Hex3HexNAc6dHex1 |
| 33 | 2352.10 | 2350.72 | NeuAc3Hex45HexNAc4 |
| 34 | 2369.13 | 2369.21 | NeuAc2Hex5HexNAc4dHex1 |
| 35 | 2402.19 | 2401.80 | NeuAc1Hex4HexNAc6dHex1SO3 |
| 36 | 2410.18 | 2409.32 | NeuAc1Hex4HexNAc5dHex3 |
| 37 | 2443.21 | 2443.01 | NeuAc1Hex5HexNAc6dHex1 |
| 38 | 2451.24 | 2449.46 | NeuAc2Hex3HexNAc6dHex1 |
| 39 | | 2547.32 | unknown |
| 40 | 2572.32 | 2572.25 | NeuAc2Hex5HexNAc5dHex1 |
| 41 | 2588.32 | 2588.01 | NeuAc2Hex6HexNAc5 |

(continued)

| No. | calc. m/z | exp. m/z | proposed composition |
|---|---|---|---|
| 42 | 2734.46 | 2734.46 | NeuAc2Hex6HexNAc5dHex1 |
| 43 | 2775.52 | 2773.43 | NeuAc2Hex5HexNAc6dHex1 |
| 44 | 2808.54 | 2808.40 | NeuAc1Hex7HexNAc6dHex1 |
| 45 | 2816.57 | 2814.30 | NeuAc2Hex4HexNAc7dHex1 |
| 46 | 2879.58 | 2879.54 | NeuAc3Hex6HexNAc5 |
| 47 | 3025.72 | 3025.72 | NeuAc3Hex6HexNAc5dHex1 |
| 48 | 3107.82 | 3104.91 | NeuAc3Hex4HexNAc7dHex1 |
| 49 | 3244.91 | 3245.37 | NeuAc3Hex7HexNAc6 |
| 50 | 3391.05 | 3390.79 | NeuAc3Hex7HexNAc6dHex1 |
| 51 | 3536.17 | 3536.17 | NeuAc4Hex7HexNAc6 |
| 52 | 3682.31 | 3682.59 | NeuAc4Hex7HexNAc6dHex1 |

**Table 4.** Preferred neutral glycan compositions. Calculated mass-to-charge ratios (calc. m/z) refer to the first isotope signal of $[M+Na]^+$ ion.

| **Proposed composition** | **calc. m/z** |
|---|---|
| HexHexNAc | 406,13 |
| Hex3 | 527,16 |
| HexHexNAcdHex | 552,19 |
| Hex2HexNAc | 568,19 |
| HexHexNAc2 | 609,21 |
| Hex4 | 689,21 |
| Hex2HexNAcdHex | 714,24 |
| Hex3HexNAc | 730,24 |
| HexHexNAc2dHex | 755,27 |
| Hex2HexNAc2 | 771,26 |
| HexHexNAc3 | 812,29 |
| Hex5 | 851,28 |
| Hex2HexNAcdHex2 | 860,30 |
| Hex4HexNAc | 892,29 |
| HexHexNAc2dHex2 | 901,33 |
| Hex2HexNAc2dHex | 917,32 |
| Hex3HexNAc2 | 933,32 |
| HexHexNAc3dHex | 958,35 |
| Hex2HexNAc3 | 974,34 |
| Hex2HexNAcdHex3 | 1006,36 |
| Hex6 | 1013,32 |
| HexHexNAc4 | 1015,37 |
| HexHexNAc5dHex | 1364,51 |
| Hex3HexNAc2dHex3 | 1371,49 |
| Hex7HexNAc | 1378,45 |
| Hex4HexNAc2dHex2 | 1387,49 |
| Hex2HexNAc5 | 1380,50 |
| Hex5NexNAc2dHex | 1403,48 |
| Hex2HexNAc3dHex3 | 1412,52 |
| Hex6HexNAc2 | 1419,48 |
| HexHexNAc6 | 1421,53 |
| Hex3HexNAc3dHex2 | 1428,51 |
| Hex4HexNAc3dHex | 1444,51 |
| HexHexNAc4dHex3 | 1453,54 |
| Hex5HexNAc3 | 1460,50 |
| Hex2HexNAc4dHex2 | 1469,54 |
| Hex3HexNAc4dHex | 1485,53 |
| Hex9 | 1499,48 |
| Hex4HexNAc4 | 1501,53 |
| HexHexNAc5dHex2 | 1510,57 |
| Hex3HexNAc2dHex4 | 1517,55 |
| Hex2HexNAc5dHex | 1526,56 |
| Hex4HexNAc2dHex3 | 1533,54 |
| Hex8HexNAc | 1540,50 |
| Hex3HexNAc5 | 1542,56 |

(continued)

| | | | | |
|---|---|---|---|---|
| Hex3HexNAcdHex2 | 1022,35 | | Hex5HexNAc2dHex2 | 1549,54 |
| Hex5HexNAc | 1054,34 | | Hex6HexNAc2dHex | 1565,53 |
| Hex2HexNAc2dHex2 | 1063,38 | | Hex3HexNAc3dHex3 | 1574,57 |
| Hex3HexNAc2dHex | 1079,38 | | Hex7HexNAc2 | 1581,53 |
| Hex4HexNAc2 | 1095,37 | | Hex2HexNAc6 | 1583,58 |
| HexHexNAc3dHex2 | 1104,41 | | Hex4HexNAc3dHex2 | 1590,57 |
| Hex2HexNAc3dHex | 1120,40 | | Hex5HexNAc3dHex | 1606,56 |
| Hex3HexNAc3 | 1136,40 | | Hex2HexNAc4dHex3 | 1615,60 |
| Hex2HexNAcdHex4 | 1152,42 | | Hex6HexNAc3 | 1622,56 |
| HexHexNAc4dHex | 1161,43 | | Hex3HexNAc4dHex2 | 1631,59 |
| Hex7 | 1175,37 | | Hex4HexNAc4dHex | 1647,59 |
| Hex2HexNAc4 | 1177,42 | | Hex10 | 1661,53 |
| Hex2HexNAc2dHex3 | 1209,44 | | Hex5HexNAc4 | 1663,58 |
| Hex6HexNAc | 1216,40 | | Hex2HexNAc5dHex2 | 1672,62 |
| HexHexNAc5 | 1218,45 | | Hex3HexNAc5dHex | 1688,61 |
| Hex3HexNAc2dHex2 | 1225,43 | | Hex5HexNAc2dHex3 | 1695,60 |
| Hex4HexNAc2dHex | 1241,43 | | Hex9HexNAc | 1702,56 |
| Hex5HexNAc2 | 1257,42 | | Hex4HexNAx5 | 1704,61 |
| Hex2HexNAc3dHex2 | 1266,46 | | Hex6HexNAc2dHex2 | 1711,59 |
| Hex3HexNAc3dHex | 1282,45 | | Hex3HexNAc3dHex4 | 1720,63 |
| Hex4HexNAc3 | 1298,45 | | Hex7HexNAc2dHex | 1727,59 |
| HexHexNAc4dHex2 | 1307,49 | | Hex2HexNAc6dHex | 1729,64 |
| Hex2HexNAc4dHex | 1323,48 | | Hex4HexNAc3dHex3 | 1736,62 |
| Hex8 | 1337,42 | | Hex8HexNAc2 | 1743,58 |
| Hex3HexNAc4 | 1339,48 | | Hex3HexNAc6 | 1745,64 |
| Hex2HexNAc2dHex4 | 1355,50 | | Hex5HexNAc3dHex2 | 1752,62 |
| Hex6HexNAc3dHex | 1768,81 | | Hex4HexNAc7 | 2110,77 |
| Hex3HexNAc4dHex3 | 1777,65 | | Hex6HexNAc4dHex2 | 2117,75 |
| Hex7HexNAc3 | 1784,61 | | Hex3HexNAc5dHex4 | 2128,79 |
| Hex4HexNAc4dHex2 | 1793,84 | | Hex7HexNAc4dHex | 2133,75 |
| HexSHexNAc4dHex | 1809,64 | | Hex4HexNAcSdHex3 | 2142,78 |
| Hex2HexNAc5dHex3 | 1818,68 | | Hex13 | 2147,89 |
| Hex11 | 1823,58 | | Hex8HexNAc4 | 2149,74 |
| Hex6HexNAc4 | 1825,83 | | Hex5HexNAc5dHex2 | 2158,78 |
| Hex3HexNAc5dWex2 | 1834,67 | | Hex6HexNAc5dHex | 2174,77 |
| Hex4HexNAc5dHex | 1850,67 | | Hex8HexNAc2dHex3 | 2181,78 |
| Hex6HexNAc2dHex3 | 1857,65 | | Hex3HexNAc6dHex3 | 2183,81 |
| Hex10HexNAc | 1864.61 | | Hex12HexNac | 2188,71 |
| Hex5HexNAc5 | 1866,68 | | Hex7HexNAc5 | 2190,77 |

(continued)

| | | | | |
|---|---|---|---|---|
| Hex7HexNAc2dHex2 | 1873,64 | | Hex4HexNAc6dHex2 | 2199,80 |
| Hex2HexNAc6dHex2 | 1875,70 | | Hex5HexNAc6dHex | 2215,80 |
| Hex4HexNAc3dHex4 | 1882,68 | | Hex7HexNAc3dHex3 | 2222,78 |
| Hex8HexNAc2dHex | 1889,64 | | Hex2HexNAc7dHex3 | 2224,84 |
| Hex3HexNAc6dHex | 1891,69 | | Hex11HexNAc2 | 2229,74 |
| Hex5HexNAc3dHex3 | 1898,68 | | Hex6HexNAc6 | 2231,79 |
| Hex9HexNAc2 | 1905,63 | | Hex8HexNAc3dHex2 | 2238,78 |
| Hex4HexNAc6 | 1907,69 | | Hex3HexNAc7dHex2 | 2240,83 |
| Hex6HexNAc3dHex2 | 1914,87 | | Hex5HexNAc4dHex4 | 2247,81 |
| Hex3HexNAc4dHex4 | 1923,71 | | Hex4HexNAc7dHex | 2256,83 |
| Hex7HexNAc3dHex | 1930,67 | | Hex6HexNAc4dHex3 | 2263,81 |
| Hex2HexNAc7dHex | 1932,72 | | Hex5HexNAc7 | 2272,82 |
| Hex4HexNAc4dHex3 | 1939,70 | | Hex7HexNAc4dHex2 | 2279,80 |
| Hex8HexNAc3 | 1946,68 | | Hex4HexNAc5dHex4 | 2288,84 |
| Hex5HexNAc4dHex2 | 1955,70 | | Hex5HexNAc5dHex3 | 2304,84 |
| Hex6HexNAc4dHex | 1971,69 | | Hex14 | 2309,74 |
| Hex3HexNAc5dHex3 | 1980,73 | | Hex9HexNAc4 | 2311,79 |
| Hex12 | 1985,63 | | Hex6HexNAc5dHex2 | 2320,83 |
| Hex7HexNAc4 | 1987,89 | | Hex7HexNAc5dHex | 2338,82 |
| Hex4HexNAc5dHex2 | 1998,72 | | Hex4HexNAc6dHex3 | 2345,88 |
| Hex5HexNAc5dHex | 2012,72 | | Hex8HexNAc5 | 2352,82 |
| Hex7HexNAc2dHex3 | 2019,70 | | Hex5HexNAc6dHex2 | 2361,86 |
| Hex2HexNAc6dHex3 | 2021,76 | | Hex6HexNAcBdHex | 2377,85 |
| Hex11HexNAc | 2026,66 | | Hex8HexNAc3dHex3 | 2384,83 |
| Hex6HexNAcS | 2028,71 | | Hex3HexNAc7dHex3 | 2388,89 |
| Hex8HexNAc2dHex2 | 2035,70 | | Hex12HexNac2 | 2391,79 |
| Hex3HexNAc6dHex2 | 2037,75 | | Hex7HexNAc6 | 2393,85 |
| Hex5HexNAc3dHex4 | 2044,73 | | Hex4HexNAc7dHex2 | 2402,88 |
| Hex4HexNAc6dHex | 2053,75 | | HexBHexNAc4dHex4 | 2409,87 |
| Hex6HexNAc3dHex3 | 2060,73 | | Hex5HexNAc7dHex | 2418,88 |
| Hex10HexNAc2 | 2067,69 | | Hex7HexNAc4dHex3 | 2425,86 |
| Hex5HexNAc6 | 2069,74 | | Hex6HexNAc7 | 2434,87 |
| Hex7HexNAc3dHex2 | 2076,72 | | Hex5HexNAc5dHex4 | 2450,89 |
| Hex2HexNAc7dHex2 | 2078,78 | | Hex6HexNAc5dHex3 | 2466,89 |
| Hex4HexNAc4dHex4 | 2085,76 | | Hex15 | 2471,79 |
| Hex8HexNAc3dHex | 2092,72 | | Hex7HexNAc5dHex2 | 2482,88 |
| Hex3HexNAc7dHex | 2094,77 | | Hex8HexNAc5dHex | 2498,88 |
| Hex5HexNAc4dHex3 | 2101,76 | | Hex5HexNAc6dHex3 | 2507,91 |
| Hex9HexNAc3 | 2108,71 | | Hex6HexNAc6dHex2 | 2523,91 |

(continued)

| | |
|---|---|
| Hex7HexNAc6dHex | 2539,90 |
| Hex4HexNAc7dHex3 | 2548,94 |
| Hex13HexNAc2 | 2553,85 |
| Hex8HexNAc6 | 2555,90 |
| Hex5HexNAc7dHex2 | 2564,94 |
| Hex6HexNAc7dHex | 2580,93 |
| Hex6HexNAc5dHex4 | 2612,95 |
| Hex7HexNAc5dHex3 | 2628,94 |
| Hex16 | 2633,85 |
| Hex8HexNAc5dHex2 | 2644,94 |
| Hex6HexNAc6dHex3 | 2669,97 |
| Hex7HexNAc6dHex2 | 2685,96 |
| Hex5HexNAc7dHex3 | 2710,99 |
| Hex14HexNAc2 | 2715,90 |
| Hex6HexNAc7dHex2 | 2726,99 |
| Hex7HexNAc7dHex | 2742,98 |
| Hex8HexNAc7 | 2758,98 |
| Hex7Hexnac5dHex4 | 2775,00 |
| Hex8HexNAc5dHex3 | 2790,99 |
| Hex17 | 2795,90 |
| Hex7HexNAc6dHex3 | 2832,02 |
| Hex16HexNAc | 2836,92 |
| Hex9HexNAc6dHex | 2864,01 |
| Hex6HexNAc7dHex3 | 2873,05 |
| Hex15HexNAc2 | 2877,95 |
| Hex8HexNAc7dHex | 2905,04 |
| Hex8Hexnac5dHex4 | 2937,05 |
| Hex18 | 2957,95 |
| Hex7HexNAc6dHex4 | 2978,08 |
| Hex17HexNAc | 2998,98 |
| Hex8HexNAc7dHex2 | 3051,09 |
| Hex9HexNAc8 | 3124,11 |
| Hex8HexNAc6dHex4 | 3140,13 |
| Hex8HexNAc7dHex3 | 3197,15 |
| Hex9HexNAcBdHex / Hex7HexNAc6dHex6 | 3270,17 |
| Hex9HexNAc6dHex4 | 3302,18 |
| Hex8HexNAc7dHex4 | 3343,21 |
| Hex9HexNAc8dHex2 | 3418,23 |

(continued)

| | |
|---|---|
| Hex10HexNAc6dHex4 | 3464,24 |
| Hex10HexNAc9 | 3489,24 |
| Hex9HexNAc8dHex3 | 3562,28 |
| Hex11HexNAc6dHex4 | 3626,29 |
| Hex10HexNAc9dHex | 3635,30 |
| Hex9HexNAc8dHex4 | 3708,34 |
| Hex10HexNAc9dHex2 / Hex8HexNAc7dHex7 | 3781,36 |
| Hex9HexNAc8dHex5 / Hex7HexNAc6dHex10 | 3854,40 |

**Table 5.** NMR analysis of large neutral N-glycan fraction from pancreatic cancer sample. Reference glycans A-C are as in **Figure 14.**

| | | | ppm | | | |
|---|---|---|---|---|---|---|
| **Residue** | **Linkage** | **Proton** | **A** | **B** | **C** | **Sample** |
| D-GlcNAc | | H-1α | 5.188 | 5.191 | 5.187 | 5.187 |
| | | H-1β | 4.695 | 4.690 | 4.693 | 4.693 |
| | | NAc | 2.038<br>2.036 | 2.042 | 2.037 | 2.036 |
| β-D-GlcNAc | 4 | H-1 | 4.601<br>4.592 | 4.596 | 4.586 | 4.952 |
| | | NAc | 2.064<br>2.063 | 2.072 | 2.063 | 2.062 |
| β-D-Man | 4,4 | H-1 | 4.780 | 4.775 | 4.771 | under HDO |
| | | H-2 | 4.250 | 4.238 | 4.234 | 4.232/4.253 |
| α-D-Man | 6,4,4 | H-1 | 4.870 | 4.869 | 4.870 | 4.870 |
| | | H-2 | 4.145 | 4.149 | 4.149 | 4.146 |
| α-D-Man | 6,6,4,4 | H-1 | 4.907 | 5.153 | 5.151 | 4.907/5.148 |
| | | H-2 | 3.984 | 4.025 | 4.021 | 3.984 |
| α-D-Man | 2,6,6,4,4 | H-1 | - | 5.047 | 5.042 | 5.041 |
| | | H-2 | - | 4.074 | 4.069 | 4.067 |
| α-D-Man | 3,6,4,4 | H-1 | 5.092 | 5.414 | 5.085 | 5.090/5.407 |
| | | H-2 | 4.065 | 4.108 | 4.069 | 4.067/4.108 |
| α-D-Man | 2,3,6,4,4 | H-1 | - | 5.047 | - | 5.041 |
| | | H-2 | - | 4.074 | - | 4.067 |
| α-D-Man | 3,4,4 | H-1 | 5.097 | 5.343 | 5.341 | 5.090/5.345 |
| | | H-2 | 4.076 | 4.108 | 4.099 | 4.067/4.108 |
| α-D-Man | 2,3,4,4 | H-1 | - | 5.317 | 5.309 | 5.308 |
| | | H-2 | - | 4.108 | 4.099 | 4.108 |
| α-D-Man | 2,2,3,4,4 | H-1 | - | 5.047 | 5.042 | 5.041 |
| | | H-2 | - | 4.074 | 4.069 | 4.067 |

**Table 6.** NMR analysis of small N-glycan fraction from pancreatic cancer sample. Reference glycans D-G are as in **Figure 15.**

| | | | ppm | |
|---|---|---|---|---|
| **Residue** | **Linkage** | **Proton** | **D** | **Sample** |
| D-GlcNAc | | H-1α | 5.188 | 5.188 |
| | | H-1β | 4.696 | under HDO |
| | | NAc | 2.038 | 2.037 |
| β-D-GlcNAc | 4 | H-1α | 4.612 | 4.606 |
| | | H-1β | 4.603 | |
| | | NAc | 2.078 | 2.078 |
| β-D-Man | 4,4 | H-1 | 4.780 | under HDO |
| | | H-2 | 4.254 | 4.250/4.231 |
| α-D-Man | 6,4,4 | H-1 | 4.915 | 4.915 |
| | | H-2 | 3.974 | 3.970 |
| α-D-Man | 3,4,4 | H-1 | 5.101 | 5.100/5.350 |
| | | H-2 | 4.067 | 4.067/4.103/4.082 |

| | | | ppm | | | |
|---|---|---|---|---|---|---|
| **Residue** | **Linkage** | **Proton** | **E** | **F** | **G** | **Sample** |
| β-D-GlcNAc | | H-1α | 5.213 | 5.212 | 5.212 | 5.210 |
| | | H-1β | 4.722 | 4.721 | 4.72 | under HDO |
| | | Nacα | 2.057 | 2.057 | 2.057 | |
| | | Nacβ | 2.054 | 2.054 | 2.054 | 2.056 |
| β-D-Man | 4 | H-1(α) | 4.791 | 4.781 | 4.780 | under HDO under HDO |
| | | H-1(β) | 4.783 | 4.774 | 4.773 | |
| | | H-2(α) | 4.265 | 4.246 | 4.244 | 4.250 |
| | | H-2(β) | 4.254 | 4.238 | 4.235 | 4.231 |
| α-D-Man | 6,4 | H-1 | 4.917 | 4.918 | 4.917 | 4.915 |
| | | H-2 | 3.973 | 3.977 | 3.98 | 3.970 |
| α-D-Man | 3,4 | H-1 | 5.104 | 5.352 | 5.345 | 5.100/5.350 |
| | | H-2 | 4.070 | 4.105 | 4.080 | 4.067/4.103/4.082 |
| α-D-Man | 2,3,4 | H-1 | - | 5.051 | 5.303 | 5.046/5.304 |
| | | H-2 | - | 4.069 | 4.105 | 4.067/4.101 |
| α-D-Man | 2,2,3,4 | H-1 | - | - | 5.043 | 5.046 |
| | | H-2 | - | - | 4.064 | 4.067 |

**Table 7.**

| $Hex_{5-9}HexNAc_2$ (including high-mannose type N-glycans) Proposed composition | m/z | Human tissue | Human cell line |
|---|---|---|---|
| Hex5HexNAc2 | 1257 | + | + |
| Hex6HexNAc2 | 1419 | + | + |
| Hex7HexNAc2 | 1581 | + | + |
| Hex8HexNAc2 | 1743 | + | + |
| Hex9HexNAc2 | 1905 | + | + |

| $Hex_{1-4}HexNAc_2dHex_{0-1}$ (including low-mannose type N-glycans) Proposed composition | m/z | Human tissue | Human cell line |
|---|---|---|---|
| HexHexNAc2 | 609 | + | |
| HexHexNAc2dHex | 755 | + | |
| Hex2HexNAc2 | 771 | + | + |
| Hex2HexNAc2dHex | 917 | + | + |
| Hex3HexNAc2 | 933 | + | + |
| Hex3HexNAc2dHex | 1079 | + | + |
| Hex4HexNAc2 | 1095 | + | + |
| Hex4HexNAc2dHex | 1241 | + | + |

| $Hex_{10-12}HexNAc_2$ (including glucosylated high-mannose type N-glycans) Proposed composition | m/z | Human cells | Human cell line |
|---|---|---|---|
| Hex10HexNAc2 | 2067 | + | + |
| Hex11HexNAc2 | 2229 | + | |
| Hex12HexNAc2 | 2391 | + | |

| $Hex_{5-9}HexNAc_2dHex_1$ (including fucosylated high-mannose type N-glycans) Proposed composition | m/z | Human tissue | Human cell line |
|---|---|---|---|
| Hex5HexNAc2dHex | 1403 | + | + |
| Hex6HexNAc2dHex | 1565 | + | + |

| HexNAc=3 and Hex≥2 (including hybrid-type and monoantennary N-glycans) Proposed composition | m/z | Human cells | Human cell line |
|---|---|---|---|
| Hex2HexNAc3 | 974 | + | |
| Hex2HexNAc3dHex | 1120 | + | |
| Hex3HexNAc3 | 1136 | + | + |
| Hex2HexNAc3dHex2 | 1266 | + | |
| Hex3HexNAc3dHex | 1282 | + | + |
| Hex4HexNAc3 | 1298 | + | + |
| Hex3HexNAc3dHex2 | 1428 | + | |
| Hex4HexNAc3dHex | 1444 | + | + |
| Hex5HexNAc3 | 1460 | + | + |
| Hex4HexNAc3dHex2 | 1590 | + | + |
| Hex5HexNAc3dHex | 1606 | + | + |
| Hex6HexNAc3 | 1622 | + | + |
| Hex5HexNAc3dHex2 | 1752 | + | + |
| Hex6HexNAc3dHex | 1768 | + | + |
| Hex7HexNAc3 | 1784 | + | + |
| Hex7HexNAc3dHex | 1930 | + | + |
| Hex8HexNAc3 | 1946 | + | |

| HexNAc≥4 and Hex≥3 (including complex-type N-glycans) Proposed composition | m/z | Human cells | Human cell line |
|---|---|---|---|
| Hex3HexNAc4 | 1339 | + | + |
| Hex3HexNAc4dHex | 1485 | + | + |
| Hex4HexNAc4 | 1501 | + | + |
| Hex3HexNAc5 | 1542 | + | + |
| Hex4HexNAc4dHex | 1647 | + | + |
| Hex5HexNAc4 | 1663 | + | + |
| Hex3HexNAc5dHex | 1688 | + | + |
| Hex4HexNAx5 | 1704 | + | + |
| Hex4HexNAc4dHex2 | 1793 | + | + |
| Hex5HexNAc4dHex | 1809 | + | + |
| Hex6HexNAc4 | 1825 | + | + |
| Hex4HexNAc5dHex | 1850 | + | + |
| Hex5HexNAc5 | 1866 | + | |
| Hex3HexNAc6dHex | 1891 | + | + |
| Hex5HexNAc4dHex2 | 1955 | + | + |
| Hex6HexNAc4dHex | 1971 | + | + |
| Hex7HexNAc4 | 1987 | + | + |
| Hex4HexNAc5dHex2 | 1996 | + | + |
| Hex5HexNAc5dHex | 2012 | + | |
| Hex6HexNAc5 | 2028 | + | + |
| Hex5HexNAc4dHex3 | 2101 | + | + |
| Hex6HexNAc4dHex2 | 2117 | + | |
| Hex7HexNAc4dHex | 2133 | + | + |
| Hex4HexNAc5dHex3 | 2142 | + | |
| Hex8HexNAc4 | 2149 | + | + |
| Hex5HexNAc5dHex2 | 2158 | + | |
| Hex6HexNAc5dHex | 2174 | + | + |
| Hex7HexNAc5 | 2190 | + | + |
| Hex5HexNAc6dHex | 2215 | + | + |
| Hex6HexNAc6 | 2231 | + | |
| Hex5HexNAc4dHex4 | 2247 | + | + |
| Hex7HexNAc4dHex2 | 2279 | + | |
| Hex5HexNAc5dHex3 | 2304 | + | + |
| Hex6HexNAc5dHex2 | 2320 | + | + |
| Hex7HexNAc5dHex | 2336 | + | |
| Hex8HexNAc5 | 2352 | + | + |
| Hex7HexNAc6 | 2393 | + | + |
| Hex7HexNAc4dHex3 | 2425 | + | |
| Hex6HexNAc5dHex3 | 2466 | + | |
| Hex8HexNAc5dHex | 2498 | + | |
| Hex7HexNAc6dHex | 2539 | + | + |
| Hex6HexNAc5dHex4 | 2612 | + | + |
| Hex8HexNAc7 | 2758 | + | |
| Hex7Hexnac5dHex4 | 2775 | + | + |
| Hex8HexNAc5dHex4 | 2937 | + | + |
| Hex8HexNAc6dHex4 | 3140 | + | + |
| Hex9HexNAc6dHex4 | 3302 | + | + |
| Hex10HexNAc6dHex4 | 3464 | + | + |
| Hex11HexNAc6dHex4 | 3626 | + | + |

| $Hex_{1-9}HexNAc_1$ (including soluble glycans) Proposed composition | m/z | Human cells | Human cell line |
|---|---|---|---|
| Hex2HexNAc | 568 | + | |
| Hex3HexNAc | 730 | + | + |
| Hex4HexNAc | 892 | + | |
| Hex5HexNAc | 1054 | + | + |
| Hex6HexNAc | 1216 | + | + |
| Hex7HexNAc | 1378 | + | + |
| Hex8HexNAc | 1540 | + | + |
| Hex9HexNAc | 1702 | + | |

| **HexNAc≥3 and dHex≥1** (including fucosylated hybrid/monoant. N-glycans) Proposed composition | m/z | Human cells | Human cell line |
|---|---|---|---|
| Hex2HexNAc3dHex | 1120 | + | |
| Hex2HexNAc3dHex2 | 1266 | + | |
| Hex3HexNAc3dHex | 1282 | + | + |
| Hex3HexNAc3dHex2 | 1428 | + | |
| Hex4HexNAc3dHex | 1444 | + | + |
| Hex4HexNAc3dHex2 | 1590 | + | + |
| Hex5HexNAc3dHex | 1606 | + | + |
| Hex5HexNAc3dHex2 | 1752 | + | + |
| Hex6HexNAc3dHex | 1768 | + | + |
| Hex7HexNAc3dHex | 1930 | + | + |
| Hex3HexNAc4dHex | 1485 | + | + |
| Hex4HexNAc4dHex | 1647 | + | + |
| Hex3HexNAc5dHex | 1688 | + | + |
| Hex4HexNAc4dHex2 | 1793 | + | + |
| Hex5HexNAc4dHex | 1809 | + | + |
| Hex4HexNAc5dHex | 1850 | + | + |
| Hex3HexNAc6dHex | 1891 | + | + |
| Hex5HexNAc4dHex2 | 1955 | + | + |
| Hex6HexNAc4dHex | 1971 | + | + |
| Hex4HexNAc5dHex2 | 1996 | + | + |
| Hex5HexNAc5dHex | 2012 | + | |
| Hex5HexNAc4dHex3 | 2101 | + | + |
| Hex6HexNAc4dHex2 | 2117 | + | |
| Hex7HexNAc4dHex | 2133 | + | + |
| Hex4HexNAc5dHex3 | 2142 | + | |
| Hex5HexNAc5dHex2 | 2158 | + | |
| Hex6HexNAc5dHex | 2174 | + | + |
| Hex5HexNAc6dHex | 2215 | + | + |
| Hex5HexNAc4dHex4 | 2247 | + | + |
| Hex7HexNAc4dHex2 | 2279 | + | |
| Hex5HexNAc5dHex3 | 2304 | + | + |
| Hex6HexNAc5dHex2 | 2320 | + | + |
| Hex7HexNAc5dHex | 2336 | + | |
| Hex7HexNAc4dHex3 | 2425 | + | |
| Hex6HexNAc5dHex3 | 2466 | + | |
| Hex8HexNAc5dHex | 2498 | + | |
| Hex7HexNAc6dHex | 2539 | + | + |
| Hex6HexNAc5dHex4 | 2612 | + | + |
| Hex7Hexnac5dHex4 | 2775 | + | + |
| Hex8HexNAc5dHex4 | 2937 | + | + |
| Hex8HexNAc6dHex4 | 3140 | + | + |
| Hex9HexNAc6dHex4 | 3302 | + | + |
| Hex10HexNAc6dHex4 | 3464 | + | + |
| Hex11HexNAc6dHex4 | 3626 | + | + |

| **HexNAc=Hex≥5** (terminal HexNAc, N=H) Proposed composition | m/z | Human cells | Human cell line |
|---|---|---|---|
| Hex5HexNAc5 | 1866 | + | |
| Hex5HexNAc5dHex | 2012 | + | |
| Hex5HexNAc5dHex2 | 2158 | + | |
| Hex6HexNAc6 | 2231 | + | |

| **HexNAc≥3 and dHex≥2** (including multifucosylated hybrid/monoant. N-glycans) Proposed composition | m/z | Human cells | Human cell line |
|---|---|---|---|
| Hex2HexNAc3dHex2 | 1266 | + | |
| Hex3HexNAc3dHex2 | 1428 | + | |
| Hex4HexNAc3dHex2 | 1590 | + | + |
| Hex5HexNAc3dHex2 | 1752 | + | + |
| Hex4HexNAc4dHex2 | 1793 | + | + |
| Hex5HexNAc4dHex2 | 1955 | + | + |
| Hex4HexNAc5dHex2 | 1996 | + | + |
| Hex5HexNAc4dHex3 | 2101 | + | + |
| Hex6HexNAc4dHex2 | 2117 | + | |
| Hex4HexNAc5dHex3 | 2142 | + | |
| Hex5HexNAc5dHex2 | 2158 | + | |
| Hex5HexNAc4dHex4 | 2247 | + | + |
| Hex7HexNAc4dHex2 | 2279 | + | |
| Hex5HexNAc5dHex3 | 2304 | + | + |
| Hex6HexNAc5dHex2 | 2320 | + | + |
| Hex7HexNAc4dHex3 | 2425 | + | |
| Hex6HexNAc5dHex3 | 2466 | + | |
| Hex6HexNAc5dHex4 | 2612 | + | + |
| Hex7Hexnac5dHex4 | 2775 | + | + |
| Hex8HexNAc5dHex4 | 2937 | + | + |
| Hex8HexNAc6dHex4 | 3140 | + | + |
| Hex9HexNAc6dHex4 | 3302 | + | + |
| Hex10HexNAc6dHex4 | 3464 | + | + |
| Hex11HexNAc6dHex4 | 3626 | + | + |

| **HexNAc>Hex≥2** (terminal HexNAc, N>H) Proposed composition | m/z | Human cells | Human cell line |
|---|---|---|---|
| Hex2HexNAc3 | 974 | + | |
| Hex2HexNAc3dHex | 1120 | + | |
| Hex2HexNAc3dHex2 | 1266 | + | |
| Hex3HexNAc4 | 1339 | + | + |
| Hex3HexNAc4dHex | 1485 | + | + |
| Hex3HexNAc5 | 1542 | + | + |
| Hex3HexNAc5dHex | 1688 | + | + |
| Hex4HexNAx5 | 1704 | + | + |
| Hex4HexNAc5dHex | 1850 | + | + |
| Hex3HexNAc6dHex | 1891 | + | + |
| Hex4HexNAc5dHex2 | 1996 | + | + |
| Hex4HexNAc5dHex3 | 2142 | + | |
| Hex5HexNAc6dHex | 2215 | + | + |

**Table 8.**

| HexNAc=3 and Hex≥2 (including hybrid-type and monoantennary N-glycans) Proposed composition | m/z | Human cells | Human cell line |
|---|---|---|---|
| Hex3HexNAc3SP | 1192 | + | + |
| Hex3HexNAc3dHexSP | 1338 | + | + |
| Hex4HexNAc3SP | 1354 | + | + |
| NeuAcHex3HexNAc3 | 1403 | + | + |
| NeuGcHex3HexNAc3 | 1419 | + | |
| Hex4HexNAc3dHexSP | 1500 | + | + |
| Hex5HexNAc3SP | 1516 | + | + |
| NeuAcHex3HexNAc3dHex | 1549 | + | + |
| NeuAcHex3HexNAc3SP2 | 1563 | + | |
| NeuAcHex4HexNAc3 | 1565 | + | + |
| NeuGcHex4HexNAc3 | 1581 | + | + |
| Hex4HexNAc3dHex2SP | 1646 | + | |
| Hex5HexNAc3dHexSP | 1662 | + | + |
| Hex6HexNAc3SP and/or NeuAc2Hex2HexNAc3dHex | 1678 | + | + |
| NeuAc2Hex3HexNAc3 | 1694 | + | + |
| NeuAcHex3HexNAc3dHexSP2 | 1709 | + | |
| NeuAcHex4HexNAc3dHex | 1711 | + | + |
| NeuAcHex5HexNAc3 and/or NeuGcHex4HexNAc3dHex | 1727 | + | + |
| NeuGcHex5HexNAc3 | 1743 | + | + |
| NeuAcHex4HexNAc3dHexSP | 1791 | + | |
| Hex5HexNAc3dHex2SP | 1808 | + | |
| Hex6NexNAc3dHexSP | 1824 | + | + |
| NeuAc2Hex3HexNAc3dHex | 1840 | + | + |
| NeuAc2Hex4HexNAc3 | 1856 | + | |
| NeuAcHex4HexNAc3dHex2 | 1857 | + | |
| NeuAcHex5HexNAc3dHex and/or NeuGcHex4HexNAc3dHex2 | 1873 | + | + |
| NeuAcHex5HexNAc3SP2 | 1887 | + | |
| NeuAcHex6HexNAc3 | 1889 | + | + |
| Hex8HexNAc3SP and/or NeuAc2Hex4HexNAc3dHex | 2002 | + | + |
| NeuAcHex4HexNAc3dHex3 | 2003 | + | |
| NeuAc2Hex5HexNAc3 and/or NeuGcNeuAcHex4HexNAc3dHex | 2018 | + | + |
| NeuAcHex5HexNAc3dHex2 | 2019 | + | + |
| NeuGcNeuAcHex5HexNAc3 and/or NeuGc2Hex4HexNAc3dHex | 2034 | + | |
| NeuAcHex6HexNAc3dHex | 2035 | + | + |
| NeuGc2Hex5HexNAc3 | 2050 | + | |
| NeuAcHex7HexNAc3 | 2051 | + | + |
| NeuAc2Hex4HexNAc3dHexSP and/or Hex8HexNAc3SP2 | 2082 | + | |
| NeuAcHex6HexNAc3dHexSP | 2115 | + | |
| Hex8HexNAc3dHexSP and/or NeuAc2Hex4HexNAc3dHex2 | 2148 | + | |
| NeuAc2Hex5HexNAc3dHex and/or Hex6HexNAc5SP2 | 2164 | + | + |
| NeuAcHex5HexNAc3dHex3 | 2165 | + | + |
| NeuAcHex8HexNAc3SP and/or NeuAc3Hex4HexNAc3dHex | 2293 | + | |
| NeuAc2Hex5HexNAc3dHex2 and/or NeuGcNeuAcHex4HexNAc3dHex3 | 2310 | + | |
| NeuAc3Hex5HexNAc3SP | 2389 | + | |
| NeuAc2Hex5HexNAc3dHex2SP | 2390 | + | + |
| NeuAc2Hex6HexNAc3dHexSP | 2406 | + | |
| NeuAcHex8HexNAc3dHexSP and/or NeuAc3Hex4HexNAc3dHex2 | 2439 | + | |
| NeuAcHex9HexNAc3dHex | 2521 | + | |

| HexNAc≥4 and Hex≥3 (including complex-type N-glycans) Proposed composition | m/z | Human cells | Human cell line |
|---|---|---|---|
| Hex4HexNAc4SP | 1557 | + | + |
| NeuAcHex3HexNAc4 | 1606 | + | |
| Hex4HexNAc4SP2 | 1637 | + | |
| Hex4HexNAc4dHexSP | 1703 | + | + |
| Hex4HexNAc4SP3 and/or Hex7HexNAc2SP2 | 1717 | + | |
| Hex5HexNAc4SP | 1719 | + | + |
| NeuAcHex4HexNAc4 | 1768 | + | + |
| NeuGcHex4HexNac4 | 1784 | + | |
| Hex5HexNAc4SP2 and/or Hex8HexNAc2SP | 1799 | + | |
| NeuAcHex3HexNac5 | 1809 | + | |
| NeuGcHex3HexNAc5 | 1825 | + | + |
| Hex5HexNAc4dHexSP | 1865 | + | + |
| Hex6HexNAc4SP | 1881 | + | + |
| Hex4HexNAc5dHexSP | 1906 | + | |
| NeuAcHex4HexNAc4dHex | 1914 | + | + |
| NeuAcHex4HexNAc4SP2 | 1928 | + | |
| NeuAcHex5HexNAc4 | 1930 | + | + |
| NeuGcHex5HexNAc4 | 1946 | + | + |
| NeuAcHex4HexNAc5 | 1971 | + | |
| NeuAcHex5HexNAc4Ac | 1972 | + | |
| Hex5HexNAc5SP2 | 2002 | + | |
| NeuAcHex5HexNAc4SP | 2010 | + | + |
| Hex5HexNAc4dHex2SP | 2011 | + | |
| NeuGcHex5HexNAc4SP | 2026 | + | |
| Hex6HexNAc4dHexSP | 2027 | + | + |
| Hex7HexNAc4SP and/or Hex4HexNAc6SP2 and/or NeuAc2Hex3HexNAc4dHex | 2043 | + | |
| NeuAcHex4HexNAc5SP | 2051 | + | |
| Hex4HexNAc5dHex2SP | 2052 | + | |
| NeuAc2Hex4HexNAc4 | 2059 | + | |
| NeuAcHex4HexNAc4dHex2 | 2060 | + | + |
| NeuAcHex4HexNAc4dHexSP2 | 2074 | + | |
| NeuAcHex5HexNAc4dHex | 2076 | + | + |
| NeuAcHex6HexNAc4 and/or NeuGcHex5HexNAc4dHex | 2092 | + | + |
| NeuAcHex3HexNAc5dHex2 and/or NeuAc2Hex4HexNAc4Ac | 2101 | + | |
| NeuGcHex6HexNAc4 | 2108 | + | |
| NeuAcHex4HexNAc5dHex | 2117 | + | |
| Hex4HexNAc5dHex2SP2 | 2132 | + | |
| NeuAcHex5HexNAc5 | 2133 | + | + |
| NeuAc2Hex4HexNAc4SP | 2139 | + | |
| NeuAcHex5HexNAc4dHexSP | 2156 | + | + |
| Hex5HexNAc4dHex3SP | 2157 | + | |
| Hex6HexNAc5SP2 | 2164 | + | |
| NeuAcHex6HexNAc4SP and/or NeuGcHex5HexNAc4dHexSP | 2172 | + | + |
| Hex6HexNAc4dHex2SP and/or Hex3HexNAc6dHex2SP2 | 2173 | + | |
| NeuAcHex4HexNAc6 | 2174 | + | |
| NeuAc3Hex3HexNAc4 and/or NeuGcHex6HexNAc4SP and/or NeuAc2NeuGcHex2HexNAc4dHex | 2188 | + | |
| NeuAc2Hex3HexNAc4dHex2 and/or Hex7HexNAc4dHexSP and/or Hex4HexNAc6dHexSP2 | 2189 | + | + |
| NeuAcHex3HexNAc4dHex4 | 2190 | + | + |
| Hex4HexNAc5dHex3SP | 2198 | + | + |
| NeuAc2Hex4HexNAc4dHex | 2205 | + | |
| NeuAc2Hex4HexNAc4SP2 | 2219 | + | |
| NeuAc2Hex5HexNAc4 | 2221 | + | + |
| NeuAcHex5HexNAc4dHex2 | 2222 | + | + |
| Hex6HexNAc5dHexSP | 2230 | + | |
| NeuGcNeuAcHex5HexNAc4 | 2237 | + | |
| NeuAcHex6HexNAc4dHex and/or NeuGcHex5HexNAc4dHex2 | 2238 | + | + |
| NeuAc2Hex3HexNAc5dHex and/or Hex7HexNAc5SP | 2246 | + | |

| | | | |
|---|---|---|---|
| NeuGc2Hex5HexNAc4 | 2253 | + | |
| NeuAcHex7HexNAc4 and/or NeuGcHex6HexNAc4dHex | 2254 | + | + |
| NeuAc2Hex4HexNAc5 | 2262 | + | |
| NeuAcHex4HexNAc5dHex2 and/or NeuAc2Hex5HexNAc4Ac | 2263 | + | |
| Hex5HexNAc6dHexSP | 2271 | + | |
| NeuAcHex5HexNAc5dHex | 2279 | + | + |
| NeuAc2Hex4HexNAc4dHexSP and/or Hex11HexNAc2SP | 2285 | + | |
| NeuAcHex6HexNAc5 | 2295 | + | + |
| NeuAc2Hex5HexNAc4SP | 2301 | + | |
| NeuAcHex5HexNAc4dHex2SP | 2302 | + | |
| NeuAc2Hex5HexNAc4Ac2 | 2305 | + | |
| NeuAcHex6HexNAc4dHexSP | 2318 | + | + |
| Hex6HexNAc4dHex3SP and/or NeuGcNeuAcHex3HexNAc6 | 2319 | + | |
| NeuAcHex4HexNAc6dHex | 2320 | + | |
| NeuAcHex5HexNAc5dHexAc | 2321 | + | |
| Hex7HexNAc4dHex2SP and/or Hex4HexNAc6dHex2SP2 | 2335 | + | |
| NeuAcHex5HexNAc6 | 2336 | + | + |
| NeuAc3Hex4HexNac4 | 2350 | + | |
| NeuAc2Hex4HexNAc4dHexSP | 2365 | + | |
| NeuAc2Hex5HexNAc4dHex | 2367 | + | + |
| NeuAcHex5HexNAc4dHex3 | 2368 | + | + |
| NeuAc2Hex6HexNAc4 and/or NeuGcNeuAcHex5HexNAc4dHex | 2383 | + | + |
| NeuAcHex6HexNAc4dHex2 and/or NeuGcHex5HexNAc4dHex3 | 2384 | + | + |
| NeuAc2Hex3HexNAc5dHex2 and/or Hex7HexNAc5dHexSP | 2392 | + | + |
| NeuAcHex3HexNAc5dHex4 | 2393 | + | |
| NeuGc2Hex5HexNAc4dHex | 2399 | + | |
| NeuAcHex4HexNAc6dHexSP and/or NeuGcHex6HexNAc4dHex2 and/or NeuAcHex7HexNAc4dHex | 2400 | + | |
| Hex4HexNAc6dHex3SP | 2401 | + | |
| NeuAc2Hex4HexNAc5dHex | 2408 | + | |
| NeuAcHex4HexNAc5dHex3 and/or NeuAc2Hex5HexNAc4dHexAc | 2409 | + | |
| NeuAc2Hex5HexNAc5 | 2424 | + | |
| NeuAcHex5HexNAc5dHex2 | 2425 | + | + |
| NeuAcHex6HexNAc5dHex | 2441 | + | + |
| NeuAc2Hex5HexNAc4dHexSP | 2447 | + | + |
| NeuAcHex5HexNAc4dHex3SP | 2448 | + | |
| NeuAcHex7HexNAc5 and/or NeuGcHex6HexNAc5dHex | 2457 | + | + |
| NeuGcHex7HexNAc5 | 2473 | + | |
| NeuAcHex5HexNAc6dHex | 2482 | + | |
| NeuAcHex4HexNAc5dHex3SP | 2489 | + | |
| Hex6HexNAc7SP | 2490 | + | |
| NeuAc3Hex5HexNAc4 | 2512 | + | |
| NeuAc2Hex5HexNAc4dHex2 | 2513 | + | + |
| NeuAcHex5HexNAc4dHex4 | 2514 | + | |
| NeuAcHex6HexNAc5dHexSP and/or NeuAc3Hex2HexNAc5dHex2 | 2521 | + | |
| Hex6HexNAc5dHex3SP | 2522 | + | |
| NeuGcNeuAc2Hex5HexNAc4 | 2528 | + | |
| NeuAc2Hex6HexNAc4dHex and/or NeuGcNeuAcHex5HexNAc4dHex2 | 2529 | + | |
| NeuGc2NeuAcHex5HexNAc4 | 2544 | + | |
| NeuGc2Hex5HexNAc4dHex2 and/or NeuGcNeuAcHex6HexNAc4dHex | 2545 | + | |
| NeuGc3Hex5HexNAc4 | 2560 | + | |
| NeuGc2Hex6HexNAc4dHex | 2561 | + | |
| NeuAc2Hex5HexNAc5dHex | 2570 | + | + |
| NeuAcHex5HexNAc5dHex3 | 2571 | + | |
| NeuAc2Hex6HexNAc5 | 2586 | + | + |
| NeuAcHex6HexNAc5dHex2 | 2587 | + | + |
| Hex7HexNAc6dHexSP | 2595 | + | |
| NeuGcNeuAcHex6HexNAc5 | 2602 | + | |
| NeuAcHex7HexNAc5dHex and/or NeuGcHex6HexNAc5dHex2 | 2603 | + | + |
| NeuAcHex8HexNAc5 and/or NeuGcHex7HexNAc5dHex | 2619 | + | |
| NeuAc2Hex5HexNAc6 | 2627 | + | |

| | | | |
|---|---|---|---|
| NeuGcHex8HexNAc5 and/or NeuAcHex4HexNAc5dHex4SP | 2635 | + | |
| NeuAcHex6HexNAc6dHex | 2644 | + | + |
| NeuAc2Hex5HexNAc4dHex3 | 2659 | + | |
| NeuAcHex7HexNAc6 | 2660 | + | + |
| NeuGcNeuAc2Hex5HexNAc4dHex and/or NeuAc3Hex6HexNAc4 | 2674 | + | |
| NeuAc2Hex4HexNAc5dHex2SP2 | 2714 | + | |
| NeuAcHex4HexNAc5dHex4SP2 and/or NeuAc3Hex5HexNAc5 | 2715 | + | + |
| NeuAc2Hex5HexNAc5dHex2 | 2716 | + | |
| NeuAc2Hex6HexNAc5dHex | 2732 | + | + |
| NeuAcHex6HexNAc5dHex3 | 2733 | + | + |
| NeuGcNeuAcHex6HexNAc5dHex | 2748 | + | |
| NeuAcHex8HexNAc5dHex | 2765 | + | + |
| NeuGcHex8HexNAc5dHex and/or NeuAcHex9HexNAc5 | 2781 | + | |
| NeuAcHex6HexNAc6dHex2 | 2791 | + | |
| NeuAc3Hex5HexNAc4dHex2 and/or NeuAcHex6HexNAc6dHexSP2 | 2804 | + | |
| Hex6HexNAc6dHex3SP2 | 2805 | + | |
| NeuAcHex7HexNAc6dHex | 2807 | + | + |
| NeuAc2Hex6HexNAc5dHexSP | 2812 | + | |
| NeuAcHex6HexNAc5dHex3SP | 2813 | + | |
| NeuGcNeuAc3Hex5HexNAc4 | 2819 | + | |
| NeuAc3Hex6HexNAc4dHex and/or NeuGcNeuAc2Hex5HexNAc4dHex2 | 2820 | + | |
| NeuAc3Hex6HexNAc5 | 2878 | + | + |
| NeuAc2Hex6HexNAc5dHex2 | 2879 | + | + |
| NeuAcHex6HexNAc5dHex4 | 2880 | + | + |
| NeuGcNeuAc2Hex6HexNAc5 | 2894 | + | |
| NeuAc2Hex7HexNAc5dHex and/or NeuGcNeuAcHex6HexNAc5dHex2 | 2895 | + | + |
| NeuAc3Hex6HexNAc4dHexSP and/or NeuGcNeuAc2Hex5HexNAc4dHex2SP | 2900 | + | |
| NeuGc2Hex6HexNAc5dHex2 | 2911 | + | |
| NeuAc2Hex5HexNAc6dHex2 | 2920 | + | |
| NeuGc3Hex6HexNAc5 | 2925 | + | |
| NeuGcNeuAc2Hex5HexNAc6 | 2935 | + | |
| NeuAc2Hex6HexNAc6dHex and/or NeuGcNeuAcHex5HexNAc6dHex2 | 2936 | + | |
| NeuAcHex6HexNAc6dHex3 | 2937 | + | |
| NeuGc2NeuAcHex5HexNAc6 and/or NeuAc3Hex5HexNAc4dHex3 | 2951 | + | |
| NeuAc2Hex7HexNAc6 | 2952 | + | |
| NeuAcHex7HexNAc6dHex2 | 2953 | + | + |
| Hex8HexNAc7dHexSP | 2961 | + | |
| NeuAc2Hex4HexNAc7dHex2 | 2961 | + | |
| NeuAcHex7HexNAc7dHex | 3010 | + | |
| NeuAc3Hex6HexNAc5dHex | 3024 | + | + |
| NeuAc2Hex6HexNAc5dHex3 | 3025 | + | + |
| NeuAcHex8HexNAc7 | 3026 | + | |
| NeuGc3Hex6HexNAc5dHex and/or NeuGc2NeuAcHex7HexNAc5 | 3072 | + | |
| NeuAc3Hex6HexNAc6 | 3081 | + | + |
| NeuAc2Hex6HexNAc6dHex2 | 3082 | + | |
| NeuAc2Hex7HexNAc6dHex | 3098 | + | + |
| NeuAcHex7HexNAc6dHex3 | 3099 | + | + |
| NeuAc3Hex6HexNAc5dHexSP | 3104 | + | |
| NeuAc2Hex6HexNAc5dHex3SP | 3105 | + | |
| NeuAc3Hex6HexNAc5dHex2 | 3170 | + | |
| NeuAc2Hex6HexNAc5dHex4 | 3171 | + | |
| NeuAcHex8HexNAc7dHex | 3172 | + | |
| NeuAc3Hex6HexNAc6dHex | 3227 | + | |
| NeuAc2Hex6HexNAc6dHex3 | 3228 | + | |
| NeuAc3Hex7HexNAc6 | 3243 | + | |
| NeuAc2Hex7HexNAc6dHex2 | 3244 | + | + |
| NeuAcHex7HexNAc6dHex4 | 3245 | + | + |
| NeuAc2Hex7HexNAc7dHex | 3301 | + | |
| NeuAcHex7HexNAc7dHex3 | 3302 | + | |
| NeuAc2Hex8HexNAc7 | 3317 | + | |
| NeuAcHex8HexNAc7dHex2 | 3318 | + | |
| NeuAc3Hex7HexNAc6dHex | 3389 | + | + |
| NeuAc2Hex7HexNAc6dHex3 | 3390 | + | + |
| NeuAcHex7HexNAc6dHex5 and/or NeuAcHex9HexNAc8 | 3391 | + | |
| NeuAc2Hex8HexNAc7dHex | 3463 | + | |

| | | | |
|---|---|---|---|
| NeuAcHex8HexNAc7dHex3 | 3464 | + | |
| NeuAc2Hex7HexNAc6dHex4 | 3536 | + | |
| NeuAcHex9HexNAc8dHex | 3537 | + | |
| NeuAc3Hex8HexNAc7 | 3608 | + | |
| NeuAc2Hex8HexNac7dHex2 | 3609 | + | |
| NeuAcHex8HexNac7dHex4 | 3610 | + | |
| NeuAc4Hex7HexNAc6dHex | 3680 | + | |
| NeuAc3Hex7HexNAc6dHex3 | 3681 | + | |
| NeuAc2Hex9HexNAc8 | 3682 | + | |
| NeuAcHex9HexNAc8dHex2 | 3683 | + | |
| NeuAc3Hex8HexNAc7dHex | 3754 | + | |
| NeuAc2Hex8HexNAc7dHex3 | 3755 | + | |
| NeuAcHex10HexNAc9 and/or NeuAcHex8HexNAc7dHex5 | 3756 | + | |
| NeuAc4Hex6HexNAc8 | 3778 | + | |
| NeuAc3Hex7HexNAc6dHex4 | 3827 | + | |
| NeuAc2Hex9HexNAc8dHex | 3828 | + | |
| NeuAcHex9HexNAc8dHex3 | 3829 | + | |
| NeuAc2Hex8HexNAc7dHex4 | 3901 | + | |
| NeuAc2Hex9HexNAc8dHex2 | 3974 | + | |
| NeuAcHex9HexNAc8dHex4 | 3975 | + | |
| NeuAc4Hex8HexNAc7dHex | 4045 | + | |
| NeuAc3Hex8HexNAc7dHex3 | 4046 | + | |
| NeuAc2Hex10HexNAc9 and/or NeuAc2Hex8HexNAc7dHex5 | 4047 | + | |
| NeuAc3Hex9HexNAc8dHex | 4119 | + | |
| NeuAc2Hex9HexNAc8dHex3 | 4120 | + | |

| **HexNAc≥3 and dHex≥1 (including fucosylated N-glycans)**<br>**Proposed composition** | **m/z** | **Human cells** | **Human cell line** |
|---|---|---|---|
| Hex3HexNAc3dHexSP | 1338 | + | + |
| Hex4HexNAc3dHexSP | 1500 | + | + |
| NeuAcHex3HexNAc3dHex | 1549 | + | + |
| Hex4HexNAc3dHex2SP | 1646 | + | |
| Hex5HexNAc3dHexSP | 1662 | + | + |
| Hex6HexNAc3SP and/or NeuAc2Hex2HexNAc3dHex | 1678 | + | + |
| NeuAcHex3HexNAc3dHexSP2 | 1709 | + | |
| NeuAcHex4HexNAc3dHex | 1711 | + | + |
| NeuAcHex5HexNAc3 and/or NeuGcHex4HexNAc3dHex | 1727 | + | + |
| NeuAcHex4HexNAc3dHexSP | 1791 | + | |
| Hex5HexNAc3dHex2SP | 1808 | + | |
| Hex6NexNAc3dHexSP | 1824 | + | + |
| NeuAc2Hex3HexNAc3dHex | 1840 | + | + |
| NeuAcHex4HexNAc3dHex2 | 1857 | + | |
| NeuAcHex5HexNAc3dHex and/or NeuGcHex4HexNAc3dHex2 | 1873 | + | + |
| Hex8HexNAc3SP and/or NeuAc2Hex4HexNAc3dHex | 2002 | + | + |
| NeuAcHex4HexNAc3dHex3 | 2003 | + | |
| NeuAc2Hex5HexNAc3 and/or NeuGcNeuAcHex4HexNAc3dHex | 2018 | + | + |
| NeuAcHex5HexNAc3dHex2 | 2019 | + | + |
| NeuGcNeuAcHex5HexNAc3 and/or NeuGc2Hex4HexNAc3dHex | 2034 | + | |
| NeuAcHex6HexNAc3dHex | 2035 | + | + |
| NeuAc2Hex4HexNAc3dHexSP and/or Hex8HexNAc3SP2 | 2082 | + | |
| NeuAcHex6HexNAc3dHexSP | 2115 | + | |
| Hex8HexNAc3dHexSP and/or NeuAc2Hex4HexNAc3dHex2 | 2148 | + | |
| NeuAc2Hex5HexNAc3dHex and/or Hex6HexNAc5SP2 | 2164 | + | + |
| NeuAcHex5HexNAc3dHex3 | 2165 | + | + |
| NeuAcHex8HexNAc3SP and/or NeuAc3Hex4HexNAc3dHex | 2293 | + | |
| NeuAc2Hex5HexNAc3dHex2 and/or NeuGcNeuAcHex4HexNAc3dHex3 | 2310 | + | |
| NeuAc2Hex5HexNAc3dHex2SP | 2390 | + | + |
| NeuAc2Hex6HexNAc3dHexSP | 2406 | + | |
| NeuAcHex8HexNAc3dHexSP and/or NeuAc3Hex4HexNAc3dHex2 | 2439 | + | |
| NeuAcHex9HexNAc3dHex | 2521 | + | |
| Hex4HexNAc4dHexSP | 1703 | + | + |
| Hex5HexNAc4dHexSP | 1865 | + | + |
| Hex4HexNAc5dHexSP | 1906 | + | |
| NeuAcHex4HexNAc4dHex | 1914 | + | + |
| Hex5HexNAc4dHex2SP | 2011 | + | |
| Hex6HexNAc4dHexSP | 2027 | + | + |
| Hex7HexNAc4SP and/or Hex4HexNAc6SP2 and/or NeuAc2Hex3HexNAc4dHex | 2043 | + | |
| Hex4HexNAc5dHex2SP | 2052 | + | |
| NeuAcHex4HexNAc4dHex2 | 2060 | + | + |
| NeuAcHex4HexNAc4dHexSP2 | 2074 | + | |
| NeuAcHex5HexNAc4dHex | 2076 | + | + |
| NeuAcHex6HexNAc4 and/or NeuGcHex5HexNAc4dHex | 2092 | + | + |
| NeuAcHex3HexNAc5dHex2 and/or NeuAc2Hex4HexNAc4Ac | 2101 | + | |
| NeuAcHex4HexNAc5dHex | 2117 | + | |
| Hex4HexNAc5dHex2SP2 | 2132 | + | |
| NeuAcHex5HexNAc4dHexSP | 2156 | + | + |
| Hex5HexNAc4dHex3SP | 2157 | + | |
| NeuAcHex6HexNAc4SP and/or NeuGcHex5HexNAc4dHexSP | 2172 | + | + |
| Hex6HexNAc4dHex2SP and/or Hex3HexNAc6dHex2SP2 | 2173 | + | |
| NeuAc3Hex3HexNAc4 and/or NeuGcHex6HexNAc4SP and/or NeuAc2NeuGcHex2HexNAc4dHex | 2188 | + | |

| | | | |
|---|---|---|---|
| NeuAc2Hex3HexNAc4dHex2 and/or Hex7HexNAc4dHexSP and/or Hex4HexNAc6dHexSP2 | 2189 | + | + |
| NeuAcHex3HexNAc4dHex4 | 2190 | + | + |
| Hex4HexNAc5dHex3SP | 2198 | + | + |
| NeuAc2Hex4HexNAc4dHex | 2205 | + | |
| NeuAcHex5HexNAc4dHex2 | 2222 | + | + |
| Hex6HexNAc5dHexSP | 2230 | + | |
| NeuAcHex6HexNAc4dHex and/or NeuGcHex5HexNAc4dHex2 | 2238 | + | + |
| NeuAc2Hex3HexNAc5dHex and/or Hex7HexNAc5SP | 2246 | + | |
| NeuAcHex7HexNAc4 and/or NeuGcHex6HexNAc4dHex | 2254 | + | + |
| NeuAcHex4HexNAc5dHex2 and/or NeuAc2Hex5HexNAc4Ac | 2263 | + | |
| Hex5HexNAc6dHexSP | 2271 | + | |
| NeuAcHex5HexNAc5dHex | 2279 | + | + |
| NeuAc2Hex4HexNAc4dHexSP and/or Hex11HexNAc2SP | 2285 | + | |
| NeuAcHex5HexNAc4dHex2SP | 2302 | + | |
| NeuAcHex6HexNAc4dHexSP | 2318 | + | + |
| Hex6HexNAc4dHex3SP and/or NeuGcNeuAcHex3HexNAc6 | 2319 | + | |
| NeuAcHex4HexNAc6dHex | 2320 | + | |
| NeuAcHex5HexNAc5dHexAc | 2321 | + | |
| Hex7HexNAc4dHex2SP and/or Hex4HexNAc6dHex2SP2 | 2335 | + | |
| NeuAc2Hex4HexNAc4dHexSP | 2365 | + | |
| NeuAc2Hex5HexNAc4dHex | 2367 | + | + |
| NeuAcHex5HexNAc4dHex3 | 2368 | + | + |
| NeuAc2Hex6HexNAc4 and/or NeuGcNeuAcHex5HexNAc4dHex | 2383 | + | + |
| NeuAcHex6HexNAc4dHex2 and/or NeuGcHex5HexNAc4dHex3 | 2384 | + | + |
| NeuAc2Hex3HexNAc5dHex2 and/or Hex7HexNAc5dHexSP | 2392 | + | + |
| NeuAcHex3HexNAc5dHex4 | 2393 | + | |
| NeuGc2Hex5HexNAc4dHex | 2399 | + | |
| NeuAcHex4HexNAc6dHexSP and/or NeuGcHex6HexNAc4dHex2 and/or NeuAcHex7HexNAc4dHex | 2400 | + | |
| Hex4HexNAc6dHex3SP | 2401 | + | |
| NeuAc2Hex4HexNAc5dHex | 2408 | + | |
| NeuAcHex4HexNAc5dHex3 and/or NeuAc2Hex5HexNAc4dHexAc | 2409 | + | |
| NeuAcHex5HexNAc5dHex2 | 2425 | + | + |
| NeuAcHex6HexNAc5dHex | 2441 | + | + |
| NeuAc2Hex5HexNAc4dHexSP | 2447 | + | + |
| NeuAcHex5HexNAc4dHex3SP | 2448 | + | |
| NeuAcHex7HexNAc5 and/or NeuGcHex6HexNAc5dHex | 2457 | + | + |
| NeuAcHex5HexNAc6dHex | 2482 | + | |
| NeuAcHex4HexNAc5dHex3SP | 2489 | + | |
| NeuAc2Hex5HexNAc4dHex2 | 2513 | + | + |
| NeuAcHex5HexNAc4dHex4 | 2514 | + | |
| NeuAcHex6HexNAc5dHexSP and/or NeuAc3Hex2HexNAc5dHex2 | 2521 | + | |
| Hex6HexNAc5dHex3SP | 2522 | + | |
| NeuAc2Hex6HexNAc4dHex and/or NeuGcNeuAcHex5HexNAc4dHex2 | 2529 | + | |
| NeuGc2Hex5HexNAc4dHex2 and/or NeuGcNeuAcHex6HexNAc4dHex | 2545 | + | |
| NeuGc2Hex6HexNAc4dHex | 2561 | + | |
| NeuAc2Hex5HexNAc5dHex | 2570 | + | + |
| NeuAcHex5HexNAc5dHex3 | 2571 | + | |
| NeuAcHex6HexNAc5dHex2 | 2587 | + | + |
| Hex7HexNAc6dHexSP | 2595 | + | |
| NeuAcHex7HexNAc5dHex and/or NeuGcHex6HexNAc5dHex2 | 2603 | + | + |
| NeuAcHex8HexNAc5 and/or NeuGcHex7HexNAc5dHex | 2619 | + | |
| NeuGcHex8HexNAc5 and/or NeuAcHex4HexNAc5dHex4SP | 2635 | + | |
| NeuAcHex6HexNAc6dHex | 2644 | + | + |
| NeuAc2Hex5HexNAc4dHex3 | 2659 | + | |
| NeuGcNeuAc2Hex5HexNAc4dHex | 2674 | + | |

| | | | |
|---|---|---|---|
| and/or NeuAc3Hex6HexNAc4 | | | |
| NeuAc2Hex4HexNAc5dHex2SP2 | 2714 | + | |
| NeuAcHex4HexNAc5dHex4SP2 and/or NeuAc3Hex5HexNAc5 | 2715 | + | + |
| NeuAc2Hex5HexNAc5dHex2 | 2716 | + | |
| NeuAc2Hex6HexNAc5dHex | 2732 | + | + |
| NeuAcHex6HexNAc5dHex3 | 2733 | + | + |
| NeuGcNeuAcHex6HexNAc5dHex | 2748 | + | |
| NeuAcHex8HexNAc5dHex | 2765 | + | + |
| NeuGcHex8HexNAc5dHex and/or NeuAcHex9HexNAc5 | 2781 | + | |
| NeuAcHex6HexNAc6dHex2 | 2791 | + | |
| NeuAc3Hex5HexNAc4dHex2 and/or NeuAcHex6HexNAc6dHexSP2 | 2804 | + | |
| Hex6HexNAc6dHex3SP2 | 2805 | + | |
| NeuAcHex7HexNAc6dHex | 2807 | + | + |
| NeuAc2Hex6HexNAc5dHexSP | 2812 | + | |
| NeuAcHex6HexNAc5dHex3SP | 2813 | + | |
| NeuAc3Hex6HexNAc4dHex and/or NeuGcNeuAc2Hex5HexNAc4dHex2 | 2820 | + | |
| NeuAc2Hex6HexNAc5dHex2 | 2879 | + | + |
| NeuAcHex6HexNAc5dHex4 | 2880 | + | + |
| NeuAc2Hex7HexNAc5dHex and/or NeuGcNeuAcHex6HexNAc5dHex2 | 2895 | + | + |
| NeuAc3Hex6HexNAc4dHexSP and/or NeuGcNeuAc2Hex5HexNAc4dHex2SP | 2900 | + | |
| NeuGc2Hex6HexNAc5dHex2 | 2911 | + | |
| NeuAc2Hex5HexNAc6dHex2 | 2920 | + | |
| NeuAc2Hex6HexNAc6dHex and/or NeuGcNeuAcHex5HexNAc6dHex2 | 2936 | + | |
| NeuAcHex6HexNAc6dHex3 | 2937 | + | |
| NeuGc2NeuAcHex5HexNAc6 and/or NeuAc3Hex5HexNAc4dHex3 | 2951 | + | |
| NeuAcHex7HexNAc6dHex2 | 2953 | + | + |
| Hex8HexNAc7dHexSP | 2961 | + | |
| NeuAc2Hex4HexNAc7dHex2 | 2961 | + | |
| NeuAcHex7HexNAc7dHex | 3010 | + | |
| NeuAc3Hex6HexNAc5dHex | 3024 | + | + |
| NeuAc2Hex6HexNAc5dHex3 | 3025 | + | + |
| NeuGc3Hex6HexNAc5dHex and/or NeuGc2NeuAcHex7HexNAc5 | 3072 | + | |
| NeuAc2Hex6HexNAc6dHex2 | 3082 | + | |
| NeuAc2Hex7HexNAc6dHex | 3098 | + | + |
| NeuAcHex7HexNAc6dHex3 | 3099 | + | + |
| NeuAc3Hex6HexNAc5dHexSP | 3104 | + | |
| NeuAc2Hex6HexNAc5dHex3SP | 3105 | + | |
| NeuAc3Hex6HexNAc5dHex2 | 3170 | + | |
| NeuAc2Hex6HexNAc5dHex4 | 3171 | + | |
| NeuAcHex8HexNAc7dHex | 3172 | + | |
| NeuAc3Hex6HexNAc6dHex | 3227 | + | |
| NeuAc2Hex6HexNAc6dHex3 | 3228 | + | |
| NeuAc2Hex7HexNAc6dHex2 | 3244 | + | + |
| NeuAcHex7HexNAc6dHex4 | 3245 | + | + |
| NeuAc2Hex7HexNAc7dHex | 3301 | + | |
| NeuAcHex7HexNAc7dHex3 | 3302 | + | |
| NeuAcHex8HexNAc7dHex2 | 3318 | + | |
| NeuAc3Hex7HexNAc6dHex | 3389 | + | + |
| NeuAc2Hex7HexNAc6dHex3 | 3390 | + | + |
| NeuAcHex7HexNAc6dHex5 and/or NeuAcHex9HexNAc8 | 3391 | + | |
| NeuAc2Hex8HexNAc7dHex | 3463 | + | |
| NeuAcHex8HexNAc7dHex3 | 3464 | + | |
| NeuAc2Hex7HexNAc6dHex4 | 3536 | + | |
| NeuAcHex9HexNAc8dHex | 3537 | + | |
| NeuAc2Hex8HexNac7dHex2 | 3609 | + | |
| NeuAcHex8HexNac7dHex4 | 3610 | + | |
| NeuAc4Hex7HexNAc6dHex | 3680 | + | |
| NeuAc3Hex7HexNAc6dHex3 | 3681 | + | |
| NeuAcHex9HexNAc8dHex2 | 3683 | + | |
| NeuAc3Hex8HexNAc7dHex | 3754 | + | |
| NeuAc2Hex8HexNAc7dHex3 | 3755 | + | |
| NeuAcHex10HexNAc9 and/or NeuAcHex8HexNAc7dHex5 | 3756 | + | |
| NeuAc3Hex7HexNAc6dHex4 | 3827 | + | |
| NeuAc2Hex9HexNAc8dHex | 3828 | + | |
| NeuAcHex9HexNAc8dHex3 | 3829 | + | |
| NeuAc2Hex8HexNAc7dHex4 | 3901 | + | |

| | | | |
|---|---|---|---|
| NeuAc2Hex9HexNAc8dHex2 | 3974 | + | |
| NeuAcHex9HexNAc8dHex4 | 3975 | + | |
| NeuAc4Hex8HexNAc7dHex | 4045 | + | |
| NeuAc3Hex8HexNAc7dHex3 | 4046 | + | |
| NeuAc2Hex10HexNAc9 and/or NeuAc2Hex8HexNAc7dHex5 | 4047 | + | |
| NeuAc3Hex9HexNAc8dHex | 4119 | + | |
| NeuAc2Hex9HexNAc8dHex3 | 4120 | + | |

| **HexNAc≥3 and dHex≥2** (including multifucosylated N-glycans)<br>**Proposed composition** | **m/z** | **Human cells** | **Human cell line** |
|---|---|---|---|
| Hex4HexNAc3dHex2SP | 1646 | + | |
| NeuAcHex3HexNAc3dHexSP2 | 1709 | + | |
| Hex5HexNAc3dHex2SP | 1808 | + | |
| NeuAcHex4HexNAc3dHex2 | 1857 | + | |
| NeuAcHex5HexNAc3dHex and/or NeuGcHex4HexNAc3dHex2 | 1873 | + | + |
| NeuAcHex4HexNAc3dHex3 | 2003 | + | |
| NeuAcHex5HexNAc3dHex2 | 2019 | + | + |
| Hex8HexNAc3dHexSP and/or NeuAc2Hex4HexNAc3dHex2 | 2148 | + | |
| NeuAcHex5HexNAc3dHex3 | 2165 | + | + |
| NeuAc2Hex5HexNAc3dHex2 and/or NeuGcNeuAcHex4HexNAc3dHex3 | 2310 | + | |
| NeuAc2Hex5HexNAc3dHex2SP | 2390 | + | + |
| NeuAcHex8HexNAc3dHexSP and/or NeuAc3Hex4HexNAc3dHex2 | 2439 | + | |
| Hex5HexNAc4dHex2SP | 2011 | + | |
| Hex4HexNAc5dHex2SP | 2052 | + | |
| NeuAcHex4HexNAc4dHex2 | 2060 | + | + |
| NeuAcHex3HexNAc5dHex2 and/or NeuAc2Hex4HexNAc4Ac | 2101 | + | |
| Hex4HexNAc5dHex2SP2 | 2132 | + | |
| Hex5HexNAc4dHex3SP | 2157 | + | |
| NeuAcHex6HexNAc4SP and/or NeuGcHex5HexNAc4dHexSP | 2172 | + | + |
| Hex6HexNAc4dHex2SP and/or Hex3HexNAc6dHex2SP2 | 2173 | + | |
| NeuAc2Hex3HexNAc4dHex2 and/or Hex7HexNAc4dHexSP and/or Hex4HexNAc6dHexSP2 | 2189 | + | + |
| NeuAcHex3HexNAc4dHex4 | 2190 | + | + |
| Hex4HexNAc5dHex3SP | 2198 | + | + |
| NeuAcHex5HexNAc4dHex2 | 2222 | + | + |
| NeuAcHex6HexNAc4dHex and/or NeuGcHex5HexNAc4dHex2 | 2238 | + | + |
| NeuAcHex4HexNAc5dHex2 and/or NeuAc2Hex5HexNAc4Ac | 2263 | + | |
| NeuAc2Hex4HexNAc4dHexSP and/or Hex11HexNAc2SP | 2285 | + | |
| NeuAcHex5HexNAc4dHex2SP | 2302 | + | |
| Hex6HexNAc4dHex3SP and/or NeuGcNeuAcHex3HexNAc6 | 2319 | + | |
| Hex7HexNAc4dHex2SP and/or Hex4HexNAc6dHex2SP2 | 2335 | + | |
| NeuAc2Hex4HexNAc4dHexSP | 2365 | + | |
| NeuAcHex5HexNAc4dHex3 | 2368 | + | + |
| NeuAcHex6HexNAc4dHex2 and/or NeuGcHex5HexNAc4dHex3 | 2384 | + | + |
| NeuAc2Hex3HexNAc5dHex2 and/or Hex7HexNAc5dHexSP | 2392 | + | + |
| NeuAcHex3HexNAc5dHex4 | 2393 | + | |
| NeuAcHex4HexNAc6dHexSP and/or NeuGcHex6HexNAc4dHex2 and/or NeuAcHex7HexNAc4dHex | 2400 | + | |
| Hex4HexNAc6dHex3SP | 2401 | + | |
| NeuAcHex4HexNAc5dHex3 and/or NeuAc2Hex5HexNAc4dHexAc | 2409 | + | |
| NeuAcHex5HexNAc5dHex2 | 2425 | + | + |
| NeuAcHex5HexNAc4dHex3SP | 2448 | + | |
| NeuAcHex4HexNAc5dHex3SP | 2489 | + | |
| NeuAc2Hex5HexNAc4dHex2 | 2513 | + | + |
| NeuAcHex5HexNAc4dHex4 | 2514 | + | |
| NeuAcHex6HexNAc5dHexSP and/or NeuAc3Hex2HexNAc5dHex2 | 2521 | + | |
| Hex6HexNAc5dHex3SP | 2522 | + | |
| NeuAc2Hex6HexNAc4dHex and/or NeuGcNeuAcHex5HexNAc4dHex2 | 2529 | + | |
| NeuGc2Hex5HexNAc4dHex2 and/or NeuGcNeuAcHex6HexNAc4dHex | 2545 | + | |
| NeuAcHex5HexNAc5dHex3 | 2571 | + | |
| NeuAcHex6HexNAc5dHex2 | 2587 | + | + |
| NeuAcHex7HexNAc5dHex and/or NeuGcHex6HexNAc5dHex2 | 2603 | + | + |
| NeuGcHex8HexNAc5 and/or NeuAcHex4HexNAc5dHex4SP | 2635 | + | |
| NeuAc2Hex5HexNAc4dHex3 | 2659 | + | |
| NeuAc2Hex4HexNAc5dHex2SP2 | 2714 | + | |
| NeuAcHex4HexNAc5dHex4SP2 and/or NeuAc3Hex5HexNAc5 | 2715 | + | + |
| NeuAc2Hex5HexNAc5dHex2 | 2716 | + | |
| NeuAcHex6HexNAc5dHex3 | 2733 | + | + |
| NeuAcHex6HexNAc6dHex2 | 2791 | + | |
| NeuAc3Hex5HexNAc4dHex2 and/or NeuAcHex6HexNAc6dHexSP2 | 2804 | + | |
| Hex6HexNAc6dHex3SP2 | 2805 | + | |
| NeuAcHex6HexNAc5dHex3SP | 2813 | + | |
| NeuAc3Hex6HexNAc4dHex and/or NeuGcNeuAc2Hex5HexNAc4dHex2 | 2820 | + | |
| NeuAc2Hex6HexNAc5dHex2 | 2879 | + | + |
| NeuAcHex6HexNAc5dHex4 | 2880 | + | + |
| NeuAc2Hex7HexNAc5dHex and/or NeuGcNeuAcHex6HexNAc5dHex2 | 2895 | + | + |
| NeuAc3Hex6HexNAc4dHexSP and/or NeuGcNeuAc2Hex5HexNAc4dHex2SP | 2900 | + | |
| NeuGc2Hex6HexNAc5dHex2 | 2911 | + | |
| NeuAc2Hex5HexNAc6dHex2 | 2920 | + | |
| NeuAc2Hex6HexNAc6dHex and/or NeuGcNeuAcHex5HexNAc6dHex2 | 2936 | + | |
| NeuAcHex6HexNAc6dHex3 | 2937 | + | |
| NeuGc2NeuAcHex5HexNAc6 and/or NeuAc3Hex5HexNAc4dHex3 | 2951 | + | |
| NeuAcHex7HexNAc6dHex2 | 2953 | + | + |
| NeuAc2Hex4HexNAc7dHex2 | 2961 | + | |
| NeuAc2Hex6HexNAc5dHex3 | 3025 | + | + |
| NeuAc2Hex6HexNAc6dHex2 | 3082 | + | |
| NeuAcHex7HexNAc6dHex3 | 3099 | + | + |
| NeuAc3Hex6HexNAc5dHexSP | 3104 | + | |
| NeuAc2Hex6HexNAc5dHex3SP | 3105 | + | |
| NeuAc3Hex6HexNAc5dHex2 | 3170 | + | |
| NeuAc2Hex6HexNAc5dHex4 | 3171 | + | |
| NeuAc2Hex6HexNAc6dHex3 | 3228 | + | |
| NeuAc2Hex7HexNAc6dHex2 | 3244 | + | + |
| NeuAcHex7HexNAc6dHex4 | 3245 | + | + |
| NeuAcHex7HexNAc7dHex3 | 3302 | + | |
| NeuAcHex8HexNAc7dHex2 | 3318 | + | |
| NeuAc2Hex7HexNAc6dHex3 | 3390 | + | + |
| NeuAcHex7HexNAc6dHex5 and/or NeuAcHex9HexNAc8 | 3391 | + | |
| NeuAcHex8HexNAc7dHex3 | 3464 | + | |
| NeuAc2Hex7HexNAc6dHex4 | 3536 | + | |
| NeuAc2Hex8HexNac7dHex2 | 3609 | + | |
| NeuAcHex8HexNac7dHex4 | 3610 | + | |
| NeuAc3Hex7HexNAc6dHex3 | 3681 | + | |
| NeuAcHex9HexNAc8dHex2 | 3683 | + | |
| NeuAc2Hex8HexNAc7dHex3 | 3755 | + | |
| NeuAcHex10HexNAc9 and/or NeuAcHex8HexNAc7dHex5 | 3756 | + | |
| NeuAc3Hex7HexNAc6dHex4 | 3827 | + | |
| NeuAcHex9HexNAc8dHex3 | 3829 | + | |
| NeuAc2Hex8HexNAc7dHex4 | 3901 | + | |
| NeuAc2Hex9HexNAc8dHex2 | 3974 | + | |
| NeuAcHex9HexNAc8dHex4 | 3975 | + | |
| NeuAc3Hex8HexNAc7dHex3 | 4046 | + | |
| NeuAc2Hex10HexNAc9 and/or NeuAc2Hex8HexNAc7dHex5 | 4047 | + | |
| NeuAc2Hex9HexNAc8dHex3 | 4120 | + | |

| HexNAc>Hex≥3 (terminal HexNAc, N>H) Proposed composition | m/z | Human cells | Human cell line |
|---|---|---|---|
| Hex6HexNAc3SP and/or NeuAc2Hex2HexNAc3dHex | 1678 | + | + |
| NeuAcHex3HexNAc4 | 1606 | + | |
| NeuAcHex3HexNac5 | 1809 | + | |
| NeuGcHex3HexNAc5 | 1825 | + | + |
| Hex4HexNAc5dHexSP | 1906 | + | |
| NeuAcHex4HexNAc5 | 1971 | + | |
| Hex7HexNAc4SP and/or Hex4HexNAc6SP2 and/or NeuAc2Hex3HexNAc4dHex | 2043 | + | |
| NeuAcHex4HexNAc5SP | 2051 | + | |
| Hex4HexNAc5dHex2SP | 2052 | + | |
| NeuAcHex3HexNAc5dHex2 and/or NeuAc2Hex4HexNAc4Ac | 2101 | + | |
| NeuAcHex4HexNAc5dHex | 2117 | + | |
| Hex4HexNAc5dHex2SP2 | 2132 | + | |
| Hex6HexNAc4dHex2SP and/or Hex3HexNAc6dHex2SP2 | 2173 | + | |
| NeuAcHex4HexNAc6 | 2174 | + | |
| NeuAc3Hex3HexNAc4 and/or NeuGcHex6HexNAc4SP and/or NeuAc2NeuGcHex2HexNAc4dHex | 2188 | + | |
| NeuAc2Hex3HexNAc4dHex2 and/or Hex7HexNAc4dHexSP and/or Hex4HexNAc6dHexSP2 | 2189 | + | + |
| NeuAcHex3HexNAc4dHex4 | 2190 | + | + |
| Hex4HexNAc5dHex3SP | 2198 | + | + |
| NeuAc2Hex4HexNAc5 | 2262 | + | |
| NeuAcHex4HexNAc5dHex2 and/or NeuAc2Hex5HexNAc4Ac | 2263 | + | |
| Hex5HexNAc6dHexSP | 2271 | + | |
| NeuAcHex4HexNAc6dHex | 2320 | + | |
| Hex7HexNAc4dHex2SP and/or Hex4HexNAc6dHex2SP2 | 2335 | + | |
| NeuAcHex5HexNAc6 | 2336 | + | + |
| NeuAc2Hex3HexNAc5dHex2 and/or Hex7HexNAc5dHexSP | 2392 | + | + |
| NeuAcHex3HexNAc5dHex4 | 2393 | + | |
| NeuAcHex4HexNAc6dHexSP and/or NeuGcHex6HexNAc4dHex2 and/or NeuAcHex7HexNAc4dHex | 2400 | + | |
| Hex4HexNAc6dHex3SP | 2401 | + | |
| NeuAc2Hex4HexNAc5dHex | 2408 | + | |
| NeuAcHex4HexNAc5dHex3 and/or NeuAc2Hex5HexNAc4dHexAc | 2409 | + | |
| NeuAcHex5HexNAc6dHex | 2482 | + | |
| NeuAcHex4HexNAc5dHex3SP | 2489 | + | |
| Hex6HexNAc7SP | 2490 | + | |
| NeuGc3Hex5HexNAc4 | 2560 | + | |
| NeuAc2Hex5HexNAc6 | 2627 | + | |
| NeuAc2Hex4HexNAc5dHex2SP2 | 2714 | + | |
| NeuAcHex4HexNAc5dHex4SP2 and/or NeuAc3Hex5HexNAc5 | 2715 | + | + |
| NeuAc2Hex5HexNAc6dHex2 | 2920 | + | |
| NeuGcNeuAc2Hex5HexNAc6 | 2935 | + | |
| NeuAc2Hex4HexNAc7dHex2 | 2961 | + | |
| NeuAc4Hex6HexNAc8 | 3778 | + | |

| HexNAc=Hex≥5 (terminal HexNAc, N=H) Proposed composition | m/z | Human cells | Human cell line |
|---|---|---|---|
| Hex5HexNAc5SP2 | 2002 | + | |
| NeuAcHex5HexNAc5 | 2133 | + | + |
| NeuAcHex5HexNAc5dHex | 2279 | + | + |
| NeuAcHex5HexNAc5dHexAc | 2321 | + | |
| NeuAc2Hex5HexNAc5 | 2424 | + | |
| NeuAcHex5HexNAc5dHex2 | 2425 | + | + |
| NeuAc2Hex5HexNAc5dHex | 2570 | + | + |
| NeuAcHex5HexNAc5dHex3 | 2571 | + | |
| NeuAcHex6HexNAc6dHex | 2644 | + | + |
| NeuAcHex4HexNAc5dHex4SP2 and/or NeuAc3Hex5HexNAc5 | 2715 | + | + |
| NeuAc2Hex5HexNAc5dHex2 | 2716 | + | |
| NeuAcHex6HexNAc6dHex2 | 2791 | + | |
| Hex6HexNAc6dHex3SP2 | 2805 | + | |
| NeuAc2Hex6HexNAc6dHex and/or NeuGcNeuAcHex5HexNAc6dHex2 | 2936 | + | |
| NeuAcHex6HexNAc6dHex3 | 2937 | + | |
| NeuAcHex7HexNAc7dHex | 3010 | + | |
| NeuAc3Hex6HexNAc6 | 3081 | + | + |
| NeuAc2Hex6HexNAc6dHex2 | 3082 | + | |
| NeuAc3Hex6HexNAc6dHex | 3227 | + | |
| NeuAc2Hex6HexNAc6dHex3 | 3228 | + | |
| NeuAc2Hex7HexNAc7dHex | 3301 | + | |
| NeuAcHex7HexNAc7dHex3 | 3302 | + | |

| SP≥1 (including sulphated and/or phosphorylated glycans) Proposed composition | m/z | Human cells | Human cell line |
|---|---|---|---|
| Hex3HexNAc2SP | 989 | + | |
| Hex3HexNAc2dHexSP | 1135 | + | |
| Hex4HexNAc2SP | 1151 | + | |
| Hex3HexNAc3SP | 1192 | + | + |
| Hex5HexNAc2SP | 1313 | + | |
| Hex3HexNAc3dHexSP . | 1338 | + | + |
| Hex4HexNAc3SP | 1354 | + | + |
| Hex5HexNAc2dHexSP | 1459 | + | + |
| Hex6HexNAc2SP | 1475 | + | |
| Hex4HexNAc3dHexSP | 1500 | + | + |
| Hex5HexNAc3SP | 1516 | + | |
| Hex6HexNAc2SP2 | 1555 | + | |
| Hex4HexNAc4SP | 1557 | + | + |
| NeuAcHex3HexNAc3SP2 | 1563 | + | |
| Hex6HexNAc2dHexSP | 1621 | + | + |
| Hex4HexNAc4SP2 and/or Hex7HexNAc2SP | 1637 | + | |
| Hex4HexNAc3dHex2SP | 1646 | + | |
| Hex5HexNAc3dHexSP | 1662 | + | + |
| Hex6HexNAc3SP | 1678 | + | |
| Hex4HexNAc4dHexSP | 1703 | + | + |
| NeuAcHex3HexNAc3dHexSP2 | 1709 | + | |
| Hex4HexNAc4SP3 and/or Hex7HexNAc2SP2 | 1717 | + | |
| Hex5HexNAc4SP | 1719 | + | + |
| Hex7HexNAc2dHexSP | 1783 | + | |
| NeuAcHex4HexNAc3dHexSP | 1791 | + | |
| Hex5HexNAc4SP2 and/or Hex8HexNAc2SP | 1799 | + | |
| Hex5HexNAc3dHex2SP | 1808 | + | |
| NeuAc2Hex5HexNAc2 and/or NeuAc2Hex2HexNAc4SP | 1815 | + | |
| Hex6NexNAc3dHexSP | 1824 | + | + |
| Hex5HexNAc4dHexSP | 1865 | + | + |
| Hex6HexNAc4SP | 1881 | + | + |
| Hex4HexNAc5dHexSP | 1906 | + | |
| NeuAcHex6HexNAc2dHexSP and/or NeuAcHex3HexNAc4dHexSP2 | 1912 | + | |
| NeuAcHex4HexNAc4SP2 | 1928 | + | |
| Hex8HexNAc3SP and/or Hex5HexNAc5SP2 and/or NeuAc2Hex4HexNAc3dHex | 2002 | + | + |
| NeuAcHex5HexNAc4SP | 2010 | + | + |
| Hex5HexNAc4dHex2SP | 2011 | + | |
| NeuGcHex5HexNAc4SP | 2026 | + | |
| Hex6HexNAc4dHexSP | 2027 | + | + |
| Hex7HexNAc4SP and/or Hex4HexNAc6SP2 and/or NeuAc2Hex3HexNAc4dHex | 2043 | + | |
| NeuAcHex7HexNAc3 and/or NeuAcHex4HexNAc5SP | 2051 | + | + |
| Hex4HexNAc5dHex2SP | 2052 | + | |
| NeuAcHex4HexNAc4dHexSP2 | 2074 | + | |
| NeuAc2Hex4HexNAc3dHexSP and/or Hex8HexNAc3SP2 and/or Hex5HexNAc5SP3 | 2082 | + | |
| NeuAcHex6HexNAc3dHexSP | 2115 | + | |
| Hex8HexNAc3dHexSP and/or NeuAc2Hex4HexNAc3dHex2 | 2148 | + | |
| NeuAcHex5HexNAc4dHexSP and/or NeuAcHex8HexNAc2dHex | 2156 | + | + |
| Hex5HexNAc4dHex3SP | 2157 | + | |
| NeuAc2Hex5HexNAc3dHex and/or Hex6HexNAc5SP2 | 2164 | + | + |
| NeuAcHex6HexNAc4SP and/or NeuGcHex5HexNAc4dHexSP and/or NeuAcHex9HexNAc2 | 2172 | + | + |
| Hex6HexNAc4dHex2SP and/or Hex3HexNAc6dHex2SP2 | 2173 | + | |
| NeuAc3Hex3HexNAc4 and/or NeuGcHex6HexNAc4SP and/or NeuAc2NeuGcHex2HexNAc4dHex | 2188 | + | |
| NeuAc2Hex3HexNAc4dHex2 and/or Hex7HexNAc4dHexSP and/or Hex4HexNAc6dHexSP2 | 2189 | + | + |
| Hex4HexNAc5dHex3SP | 2198 | + | + |
| NeuAc2Hex4HexNAc4SP2 | 2219 | + | |
| Hex6HexNAc5dHexSP | 2230 | + | |
| NeuAc2Hex3HexNAc5dHex and/or Hex7HexNAc5SP | 2246 | + | |
| NeuAc2Hex4HexNAc4dHexSP and/or Hex11HexNAc2SP | 2285 | + | |
| NeuAcHex8HexNAc3SP and/or NeuAc3Hex4HexNAc3dHex | 2293 | + | |
| NeuAc2Hex5HexNAc4SP | 2301 | + | |
| NeuAcHex5HexNAc4dHex2SP | 2302 | + | |
| NeuAcHex6HexNAc4dHexSP | 2318 | + | + |
| Hex6HexNAc4dHex3SP | 2319 | + | |
| Hex7HexNAc4dHex2SP and/or Hex4HexNAc6dHex2SP2 | 2335 | + | |
| NeuAc2Hex4HexNAc4dHexSP | 2365 | + | |
| NeuAc3Hex5HexNAc3SP and/or NeuAc2Hex5HexNAc4Ac4 | 2389 | + | |
| NeuAc2Hex5HexNAc3dHex2SP | 2390 | + | + |
| NeuAc2Hex3HexNAc5dHex2 and/or Hex7HexNAc5dHexSP | 2392 | + | + |
| NeuAcHex4HexNAc6dHexSP and/or NeuGcHex6HexNAc4dHex2 and/or NeuAcHex7HexNAc4dHex | 2400 | + | |
| NeuAc2Hex6HexNAc3dHexSP | 2406 | + | |
| NeuAcHex8HexNAc3dHexSP and/or NeuAc3Hex4HexNAc3dHex2 | 2439 | + | |
| NeuAc2Hex5HexNAc4dHexSP and/or NeuAc2Hex8HexNAc2dHex and/or Hex12HexNAc2SP | 2447 | + | + |
| NeuAcHex5HexNAc4dHex3SP and/or NeuAcHex8HexNAc2dHex3 | 2448 | + | |
| NeuAcHex7HexNAc3dHex3 and/or NeuAcHex4HexNAc5dHex3SP | 2489 | + | |
| Hex6HexNAc7SP | 2490 | + | |
| NeuAcHex6HexNAc5dHexSP and/or NeuAcHex9HexNAc3dHex and/or NeuAc3Hex2HexNAc5dHex2 | 2521 | + | |
| Hex6HexNAc5dHex3SP | 2522 | + | |
| Hex7HexNAc6dHexSP | 2595 | + | |
| NeuGcHex8HexNAc5 and/or NeuAcHex4HexNAc5dHex4SP | 2635 | + | |
| NeuAc2Hex5HexNAc5dHexSP | 2650 | + | |
| Hex7HexNAc7SP | 2652 | + | |
| Hex6HexNAc5dHex4SP | 2668 | + | |
| NeuGcHex6HexNAc5dHexSP and/or NeuAcHex7HexNAc5dHexSP | 2683 | + | |
| NeuAc2Hex4HexNAc5dHex2SP2 | 2714 | + | |
| NeuAcHex4HexNAc5dHex4SP2 and/or NeuAc3Hex5HexNAc5 | 2715 | + | |
| Hex6HexNAc6dHex3SP | 2725 | + | |
| Hex7HexNAc6dHex2SP | 2741 | + | |
| NeuAcHex6HexNAc5dHex2SP2 | 2747 | + | |
| NeuAc2Hex4HexNAc6dHex2 and/or Hex8HexNAc6dHexSP | 2757 | + | |
| Hex7HexNAc7dHexSP | 2798 | + | |
| NeuAc3Hex5HexNAc4dHex2 and/or NeuAcHex6HexNAc6dHexSP2 | 2804 | + | |
| Hex6HexNAc6dHex3SP2 | 2805 | + | |
| NeuAc2Hex6HexNAc5dHexSP | 2812 | + | |
| NeuAcHex6HexNAc5dHex3SP | 2813 | + | |
| Hex8HexNAc7SP | 2814 | + | |
| Hex6HexNAc6dHex4SP | 2871 | + | |
| NeuAcHex7HexNAc6dHexSP and/or NeuAcHex10HexNAc4dHex | 2887 | + | |
| Hex7HexNAc6dHex3SP | 2887 | + | |
| NeuAc3Hex6HexNAc4dHexSP and/or NeuGcNeuAc2Hex5HexNAc4dHex2SP | 2900 | + | |
| NeuAc3Hex4HexNAc6dHex and/or NeuAcHex8HexNAc6SP | 2903 | + | |
| Hex7HexNAc7dHex2SP | 2945 | + | |
| NeuAc2Hex6HexNAc5dHex2SP | 2958 | + | |
| NeuAcHex6HexNAc5dHex4SP | 2960 | + | |
| Hex8HexNAc7dHexSP | 2961 | + | |
| Hex8HexNAc8SP | 3018 | + | |

| | | | |
|---|---|---|---|
| Hex7HexNAc6dHex4SP | 3034 | + | |
| Hex7HexNAc7dHex3SP | 3091 | + | |
| NeuAc3Hex6HexNAc5dHexSP | 3104 | + | |
| NeuAc2Hex6HexNAc5dHex3SP | 3105 | + | |
| NeuAcHex8HexNAc7SP and/or NeuAc3Hex4HexNAc7dHex | 3106 | + | |
| Hex8HexNAc7dHex2SP and/or NeuAc2Hex4HexNAc7dHex3 | 3107 | + | |
| NeuAc2Hex7HexNAc6dHexSP | 3178 | + | |
| Hex7HexNAc7dHex4SP | 3237 | + | |
| NeuAc3Hex7HexNAc5dHexSP and/or NeuGcNeuAc2Hex6HexNAc5dHex2SP | 3266 | + | |
| NeuAc3Hex5HexNAc7dHex and/or NeuGcHex8HexNAc7dHexSP | 3268 | + | |
| NeuAc4Hex4HexNAc5dHex2SP2 | 3297 | + | |
| NeuAc3Hex4HexNAc5dHex4SP2 | 3298 | + | |
| Hex8HexNAc8dHex3SP and/or NeuAc2Hex4HexNAc8dHex4 | 3456 | + | |
| NeuAc3Hex7HexNAc6dHexSP | 3469 | + | |
| NeuAc2Hex7HexNAc6dHex3SP | 3470 | + | |

**Table 9.** Structural classification of neutral glycan fraction glycan signals isolated from **normal human** lung tissue (1. column), human **lung cancer** tissue (2. column), normal **human serum** (5. column), and a cultured **human cell line** (6. column). Acidic glycan fraction glycans analyzed as neutral desialylated glycan signals together with the corresponding neutral glycan fraction are similarly classified from the same human tissue samples (3. and 4. column, **total normal** and **total cancer**).

| **Structural features of Neutral N-glycans** | | % | | | | | |
|---|---|---|---|---|---|---|---|
| structural feature | proposed composition | normal lung | lung cancer | total normal | total cancer | human serum | human cell line |
| $Hex_{5-9}HexNAc_2$ | high-mannose | 47,0 | 46,0 | 17,8 | 22,3 | 25,7 | 53,7 |
| $Hex_{1-4}HexNAc_2dHex_{0-1}$ | low-mannose | 28,0 | 19,5 | 15,5 | 24,4 | 0,7 | 8,5 |
| $Hex_{10-12}HexNAc_2$ | high-mannose / Glc | 0,0 | 0,0 | 0,0 | 0,0 | 0,0 | 1,9 |
| $Hex_{5-6}HexNAc_2dHex_1$ | low-mannose + Fuc | 0,7 | 0,0 | 0,3 | 0,2 | 0,0 | 1,0 |
| $n_{HexNAc} = 3$ ja $n_{Hex} \geq 2$ | hybrid / monoantennary | 7,9 | 8,7 | 8,4 | 7,1 | 6,6 | 7,3 |
| $n_{HexNAc} \geq 4$ ja $n_{Hex} \geq 2$ | complex type | 15,8 | 24,4 | 57,8 | 46,0 | 66,2 | 9,3 |
| $Hex_{1-9}HexNAc$ | soluble | 0,7 | 0,5 | 0,0 | 0,0 | 0,8 | 11,3 |
| other | - | 0,0 | 0,9 | 0,2 | 0,0 | 0,0 | 6,9 |
| $n_{dHex} \geq 1$ | fucosylation | 19,4 | 33,6 | 42,8 | 34,6 | 50,5 | 13,9 |
| $n_{dHex} \geq 2$ | α2/3/4- Fuc | 0,0 | 0,8 | 0,3 | 1,1 | 0,0 | 1,3 |
| $n_{HexNAc} > n_{Hex} \geq 2$ | terminal HexNAc | 3,9 | 17,8 | 3,8 | 7,1 | 21,8 | 4,2 |
| $n_{HexNAc} = n_{Hex} \geq 3$ | terminal HexNAc | 6,9 | 8,2 | 8,2 | 5,0 | 31,4 | 1,9 |

**Table 10. N-glycan structural classification of lysosomal protein sample.**

| **Neutral N-glycan structural features:** | | |
|---|---|---|
| Glycan feature | Proposed structure | Proportion, % |
| $Hex_{5-10}HexNAc_2$ | High-mannose type / $Glc_1$ | 46 |
| $Hex_{1-4}HexNAc_2dHex_{0-1}$ | Low-mannose type | 49 |
| $n_{HexNAc} = 3$ ja $n_{Hex} \geq 2$ | Hybrid-type / Monoantennary | 2 |
| $n_{HexNAc} \geq 4$ ja $n_{Hex} \geq 2$ | Complex-type | 0,6 |
| Other | - | <3 |
| $n_{dHex} \geq 1$ | Fucosylation | 29 |
| $n_{dHex} \geq 2$ | α2/3/4-linked Fuc | 0,8 |
| $n_{HexNAc} > n_{Hex} \geq 2$ | Terminal HexNAc (N>H) | 0,2 |
| $n_{HexNAc} = n_{Hex} \geq 5$ | Terminal HexNAc (N=H) | - |
| | | |
| **Acidic N-glycan structural features:** | | |
| Glycan feature | Proposed structure | Proportion, % |
| $n_{HexNAc} = 3$ ja $n_{Hex} \geq 3$ | Hybrid-type / Monoantennary | 46 |
| $n_{HexNAc} \geq 4$ jan$_{Hex} \geq 3$ | Complex-type | 37 |
| muut | - | 17 |
| $n_{dHex} \geq 1$ | Fucosylation | 80 |
| $n_{dHex} \geq 2$ | α2/3/4-linked Fuc | 10 |
| $n_{HexNAc} > n_{Hex} \geq 2$ | Terminal HexNAc (N>H) | 0,1 |
| $n_{HexNAc} = n_{Hex} \geq 5$ | Terminal HexNAc (N=H) | 0,4 |
| + 80 Da | Sulphate or phosphate ester | 17 |

**Table 11. Identification of disease-specific glycosylation by quantitative glycome analysis.**

| Composition | m/z | Class | I | II | m/z | Class | Abs. differ. | m/z | Class | Rel. differ. |
|---|---|---|---|---|---|---|---|---|---|---|
| Hex1HexNAc2 | 609 | NL | 0,00 | 0,00 | 771 | NL | 12,8 | 1955 | NCE | new |
| Hex2HexNAc1dHex1 | 714 | NOF | 0,00 | 0,00 | 1485 | NCFT | 3,5 | 2685 | NCE | new |
| Hex3HexNAc1 | 730 | NS | 0,00 | 0,00 | 1743 | NM | 2,1 | 2905 | NCF | new |
| Hex1HexNAc2dHex1 | 755 | NLF | 2,47 | 0,00 | 1905 | NM | 1,8 | 771 | NL | 2,4 |
| Hex2HexNAc2 | 771 | NL | 5,44 | 18,25 | 1419 | NM | 1,4 | 1905 | NM | 2,2 |
| Hex2HexNAc2dHex1 | 917 | NLF | 1,81 | 2,61 | 917 | NLF | 0,8 | 1485 | NCFT | 1,3 |
| Hex3HexNAc2 | 933 | NL | 2,47 | 1,12 | 1581 | NM | 0,5 | 2394 | NC | 1,3 |
| Hex2HexNAc3 | 974 | NH-T | 0,00 | 0,00 | 1955 | NCE | 0,4 | 1743 | NM | 1,2 |
| Hex2HexNAc2dHex2 | 1063 | NOE | 0,00 | 0,00 | 2685 | NCE | 0,4 | 917 | NLF | 0,4 |
| Hex3HexNAc2dHex1 | 1079 | NLF | 1,81 | 1,12 | 2905 | NCF | 0,4 | 1419 | NM | 0,4 |
| Hex4HexNAc2 | 1095 | NL | 1,48 | 1,30 | 2539 | NCF | 0,3 | 2539 | NCF | 0,4 |
| Hex2HexNAc3dHex1 | 1120 | NHFT | 0,00 | 0,00 | 2394 | NC | 0,2 | 1581 | NM | 0,2 |
| Hex3HexNAc3 | 1136 | NH | 0,82 | 0,00 | 2175 | NCF | 0,2 | 1282 | NHF | 0,1 |
| Hex2HexNAc2dHex3 | 1209 | NOE | 0,00 | 0,00 | 1622 | NH | 0,2 | 2012 | NCFB | 0,1 |
| Hex3HexNAc2dHex2 | 1225 | NOE | 0,00 | 0,00 | 1282 | NHF | 0,1 | 1622 | NH | 0,1 |
| Hex4HexNAc2dHex1 | 1241 | NLF | 0,00 | 0,00 | 2012 | NCFB | 0,1 | 1339 | NH-T | 0,1 |
| Hex5HexNAc2 | 1257 | NM | 8,90 | 7,64 | 1339 | NH-T | 0,0 | 2320 | NCE | 0,1 |
| Hex2HexNAc3dHex2 | 1266 | NHET | 0,00 | 0,00 | 2320 | NCE | 0,0 | 2175 | NCF | 0,0 |
| Hex3HexNAc3dHex1 | 1282 | NHF | 0,82 | 0,93 | 609 | NL | 0,0 | 609 | NL | 0,0 |
| Hex4HexNAc3 | 1298 | NH | 1,48 | 1,12 | 714 | NOF | 0,0 | 714 | NOF | 0,0 |
| Hex3HexNAc4 | 1339 | NH-T | 0,33 | 0,37 | 730 | NS | 0,0 | 730 | NS | 0,0 |
| Hex5HexNAc2dHex1 | 1403 | NMF | 0,33 | 0,19 | 974 | NH-T | 0,0 | 974 | NH-T | 0,0 |
| Hex6HexNAc2 | 1419 | NM | 3,95 | 5,40 | 1063 | NOE | 0,0 | 1063 | NOE | 0,0 |
| Hex3HexNAc3dHex2 | 1428 | NHE | 0,00 | 0,00 | 1120 | NHFT | 0,0 | 1120 | NHFT | 0,0 |
| Hex4HexNAc3dHex1 | 1444 | NHF | 1,65 | 1,30 | 1209 | NOE | 0,0 | 1209 | NOE | 0,0 |
| Hex5HexNAc3 | 1460 | NH | 2,47 | 2,42 | 1225 | NOE | 0,0 | 1225 | NOE | 0,0 |
| Hex3HexNAc4dHex1 | 1485 | NCFT | 2,64 | 6,15 | 1241 | NLF | 0,0 | 1241 | NLF | 0,0 |
| Hex4HexNAc4 | 1501 | NC | 1,32 | 0,93 | 1266 | NHET | 0,0 | 1266 | NHET | 0,0 |
| Hex3HexNAc5 | 1542 | NC-T | 0,00 | 0,00 | 1428 | NHE | 0,0 | 1428 | NHE | 0,0 |
| Hex7HexNAc2 | 1581 | NM | 2,31 | 2,79 | 1542 | NC-T | 0,0 | 1542 | NC-T | 0,0 |
| Hex6HexNAc3 | 1622 | NH | 1,15 | 1,30 | 1688 | NCFT | 0,0 | 1688 | NCFT | 0,0 |
| Hex4HexNAc4dHex1 | 1647 | NCF | 3,95 | 2,23 | 2028 | NC | 0,0 | 2028 | NC | 0,0 |
| Hex5HexNAc4 | 1663 | NC | 17,63 | 13,97 | 1460 | NH | -0,1 | 1460 | NH | 0,0 |
| Hex3HexNAc5dHex1 | 1688 | NCFT | 0,00 | 0,00 | 1850 | NCFT | -0,1 | 1095 | NL | -0,1 |
| Hex4HexNAc5 | 1704 | NC-T | 0,16 | 0,00 | 1403 | NMF | -0,1 | 1257 | NM | -0,1 |
| Hex8HexNAc2 | 1743 | NM | 1,81 | 3,91 | 1704 | NC-T | -0,2 | 1850 | NCFT | -0,2 |
| Hex5HexNAc4dHex1 | 1809 | NCF | 20,59 | 11,73 | 1095 | NL | -0,2 | 1663 | NC | -0,2 |
| Hex6HexNAc4 | 1825 | NC | 2,47 | 0,56 | 1444 | NHF | -0,3 | 1444 | NHF | -0,2 |
| Hex4HexNAc5dHex1 | 1850 | NCFT | 0,66 | 0,56 | 1298 | NH | -0,4 | 1298 | NH | -0,2 |
| Hex5HexNAc5 | 1866 | NC-B | 0,49 | 0,00 | 1501 | NC | -0,4 | 1501 | NC | -0,3 |
| Hex9HexNAc2 | 1905 | NM | 0,82 | 2,61 | 1866 | NC-B | -0,5 | 1079 | NLF | -0,4 |
| Hex5HexNAc4dHex2 | 1955 | NCE | 0,00 | 0,37 | 1079 | NLF | -0,7 | 1809 | NCF | -0,4 |
| Hex5HexNAc5dHex1 | 2012 | NCFB | 0,82 | 0,93 | 1136 | NH | -0,8 | 1647 | NCF | -0,4 |
| Hex6HexNAc5 | 2028 | NC | 1,32 | 1,30 | 1257 | NM | -1,3 | 1403 | NMF | -0,4 |
| Hex6HexNAc5dHex1 | 2175 | NCF | 4,12 | 4,28 | 933 | NL | -1,4 | 933 | NL | -0,5 |
| Hex6HexNAc5dHex2 | 2320 | NCE | 0,33 | 0,37 | 1647 | NCF | -1,7 | 1825 | NC | -0,8 |
| Hex7HexNAc6 | 2394 | NC | 0,16 | 0,37 | 1825 | NC | -1,9 | 1704 | NC-T | gone |
| Hex7HexNAc6dHex1 | 2539 | NCF | 0,82 | 1,12 | 755 | NLF | -2,5 | 1866 | NC-B | gone |
| Hex7HexNAc6dHex2 | 2685 | NCE | 0,00 | 0,37 | 1663 | NC | -3,7 | 1136 | NH | gone |
| Hex8HexNAc7dHex1 | 2905 | NCF | 0,00 | 0,37 | 1809 | NCF | -8,9 | 755 | NLF | gone |

**Table 12. Detected tissue material N-linked and soluble glycome compositions.**

| **Neutral N-glycan structural features:** | | |
|---|---|---|
| Glycan feature | Proposed structure | Proportion, % |
| $Hex_{5-10}HexNAc_2$ | High-mannose type / $Glc_1$ | 10-60 |
| $Hex_{1-4}HexNAC_2dHex_{0-1}$ | Low-mannose type | 0-50 |
| $n_{HexNAc} = 3$ ja $n_{Hex} \geq 2$ | Hybrid-type / Monoantennary | 5-20 |
| $n_{HexNac} \geq 4$ ja $n_{Hex} \geq 2$ | Complex-type | 5-75 |
| $Hex_{1-9}HexNAc_1$ | Soluble | 0-10 |
| $n_{dHex} \geq 1$ | Fucosylation | 10-80 |
| $n_{dHex} \geq 2$ | α2/314-linked Fuc | 0-40 |
| $n_{HexNAc} > n_{Hex} \geq 2$ | Terminal HexNAc (N>H) | 1-30 |
| $n_{HexNAc} = n_{Hex} \geq 5$ | Terminal HexNAc (N=H) | 1-40 |
| | | |
| **Acidic N-glycan structural features:** | | all |
| Glycan feature | Proposed structure | Proportion, % |
| $n_{HexNAc} = 3$ ja $n_{Hex} \geq 3$ | Hybrid-type / Monoantennary | 5-60 |
| $n_{HexNAc} \geq 4$ ja $n_{Hex} \geq 3$ | Complex-type | 40-95 |
| $n_{dHex} \geq 1$ | Fucosylation | 20-90 |
| $n_{dHex} \geq 2$ | α2/3/4-linked Fuc | 0-50 |
| $n_{HexNac} > n_{Hex} \geq 2$ | Terminal HexNAc (N>H) | 0-40 |
| $n_{HexNAc} = n_{Hex} \geq 5$ | Terminal HexNAc, (N=H) | 0-40 |
| + 80 Da | Sulphate or phosphate ester | 0-25 |

**Table 13. Breast carcinoma acidic protein-linked glycan structural classification.**

| **Acidic N-glycan classification:**<br>Ductale type breast carcinoma, normal and tumor tissues<br>Lymph node metastasis, normal and metastatic tissues | | Proportion of acidic glycans, % | | | |
|---|---|---|---|---|---|
| Composition | Classification | **normal** | **cancer** | **LNN** | **LNM** |
| $n_{dHex} \geq 1$ | Fucosylated | 61 | 48 | 61 | 68 |
| $n_{dHex} \geq 2$ | α2/3/4-Fuc (complex fucosylation) | 1 | 8 | 6 | 7 |
| +42 Da | Acetylated | 0.3 | 0.2 | 1 | 2 |
| +80 Da | Sulfated or phosphorylated | - | 2 | 1 | 4 |

**REFERENCES**

Arap, W., et al. (1998) Science 279(5349):377-80
Brockhausen, 1. (1999) Biochim. Biophys. Acta 1473(1):67-95
Davies, et al. (1992) J. Chromatogr. 609(1-2):125-31 1
Domon, B. & Costello, C.E. (1988) Glycoconj. J. 5:397-409
Dube, D.H. & Bertozzi, C.R. (2005) Nat. Rev. Drug Discov. 4(6):477-88
Ernst, B., Hart, G.W., and Sinay, P. (eds.) (2000) Carbohydrates in chemistry and biology, ISBN 3-527-29511-9, Weiley-VHC, Weinheim.

Fernandez, L.E., et al. (2003) Expert Rev. Vaccines 2(6) :817-23
Harvey, D.J., et al. (1993) Rapid Commun. Mass Spectrom. 7(7):614-9
Holmberg, L.A. & Sandmeier, B.M. (2001) Expert Opin. Biol. Ther. 1(5) :881-91
Huang, Y., et al. (2001) Anal. Chem. 73(24):6063-9
Kurokawa, T., et al. (2002) Eur. J. Biochem. 269:5459-73
Naven, T.J. & Harvey, D.J. (1996) Rapid Commun. Mass Spectrom. 10(11):1361-6
Nyman, T.A., et al. (1998) Eur. J. Biochem. 253(2):485-93
Papac, D.I., et al. (1996) Anal. Chem. 68(18):3215-23
Packer, N.H., et al. (1998) Glycoconj. J. 15(8):737-47
Plummer, T.H. Jr. & Tarentino, A.L. (1991) Glycobiology 1(3):257-63
Powell, A.K. & Harvey, D.J. (1996) Rapid Commun. Mass Spectrom. 10(9):1027-32
(Saarinen, J., et al. (1999) Eur. J. Biochem. 259(3):829-40
Verostek et al. (2000) Anal. Biochem. 278(2):111-22
Ylönen, A., et al. (2001) Glycobiology 11(7):523-31

## Claims

**1.** Method for diagnosing human cancer comprising the step of detecting in a patient sample an increase in expression of an oligosaccharide sequence relative to a control, wherein said oligosaccharide sequence is a low mannose N-glycan consisting of $Man_{1}$-$_{4}GlcNAc_{2}$, wherein said oligosaccharide sequence comprises one or more terminal Manα residues, wherein non-reducing end terminal structures of the oligosaccharide sequence comprise at least one terminal Man-glycan epitope selected from the group consisting of: Manα3Man, Manα6Man, and Manα3(Manα6) Man.

**2.** The method according to claim 1, wherein the oligosaccharide sequence has the structure according to Formula:

$$[\text{Man}\alpha 3]_{n2}([\text{Man}\alpha 6)_{n4})[\text{Man}\alpha 6]_{n5}\{[\text{Man}\alpha 3]_{n8}\}\text{Man}\beta 4\text{GlcNac}\beta 4\text{GlcNAcy}R_2$$

wherein n2, n4, n5, n8, and m are either independently 0 or 1, with the provisio that when n5 is 0, also n2 and n4 are 0, [ ] indicates determinant either being present or absent depending on the value of n2, n4, n5, n8, { } and () indicates a branch in the structure, y is anomeric linkage structure α and/or β or linkage from derivatized anomeric carbon, and $R_2$ is reducing end hydroxyl, chemical reducing end derivative or natural asparagine N-glycoside derivative.

**3.** The method according to claim 1, wherein the detection comprises:

contacting said sample with a substance binding to said oligosaccharide sequence, and determining the presence of a combination of said substance and said sample, or
releasing the oligosaccharide structures of said sample by enzymatic or
chemical methods to form a fraction containing free oligosaccharide structures or conjugates from said sample, and
determining the presence of said oligosaccharide sequences in said fraction, or determining the relative amounts of said oligosaccharide sequences in said fraction compared to other oligosaccharide sequences present in said fraction.

**4.** The method according to claim 3, wherein said substance binding to said oligosaccharide sequence is an aptamer, a peptide or a protein.

**5.** The method according to claim 4, wherein said protein is an antibody, an enzyme, a lectin or a fragment thereof.

**6.** The method according to claim 3, wherein said sample is a blood, tissue or serum sample.

**7.** The method according to claim 3, wherein the presence of said oligosaccharide sequence is determined by the use of mass spectrometry.

**8.** A pharmaceutical composition comprising an antibody that specifically recognises the combination of terminal structures and glycan core structures of the oligosaccharide sequence as defined in claim 1 for use in the treatment of

human cancer in a patient diagnosed according to claim 1.

9. The composition according to claim 8, wherein said antibody is human antibody or humanized antibody.

10. A pharmaceutical composition consisting of the oligosaccharide sequence as defined in claim 1 or an antigenic epitope according to Formula

$$[OS - (X)_n - L- Y]_m - Z \quad (II),$$

wherein OS is an oligosaccharide sequence as defined in claim 1, Y is a non-carbohydrate spacer or a non-glycosidically linked terminal conjugate, n is 0 or 1 and X is lactosyl-, galactosyl-, N-acetyllactosaminyl, mannosyl-, $Man_2$, $Man_3$-, $Man_3GlcNAc$, $Man_4GlcNAc$, N-acetylglucosaminyl-, or N-acetylgalactosaminyl, and OS is β2-, or β4-, or β6 linked to the mannosylresidue, for use in the treatment of human cancer in a patient diagnosed according to claim 1.

## Patentansprüche

1. Verfahren zur Diagnose von menschlichem Krebs umfassend den Schritt des Nachweisens einer Erhöhung der Expression einer Oligosaccharidsequenz in einer Patientenprobe relativ zu einer Kontrolle, wobei die Oligosaccharidsequenz ein niederes Mannose-N-Glykan ist bestehend aus $Man_{1-4}GlcNAc_2$, wobei die Oligosaccharidsequenz eine oder mehrere terminale Manα-Reste umfasst, wobei nicht-reduzierende endständige Strukturen der Oligosaccharidsequenz mindestens ein terminales Man-Glykan-Epitop umfassen, ausgewählt aus der Gruppe bestehend aus: Manα3Man, Manα6Man und Manα3(Manα6)Man.

2. Verfahren gemäß Anspruch 1, wobei die Oligosaccharidsequenz die Struktur gemäß der Formel:

$$[Man\alpha3]_{n2}([Man\alpha6)_{n4})[Man\alpha6]_{n5}\{[Man\alpha3]_{n8}\}Man\beta4GlcNac\beta4GlcNAcyR_2$$

hat, wobei n2, n4, n5, n8 und m unabhängig voreinander 0 oder 1 sind, mit der Maßgabe, dass, wenn n5 0 ist, auch n2 und n4 0 sind, [ ] angibt, dass die Determinante entweder anwesend oder abwesend ist, abhängig von dem Wert von n2, n4, n5, n8, { } und () eine Verzweigung in der Struktur angeben, y eine anomere Verknüpfungsstruktur α und/oder β oder eine Verknüpfung von derivatisiertem anomerem Kohlenstoff ist, und $R_2$ reduzierendes Endhydroxyl, ein chemisches reduzierendes Endderivat oder natürliches Asparagin-N-Glycosidderivat ist.

3. Verfahren gemäß Anspruch 1, wobei der Nachweis umfasst:

Inkontaktbringen der Probe mit einer Substanz, die an die Oligosaccharidsequenz bindet, und Bestimmen der Anwesenheit einer Kombination der Substanz und der Probe, oder
Freisetzen der Oligosaccharidstrukturen aus der Probe durch enzymatische oder chemische Verfahren unter Bildung einer Fraktion, die freie Oligosaccharidstrukturen oder Konjugate von der Probe enthält, und
Bestimmen der Anwesenheit der Oligosaccharidsequenzen in der Fraktion, oder Bestimmen der relativen Mengen der Oligosaccharidsaquenzen in der Fraktion verglichen mit anderen Oligosaccharidsequenzen, die in der Fraktion anwesend sind.

4. Verfahren gemäß Anspruch 3, wobei die Substanz, die an die Oligosaccharidsequenz bindet, ein Aptamer, ein Peptid oder ein Protein ist.

5. Verfahren gemäß Anspruch 4, wobei das Protein ein Antikörper, ein Enzym, ein Lektin oder ein Fragment davon ist.

6. Verfahren gemäß Anspruch 3, wobei die Probe eine Blut-, Gewebe- oder Serumprobe ist.

7. Verfahren gemäß Anspruch 3, wobei die Anwesenheit der Oligosaccharidsequenz durch die Verwendung von Massenspektrometrie bestimmt wird.

8. Arzneimittel umfassend einen Antikörper, der spezifisch die Kombination von terminalen Strukturen und Glykankernstrukturen der Oligosaccharidsequenz wie in Anspruch 1 definiert erkennt, zur Verwendung in der Behandlung von menschlichem Krebs in einem Patienten, der gemäß Anspruch 1 diagnostiziert wurde.

9. Mittel gemäß Anspruch 8, wobei der Antikörper ein menschlicher Antikörper oder ein humanisierter Antikörper ist.

10. Arzneimittel bestehend aus der Oligosaccharidsequenz wie in Anspruch 1 definiert oder einem antigenen Epitop gemäß der Formel:

$$[OS - (X)_n - L- Y]_m - Z \qquad (II),$$

wobei OS eine Oligosaccharidsequenz wie in Anspruch 1 definiert ist, Y ein Nicht-Kohlenhydrat-Spacer oder ein nicht-glycosidisch verknüpftes terminales Konjugat ist, n 0 oder 1 ist und X Lactosyl-, Galactosyl-, N-Acetyllactosaminyl, Mannosyl-, Man$_2$, Man$_3$-, Man$_3$GlcNAc, Man$_4$GlcNAc, N-Acetylglucosaminyl-oder N-Acetylgalactosaminyl ist und OS β2- oder β4- oder β6-verknüpft mit dem Mannosylrest ist, zur Verwendung in der Behandlung von menschlichem Krebs in einem Patienten, der gemäß Anspruch 1 diagnostiziert wurde.

## Revendications

1. Procédé de diagnostic d'un cancer humain, comprenant l'étape de détection dans un échantillon de patient d'une augmentation de l'expression d'une séquence oligosaccharidique par rapport à un témoin, dans lequel ladite séquence oligosaccharidique est un N-glycane à faible teneur en mannose consistant en Man,-$_4$GlcNAC$_2$, dans lequel ladite séquence oligosaccharidique comprend un ou plusieurs résidus Mana terminaux, dans lequel des structures terminales non réductrices de la séquence oligosaccharidique comprennent au moins un épitope terminal de type glycane de Man choisi dans le groupe constitué par . Mana3Man, Mana6Man et Mana3(Mana6)Man<

2. Procédé selon la revendication 1, dans lequel la séquence oligosaccharidique possède la structure de formule :

$$[Man\alpha3]_{n2}([Man\alpha6)_{n4})[Man\alpha6]_{n5}\{[Man\alpha3]_{n8}\}Man\beta4GlcNac\beta4GlcNAcyR_2$$

dans laquelle n2, n4, n5, n8 et m sont chacun indépendamment 0 ou 1,
à condition que quand n5 est 0, alors n2 et n4 soient également 0, [] indique un déterminant présent ou absent en fonction de la valeur de n2, n4, n5, n8, {} et () indiquent une ramification dans la structure, y est une structure de liaison anomérique a et/ou β ou une liaison provenant d'un carbone anomère dérivatisé, et $R_2$ est un hydroxyle terminal réducteur, un dérivé chimique terminal réducteur ou un dérivé naturel d'asparagine N-glycoside.

3. Procédé selon la revendication 1, dans lequel la détection comprend :

le contact dudit échantillon avec une substance se liant à ladite séquence oligosaccharidique, et la détermination de la présence d'une combinaison de ladite substance et dudit échantillon, ou
la libération des structures oligosaccharidiques dudit échantillon par des procédés enzymatiques ou chimiques pour former une fraction contenant des structures ou conjugués oligosaccharidiques libres à partir dudit échantillon, et
La détermination de la présence desdites séquences oligosaccharidiques dans ladite fraction, ou la détermination des quantités relatives desdites séquences oligosaccharidiques dans ladite fraction par rapport à d'autres séquences oligosaccharidiques présentes dans ladite fraction.

4. Procédé selon la revendication 3, dans lequel ladite substance se liant à ladite séquence oligosaccharidique est un aptamère, un peptide ou une protéine.

5. Procédé selon la revendication 4, dans lequel ladite protéine est un anticorps, une enzyme, une lectine ou l'un de leurs fragments.

6. Procédé selon la revendication 3, dans lequel ledit échantillon est un échantillon de sang, de tissu ou de sérum.

7. Procédé selon la revendication 3, dans lequel la présence de ladite séquence oligosaccharidique est déterminée par l'utilisation de la spectrométrie de masse.

8. Composition pharmaceutique comprenant un anticorps qui reconnaît spécifiquement la combinaison de structures terminales et des structures centrales de type glycane de la séquence oligosaccharidique telle que définie dans la revendication 1, pour un usage dans le traitement d'un cancer humain chez un patient diagnostiqué selon la revendication 1.

**9.** Composition selon la revendication 8, dans laquelle ledit anticorps est un anticorps humain ou un anticorps humanisé.

**10.** Composition pharmaceutique contenant la séquence oligosaccharidique telle que définie dans la revendication 1 ou un épitope antigénique de formule

$$[OS - (X)_n - L- Y]_m - Z \qquad (II),$$

dans laquelle OS est une séquence oligosaccharidique telle que définie dans la revendication 1, Y est un espaceur non glucidique ou un conjugué terminal ne contenant pas de liaison glycosidique, n est 0 ou 1 et X est un lactosyle, un galactosyle, un N-acétyllactosaminyle, un mannosyle, $Man_2$, $Man_3$, $Man_3GlcNAc$, $Man_4GlcNAc$, un N-acétylglucosaminyle ou un N-acétylgalactosaminyle, et OS est lié selon une conformation β2, P4 ou β6 à un résidu de mannose, pour un usage dans le traitement d'un cancer humain chez un patient diagnostiqué selon la revendication 1.

Figure 1.

A.

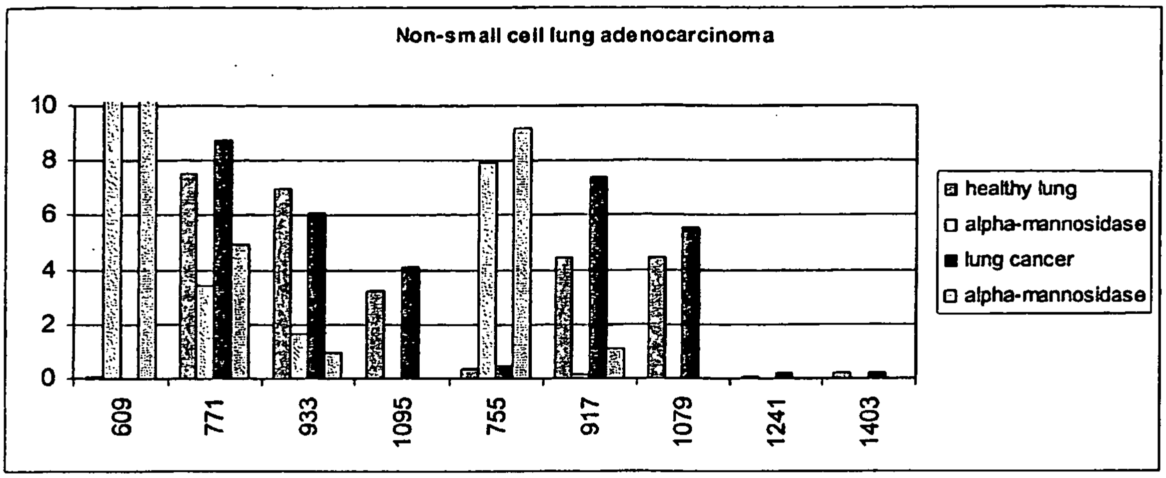
Non-small cell lung adenocarcinoma
10
8
6
4
2
0
609
771
933
1095
755
917
1079
1241
1403
healthy lung
alpha-mannosidase
lung cancer
alpha-mannosidase

B.

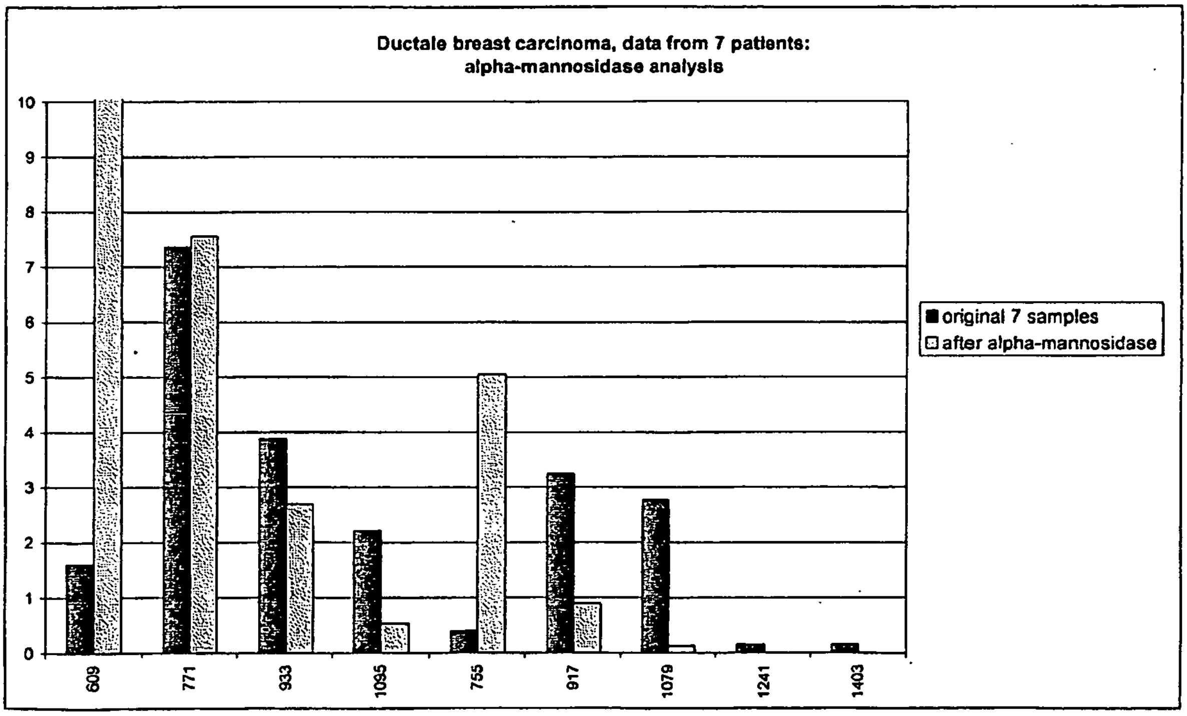
Ductale breast carcinoma, data from 7 patients:
alpha-mannosidase analysis
10
9
8
7
6
5
4
3
2
1
0
609
771
933
1095
755
917
1079
1241
1403
original 7 samples
after alpha-mannosidase

**Figure 2.**

General structure:

Manα$_{(0-3)}$Manβ4GlcNAcβ4GlcNAc(β-*N*-Asn)

Structures of N-glycan biosynthesis products:

0 – 3 Manα residues

(±)Manα2(±)Manα6
(±)Manα6
(±)Manα2(±)Manα3
Manβ4GlcNAcβ4GlcNAc(β-*N*-Asn)
(±)Manα2(±)Manα2(±)Manα3

**Figure 3.**

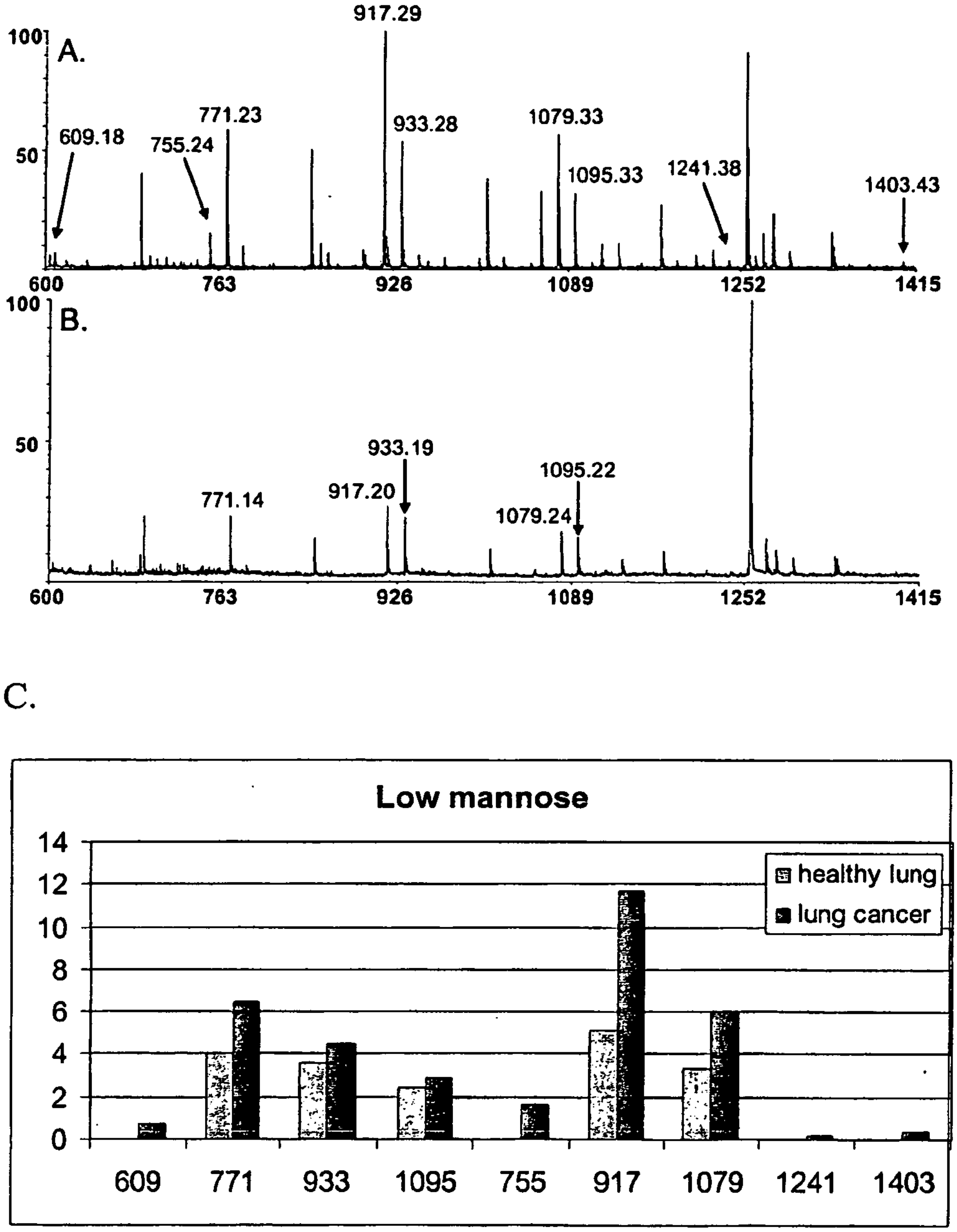
A.
917.29
771.23
933.28
1079.33
609.18
755.24
1095.33
1241.38
1403.43
100
50
600
763
926
1089
1252
1415
B.
933.19
1095.22
771.14
917.20
1079.24
100
50
600
763
926
1089
1252
1415
C.
Low mannose
14
12
10
8
6
4
2
0
healthy lung
lung cancer
609
771
933
1095
755
917
1079
1241
1403

**Figure 4.**

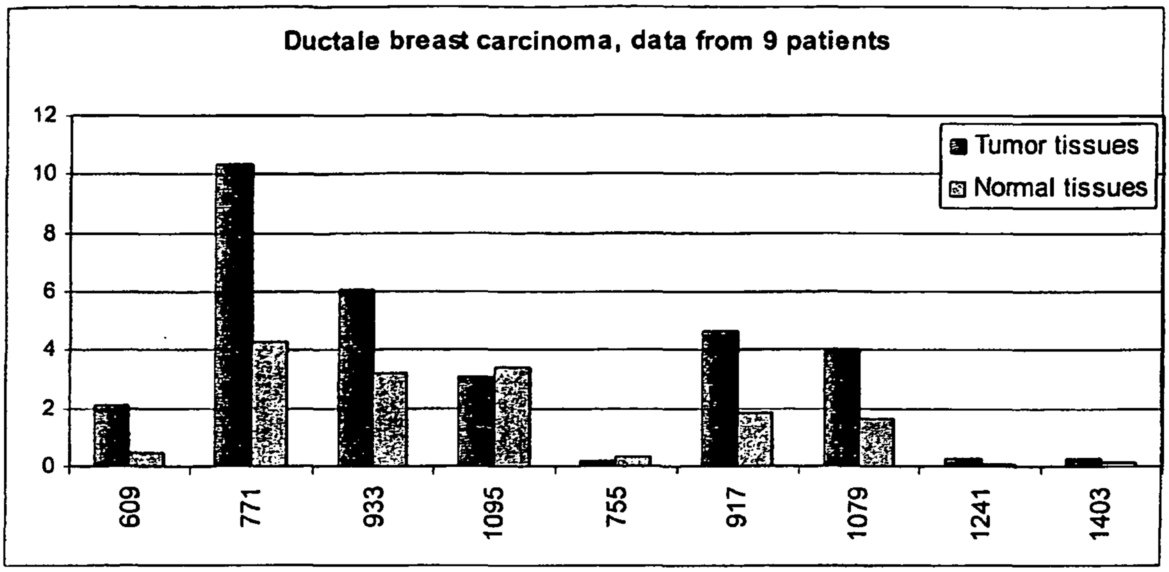
Ductale breast carcinoma, data from 9 patients
12
10
8
6
4
2
0
Tumor tissues
Normal tissues
609
771
933
1095
755
917
1079
1241
1403

**Figure 5.**

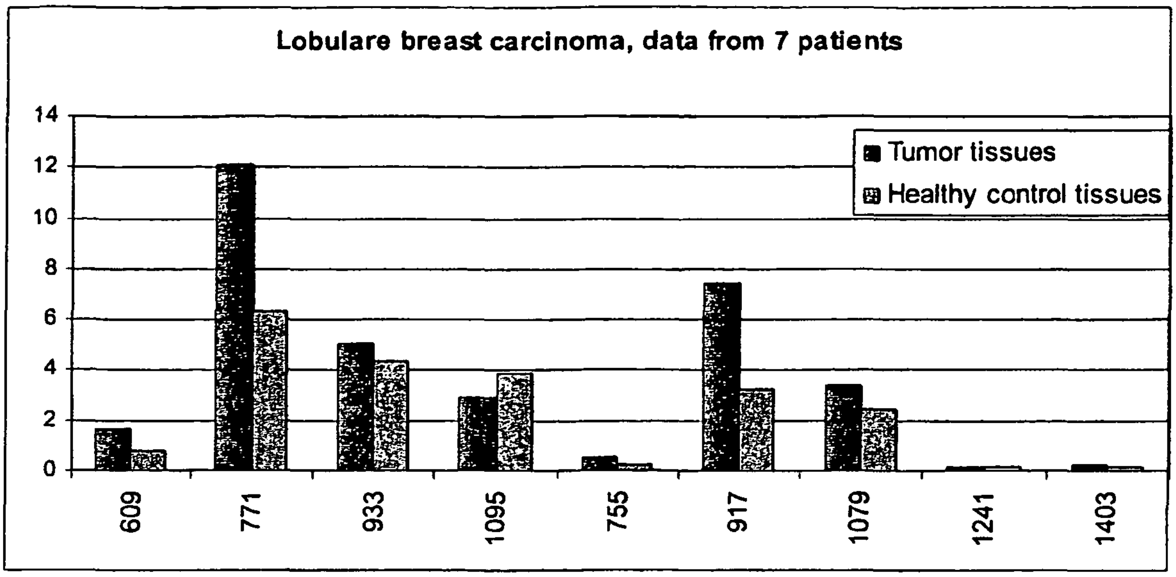
Lobulare breast carcinoma, data from 7 patients
14
12
10
8
6
4
2
0
Tumor tissues
Healthy control tissues
609
771
933
1095
755
917
1079
1241
1403

**Figure 6.**

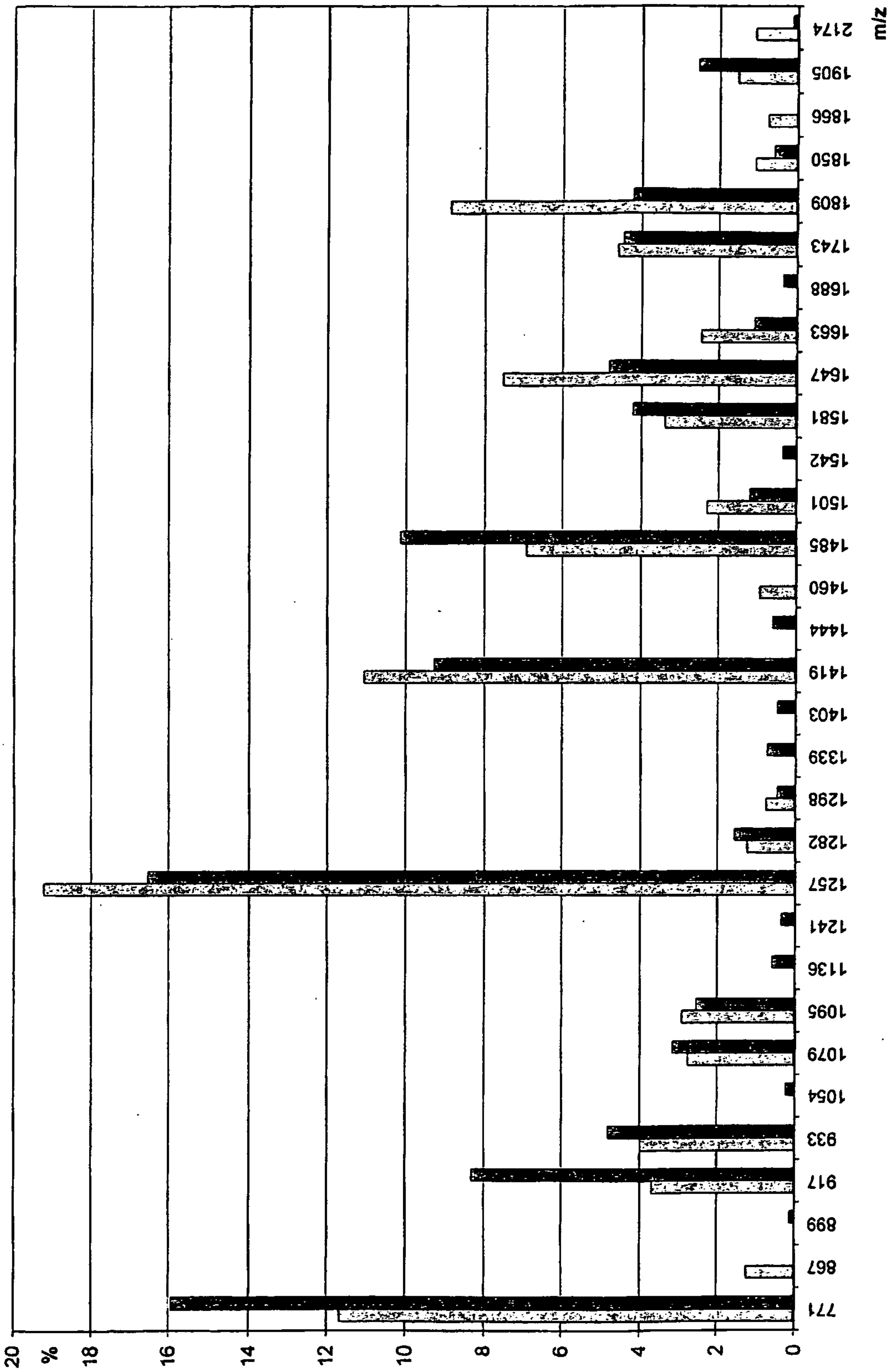
m/z
771
867
899
917
933
1054
1079
1095
1136
1241
1257
1282
1298
1339
1403
1419
1444
1460
1485
1501
1542
1581
1647
1663
1688
1743
1809
1850
1866
1905
2174
%
0
2
4
6
8
10
12
14
16
18
20

Figure 7.

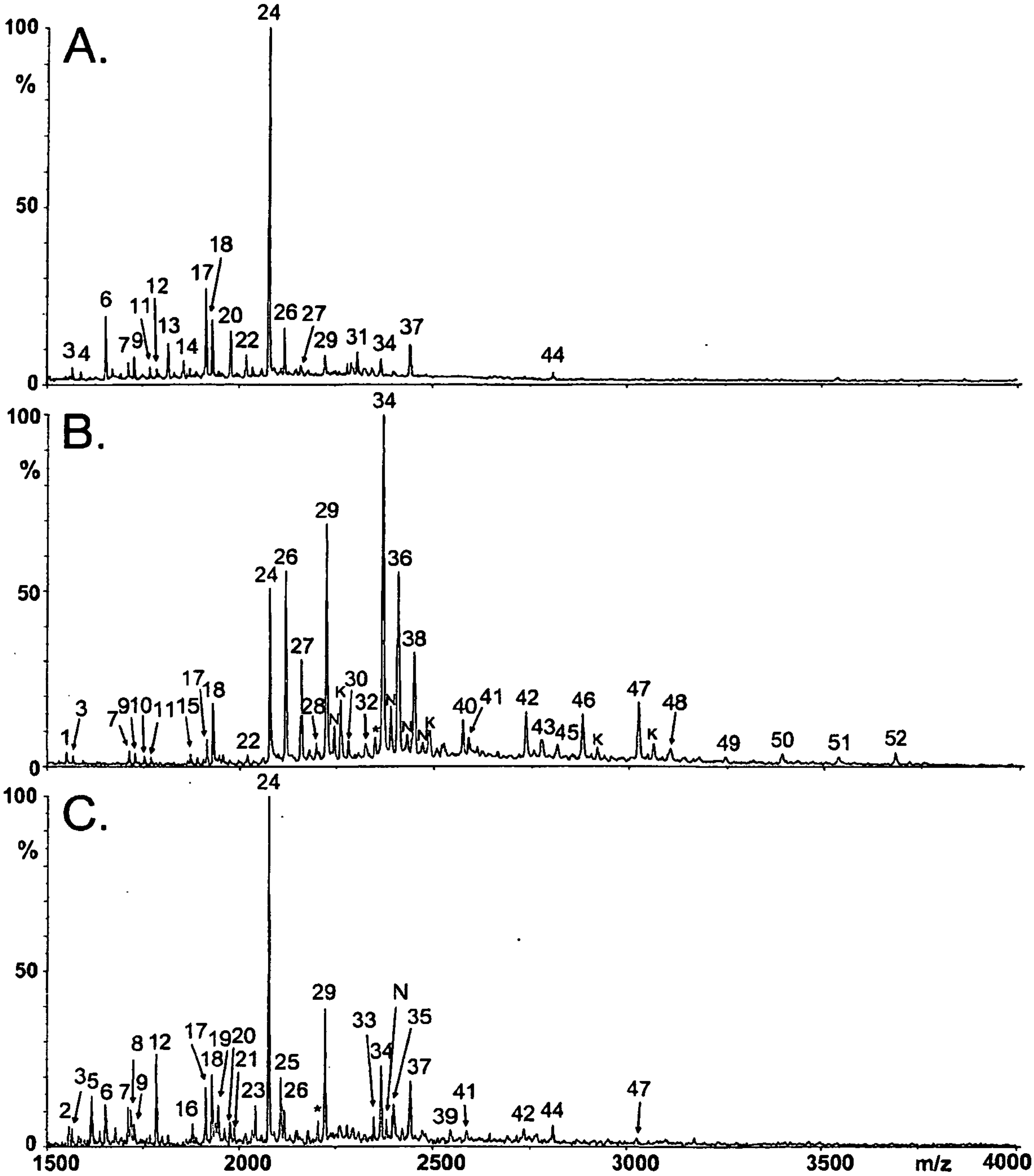

**Figure 8.**

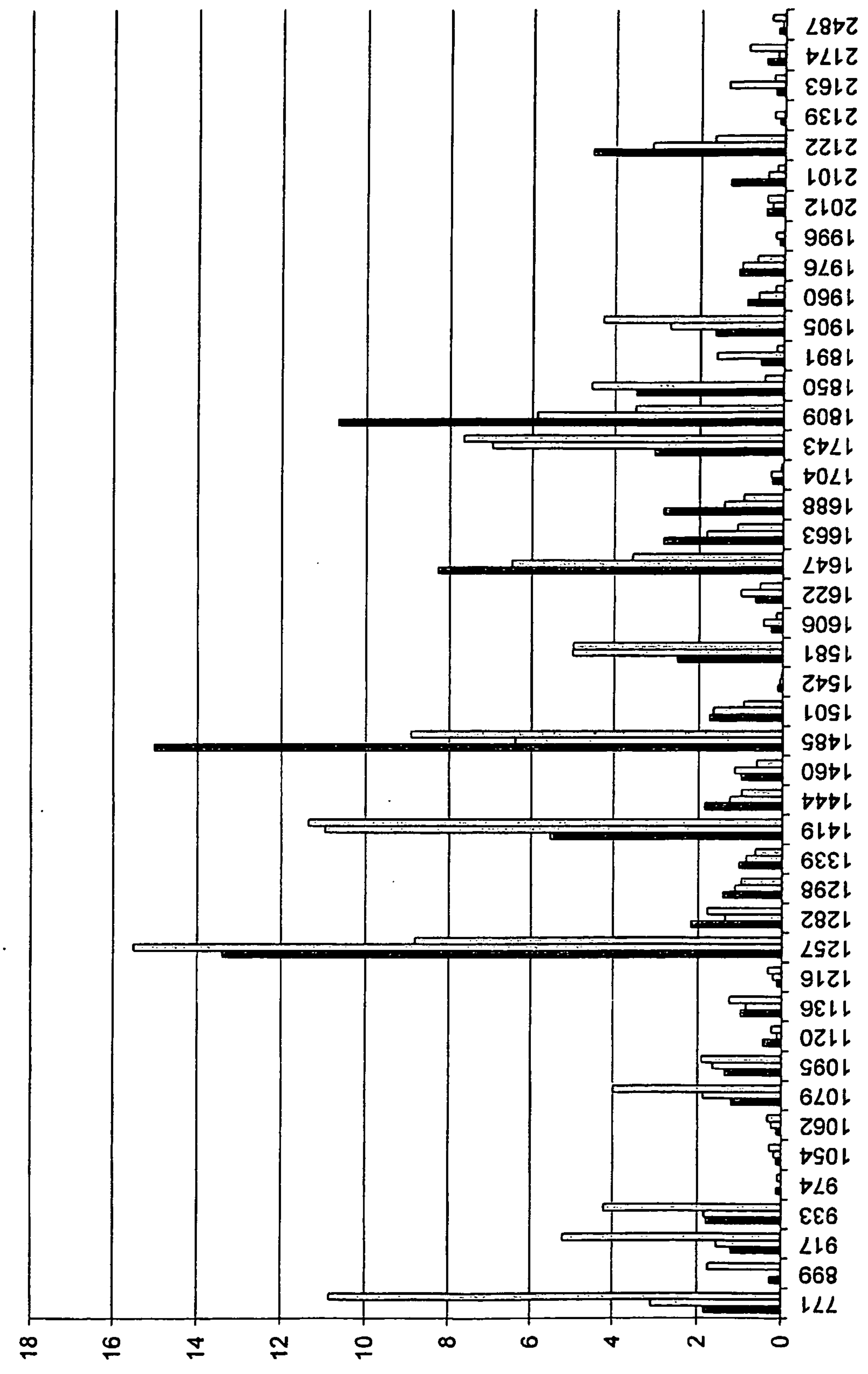

0
2
4
6
8
10
12
14
16
18
771
899
917
933
974
1054
1062
1079
1095
1120
1136
1216
1257
1282
1298
1339
1419
1444
1460
1485
1501
1542
1581
1606
1622
1647
1663
1688
1704
1743
1809
1850
1891
1905
1960
1976
1996
2012
2101
2122
2139
2163
2174
2487

Figure 9.

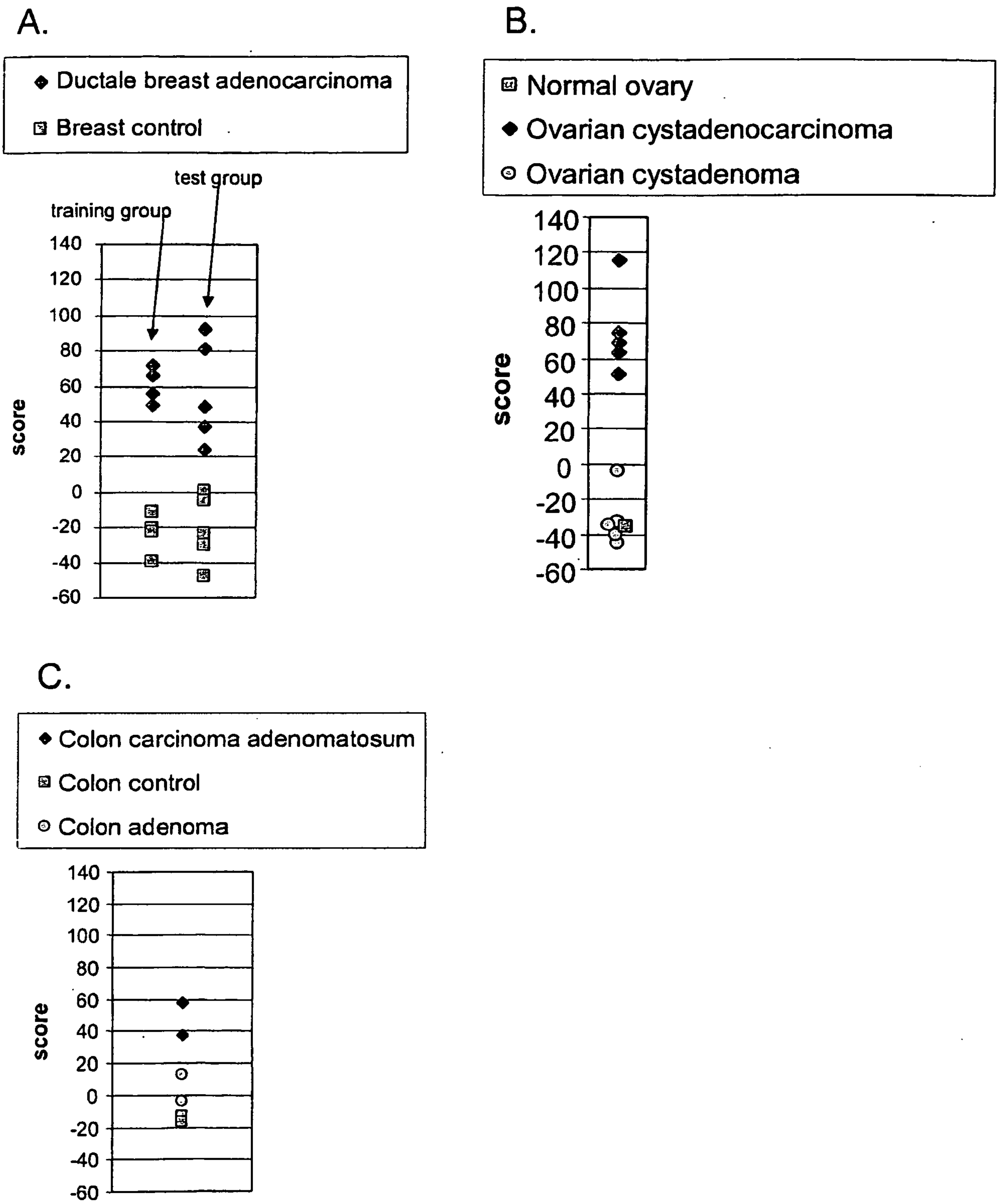
A.
Ductale breast adenocarcinoma
Breast control
test group
training group
score
140
120
100
80
60
40
20
0
-20
-40
-60
B.
Normal ovary
Ovarian cystadenocarcinoma
Ovarian cystadenoma
score
140
120
100
80
60
40
20
0
-20
-40
-60
C.
Colon carcinoma adenomatosum
Colon control
Colon adenoma
score
140
120
100
80
60
40
20
0
-20
-40
-60

**Figure 10.**

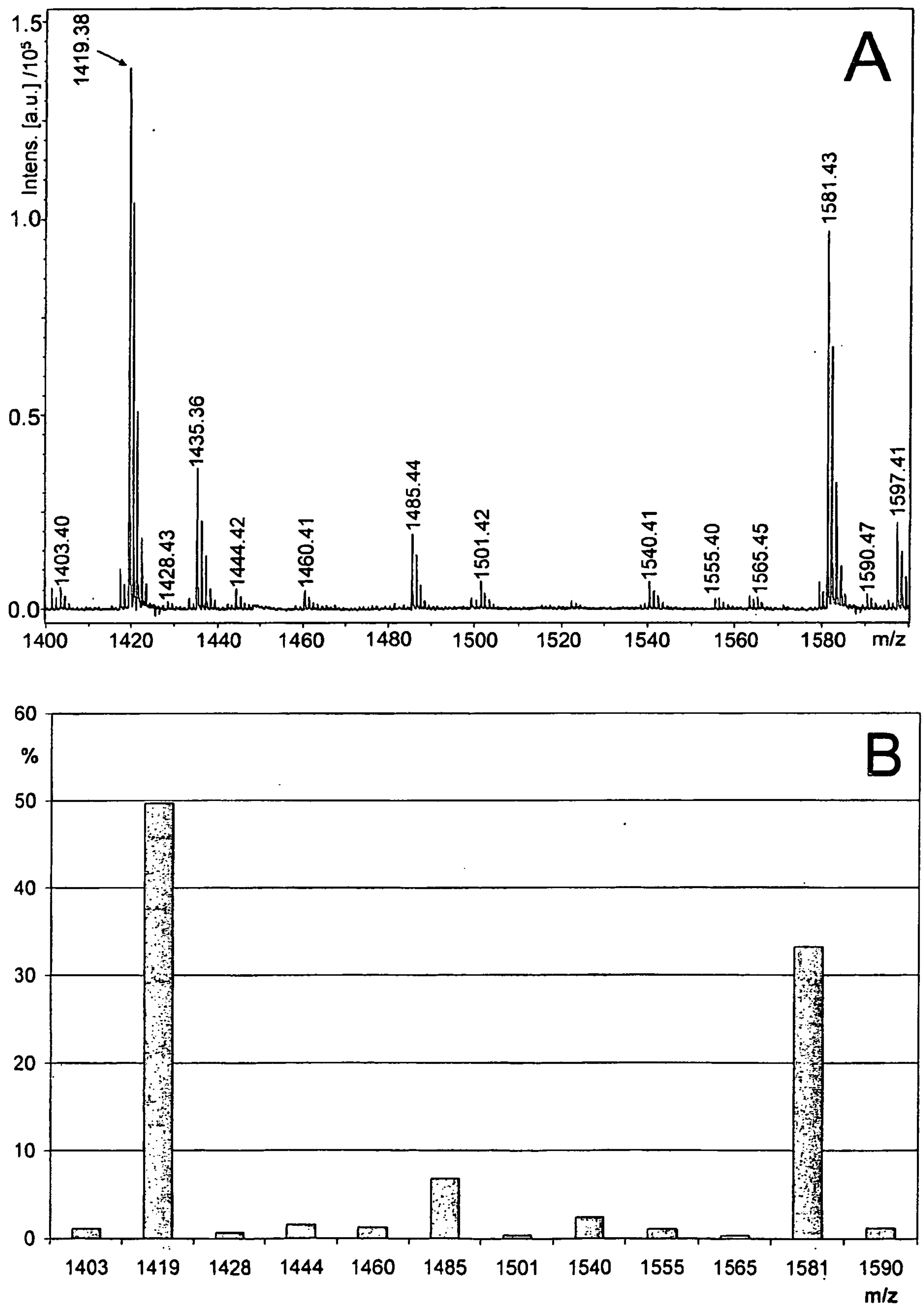

A
Intens. [a.u.] /10^5
1.5
1.0
0.5
0.0
1419.38
1581.43
1435.36
1597.41
1485.44
1501.42
1540.41
1403.40
1428.43
1444.42
1460.41
1555.40
1565.45
1590.47
1400
1420
1440
1460
1480
1500
1520
1540
1560
1580
m/z
B
%
60
50
40
30
20
10
0
1403
1419
1428
1444
1460
1485
1501
1540
1555
1565
1581
1590
m/z

**Figure 11.**

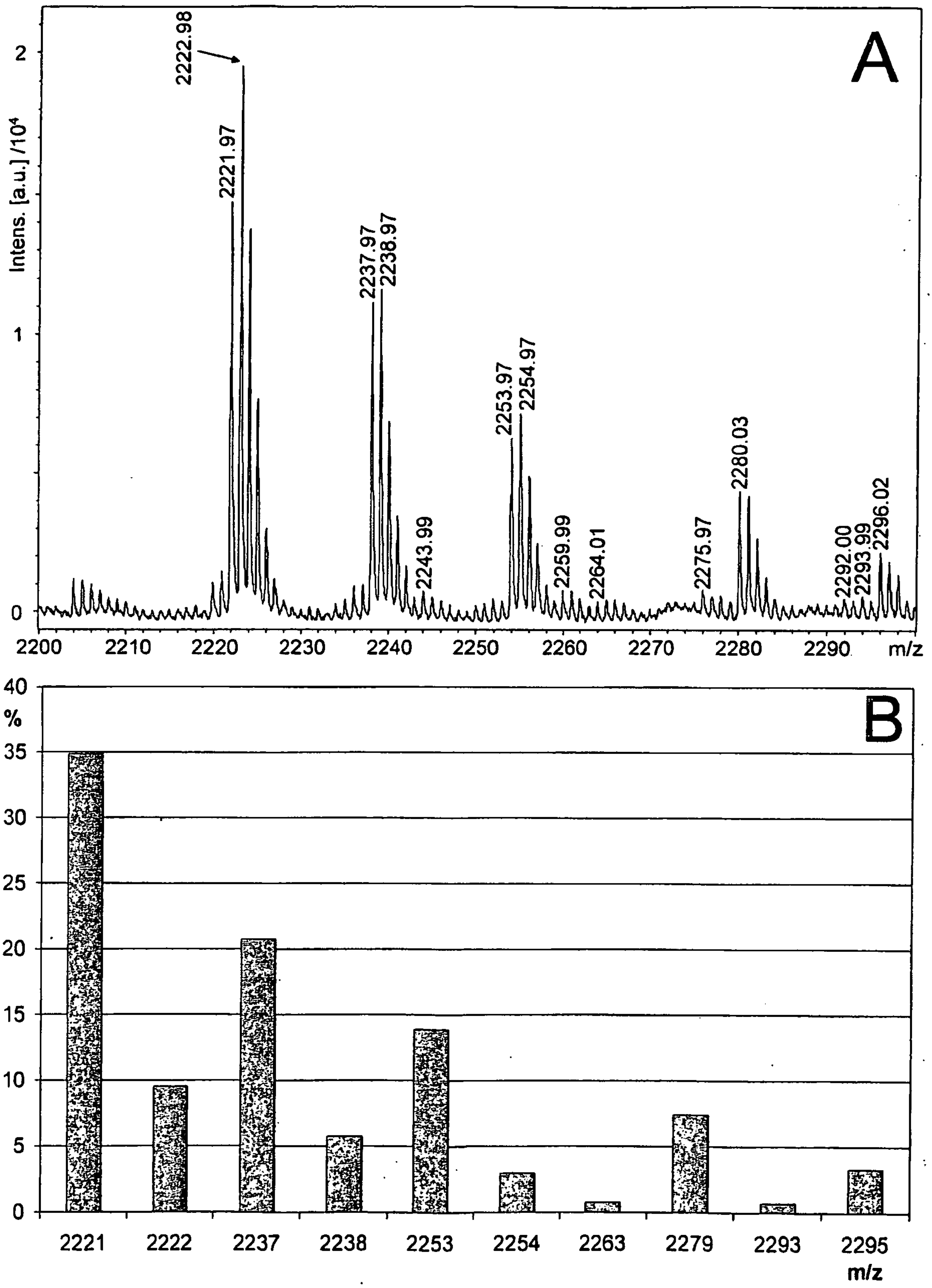
A
B
Intens. [a.u.] /10⁴
2222.98
2221.97
2237.97
2238.97
2243.99
2253.97
2254.97
2259.99
2264.01
2275.97
2280.03
2292.00
2293.99
2296.02
0
1
2
2200
2210
2220
2230
2240
2250
2260
2270
2280
2290
m/z
40
%
35
30
25
20
15
10
5
0
2221
2222
2237
2238
2253
2254
2263
2279
2293
2295
m/z

**Figure 12**

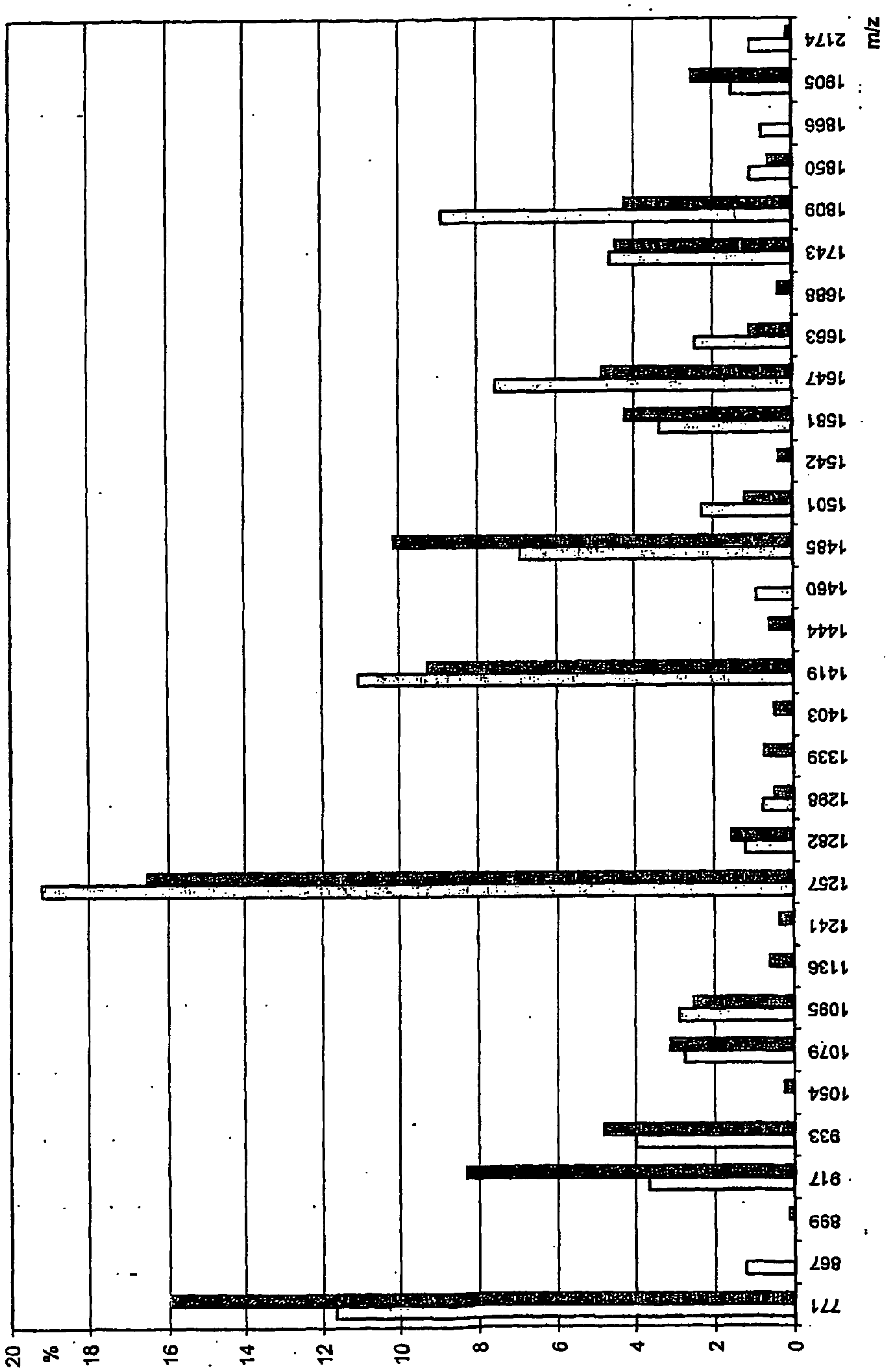

m/z
771
867
899
917
933
1054
1079
1095
1136
1241
1257
1282
1288
1339
1403
1419
1444
1460
1485
1501
1542
1581
1647
1663
1688
1743
1809
1850
1866
1805
2174
%
0
2
4
6
8
10
12
14
16
18
20

Figure 13

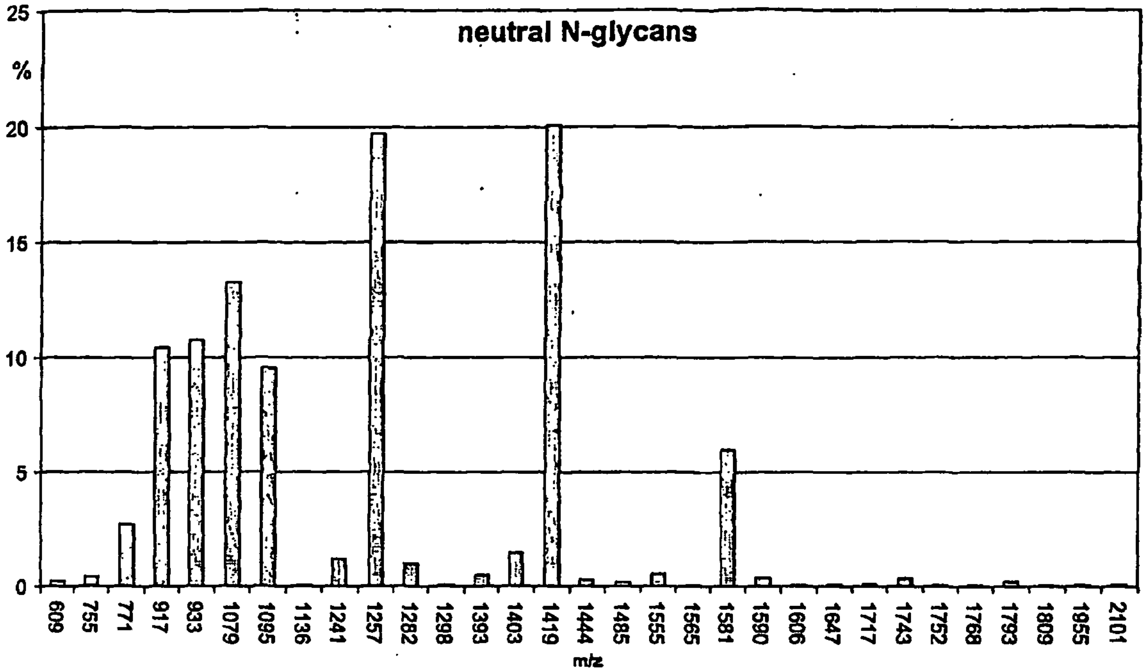
neutral N-glycans
25
%
20
15
10
5
0
609
755
771
917
933
1079
1095
1136
1241
1257
1282
1298
1393
1403
1419
1444
1485
1555
1565
1581
1590
1606
1647
1717
1743
1752
1768
1793
1809
1955
2101
m/z

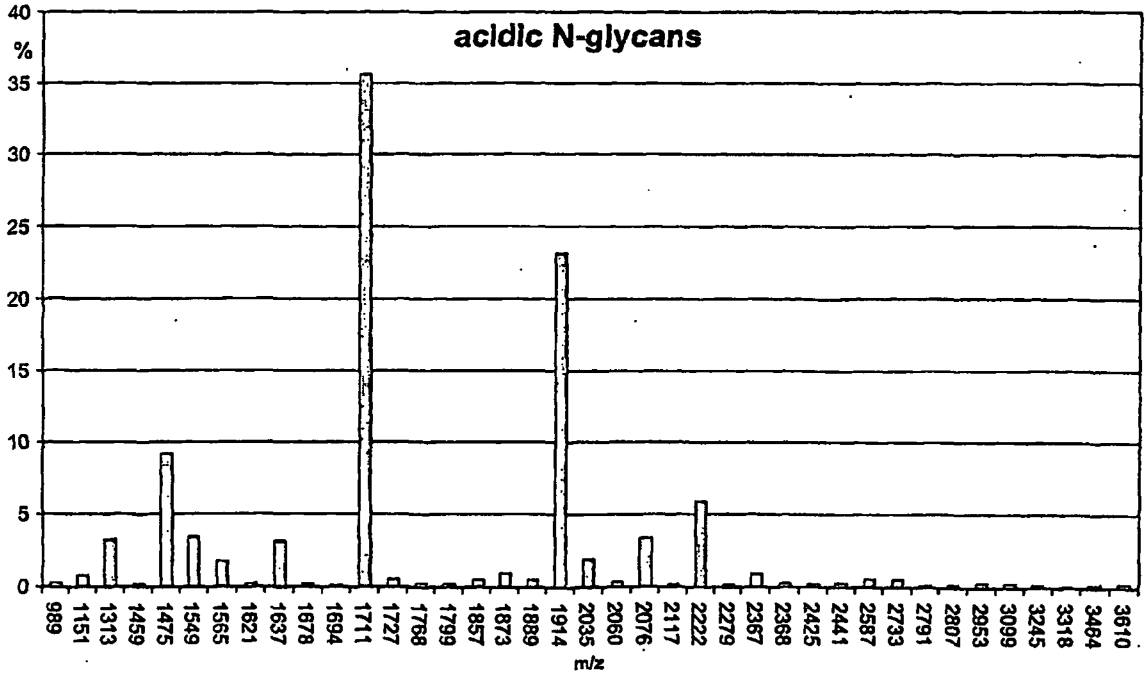
acidic N-glycans
40
%
35
30
25
20
15
10
5
0
989
1151
1313
1459
1475
1549
1565
1621
1637
1678
1694
1711
1727
1768
1799
1857
1873
1889
1914
2035
2060
2076
2117
2222
2279
2367
2368
2425
2441
2587
2733
2791
2807
2953
3099
3245
3318
3464
3610
m/z

Figure 14

**A**

```
                              a-D-Manp-(1-6)+
                                            |
                                      a-D-Manp-(1-6)+
                                            |       |
                              a-D-Manp-(1-3)+   b-D-Manp-(1-4)-b-D-GlcpNAc-(1-4)-D-GlcNAc
                                                    |
                                      a-D-Manp-(1-3)+
```

**B**

```
               a-D-Manp-(1-2)-a-D-Manp-(1-6)+
                                            |
                                      a-D-Manp-(1-6)+
                                            |       |
               a-D-Manp-(1-2)-a-D-Manp-(1-3)+   b-D-Manp-(1-4)-b-D-GlcpNAc-(1-4)-D-GlcNAc
                                                    |
a-D-Manp-(1-2)-a-D-Manp-(1-2)-a-D-Manp-(1-3)+
```

**C**

```
               a-D-Manp-(1-2)-a-D-Manp-(1-6)+
                                            |
                                      a-D-Manp-(1-6)+
                                            |       |
                              a-D-Manp-(1-3)+   b-D-Manp-(1-4)-b-D-GlcpNAc-(1-4)-D-GlcNAc
                                                    |
a-D-Manp-(1-2)-a-D-Manp-(1-2)-a-D-Manp-(1-3)+
```

**Figure 15**

**D**

```
a-D-Manp-(1-6)+
              |
        b-D-Manp-(1-4)-b-D-GlcpNAc-(1-4)-D-GlcNAc
              |
a-D-Manp-(1-3)+
```

**E**

```
a-D-Manp-(1-6)+
              |
        b-D-Manp-(1-4)-D-GlcNAc
              |
a-D-Manp-(1-3)+
```

**F**

```
              a-D-Manp-(1-6)+
                            |
                      b-D-Manp-(1-4)-D-GlcNAc
                            |
a-D-Manp-(1-2)-a-D-Manp-(1-3)+
```

**G**

```
                             a-D-Manp-(1-6)+
                                           |
                                     b-D-Manp-(1-4)-D-GlcNAc
                                           |
a-D-Manp-(1-2)-a-D-Manp-(1-2)-a-D-Manp-(1-3)+
```

**Figure 16**

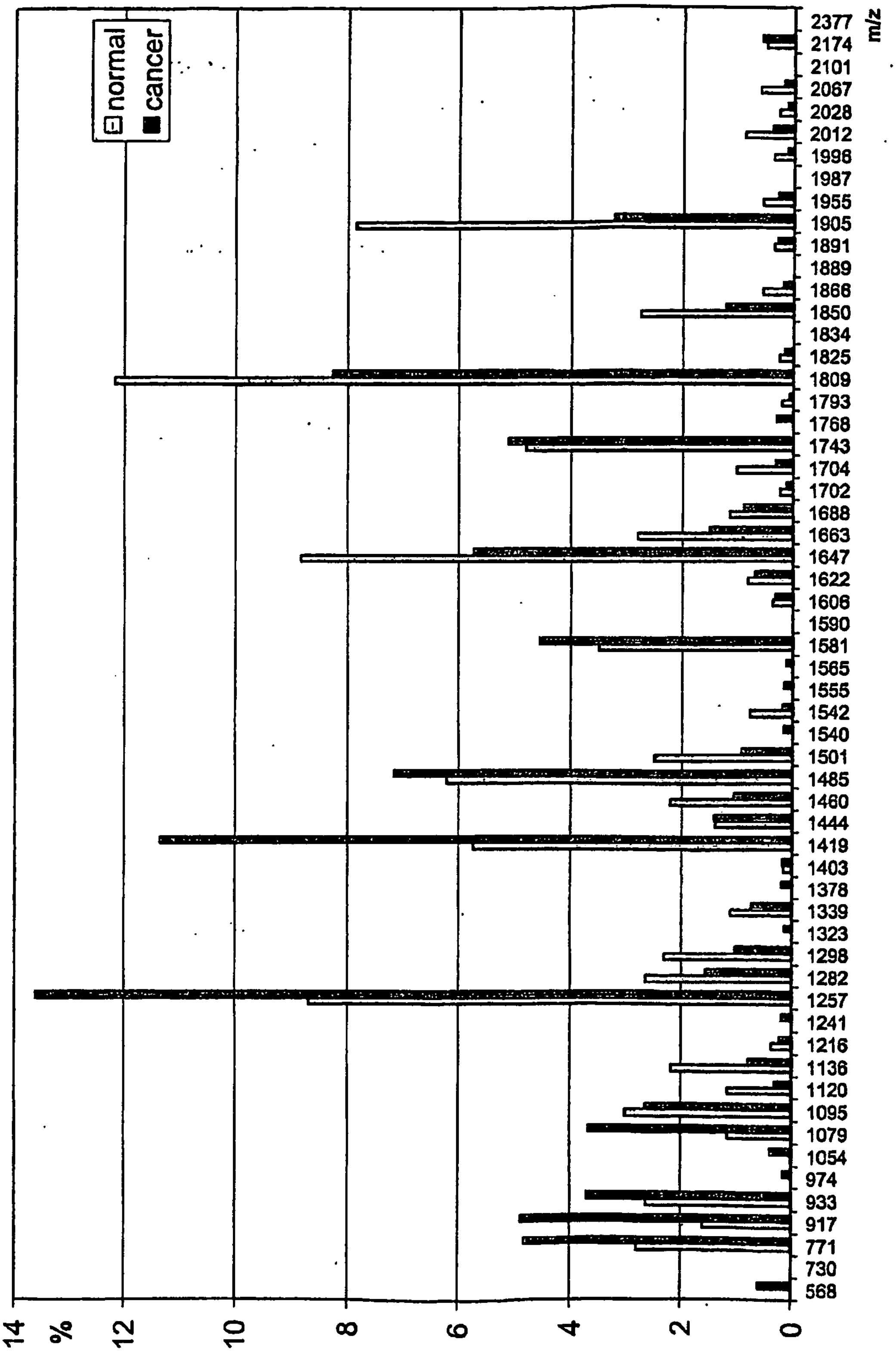
normal
cancer
%
14
12
10
8
6
4
2
0
m/z
2377
2174
2101
2067
2028
2012
1998
1987
1955
1905
1891
1889
1866
1850
1834
1825
1809
1793
1768
1743
1704
1702
1688
1663
1647
1622
1606
1590
1581
1565
1555
1542
1540
1501
1485
1460
1444
1419
1403
1378
1339
1323
1298
1282
1257
1241
1216
1136
1120
1095
1079
1054
974
933
917
771
730
568

Figure 17

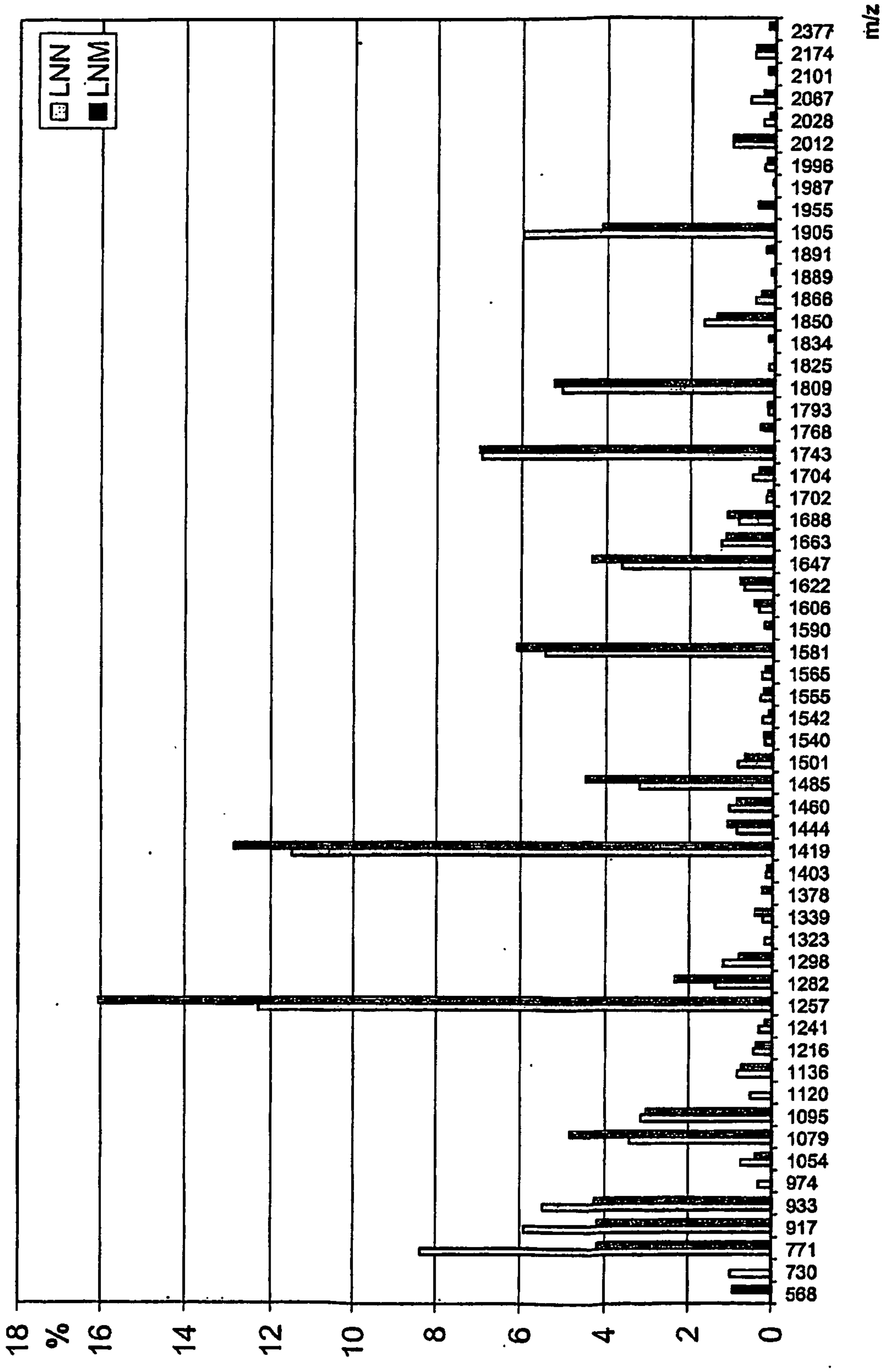
LNN
LNM
%
m/z
0
2
4
6
8
10
12
14
16
18
568
730
771
917
933
974
1054
1079
1095
1120
1136
1216
1241
1257
1282
1298
1323
1339
1378
1403
1419
1444
1460
1485
1501
1540
1542
1555
1565
1581
1590
1606
1622
1647
1663
1688
1702
1704
1743
1768
1793
1809
1825
1834
1850
1866
1889
1891
1905
1955
1987
1996
2012
2028
2087
2101
2174
2377

Figure 18

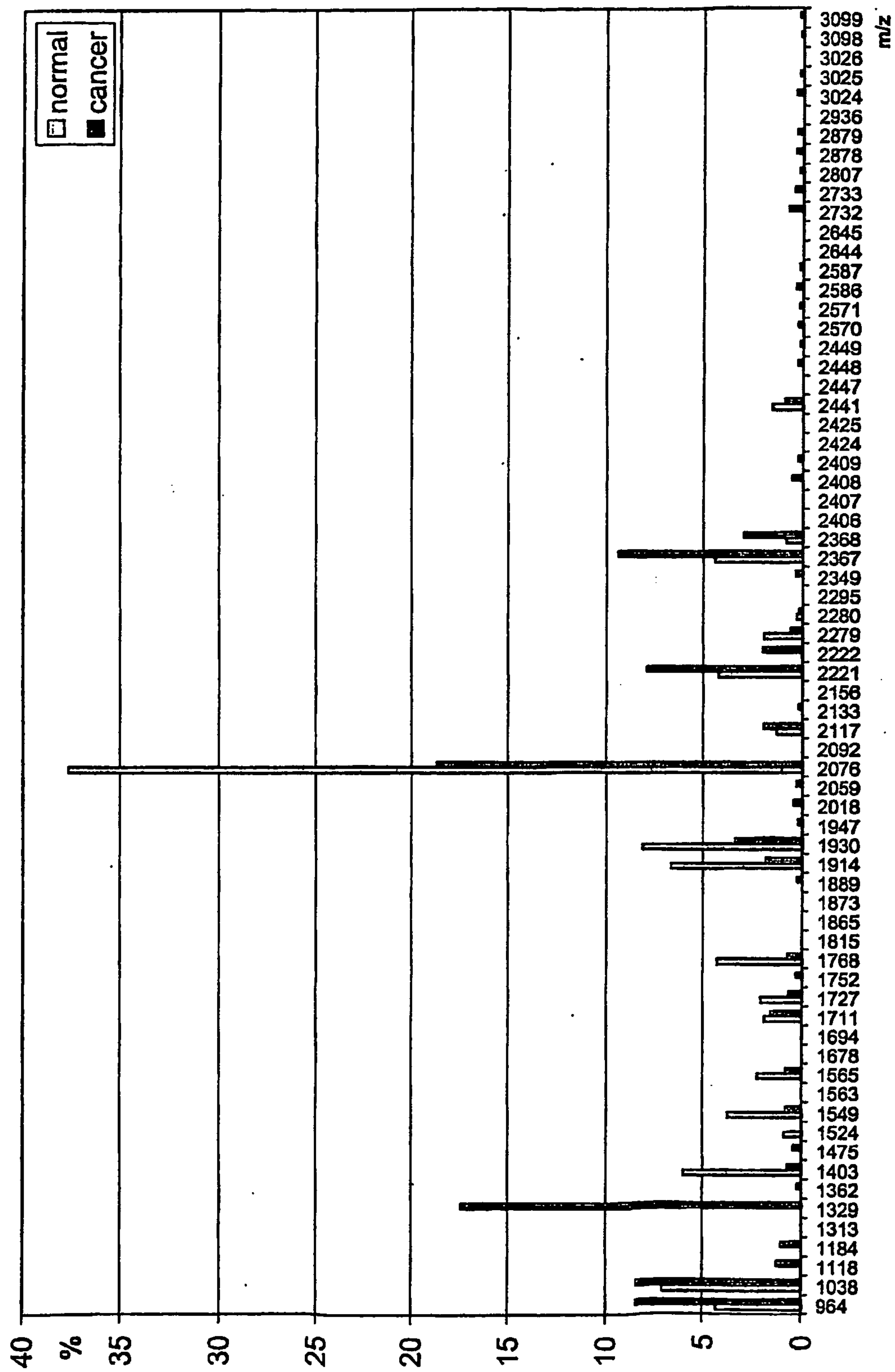
normal
cancer
%
40
35
30
25
20
15
10
5
0
m/z
3099
3098
3026
3025
3024
2936
2879
2878
2807
2733
2732
2645
2644
2587
2586
2571
2570
2449
2448
2447
2441
2425
2424
2409
2408
2407
2406
2368
2367
2349
2295
2280
2279
2222
2221
2156
2133
2117
2092
2076
2059
2018
1947
1930
1914
1889
1873
1865
1815
1768
1752
1727
1711
1694
1678
1565
1563
1549
1524
1475
1403
1362
1329
1313
1184
1118
1038
964

**Figure 19**

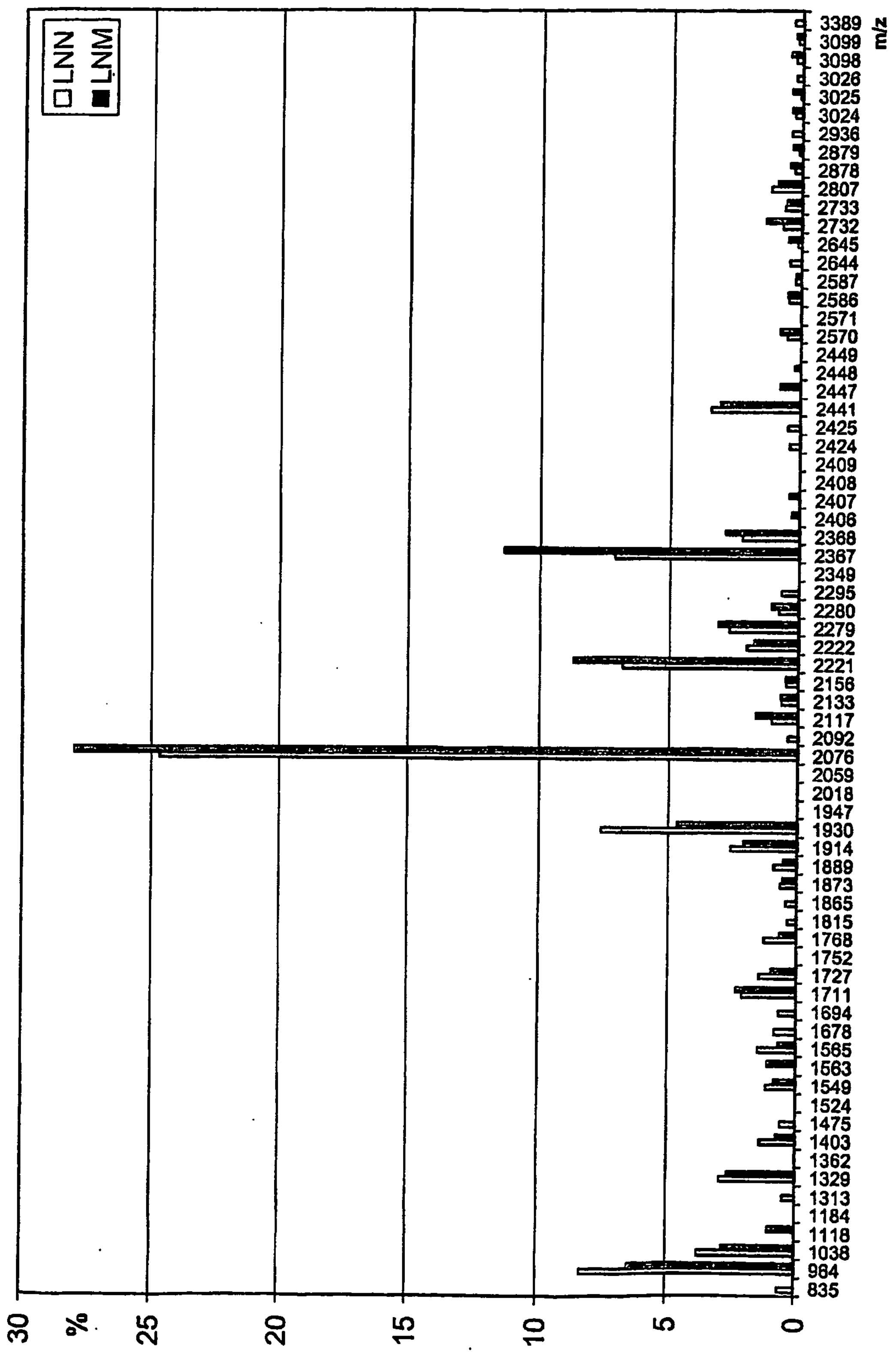
LNN
LNM
%
30
25
20
15
10
5
0
m/z
835
984
1038
1118
1184
1313
1329
1362
1403
1475
1524
1549
1563
1565
1678
1694
1711
1727
1752
1768
1815
1865
1873
1889
1914
1930
1947
2018
2059
2076
2092
2117
2133
2156
2221
2222
2279
2280
2295
2349
2367
2368
2406
2407
2408
2409
2424
2425
2441
2447
2448
2449
2570
2571
2586
2587
2644
2645
2732
2733
2807
2878
2879
2936
3024
3025
3026
3098
3099
3389

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5874060 A
- US 6025481 A
- US 4851511 A
- US 4904596 A
- US 5795961 A
- US 4725 A
- US 557 A
- US 5059520 A
- US 5171667 A
- US 5173292 A
- US 6090789 A
- US 5708163 A
- US 5902725 A
- US 6203999 B
- US 5102663 A
- US 5660834 A
- US 5747048 A
- US 5229289 A
- US 6083929 A

### Non-patent literature cited in the description


- **Hunt ; Wright.** *The Biochemical Journal,* 1985, vol. 229, 441-451
- **van Remoorte A. et al.** *Glycobiology,* 2003, vol. 13, 217-225
- **Schmitz B et al.** *Glycobiology,* 1993, vol. 3, 609-17
- *Carbohydr. Res.,* 1998, vol. 322, 167
- *Carbohydr. Res.,* 1997, vol. 297, 1
- *Eur. J. Biochem.,* 1998, vol. 257, 29
- **Harvey.** *Mass. Spec. Rev.,* 1999, vol. 18, 349
- **Fu D. ; Chen L. ; O'Neill R.A.** *Carbohydr. Res.,* 1994, vol. 261, 173-186
- **Hård K. ; Mekking A. ; Kamerling J.P ; Dacremont G.A.A. ; Vliegenthart J.F.G.** *Glycoconjugate J.,* 1991, vol. 8, 17-28
- **Hård K. ; Van Zadelhoff G. ; Moonen P. ; Kamerling J.P ; Vliegenthart J.F.G.** *Eur. J. Biochem.,* 1992, vol. 209, 895-915
- **Helin J. ; Maaheimo H. ; Seppo A. ; Keane A. ; Renkonen O.** *Carbohydr. Res.,* 1995, vol. 266, 191-209
- **Arap, W. et al.** *Science,* 1998, vol. 279 (5349), 377-80
- **Brockhausen, 1.** *Biochim. Biophys. Acta,* 1999, vol. 1473 (1), 67-95
- **Davies et al.** *J. Chromatogr.,* 1992, vol. 609 (1-2), 125-31 1
- **Domon, B. ; Costello, C.E.** *Glycoconj. J.,* 1988, vol. 5, 397-409
- **Dube, D.H. ; Bertozzi, C.R.** *Nat. Rev. Drug Discov.,* 2005, vol. 4 (6), 477-88
- Carbohydrates in chemistry and biology. 2000
- **Fernandez, L.E. et al.** *Expert Rev. Vaccines,* 2003, vol. 2 (6), 817-23
- **Harvey, D.J. et al.** *Rapid Commun. Mass Spectrom.,* 1993, vol. 7 (7), 614-9
- **Holmberg, L.A. ; Sandmeier, B.M.** *Expert Opin. Biol. Ther.,* 2001, vol. 1 (5), 881-91
- **Huang, Y. et al.** *Anal. Chem.,* vol. 73 (24), 6063-9
- **Kurokawa, T. et al.** *Eur. J. Biochem.,* vol. 269, 5459-73
- **Naven, T.J. ; Harvey, D.J.** *Rapid Commun. Mass Spectrom.,* 1996, vol. 10 (11), 1361-6
- **Nyman, T.A. et al.** *Eur. J. Biochem.,* 1998, vol. 253 (2), 485-93
- **Papac, D.I. et al.** *Anal. Chem.,* 1996, vol. 68 (18), 3215-23
- **Packer, N.H. et al.** *Glycoconj. J.,* 1998, vol. 15 (8), 737-47
- **Plummer, T.H. Jr. ; Tarentino, A.L.** *Glycobiology,* 1991, vol. 1 (3), 257-63
- **Powell, A.K. ; Harvey, D.J.** *Rapid Commun. Mass Spectrom.,* 1996, vol. 10 (9), 1027-32
- **Saarinen, J. et al.** *Eur. J. Biochem.,* 1999, vol. 259 (3), 829-40
- **Verostek et al.** *Anal. Biochem.,* 2000, vol. 278 (2), 111-22
- **Ylönen, A. et al.** *Glycobiology,* 2001, vol. 11 (7), 523-31